(19) Europäisches Patentamt
European Patent Office
Office européen des brevets

(11) **EP 4 223 754 A1**

(12) **EUROPEAN PATENT APPLICATION**
published in accordance with Art. 153(4) EPC

(43) Date of publication:
**09.08.2023 Bulletin 2023/32**

(21) Application number: **21874571.9**

(22) Date of filing: **30.09.2021**

(51) International Patent Classification (IPC):
***C07D 471/04*** *(2006.01)* ***C07D 413/14*** *(2006.01)*
***C07D 417/14*** *(2006.01)* ***C07D 487/04*** *(2006.01)*
***A61K 31/437*** *(2006.01)* ***A61K 31/444*** *(2006.01)*
***A61P 35/00*** *(2006.01)*

(52) Cooperative Patent Classification (CPC):
**A61K 31/437; A61K 31/444; A61P 35/00; C07D 413/14; C07D 417/14; C07D 471/04; C07D 487/04**

(86) International application number:
**PCT/CN2021/122040**

(87) International publication number:
**WO 2022/068917 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2020 CN 202011057860**
**10.03.2021 CN 202110261605**

(71) Applicant: **CHIA TAI TIANQING PHARMACEUTICAL GROUP CO., LTD.**
**Lianyungang,**
**Jiangsu 222062 (CN)**

(72) Inventors:
- **ZHANG, Yinsheng**
  **Nanjing, Jiangsu 211100 (CN)**
- **GAO, Yong**
  **Nanjing, Jiangsu 211100 (CN)**
- **SHI, Wei**
  **Nanjing, Jiangsu 211100 (CN)**
- **ZHAO, Damin**
  **Nanjing, Jiangsu 211100 (CN)**
- **YIN, Yuan**
  **Nanjing, Jiangsu 211100 (CN)**

(74) Representative: **Krauss, Jan**
**SKM-IP PartGmbB**
**Oberanger 45**
**80331 München (DE)**

(54) **COMPOUND AS AKT KINASE INHIBITOR**

(57) Provided is a compound as an Akt kinase inhibitor. The present invention specifically relates to a compound of formula (I) or a pharmaceutically acceptable salt thereof, a preparation method therefor, a pharmaceutical composition containing the compound, and the use thereof in the preparation of a drug for treating Akt kinase-related diseases.

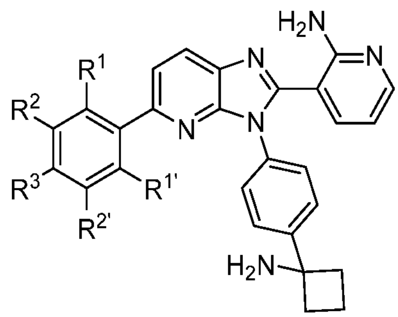

(I)

EP 4 223 754 A1

# Description

## CROSS-REFERENCE TO RELATED APPLICATIONS

The present application claims priority to and benefit of the Chinese Patent Application No. 202011057860.0 filed with China National Intellectual Property Administration on Sep. 30, 2020 and the Chinese Patent Application No. 202110261605.6 filed with China National Intellectual Property Administration on Mar. 10, 2021, the disclosure of each of which is incorporated herein by reference in its entirety.

## TECHNICAL FIELD

The present application belongs to the field of pharmaceutical chemistry, provides a compound as an Akt kinase inhibitor or a pharmaceutically acceptable salt thereof, a preparation method therefor and a pharmaceutical composition containing the compound, and relates to use thereof in preparing a drug for treating an Akt kinase-related disease of a patient in need thereof, such as treating cancer.

## BACKGROUND

Akt, also known as Protein Kinase B (PKB) or Rac, is a serine/threonine kinase of the AGC family with high homology to Protein Kinase A (PKA) and Protein Kinase C (PKC). Akt mainly has three domains: a PH domain (which has an affinity for PIP3 and is therefore critical for binding to cell membranes), a catalytic domain and a regulatory domain. Studies have shown that Akt in humans comprises three subtypes: Akt1, Akt2 and Akt3, each of which has unique function and expression pattern. Akt1, Akt2 and Akt3 are key mediators of PI3K/AKT/mTOR signaling pathway, and can promote various physiological processes such as proliferation, migration, anti-apoptotic survival and protein synthesis. Akt1 has wide expression in tissues and is mainly involved in the survival pathway and growth control of cells; Akt2 is mainly expressed in muscle and adipocytes and involved in insulin-mediated glycometabolism and the like; Akt3 is mainly expressed in testis and brain and plays an important role in maintaining normal brain volume.

Akt is one of the most frequently activated protein kinases in human cancers. Over-activation of Akt may induce cell growth, resulting in cell proliferation and helping to resist apoptosis. In cancer, Akt activity is often elevated due to oncogenic growth factors, angiogenic factors, cytokines and genetic alterations, including mutations and/or amplification of the Akt1, Akt2 and Akt3 genes. Akt signaling can be regulated from various levels, for example several molecules upstream thereof can regulate the dephosphorylation of PIP3 that can be dephosphorylated to PIP2 by PTEN and SHIP1. Akt can also be dephosphorylated by PPA2 and PHLPP, resulting in inactivation. In addition, with phosphorylation of NFkB and IRS-1, positive and negative feedback regulation and control can be performed on Akt. Studies have shown that Akt is overexpressed in various human tumors, and the abnormal function of the Akt is closely related to the occurrence, development and tolerance of chemotherapy and radiotherapy of the tumors.

According to different binding sites, Akt inhibitors may be classified into PH domain inhibitors, ATP competitive inhibitors, allosteric inhibitors, biologics and the like. Compared with Capivasertib (AZD5363) that is developed by AstraZeneca and is a pan-Akt inhibitor, ATP competitive inhibitors and allosteric inhibitors (e.g., MK-2206) do not show monotherapy activity in many clinical trials. ARQ 092 and ARQ 751 are highly selective allosteric inhibitors, both of which show better activity in early studies.

Akt has become an anti-tumor target with a great development prospect, and an Akt inhibitor with high efficiency (activity), novelty (structure) and high selectivity (safety) is an important strategy for solving the unmet medical requirements at present.

## BRIEF SUMMARY

In one aspect, the present application provides a compound of formula (I) or a pharmaceutically acceptable salt thereof,

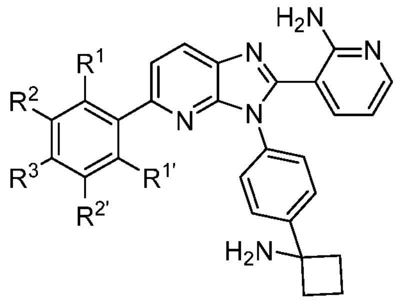

(I)

wherein,

$R^1$ and $R^{1'}$ are each independently selected from the group consisting of hydrogen and halogen;

$R^2$, $R^{2'}$ and $R^3$ are each independently selected from the group consisting of hydrogen, amino, amino-$C_1$-$C_6$ alkyl-, 7-9 membered spiro-heterocyclyl, 10 membered spiro-heterocyclyl whose ring atoms consist of a nitrogen atom and a carbon atom, 7-10 membered fused-heterocyclyl, 7-10 membered bridged heterocyclyl, 5-6 membered heterocyclyl whose ring atoms consist of a nitrogen atom and a carbon atom, 5-6 membered heterocyclyl whose ring atoms contain a silicon atom or a phosphorus atom,

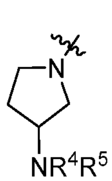, 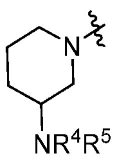, 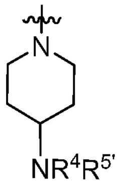, 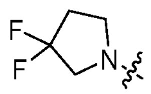 and 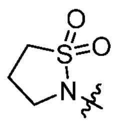,

wherein the amino or amino-$C_1$-$C_6$ alkyl- is substituted with one or more $R^{21}$, and optionally substituted with one or more $R^{21'}$, wherein the 5-6 membered heterocyclyl whose ring atoms consist of a nitrogen atom and a carbon atom is substituted with one or more $R^{22}$, and optionally substituted with one or more $R^{22'}$, wherein the 7-9 membered spiro-heterocyclyl, 10 membered spiro-heterocyclyl whose ring atoms consist of a nitrogen atom and a carbon atom, 7-10 membered heterocycloalkenyl or 5-6 membered heterocyclyl whose ring atoms contain a silicon atom or a phosphorus atom is optionally substituted with one or more $R^{23}$, wherein the 7-10 membered bridged heterocyclyl is substituted with one or more $R^{24}$,

or, $R^3$ is connected to $R^2$ or $R^{2'}$, and together with the carbon atoms connected thereto, forms morpholinyl optionally substituted with one or more $R^{25}$,

and $R^2$, $R^{2'}$ and $R^3$ are not hydrogen at the same time;

$R^{21}$ is selected from the group consisting of $C_2$-$C_6$ alkenyl-C(O)-, $C_3$-$C_{12}$ cycloalkyl, 4-12 membered heterocyclyl or $C_1$-$C_6$ alkylacylamino-$C_1$-$C_6$ alkyl-, wherein the $C_3$-$C_{12}$ cycloalkyl, 4-12 membered heterocyclyl or $C_1$-$C_6$ alkylacylamino-$C_1$-$C_6$ alkyl- is optionally substituted with one or more of the following groups selected from the group consisting of: hydroxy, $C_1$-$C_6$ alkyl and $C_1$-$C_6$ alkylacyl;

$R^{22}$ is selected from the group consisting of $C_2$-$C_6$ alkynyl-C(O)-, 4-5 membered heterocyclyl or 6 membered heterocyclyl whose ring atoms consist of a nitrogen atom and a sulfur atom, wherein the 4-5 membered heterocyclyl or 6 membered heterocyclyl whose ring atoms consist of a nitrogen atom and a sulfur atom is optionally substituted with one or more of the following groups selected from the group consisting of: 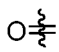, halogen, $C_1$-$C_3$ alkyl and $C_1$-$C_6$ alkylacyl;

$R^{21}$ and $R^{22'}$ are each independently selected from the group consisting of deuterium and $C_1$-$C_6$ alkyl;

$R^{23}$ and $R^{24}$ are each independently selected from the group consisting of 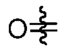, $C_1$-$C_6$ alkyl, $C_1$-$C_6$ alkylsulfonyl, $C_1$-$C_6$ alkylacyl and $C_1$-$C_6$ alkylacyl-N ($C_1$-$C_6$ alkyl)-;

$R^{25}$ is selected from $C_1$-$C_6$ alkylacyl;

$R^4$ is selected from the group consisting of $C_1$-$C_6$ alkylacyl and $C_1$-$C_6$ alkylsulfonyl;

$R^5$ is selected from $C_1$-$C_6$ alkyl;

$R^{5'}$ is selected from the group consisting of $-CD_3$ and $-CH_3$, and when $R^{5'}$ is $-CH_3$, $R^1$ or $R^{1'}$ is halogen.

In another aspect, the present application further provides a pharmaceutical composition comprising the compound or the pharmaceutically acceptable salt thereof of the present application described above.

In another aspect, the present application further provides a method for treating a disease mediated by Akt kinase in a mammal, comprising administering to a mammal in need of such treatment a therapeutically effective amount of the compound, the pharmaceutically acceptable salt thereof or the pharmaceutical composition thereof described above.

In another aspect, the present application further provides use of the compound, the pharmaceutically acceptable salt thereof or the pharmaceutical composition thereof described above in preparing a medicament for treating a disease mediated by Akt kinase.

In another aspect, the present application further provides use of the compound, the pharmaceutically acceptable salt thereof or the pharmaceutical composition thereof described above in treating a disease mediated by Akt kinase.

In another aspect, the present application further provides the compound, the pharmaceutically acceptable salt thereof or the pharmaceutical composition thereof of the present application described above for use in treating a disease mediated by Akt kinase.

## SUMMARY

In one aspect, the present application provides a compound of formula (I) or a pharmaceutically acceptable salt thereof,

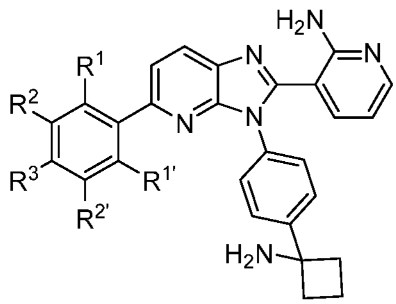

(I)

wherein,

$R^1$ and $R^{1'}$ are each independently selected from the group consisting of hydrogen and halogen;
$R^2$, $R^{2'}$ and $R^3$ are each independently selected from the group consisting of hydrogen, amino, amino-$C_1$-$C_6$ alkyl-, 7-9 membered spiro-heterocyclyl, 10 membered spiro-heterocyclyl whose ring atoms consist of a nitrogen atom and a carbon atom, 7-10 membered fused-heterocyclyl, 7-10 membered bridged heterocyclyl, 5-6 membered heterocyclyl whose ring atoms consist of a nitrogen atom and a carbon atom, 5-6 membered heterocyclyl whose ring atoms contain a silicon atom or a phosphorus atom,

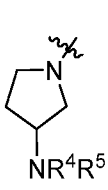, 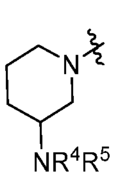, 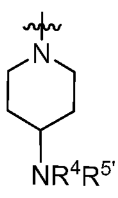, 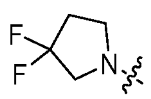 and 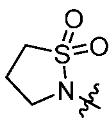,

wherein the amino or amino-$C_1$-$C_6$ alkyl- is substituted with one or more $R^{21}$, and optionally substituted with one or more $R^{21'}$, wherein the 5-6 membered heterocyclyl whose ring atoms consist of a nitrogen atom and a carbon atom is substituted with one or more $R^{22}$, and optionally substituted with one or more $R^{22'}$, wherein the 7-9 membered spiro-heterocyclyl, 10 membered spiro-heterocyclyl whose ring atoms consist of a nitrogen atom and a carbon atom, 7-10 membered heterocycloalkenyl or 5-6 membered heterocyclyl whose ring atoms contain a silicon atom or a phosphorus atom is optionally substituted with one or more $R^{23}$, wherein the 7-10 membered bridged heterocyclyl is substituted with one or more $R^{24}$,
or, $R^3$ is connected to $R^2$ or $R^{2'}$, and together with the carbon atoms connected thereto, forms morpholinyl optionally substituted with one or more $R^{25}$,
and $R^2$, $R^{2'}$ and $R^3$ are not hydrogen at the same time;

$R^{21}$ is selected from the group consisting of $C_2$-$C_6$ alkenyl-C(O)-, $C_3$-$C_{12}$ cycloalkyl, 4-12 membered heterocyclyl or $C_1$-$C_6$ alkylacylamino-$C_1$-$C_6$ alkyl-, wherein the $C_3$-$C_{12}$ cycloalkyl, 4-12 membered heterocyclyl or $C_1$-$C_6$ alkylacylamino-$C_1$-$C_6$ alkyl- is optionally substituted with one or more of the following groups selected from the group consisting of: hydroxy, $C_1$-$C_6$ alkyl and $C_1$-$C_6$ alkylacyl;
$R^{22}$ is selected from the group consisting of $C_2$-$C_6$ alkynyl-C(O)-, 4-5 membered heterocyclyl or 6 membered heterocyclyl whose ring atoms consist of a nitrogen atom and a sulfur atom, wherein the 4-5 membered heterocyclyl or 6 membered heterocyclyl whose ring atoms consist of a nitrogen atom and a sulfur atom is optionally substituted with one or more of the following groups selected from the group consisting of: 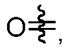, halogen, $C_1$-$C_3$ alkyl and $C_1$-$C_6$ alkylacyl;
$R^{21'}$ and $R^{22'}$ are each independently selected from the group consisting of deuterium and $C_1$-$C_6$ alkyl;
$R^{23}$ and $R^{24}$ are each independently selected from the group consisting of 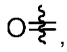, $C_1$-$C_6$ alkyl, $C_1$-$C_6$ alkylsulfonyl, $C_1$-$C_6$ alkylacyl and $C_1$-$C_6$ alkylacyl-N ($C_1$-$C_6$ alkyl)-;
$R^{25}$ is selected from $C_1$-$C_6$ alkylacyl;
$R^4$ is selected from the group consisting of $C_1$-$C_6$ alkylacyl and $C_1$-$C_6$ alkylsulfonyl;
$R^5$ is selected from $C_1$-$C_6$ alkyl;
$R^{5'}$ is selected from the group consisting of $-CD_3$ and $-CH_3$, and when $R^{5'}$ is $-CH_3$, $R^1$ or $R^{1'}$ is halogen.

In some embodiments, the present application provides a compound of formula (I) or a pharmaceutically acceptable salt thereof, wherein,

$R^1$ and $R^{1'}$ are each independently selected from the group consisting of hydrogen and halogen;
$R^2$, $R^{2'}$ and $R^3$ are each independently selected from the group consisting of hydrogen, amino, amino-$C_1$-$C_6$ alkyl-, 7-9 membered spiro-heterocyclyl, 7-10 membered fused-heterocyclyl, 7-10 membered bridged heterocyclyl, 5-6 membered heterocyclyl whose ring atoms consist of a nitrogen atom and a carbon atom, 5-6 membered heterocyclyl whose ring atoms contain a silicon atom or a phosphorus atom,

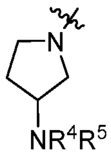, 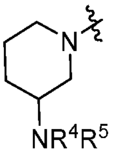, 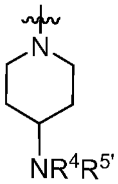, 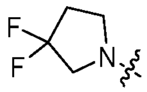 and 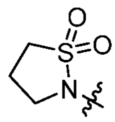,

wherein the amino or amino-$C_1$-$C_6$ alkyl- is substituted with one or more $R^{21}$, and optionally substituted with one or more $R^{21'}$, wherein the 5-6 membered heterocyclyl whose ring atoms consist of a nitrogen atom and a carbon atom is substituted with one or more $R^{22}$, and optionally substituted with one or more $R^{22'}$, wherein the 7-9 membered spiro-heterocyclyl, 7-10 membered heterocycloalkenyl or 5-6 membered heterocyclyl whose ring atoms contain a silicon atom or a phosphorus atom is optionally substituted with one or more $R^{23}$, wherein the 7-10 membered bridged heterocyclyl is substituted with one or more $R^{24}$,
or, $R^3$, together with $R^2$ or $R^{2'}$ and the carbon atoms connected thereto, forms morpholinyl optionally substituted with one or more $R^{25}$,
and $R^2$, $R^{2'}$ and $R^3$ are not hydrogen at the same time;
$R^{21}$ is selected from the group consisting of $C_2$-$C_6$ alkenyl-C(O)-, $C_3$-$C_{12}$ cycloalkyl, 4-12 membered heterocyclyl or $C_1$-$C_6$ alkylacylamino-$C_1$-$C_6$ alkyl-, wherein the $C_3$-$C_{12}$ cycloalkyl, 4-12 membered heterocyclyl or $C_1$-$C_6$ alkylacylamino-$C_1$-$C_6$ alkyl- is optionally substituted with one or more of the following groups selected from the group consisting of: hydroxy, $C_1$-$C_6$ alkyl and $C_1$-$C_6$ alkylacyl;
$R^{22}$ is selected from the group consisting of $C_2$-$C_6$ alkynyl-C(O)- and 4-5 membered heterocyclyl, wherein the 4-5 membered heterocyclyl is optionally substituted with one or more 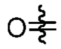;
$R^{21'}$ and $R^{22'}$ are each independently selected from $C_1$-$C_6$ alkyl;
$R^{23}$ and $R^{24}$ are each independently selected from the group consisting of 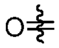, $C_1$-$C_6$ alkyl, $C_1$-$C_6$ alkylsulfonyl, $C_1$-$C_6$ alkylacyl and $C_1$-$C_6$ alkylacyl-N ($C_1$-$C_6$ alkyl)-;
$R^{25}$ is selected from $C_1$-$C_6$ alkylacyl;

$R^4$ is selected from the group consisting of $C_1$-$C_6$ alkylacyl and $C_1$-$C_6$ alkylsulfonyl;
$R^5$ is selected from $C_1$-$C_6$ alkyl;
$R^{5'}$ is selected from the group consisting of -$CD_3$ and -$CH_3$, and when $R^{5'}$ is -$CH_3$, $R^1$ or $R^{1'}$ is halogen.

In some embodiments, the present application provides a compound of formula (I) or a pharmaceutically acceptable salt thereof, wherein,

$R^1$ and $R^{1'}$ are each independently selected from the group consisting of hydrogen and halogen;
$R^2$ and $R^{2'}$ are each independently selected from the group consisting of hydrogen, amino, amino-$C_1$-$C_6$ alkyl-, 7-9 membered spiro-heterocyclyl, 10 membered spiro-heterocyclyl whose ring atoms consist of a nitrogen atom and a carbon atom, 7-10 membered fused-heterocyclyl, 7-10 membered bridged heterocyclyl, 5-6 membered heterocyclyl whose ring atoms consist of a nitrogen atom and a carbon atom, 5-6 membered heterocyclyl whose ring atoms contain a silicon atom or a phosphorus atom,

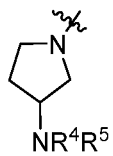, 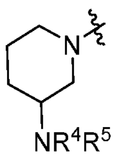, 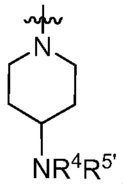, 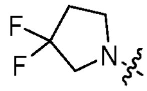 and 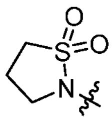,

wherein the amino or amino-$C_1$-$C_6$ alkyl- is substituted with one or more $R^{21}$, and optionally substituted with one or more $R^{21'}$, wherein the 5-6 membered heterocyclyl whose ring atoms consist of a nitrogen atom and a carbon atom is substituted with one or more $R^{22}$, and optionally substituted with one or more $R^{22'}$, wherein the 7-9 membered spiro-heterocyclyl, 10 membered spiro-heterocyclyl whose ring atoms consist of a nitrogen atom and a carbon atom, 7-10 membered heterocycloalkenyl or 5-6 membered heterocyclyl whose ring atoms contain a silicon atom or a phosphorus atom is optionally substituted with one or more $R^{23}$, wherein the 7-10 membered bridged heterocyclyl is substituted with one or more $R^{24}$,
$R^3$ is hydrogen;
or, $R^3$ is connected to $R^2$ or $R^{2'}$, and together with the carbon atoms connected thereto, forms morpholinyl optionally substituted with one or more $R^{25}$,
and $R^2$ and $R^{2'}$ are not hydrogen at the same time;
$R^{21}$ is selected from the group consisting of $C_2$-$C_6$ alkenyl-C(O)-, $C_3$-$C_{12}$ cycloalkyl, 4-12 membered heterocyclyl or $C_1$-$C_6$ alkylacylamino-$C_1$-$C_6$ alkyl-, wherein the $C_3$-$C_{12}$ cycloalkyl, 4-12 membered heterocyclyl or $C_1$-$C_6$ alkylacylamino-$C_1$-$C_6$ alkyl- is optionally substituted with one or more of the following groups selected from the group consisting of: hydroxy, $C_1$-$C_6$ alkyl and $C_1$-$C_6$ alkylacyl; $R^{22}$ is selected from the group consisting of $C_2$-$C_6$ alkynyl-C(O)-, 4-5 membered heterocyclyl or 6 membered heterocyclyl whose ring atoms consist of a nitrogen atom and a sulfur atom, wherein the 4-5 membered heterocyclyl or 6 membered heterocyclyl whose ring atoms consist of a nitrogen atom and a sulfur atom is optionally substituted with one or more of the following groups selected from the group consisting of: 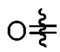, halogen, $C_1$-$C_3$ alkyl and $C_1$-$C_6$ alkylacyl;
$R^{21'}$ and $R^{22'}$ are each independently selected from the group consisting of deuterium and $C_1$-$C_6$ alkyl;
$R^{23}$ and $R^{24}$ are each independently selected from the group consisting of 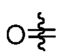, $C_1$-$C_6$ alkyl, $C_1$-$C_6$ alkylsulfonyl, $C_1$-$C_6$ alkylacyl and $C_1$-$C_6$ alkylacyl-N ($C_1$-$C_6$ alkyl)-;
$R^{25}$ is selected from $C_1$-$C_6$ alkylacyl;
$R^4$ is selected from the group consisting of $C_1$-$C_6$ alkylacyl and $C_1$-$C_6$ alkylsulfonyl;
$R^5$ is selected from $C_1$-$C_6$ alkyl;
$R^{5'}$ is selected from the group consisting of -$CD_3$ and -$CH_3$, and when $R^{5'}$ is -$CH_3$, $R^1$ or $R^{1'}$ is halogen.

In some embodiments, the present application provides a compound of formula (I) or a pharmaceutically acceptable salt thereof, wherein,

$R^1$ and $R^{1'}$ are each independently selected from the group consisting of hydrogen and halogen;
one of $R^2$ and $R^{2'}$ is hydrogen and the other is selected from the group consisting of amino, amino-$C_1$-$C_6$ alkyl-, 7-9 membered spiro-heterocyclyl, 10 membered spiro-heterocyclyl whose ring atoms consist of a nitrogen atom and a

carbon atom, 7-10 membered fused-heterocyclyl, 7-10 membered bridged heterocyclyl, 5-6 membered heterocyclyl whose ring atoms consist of a nitrogen atom and a carbon atom, 5-6 membered heterocyclyl whose ring atoms contain a silicon atom or a phosphorus atom,

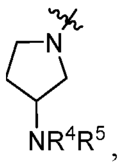, 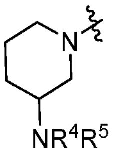, 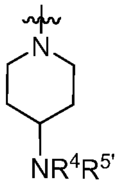, 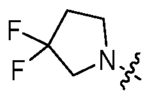 and 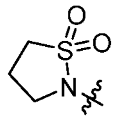,

wherein the amino or amino-$C_1$-$C_6$ alkyl- is substituted with one or more $R^{21}$, and optionally substituted with one or more $R^{21'}$, wherein the 5-6 membered heterocyclyl whose ring atoms consist of a nitrogen atom and a carbon atom is substituted with one or more $R^{22}$, and optionally substituted with one or more $R^{22'}$, wherein the 7-9 membered spiro-heterocyclyl, 10 membered spiro-heterocyclyl whose ring atoms consist of a nitrogen atom and a carbon atom, 7-10 membered heterocycloalkenyl or 5-6 membered heterocyclyl whose ring atoms contain a silicon atom or a phosphorus atom is optionally substituted with one or more $R^{23}$, wherein the 7-10 membered bridged heterocyclyl is substituted with one or more $R^{24}$,

$R^3$ is hydrogen;

or, $R^3$ is connected to $R^2$ or $R^{2'}$, and together with the carbon atoms connected thereto, forms morpholinyl optionally substituted with one or more $R^{25}$;

$R^{21}$ is selected from the group consisting of $C_2$-$C_6$ alkenyl-C(O)-, $C_3$-$C_{12}$ cycloalkyl, 4-12 membered heterocyclyl or $C_1$-$C_6$ alkylacylamino-$C_1$-$C_6$ alkyl-, wherein the $C_3$-$C_{12}$ cycloalkyl, 4-12 membered heterocyclyl or $C_1$-$C_6$ alkylacylamino-$C_1$-$C_6$ alkyl- is optionally substituted with one or more of the following groups selected from the group consisting of: hydroxy, $C_1$-$C_6$ alkyl and $C_1$-$C_6$ alkylacyl;

$R^{22}$ is selected from the group consisting of $C_2$-$C_6$ alkynyl-C(O)-, 4-5 membered heterocyclyl or 6 membered heterocyclyl whose ring atoms consist of a nitrogen atom and a sulfur atom, wherein the 4-5 membered heterocyclyl or 6 membered heterocyclyl whose ring atoms consist of a nitrogen atom and a sulfur atom is optionally substituted with one or more of the following groups selected from the group consisting of: 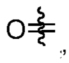, halogen, $C_1$-$C_3$ alkyl and $C_1$-$C_6$ alkylacyl;

$R^{21'}$ and $R^{22'}$ are each independently selected from the group consisting of deuterium and $C_1$-$C_6$ alkyl;

$R^{23}$ and $R^{24}$ are each independently selected from the group consisting of 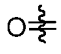, $C_1$-$C_6$ alkyl, $C_1$-$C_6$ alkylsulfonyl, $C_1$-$C_6$ alkylacyl and $C_1$-$C_6$ alkylacyl-N ($C_1$-$C_6$ alkyl)-;

$R^{25}$ is selected from $C_1$-$C_6$ alkylacyl;

$R^4$ is selected from the group consisting of $C_1$-$C_6$ alkylacyl and $C_1$-$C_6$ alkylsulfonyl;

$R^5$ is selected from $C_1$-$C_6$ alkyl;

$R^{5'}$ is selected from the group consisting of $-CD_3$ and $-CH_3$, and when $R^{5'}$ is $-CH_3$, $R^1$ or $R^{1'}$ is halogen.

In some embodiments, the 7-9 membered spiro-heterocyclyl, 7-10 membered fused-heterocyclyl and 7-10 membered bridged heterocyclyl each independently contain 1-3 heteroatoms selected from the group consisting of N, O and S.

In some embodiments, the 7-9 membered spiro-heterocyclyl, 7-10 membered fused-heterocyclyl and 7-10 membered bridged heterocyclyl each independently contain 1 or 2 heteroatoms selected from the group consisting of N, O and S.

In some embodiments, the 10 membered spiro-heterocyclyl whose ring atoms consist of a nitrogen atom and a carbon atom contains 1, 2, or 3 nitrogen atoms.

In some embodiments, the 5-6 membered heterocyclyl whose ring atoms consist of a nitrogen atom and a carbon atom contains 1, 2, or 3 nitrogen atoms.

In some embodiments, the 5-6 membered heterocyclyl whose ring atoms contain a silicon atom or a phosphorus atom contains 1 silicon atom or 1 phosphorus atom, and 1 or 2 heteroatoms selected from the group consisting of N, O and S.

In some embodiments, the 4-5 membered heterocyclyl and 4-12 membered heterocyclyl each independently contain 1-3 heteroatoms selected from the group consisting of N, O and S.

In some embodiments, $R^1$ and $R^{1'}$ are each independently selected from the group consisting of hydrogen and fluorine.

In some embodiments, $R^1$ and $R^{1'}$ are both selected from hydrogen.

In some embodiments, $R^1$ is selected from hydrogen, and $R^{1'}$ is selected from halogen; or $R^1$ is selected from halogen, and $R^{1'}$ is selected from hydrogen.

In some embodiments, $R^1$ is selected from hydrogen, and $R^{1'}$ is selected from fluorine; or $R^1$ is selected from fluorine, and $R^{1'}$ is selected from hydrogen.

In some embodiments, $R^2$, $R^{2'}$ and $R^3$ are each independently selected from the group consisting of hydrogen, amino, amino-$C_1$-$C_6$ alkyl-, 7-9 membered spiro-heterocyclyl, 7-10 membered fused-heterocyclyl, 7-10 membered bridged heterocyclyl, 5-6 membered heterocyclyl whose ring atoms consist of a nitrogen atom and a carbon atom, 5-6 membered heterocyclyl whose ring atoms contain a silicon atom or a phosphorus atom,

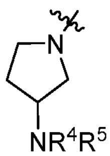 , 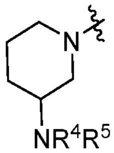 , 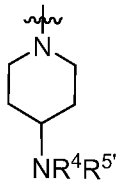 , 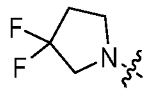 and 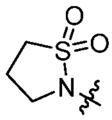 ,

wherein the amino or amino-$C_1$-$C_6$ alkyl- is substituted with one or more $R^{21}$, and optionally substituted with one or more $R^{21'}$, wherein the 5-6 membered heterocyclyl whose ring atoms consist of a nitrogen atom and a carbon atom is substituted with one or more $R^{22}$, and optionally substituted with one or more $R^{22'}$, wherein the 7-9 membered spiro-heterocyclyl, 7-10 membered heterocycloalkenyl or 5-6 membered heterocyclyl whose ring atoms contain a silicon atom or a phosphorus atom is optionally substituted with one or more $R^{23}$, wherein the 7-10 membered bridged heterocyclyl is substituted with one or more $R^{24}$,

or, $R^3$ is connected to $R^2$ or $R^{2'}$, and together with the carbon atoms connected thereto, forms morpholinyl optionally substituted with one or more $R^{25}$,
and $R^2$, $R^{2'}$ and $R^3$ are not hydrogen at the same time.

In some embodiments, $R^2$, $R^{2'}$ and $R^3$ are each independently selected from the group consisting of hydrogen, amino, amino-$C_1$-$C_4$ alkyl-, 7, 8 or 9 membered spiro-heterocyclyl, 10 membered spiro-heterocyclyl whose ring atoms consist of a nitrogen atom and a carbon atom, 7, 8 or 9 membered fused-heterocyclyl, 7-8 membered bridged heterocyclyl, 5-6 membered monoheterocyclyl whose ring atoms consist of a nitrogen atom and a carbon atom, 5-6 membered monoheterocyclyl whose ring atoms consist of a silicon atom or a phosphorus atom,

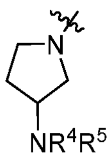 , 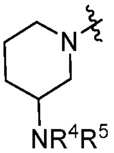 , 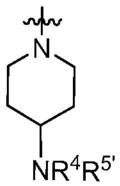 , 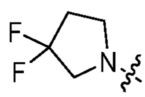

and

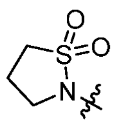 ,

wherein the amino or amino-$C_1$-$C_4$ alkyl- is substituted with one or more $R^{21}$, and optionally substituted with one or more $R^{21'}$, wherein the 5-6 membered monoheterocyclyl whose ring atoms consist of a nitrogen atom and a carbon atom is substituted with one or more $R^{22}$, and optionally substituted with one or more $R^{22'}$, wherein the 7, 8 or 9 membered spiro-heterocyclyl, 10 membered spiro-heterocyclyl whose ring atoms consist of a nitrogen atom and a carbon atom, 7, 8 or 9 membered fused-heterocyclyl, or 5-6 membered monoheterocyclyl whose ring atoms contain a silicon atom or a

phosphorus atom is optionally substituted with one or more $R^{23}$, wherein the 7-8 membered bridged heterocyclyl is substituted with one or more $R^{24}$,

or, $R^3$ is connected to $R^2$ or $R^{2'}$, and together with the carbon atoms connected thereto, forms morpholinyl optionally substituted with one or more $R^{25}$,
and $R^2$, $R^{2'}$ and $R^3$ are not hydrogen at the same time.

In some embodiments, $R^2$, $R^{2'}$ and $R^3$ are each independently selected from the group consisting of hydrogen, amino, amino-$C_1$-$C_4$ alkyl-, 7, 8 or 9 membered spiro-heterocycloalkyl, 10 membered spiro-heterocycloalkyl whose ring atoms consist of a nitrogen atom and a carbon atom, 7, 8 or 9 membered fused-heterocycloalkyl, 7-8 membered bridged heterocycloalkyl, 5-6 membered monoheterocycloalkyl whose ring atoms consist of a nitrogen atom and a carbon atom, 5-6 membered monoheterocycloalkyl whose ring atoms contain a silicon atom or a phosphorus atom,

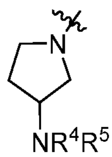,

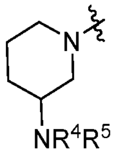, 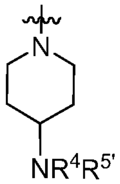, 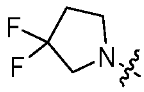 and 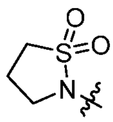,

wherein the amino or amino-$C_1$-$C_4$ alkyl- is substituted with one or more $R^{21}$, and optionally substituted with one or more $R^{21'}$, wherein the 5-6 membered monoheterocycloalkyl whose ring atoms consist of a nitrogen atom and a carbon atom is substituted with one or more $R^{22}$, and optionally substituted with one or more $R^{22'}$, wherein the 7, 8 or 9 membered spiro-heterocycloalkyl, 10 membered spiro-heterocycloalkyl whose ring atoms consist of a nitrogen atom and a carbon atom, 7, 8 or 9 membered fused-heterocycloalkyl, or 5-6 membered monoheterocycloalkyl whose ring atoms contain a silicon atom or a phosphorus atom is optionally substituted by one or more $R^{23}$, wherein the 7-8 membered bridged heterocycloalkyl is substituted by one or more $R^{24}$,

or, $R^3$ is connected to $R^2$ or $R^{2'}$, and together with the carbon atoms connected thereto, forms morpholinyl optionally substituted with one or more $R^{25}$,
and $R^2$, $R^{2'}$ and $R^3$ are not hydrogen at the same time.

In some embodiments, $R^2$, $R^{2'}$ and $R^3$ are each independently selected from the group consisting of hydrogen, amino, amino-$C_1$-$C_4$ alkyl-, 7, 8 or 9 membered spiro-heterocycloalkyl, 10 membered spiro-heterocycloalkyl whose ring atoms consist of a nitrogen atom and a carbon atom, 5 membered fused 4 membered heterocycloalkyl, 5 membered fused 5 membered heterocycloalkyl, 5 membered fused 6 membered heterocycloalkyl, 7-8 membered bridged heterocycloalkyl, 5-6 membered monoheterocycloalkyl whose ring atoms consist of a nitrogen atom and a carbon atom, 6 membered monoheterocycloalkyl whose ring atoms contain a silicon atom or a phosphorus atom,

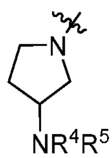,

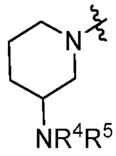, 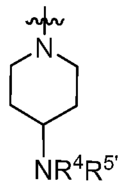, 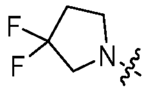 and 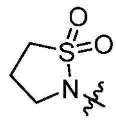,

wherein the amino or amino-$C_1$-$C_4$ alkyl- is substituted with one or more $R^{21}$, and optionally substituted with one or more $R^{21'}$, wherein the 5 membered or 6 membered monoheterocycloalkyl whose ring atoms consist of a nitrogen atom and a carbon atom is substituted with one or more $R^{22}$, and optionally substituted with one or more $R^{22'}$, wherein the 7, 8 or 9 membered spiro-heterocycloalkyl, 10 membered spiro-heterocycloalkyl whose ring atoms consist of a nitrogen atom and a carbon atom, 5 membered fused 4 membered heterocycloalkyl, 5 membered fused 5 membered heterocycloalkyl, 5 membered fused 6 membered heterocycloalkyl, or 6 membered monoheterocycloalkyl whose ring atoms contain a silicon atom or a phosphorus atom is optionally substituted with one or more $R^{23}$, wherein the 7-8 membered bridged heterocycloalkyl is substituted with one or more $R^{24}$,

or, $R^3$ is connected to $R^2$ or $R^{2'}$, and together with the carbon atoms connected thereto, forms morpholinyl optionally substituted with one or more $R^{25}$,
and $R^2$, $R^{2'}$ and $R^3$ are not hydrogen at the same time.

In some embodiments, $R^2$, $R^{2'}$ and $R^3$ are each independently selected from the group consisting of hydrogen, amino, aminomethyl,

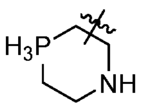, 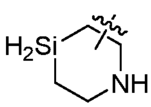, 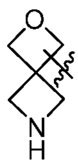, 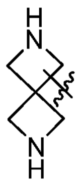, 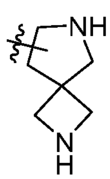, 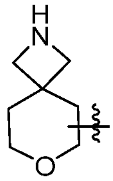,

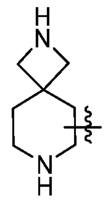, 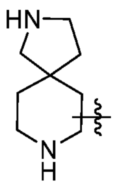, 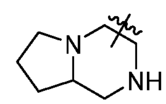, 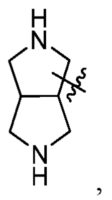, 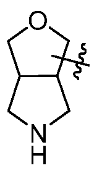, 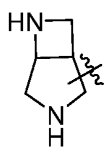, 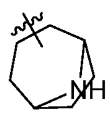, 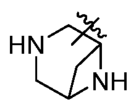, 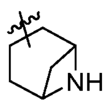,

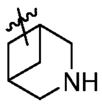,

pyrrolidinyl, piperidinyl, piperazinyl,

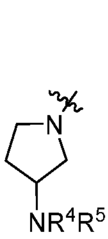, 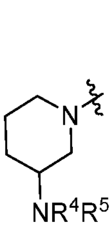, 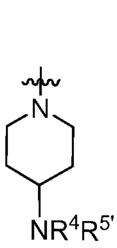, 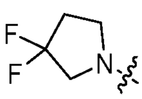

and

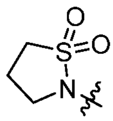,

wherein the amino or aminomethyl is substituted with one or more $R^{21}$, and optionally substituted with one or more $R^{21'}$, wherein the pyrrolidinyl, piperidinyl or piperazinyl is substituted with one or more $R^{22}$, and optionally substituted with one or more $R^{22'}$, wherein the

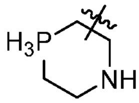,

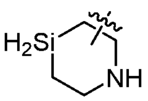, 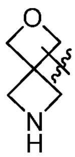, 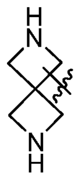, 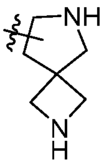, 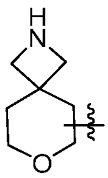, 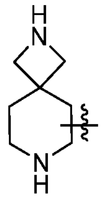, 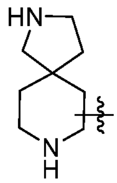, 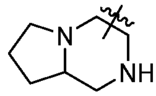, 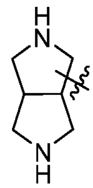,

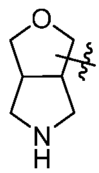 or 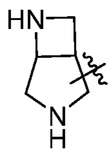

is substituted with one or more $R^{23}$, wherein the

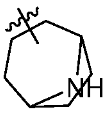, 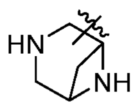,

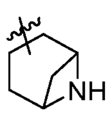 or 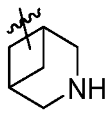

is substituted with one or more $R^{24}$,

or, $R^3$ is connected to $R^2$ or $R^{2'}$, and together with the carbon atoms connected thereto, forms a

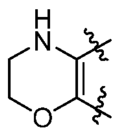

group optionally substituted with one or more $R^{25}$,
and $R^2$, $R^{2'}$ and $R^3$ are not hydrogen at the same time.

In some embodiments, $R^{2'}$, $R^{2'}$ and $R^3$ are each independently selected from the group consisting of hydrogen, amino, aminomethyl,

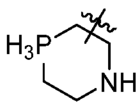, 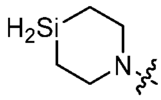, 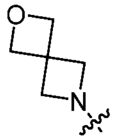, 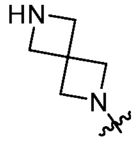, 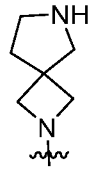, 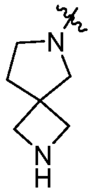,

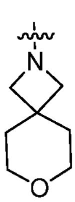, 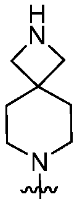, 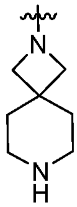, 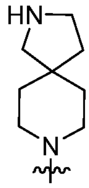, 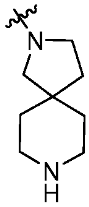, 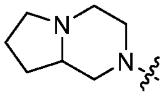, 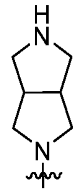, 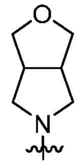, 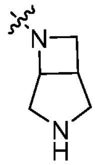, 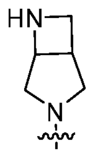,

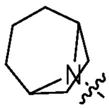, 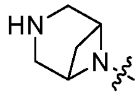, 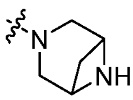, 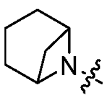, 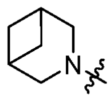, 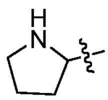, 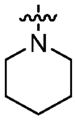, 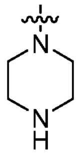, 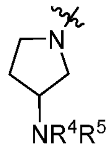,

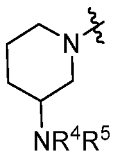, 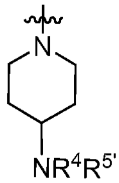, 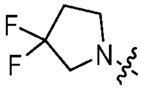 and 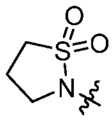,

wherein the amino or aminomethyl is substituted with one or more $R^{21}$, and optionally substituted with one or more $R^{21'}$, wherein the

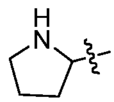, 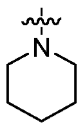

or

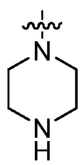

is substituted with one or more $R^{22}$, and optionally substituted with one or more $R^{22'}$, wherein the

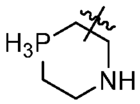, 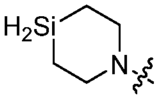, 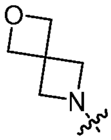, 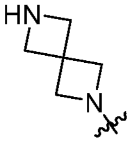, 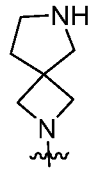, 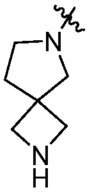, 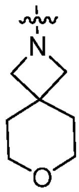, 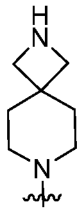,

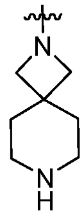, 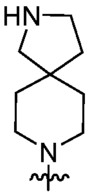, 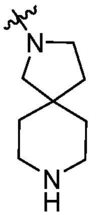, 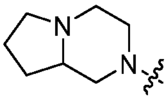, 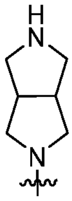, 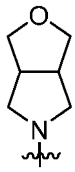, 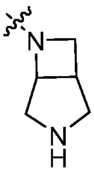 or 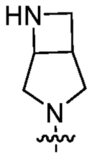

is substituted with one or more $R^{23}$, wherein the

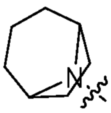, 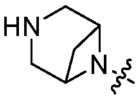, 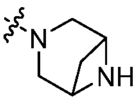, 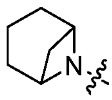 or 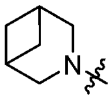

is substituted with one or more $R^{24}$,

or, $R^3$ is connected to $R^2$ or $R^{2'}$, and together with the carbon atoms connected thereto, forms a

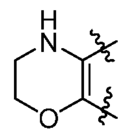

group optionally substituted with one or more $R^{25}$,
and $R^2$, $R^{2'}$ and $R^3$ are not hydrogen at the same time.

In some embodiments, $R^2$, $R^{2'}$ and $R^3$ are each independently selected from the group consisting of hydrogen, amino, aminomethyl,

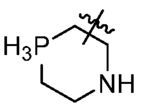, 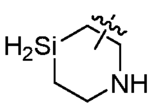, 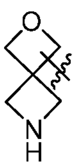, 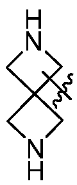, 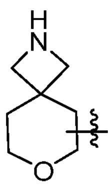, 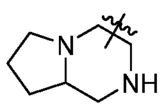,

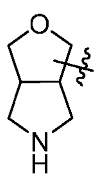, 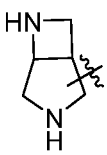, 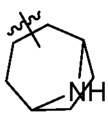,

pyrrolidinyl, piperidinyl, piperazinyl,

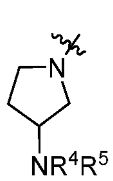, 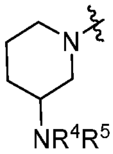, 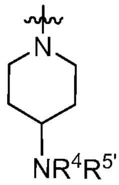,

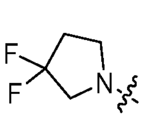 and 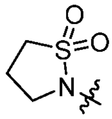,

wherein the amino and aminomethyl are substituted with one or more $R^{21}$, and optionally substituted with one or more $R^{21'}$, wherein the pyrrolidinyl, piperidinyl or piperazinyl is substituted with one or more $R^{22}$, and optionally substituted with one or more $R^{22'}$, wherein the

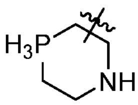, 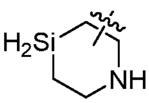, 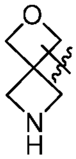, 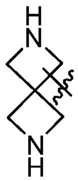, 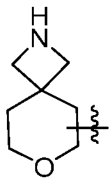, 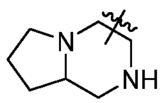, 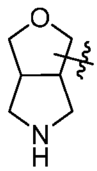 and 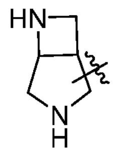

are optionally substituted with one or more $R^{23}$, wherein the

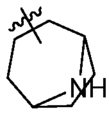

is substituted with one or more $R^{24}$,

or, $R^3$ together with $R^2$ or $R^{2'}$ and the carbon atoms connected thereto, forms a

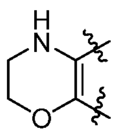

group optionally substituted with one or more $R^{25}$,
and $R^2$, $R^{2'}$ and $R^3$ are not hydrogen at the same time.

In some embodiments, $R^2$, $R^{2'}$ and $R^3$ are each independently selected from the group consisting of hydrogen, amino, aminomethyl,

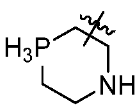, 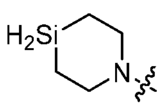, 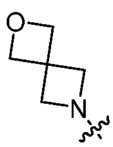, 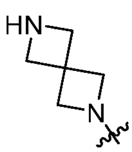, 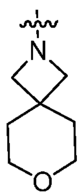,

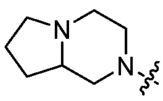, 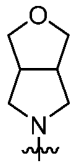, 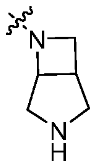, 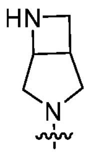, 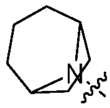, 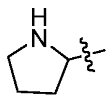, 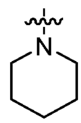, 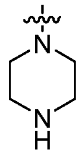, 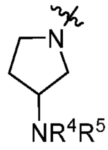, 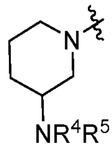,

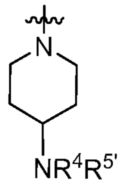, 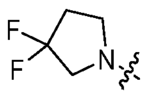 and 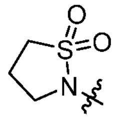,

wherein the amino and aminomethyl are substituted with one or more $R^{21}$, and optionally substituted with one or more $R^{21'}$, wherein the

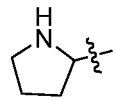, 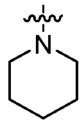

and

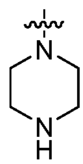

are substituted with one or more $R^{22}$, and optionally substituted with one or more $R^{22'}$, wherein the

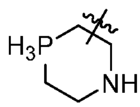, 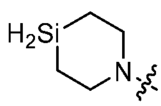, 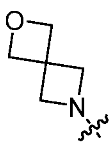, 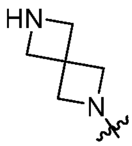, 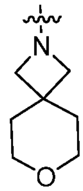, 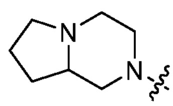, 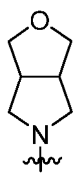, 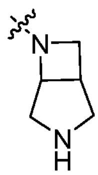

and

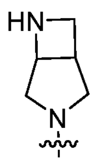

are optionally substituted with one or more $R^{23}$, wherein the

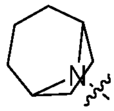

is substituted with one or more $R^{24}$,

or, $R^3$ together with $R^2$ or $R^{2'}$ and the carbon atoms connected thereto, forms a

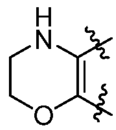

group optionally substituted with one or more $R^{25}$,
and $R^2$, $R^{2'}$ and $R^3$ are not hydrogen at the same time.

In some embodiments, one of $R^2$ and $R^{2'}$ is hydrogen and the other is selected from the group consisting of amino, amino-$C_1$-$C_6$ alkyl-, 7-9 membered spiro-heterocyclyl, 7-10 membered fused-heterocyclyl, 7-10 membered bridged heterocyclyl, 5-6 membered heterocyclyl whose ring atoms consist of a nitrogen atom and a carbon atom, 5-6 membered heterocyclyl whose ring atoms contain a silicon atom or a phosphorus atom,

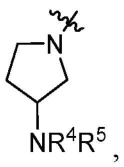 , 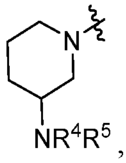 , 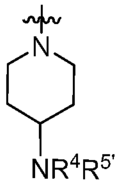 , 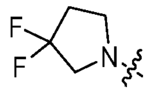 and 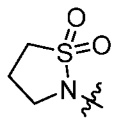 ,

wherein the amino or amino-$C_1$-$C_6$ alkyl- is substituted with one or more $R^{21}$, and optionally substituted with one or more $R^{21'}$, wherein the 5-6 membered heterocyclyl whose ring atoms consist of a nitrogen atom and a carbon atom is substituted with one or more $R^{22}$, and optionally substituted with one or more $R^{22'}$, wherein the 7-9 membered spiro-heterocyclyl, 7-10 membered heterocycloalkenyl or 5-6 membered heterocyclyl whose ring atoms contain a silicon atom or a phosphorus atom is optionally substituted with one or more $R^{23}$, wherein the 7-10 membered bridged heterocyclyl is substituted with one or more $R^{24}$,
and $R^3$ is hydrogen.

In some embodiments, one of $R^2$ and $R^{2'}$ is hydrogen and the other is selected from the group consisting of amino, amino-$C_1$-$C_4$ alkyl-, 7, 8 or 9 membered spiro-heterocyclyl, 10 membered spiro-heterocyclyl whose ring atoms consist of a nitrogen atom and a carbon atom, 7, 8 or 9 membered fused-heterocyclyl, 7-8 membered bridged heterocyclyl, 5-6 membered monoheterocyclyl whose ring atoms consist of a nitrogen atom and a carbon atom, 5-6 membered monoheterocyclyl whose ring atoms consist of a silicon atom or a phosphorus atom,

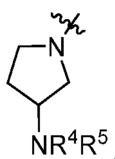 , 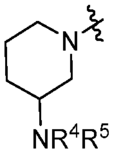 , 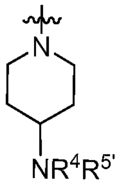 , 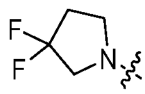

and

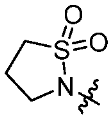 ,

wherein the amino or amino-$C_1$-$C_4$ alkyl- is substituted with one or more $R^{21}$, and optionally substituted with one or more $R^{21'}$, wherein the 5-6 membered monoheterocyclyl whose ring atoms consist of a nitrogen atom and a carbon atom is substituted with one or more $R^{22}$, and optionally substituted with one or more $R^{22'}$, wherein the 7, 8 or 9 membered

spiro-heterocyclyl, 10 membered spiro-heterocyclyl whose ring atoms consist of a nitrogen atom and a carbon atom, 7, 8 or 9 membered fused-heterocyclyl, or 5-6 membered monoheterocyclyl whose ring atoms contain a silicon atom or a phosphorus atom is optionally substituted with one or more $R^{23}$, wherein the 7-8 membered bridged heterocyclyl is substituted with one or more $R^{24}$,
and $R^3$ is hydrogen.

In some embodiments, one of $R^2$ and $R^{2'}$ is hydrogen and the other is selected from the group consisting of amino, amino-$C_1$-$C_4$ alkyl-, 7, 8 or 9 membered spiro-heterocycloalkyl, 10 membered spiro-heterocycloalkyl whose ring atoms consist of a nitrogen atom and a carbon atom, 7, 8 or 9 membered fused-heterocycloalkyl, 7-8 membered bridged heterocycloalkyl, 5-6 membered monoheterocycloalkyl whose ring atoms consist of a nitrogen atom and a carbon atom, 5-6 membered monoheterocycloalkyl whose ring atoms contain a silicon atom or a phosphorus atom,

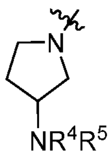,

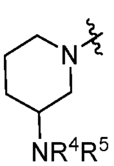, 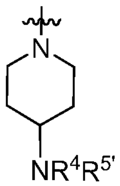, 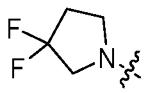 and 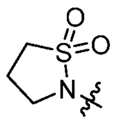,

wherein the amino or amino-$C_1$-$C_4$ alkyl- is substituted with one or more $R^{21}$, and optionally substituted with one or more $R^{21'}$, wherein the 5-6 membered monoheterocycloalkyl whose ring atoms consist of a nitrogen atom and a carbon atom is substituted with one or more $R^{22}$, and optionally substituted with one or more $R^{22'}$, wherein the 7, 8 or 9 membered spiro-heterocycloalkyl, 10 membered spiro-heterocycloalkyl whose ring atoms consist of a nitrogen atom and a carbon atom, 7, 8 or 9 membered fused-heterocycloalkyl, or 5-6 membered monoheterocycloalkyl whose ring atoms contain a silicon atom or a phosphorus atom is optionally substituted by one or more $R^{23}$, wherein the 7-8 membered bridged heterocycloalkyl is substituted by one or more $R^{24}$,
and $R^3$ is hydrogen.

In some embodiments, one of $R^2$ and $R^{2'}$ is hydrogen and the other is selected from the group consisting of amino, amino-$C_1$-$C_4$ alkyl-, 7, 8 or 9 membered spiro-heterocycloalkyl, 10 membered spiro-heterocycloalkyl whose ring atoms consist of a nitrogen atom and a carbon atom, 5 membered fused 4 membered heterocycloalkyl, 5 membered fused 5 membered heterocycloalkyl, 5 membered fused 6 membered heterocycloalkyl, 7-8 membered bridged heterocycloalkyl, 5-6 membered monoheterocycloalkyl whose ring atoms consist of a nitrogen atom and a carbon atom, 6 membered monoheterocycloalkyl whose ring atoms contain a silicon atom or a phosphorus atom,

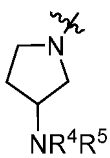,

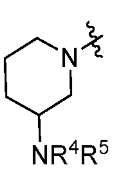, 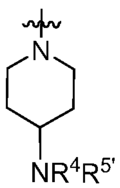, 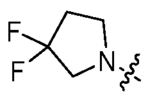 and 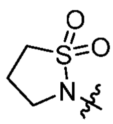,

wherein the amino or amino-$C_1$-$C_4$ alkyl- is substituted with one or more $R^{21}$, and optionally substituted with one or more $R^{21'}$, wherein the 5 membered or 6 membered monoheterocycloalkyl whose ring atoms consist of a nitrogen atom and

a carbon atom is substituted with one or more $R^{22}$, and optionally substituted with one or more $R^{22'}$, wherein the 7, 8 or 9 membered spiro-heterocycloalkyl, 10 membered spiro-heterocycloalkyl whose ring atoms consist of a nitrogen atom and a carbon atom, 5 membered fused 4 membered heterocycloalkyl, 5 membered fused 5 membered heterocycloalkyl, 5 membered fused 6 membered heterocycloalkyl, or 6 membered monoheterocycloalkyl whose ring atoms contain a silicon atom or a phosphorus atom is optionally substituted with one or more $R^{23}$, wherein the 7-8 membered bridged heterocycloalkyl is substituted with one or more $R^{24}$,
and $R^3$ is hydrogen.

In some embodiments, one of $R^2$ and $R^{2'}$ is hydrogen and the other is selected from the group consisting of amino, aminomethyl,

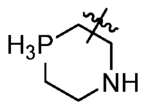, 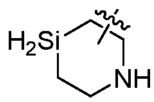, 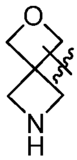, 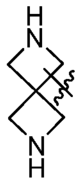, 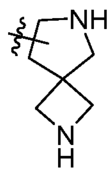, 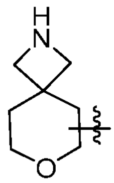,

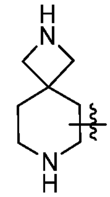, 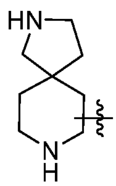, 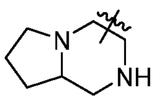, 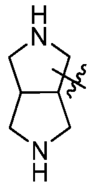, 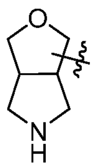, 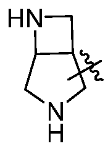, 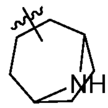, 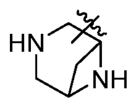, 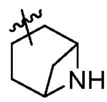,

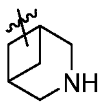,

pyrrolidinyl, piperidinyl, piperazinyl,

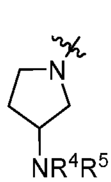, 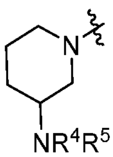, 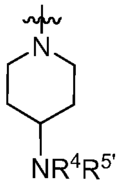, 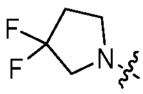

and

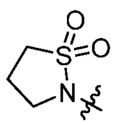,

wherein the amino or aminomethyl is substituted with one or more $R^{21}$, and optionally substituted with one or more $R^{21'}$, wherein the pyrrolidinyl, piperidinyl or piperazinyl is substituted with one or more $R^{22}$, and optionally substituted with one or more $R^{22'}$, wherein the

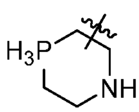,

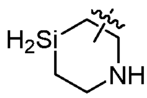, 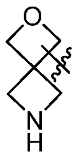, 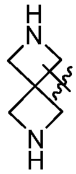, 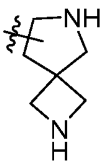, 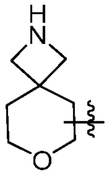, 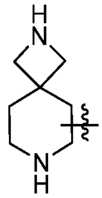, 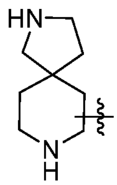, 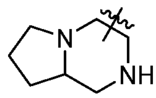, 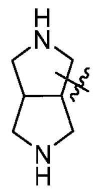,

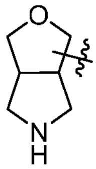 or 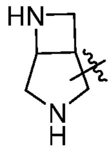

is substituted with one or more $R^{23}$, wherein the

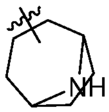, 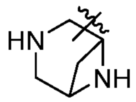,

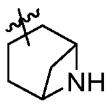 or 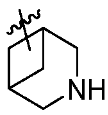

is substituted with one or more $R^{24}$,

and $R^3$ is hydrogen;
or, $R^3$ is connected to $R^2$ or $R^{2'}$, and together with the carbon atoms connected thereto, forms a

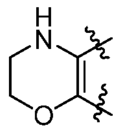

group optionally substituted with one or more $R^{25}$.

In some embodiments, one of $R^2$ and $R^{2'}$ is hydrogen and the other is selected from the group consisting of amino, aminomethyl,

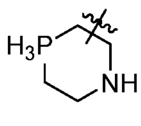, 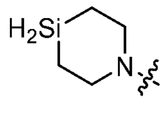, 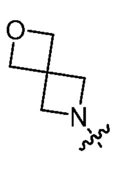, 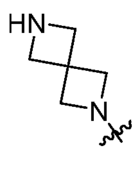, 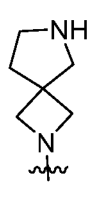,

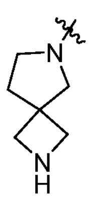, 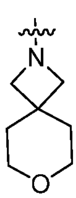, 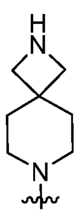, 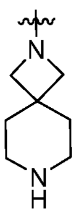, 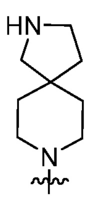, 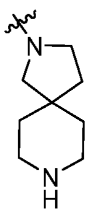, 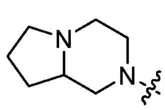, 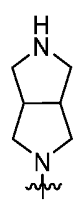, 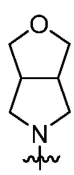, 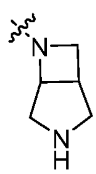, 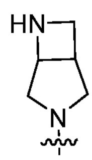,

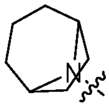, 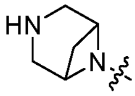, 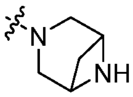, 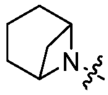, 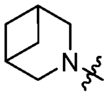, 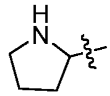, 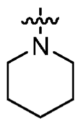, 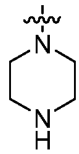, 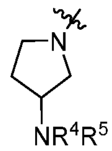,

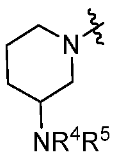, 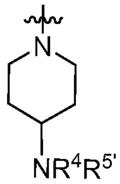, 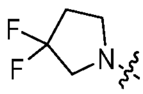 and 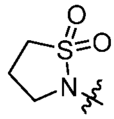,

wherein the amino or aminomethyl is substituted with one or more $R^{21}$, and optionally substituted with one or more $R^{21'}$, wherein the

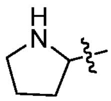, 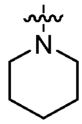

or

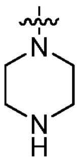

is substituted with one or more $R^{22}$, and optionally substituted with one or more $R^{22'}$, wherein the

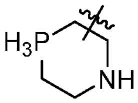, 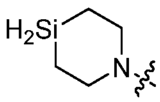, 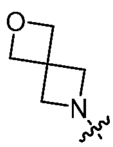, 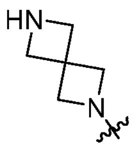, 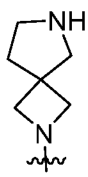, 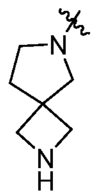, 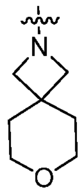, 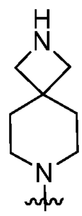,

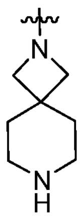, 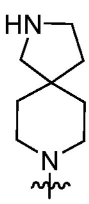, 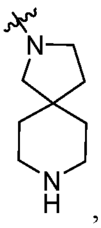, 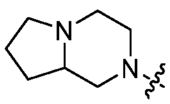, 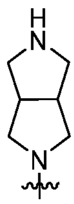, 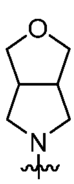, 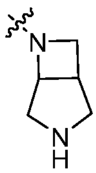 or 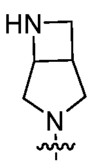

is substituted with one or more $R^{23}$, wherein the

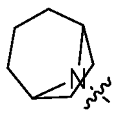, 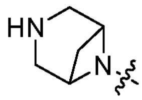, 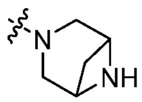, 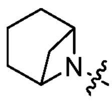 or 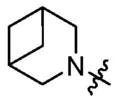

is substituted with one or more $R^{24}$,

and $R^3$ is hydrogen;
or, $R^3$ is connected to $R^2$ or $R^{2'}$, and together with the carbon atoms connected thereto, forms a

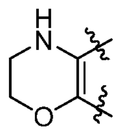

group optionally substituted with one or more $R^{25}$.

In some embodiments, $R^3$ is connected to $R^2$ or $R^{2'}$, such that the structural unit

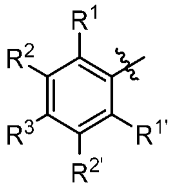

is

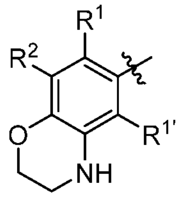 or 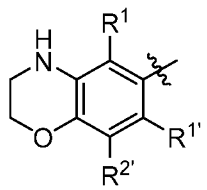

optionally substituted with one or more $R^{25}$.

In some embodiments, $R^{21}$ is selected from the group consisting of $C_2$-$C_4$ alkenyl-C(O)-, $C_5$-$C_{10}$ cycloalkyl, 4-6 membered heterocyclyl or $C_1$-$C_4$ alkylacylamino-$C_1$-$C_4$ alkyl-, wherein the $C_5$-$C_{10}$ cycloalkyl, 4-6 membered heterocyclyl or $C_1$-$C_4$ alkylacylamino-$C_1$-$C_4$ alkyl- is optionally substituted with one or more of the following groups selected from the group consisting of: hydroxy, $C_1$-$C_6$ alkyl and $C_1$-$C_4$ alkylacyl.

In some embodiments, $R^{21}$ is selected from the group consisting of $C_2$-$C_3$ alkenyl-C(O)-, $C_7$-$C_{10}$ bridged cycloalkyl, 4-6 membered monoheterocycloalkyl and $C_1$-$C_3$ alkylacylamino-$C_1$-$C_3$ alkyl-, wherein the $C_7$-$C_{10}$ bridged cycloalkyl, 4-6 membered monoheterocycloalkyl or $C_1$-$C_3$ alkylacylamino-$C_1$-$C_3$ alkyl- is optionally substituted with one or more of the following groups selected from the group consisting of: hydroxy, $C_1$-$C_4$ alkyl and $C_1$-$C_4$ alkylacyl.

In some embodiments, $R^{21}$ is selected from the group consisting of $C_2$-$C_3$ alkenyl-C(O)-, $C_{10}$ bridged cycloalkyl, 5 membered or 6 membered monoheterocycloalkyl, or $C_1$-$C_3$ alkylacylamino-$C_1$-$C_3$ alkyl-, wherein the $C_{10}$ bridged cycloalkyl, 5 membered or 6 membered monoheterocycloalkyl, or $C_1$-$C_3$ alkylacylamino-$C_1$-$C_3$ alkyl- is optionally substituted with one or more of the following groups selected from the group consisting of: hydroxy, $C_1$-$C_4$ alkyl and $C_1$-$C_4$ alkylacyl.

In some embodiments, $R^{21}$ is selected from the group consisting of $H_2C$=CHC(O)-,

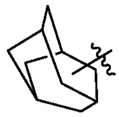,

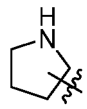, 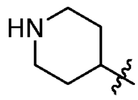

and $CH_3C(O)N(CH_3)-CH_3CH_2-$, wherein the

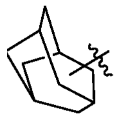, 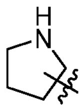 or 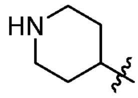

is optionally substituted with one or more of the following groups selected from the group consisting of: hydroxy and $C_1-C_4$ alkylacyl.

In some embodiments, $R^{21}$ is selected from the group consisting of $H_2C=CHC(O)-$,

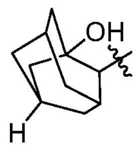,

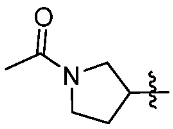, 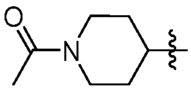

and $CH_3C(O)N(CH_3)-CH_3CH_2-$.

In some embodiments, $R^{21}$ is selected from the group consisting of $H_2C=CHC(O)-$,

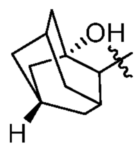,

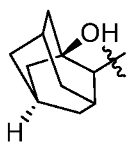, 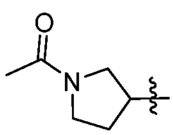, 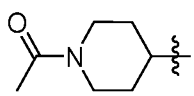

and $CH_3C(O)N(CH_3)-CH_3CH_2-$.

In some embodiments, one of $R^2$ and $R^{2'}$ is hydrogen and the other is amino, wherein the amino is substituted with one $R^{21}$, $R^{21}$ being selected from the group consisting of $C_2-C_3$ alkenyl-C(O), $C_{10}$ bridged cycloalkyl, 5 membered or 6 membered monoheterocycloalkyl, and $C_1-C_3$ alkylacylamino-$C_1-C_3$ alkyl-, wherein the $C_{10}$ bridged cycloalkyl, 5 membered or 6 membered monoheterocycloalkyl, or $C_1-C_3$ alkylacylamino-$C_1-C_3$ alkyl- is optionally substituted with one or more of the following groups selected from the group consisting of: hydroxy, $C_1-C_4$ alkyl and $C_1-C_4$ alkylacyl; and/or $R^3$ is hydrogen.

In some embodiments, one of $R^2$ and $R^{2'}$ is hydrogen and the other is amino, wherein the amino is substituted

with one $R^{21}$, $R^{21}$ being selected from the group consisting of $H_2C{=}CHC(O)$-,

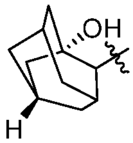,

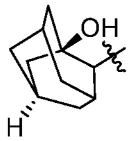, 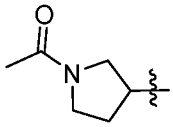, 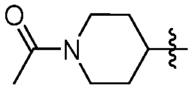

and $CH_3C(O)N(CH_3)$-$CH_3CH_2$-; and/or $R^3$ is hydrogen.

In some embodiments, one of $R^2$ and $R^{2'}$ is hydrogen and the other is amino-$C_1$-$C_6$ alkyl-, wherein the amino-$C_1$-$C_6$ alkyl- is substituted with $C_2$-$C_3$ alkenyl-C(O)-; and/or $R^3$ is hydrogen.

In some embodiments, $R^{22}$ is selected from the group consisting of $C_2$-$C_6$ alkynyl-C(O)- and 4-5 membered heterocyclyl, wherein the 4-5 membered heterocyclyl is optionally substituted with one or more 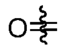.

In some embodiments, $R^{22}$ is selected from the group consisting of $C_2$-$C_3$ alkynyl-C(O)-, 4 membered or 5 membered heterocyclyl and 6 membered heterocyclyl whose ring atoms consist of a nitrogen atom and a sulfur atom, wherein the 4 membered or 5 membered heterocyclyl, or 6 membered heterocyclyl whose ring atoms consist of a nitrogen atom and a sulfur atom is optionally substituted with one or more of the following groups selected from the group consisting of: 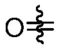, halogen, $C_1$-$C_3$ alkyl and $C_1$-$C_6$ alkylacyl.

In some embodiments, $R^{22}$ is selected from the group consisting of $C_2$-$C_3$ alkynyl-C(O)- and 4 membered or 5 membered heterocyclyl, wherein the 4 membered or 5 membered heterocyclyl is optionally substituted with one or more 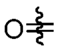.

In some embodiments, $R^{22}$ is selected from the group consisting of $C_2$-$C_3$ alkynyl-C(O)-, 4 membered or 5 membered heterocycloalkyl and

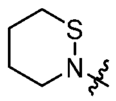,

wherein the 4 membered or 5 membered heterocycloalkyl or

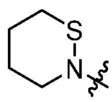

is optionally substituted with one or more of the following groups selected from the group consisting of: 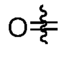, halogen, $C_1$-$C_3$ alkyl and $C_1$-$C_6$ alkylacyl.

In some embodiments, $R^{22}$ is selected from the group consisting of $C_2$-$C_3$ alkynyl-C(O)- and 4 membered or 5 membered heterocycloalkyl, wherein the 4 membered or 5 membered heterocycloalkyl is optionally substituted with one or more 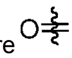.

In some embodiments, $R^{22}$ is selected from the group consisting of $H_3CC{=}CC(O)$-,

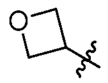,

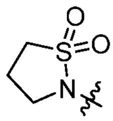, 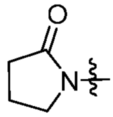, 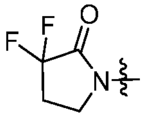, 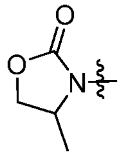, 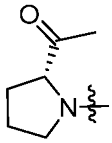, 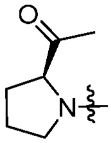, and 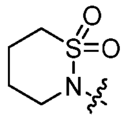.

In some embodiments, $R^{22}$ is selected from the group consisting of $H_3CC{=}CC(O)$-,

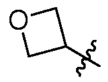,

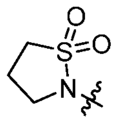 and 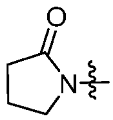.

In some embodiments, $R^{21'}$ and $R^{22'}$ are each independently selected from the group consisting of deuterium and $C_1$-$C_4$ alkyl.

In some embodiments, $R^{21'}$ and $R^{22'}$ are each independently selected from the group consisting of deuterium and methyl.

In some embodiments, $R^{21'}$ and $R^{22'}$ are each independently selected from $C_1$-$C_4$ alkyl.

In some embodiments, $R^{21'}$ and $R^{22'}$ are each independently selected from methyl.

In some embodiments, $R^{23}$ is selected from the group consisting of 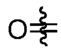, $C_1$-$C_4$ alkyl, $C_1$-$C_4$ alkylsulfonyl and $C_1$-$C_4$ alkylacyl.

In some embodiments, $R^{23}$ is selected from the group consisting of 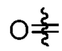, $CH_3$-, $CH_3S(O)_2$- and $CH_3C(O)$-.

In some embodiments, $R^{24}$ is selected from $C_1$-$C_4$ alkylacyl-N ($C_1$-$C_4$ alkyl)-.

In some embodiments, $R^{24}$ is selected from $CH_3C(O)N(CH_3)$-.

In some embodiments, $R^{25}$ is selected from $C_1$-$C_4$ alkylacyl.

In some embodiments, $R^{25}$ is selected from $CH_3C(O)$-.

In some embodiments, $R^4$ is selected from the group consisting of $C_1$-$C_4$ alkylacyl and $C_1$-$C_4$ alkylsulfonyl.

In some embodiments, $R^4$ is selected from the group consisting of $CH_3C(O)$- and $CH_3S(O)_2$-.

In some embodiments, $R^5$ is selected from $C_1$-$C_4$ alkyl.

In some embodiments, $R^5$ is selected from methyl.

In some embodiments, $R^4$ is selected from $CH_3C(O)$-; $R^5$ is selected from methyl.

In some embodiments, $R^4$ is selected from $CH_3S(O)_2$-; $R^5$ is selected from methyl.

In some embodiments, $R^{5'}$ is selected from $-CD_3$ and $-CH_3$, and when $R^{5'}$ is $-CH_3$, $R^1$ or $R^{1'}$ is fluorine.

In some embodiments, one of $R^2$ and $R^{2'}$ is hydrogen and the other is selected from 7-9 membered spiro-heterocyclyl, wherein the 7-9 membered spiro-heterocyclyl is optionally substituted with 1 or 2 $R^{23}$, $R^{23}$ being selected from the group consisting of 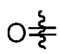,, $C_1$-$C_4$ alkyl, $C_1$-$C_4$ alkylsulfonyl and $C_1$-$C_4$ alkylacyl; and/or $R^3$ is hydrogen.

In some embodiments, one of $R^2$ and $R^{2'}$ is hydrogen and the other is selected from 7-9 membered spiro-heterocyclyl, wherein the 7-9 membered spiro-heterocyclyl is optionally substituted with 1 or 2 $R^{23}$, $R^{23}$ being selected from the group consisting of 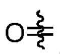, $CH_3$-, $CH_3S(O)_2$- and $CH_3C(O)$-; and/or $R^3$ is hydrogen.

In some embodiments, the 7-9 membered spiro-heterocyclyl is a 7, 8, or 9 membered monospiro heterocyclic ring containing 1 or 2 heteroatoms selected from the group consisting of N, O and S. In some embodiments, the 7-9 membered spiro-heterocyclyl is a 7, 8, or 9 membered monospiro heterocyclic ring containing 1 or 2 heteroatoms selected from the group consisting of N and O.

In some embodiments, one of $R^2$ and $R^{2'}$ is hydrogen and the other is 10 membered spiro-heterocyclyl whose ring atoms consist of a nitrogen atom and a carbon atom, wherein the 10 membered spiro-heterocyclyl is optionally substituted with 1 or 2 $R^{23}$, $R^{23}$ being selected from the group consisting of 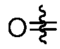 $C_1$-$C_4$ alkyl, $C_1$-$C_4$ alkylsulfonyl and $C_1$-$C_4$ alkylacyl; and/or $R^3$ is hydrogen.

In some embodiments, one of $R^2$ and $R^{2'}$ is hydrogen and the other is 10 membered spiro-heterocyclyl whose ring atoms consist of a nitrogen atom and a carbon atom, wherein the 10 membered spiro-heterocyclyl is optionally substituted with 1 or 2 $R^{23}$, $R^{23}$ being selected from the group consisting of 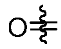 and $C_1$-$C_4$ alkyl; and/or $R^3$ is hydrogen.

In some embodiments, the 10 membered spiro-heterocyclyl contains 1 or 2 heteroatoms selected from N.

In some embodiments, one of $R^2$ and $R^{2'}$ is hydrogen and the other is selected from 7-10 membered fused-heterocyclyl, wherein the 7-10 membered fused-heterocyclyl is optionally substituted with 1 or 2 $R^{23}$, $R^{23}$ being selected from the group consisting of 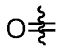 $C_1$-$C_4$ alkyl, $C_1$-$C_4$ alkylsulfonyl and $C_1$-$C_4$ alkylacyl; and/or $R^3$ is hydrogen.

In some embodiments, one of $R^2$ and $R^{2'}$ is hydrogen and the other is selected from 7-9 membered fused-heterocyclyl, wherein the 7-9 membered fused-heterocyclyl is optionally substituted with 1 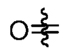 and $C_1$-$C_4$ alkylacyl; and/or $R^3$ is hydrogen.

In some embodiments, the 7-9 membered fused-heterocyclyl contains 1 or 2 heteroatoms selected from the group consisting of N, O and S. In some embodiments, the 7-9 membered fused-heterocyclyl contains 1 or 2 heteroatoms selected from the group consisting of N and O.

In some embodiments, one of $R^2$ and $R^{2'}$ is hydrogen and the other is selected from 5-6 membered heterocyclyl whose ring atoms consist of a nitrogen atom and a carbon atom, wherein the 5-6 membered heterocyclyl is substituted with one $R^{22}$ and optionally substituted with one $R^{22'}$; $R^{22}$ is selected from the group consisting of $C_2$-$C_3$ alkynyl-C(O)-, 4 membered or 5 membered heterocyclyl or 6 membered heterocyclyl whose ring atoms consist of a nitrogen atom and a sulfur atom, wherein the 4 membered or 5 membered heterocyclyl or 6 membered heterocyclyl whose ring atoms consist of a nitrogen atom and a sulfur atom is optionally substituted with 1, 2 or 3 groups selected from the group consisting of 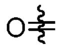, halogen, $C_1$-$C_3$ alkyl and $C_1$-$C_3$ alkylacyl; $R^{22'}$ is selected from the group consisting of deuterium and $C_1$-$C_3$ alkyl; and/or $R^3$ is hydrogen.

In some embodiments, the 5-6 membered heterocyclyl whose ring atoms consist of a nitrogen atom and a carbon atom contains 1 or 2 nitrogen atoms.

In some embodiments, one of $R^2$ and $R^{2'}$ is hydrogen and the other is selected from 7-10 membered bridged heterocyclyl, wherein the 7-10 membered bridged heterocyclyl is substituted with 1 or 2 $R^{24}$, $R^{24}$ being selected from $C_1$-$C_4$ alkylacyl-N ($C_1$-$C_4$ alkyl)-; and/or $R^3$ is hydrogen.

In some embodiments, one of $R^2$ and $R^{2'}$ is hydrogen and the other is selected from 7-10 membered bridged heterocyclyl, wherein the 7-10 membered bridged heterocyclyl is substituted with 1 $CH_3C(O)N(CH_3)$-; and/or $R^3$ is hydrogen.

In some embodiments, the 7-10 membered bridged heterocyclyl contains 1 or 2 heteroatoms selected from the group consisting of N, O and S. In some embodiments, the 7-10 membered bridged heterocyclyl contains 1 or 2 heteroatoms selected from the group consisting of N and O.

In some embodiments, one of $R^2$ and $R^{2'}$ is hydrogen and the other is selected from 5-6 membered heterocyclyl whose ring atoms contain a silicon atom and a phosphorus atom, wherein the 5-6 membered heterocyclyl is optionally substituted with 1 or 2 $R^{23}$, $R^{23}$ being selected from the group consisting of 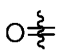 and $C_1$-$C_4$ alkyl; and/or $R^3$ is hydrogen.

In some embodiments, the 5-6 membered heterocyclyl whose ring atoms contain a silicon atom or a phosphorus atom contains 1 silicon atom or 1 phosphorus atom, and further contains 1 heteroatom selected from the group consisting

of N, O and S. In some embodiments, the 5-6 membered heterocyclyl whose ring atoms contain a silicon atom or a phosphorus atom contains 1 silicon atom or 1 phosphorus atom, and further contains 1 N atom.

In some embodiments, $R^2$, $R^{2'}$ and $R^3$ are each independently selected from the group consisting of hydrogen,

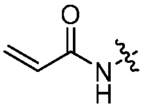, 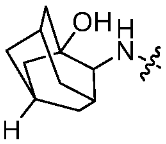, 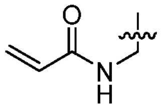, 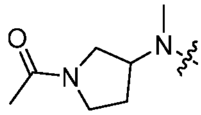, 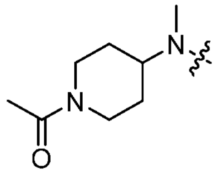,

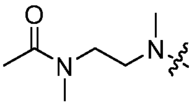, 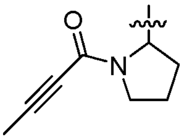, 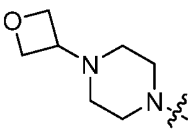, 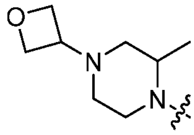, 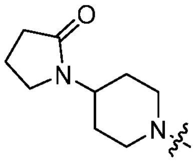,

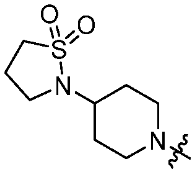, 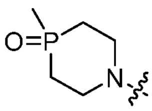, 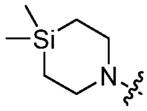, 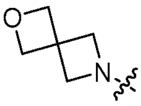, 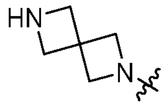, 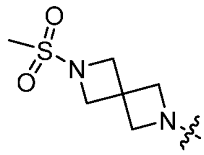,

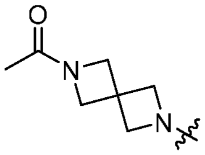, 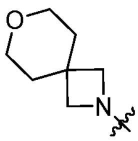, 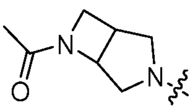, 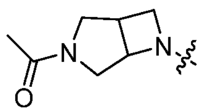, 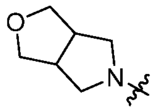, 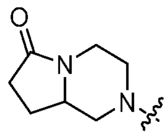,

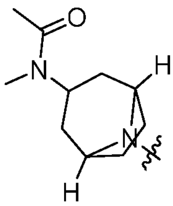, 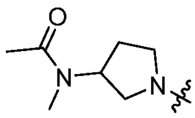, 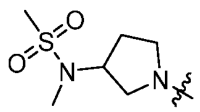, 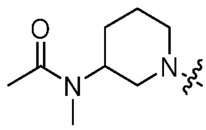, 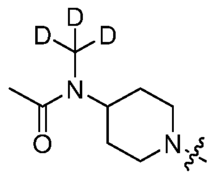,

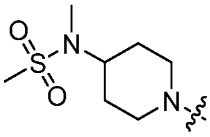, 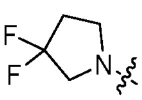 and 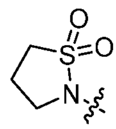,

or, $R^3$ is connected to $R^2$ or $R^{2'}$, and together with the carbon atoms connected thereto, forms a

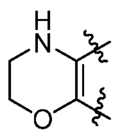 or 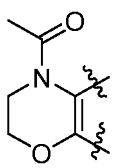

group, and $R^2$, $R^{2'}$ and $R^3$ are not hydrogen at the same time.

In some embodiments, one of $R^2$ and $R^{2'}$ is hydrogen and the other is selected from

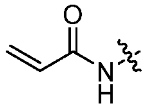,

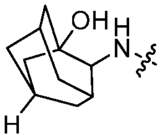, 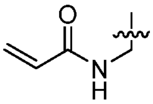, 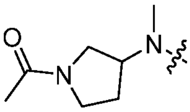, 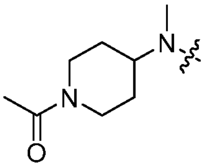, 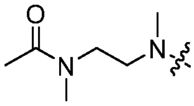, 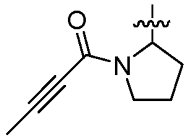,

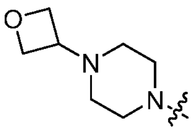, 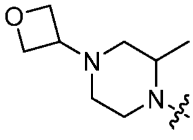, 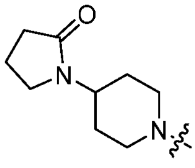, 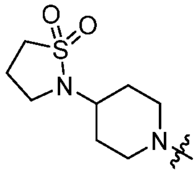, 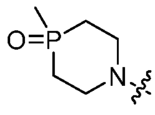, 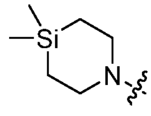,

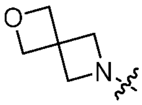, 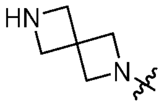, 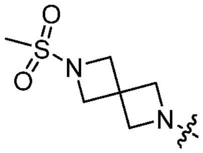, 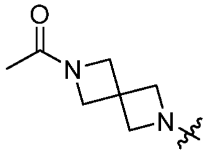, 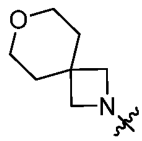, 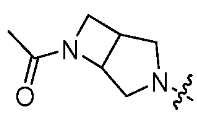,

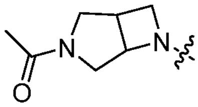, 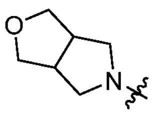, 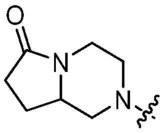, 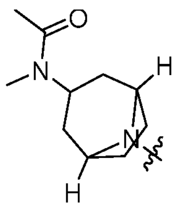, 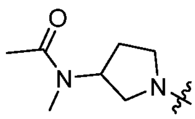, 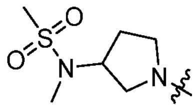,

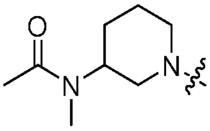, 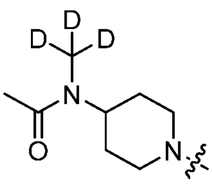, 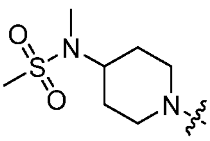, 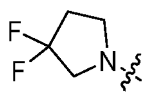 and 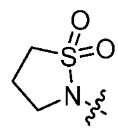;

and $R^3$ is hydrogen.

In some embodiments, $R^2$, $R^{2'}$ and $R^3$ are each independently selected from the group consisting of hydrogen,

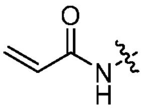, 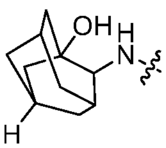, 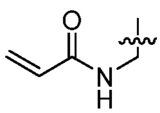, 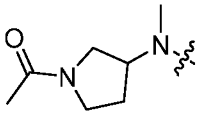, 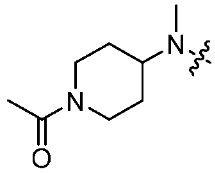,

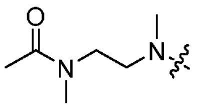, 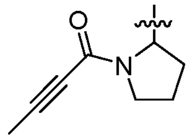, 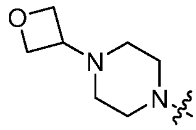, 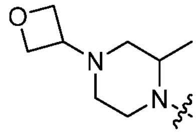, 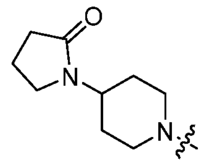,

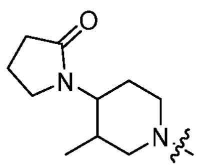, 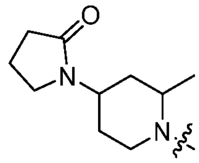, 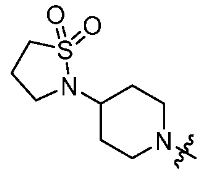, 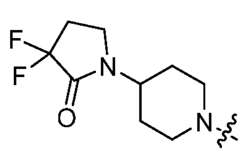, 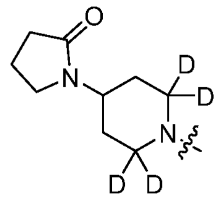,

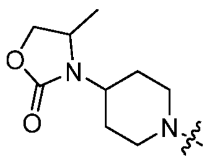, 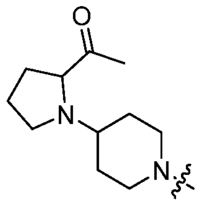, 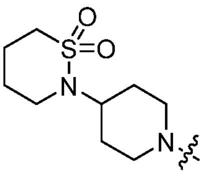, 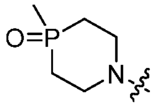, 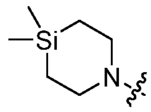, 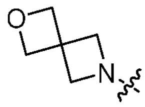,

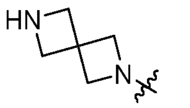, 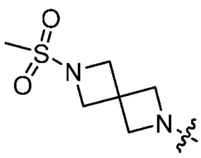, 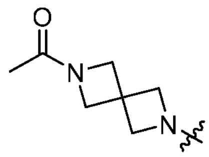, 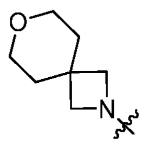, 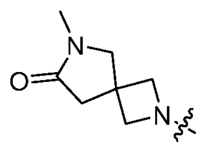,

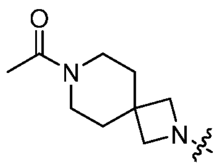, 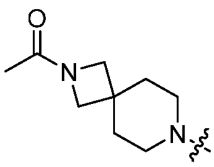, 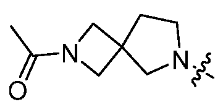, 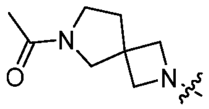, 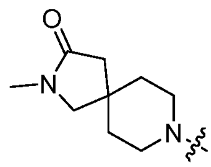,

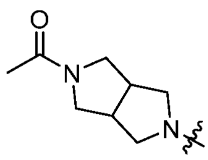, 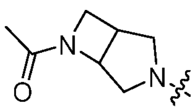, 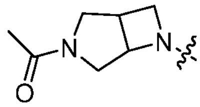, 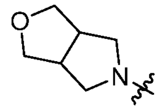, 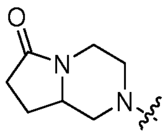, 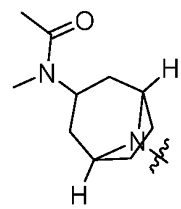,

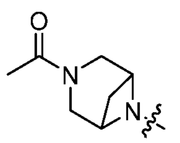, 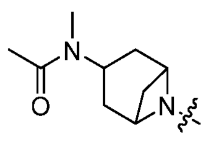, 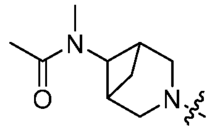, 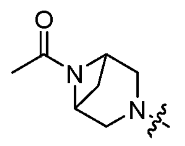, 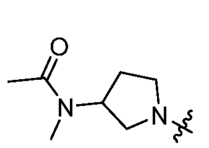,

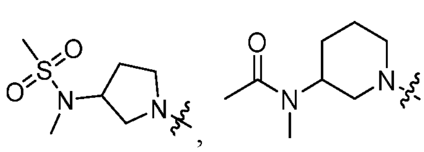, , 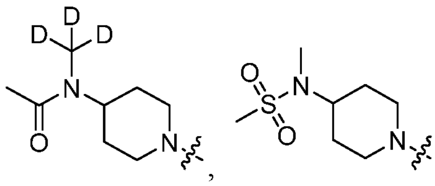, , 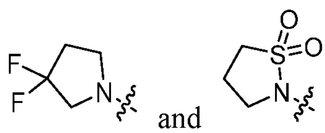 and ,

or, $R^3$ is connected to $R^2$ or $R^{2'}$, and together with the carbon atoms connected thereto, forms a

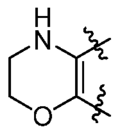 or 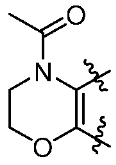

group, and $R^2$, $R^{2'}$ and $R^3$ are not hydrogen at the same time.

In some embodiments, one of $R^2$ and $R^{2'}$ is hydrogen and the other is selected from the group consisting of

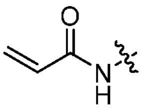, 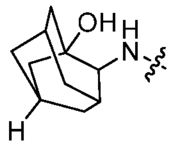, 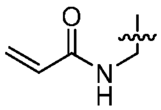, 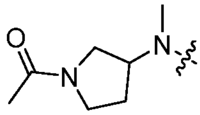, 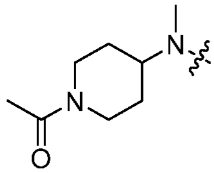,

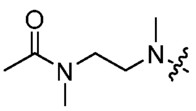, 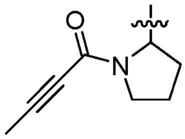, 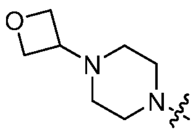, 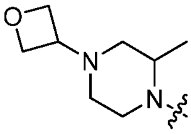, 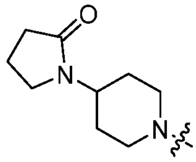,

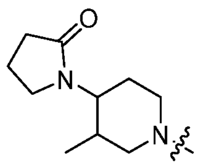, 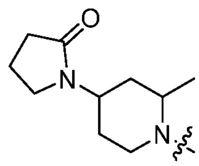, 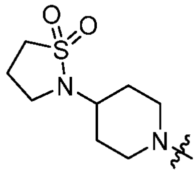, 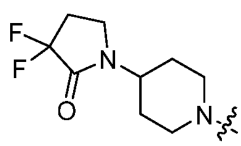, 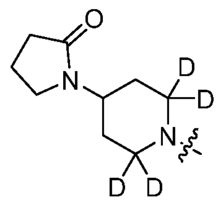,

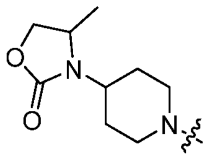, 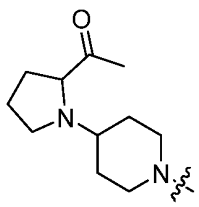, 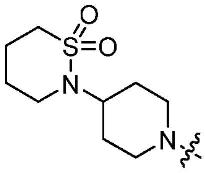, 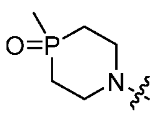, 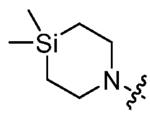, 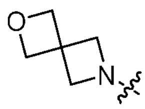,

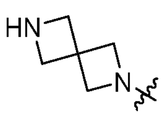, 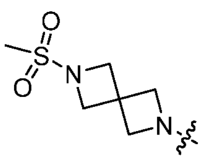, 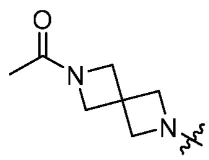, 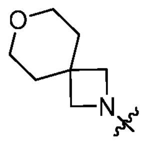, 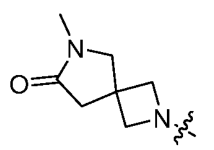,

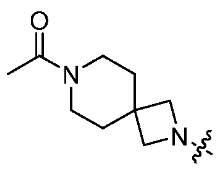, 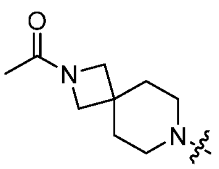, 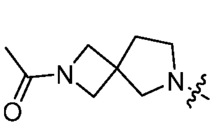, 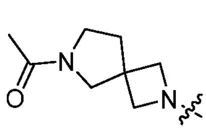, 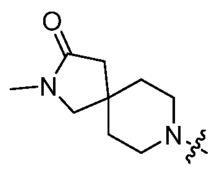,

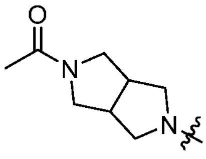, 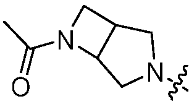, 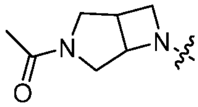, 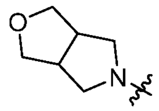, 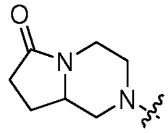, 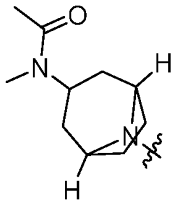,

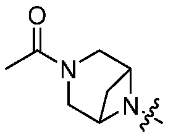, 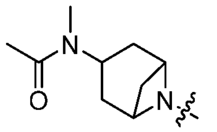, 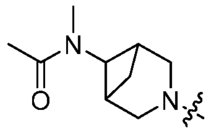, 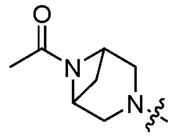, 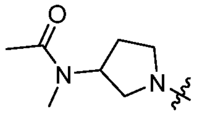,

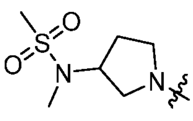, 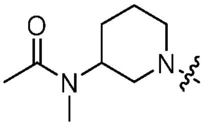, 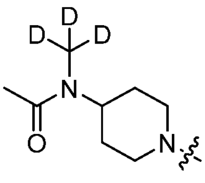, 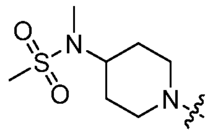, 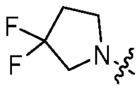 and 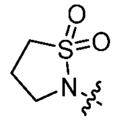;

and $R^3$ is hydrogen.

In some embodiments, $R^2$, $R^{2'}$ and $R^3$ are each independently selected from the group consisting of hydrogen,

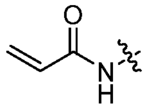, 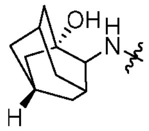, 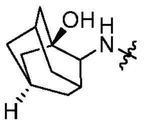, 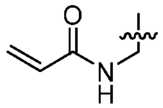, 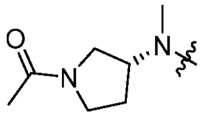, 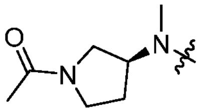,

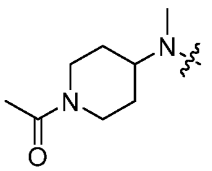, 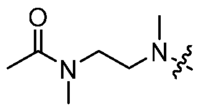, 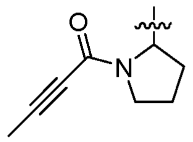, 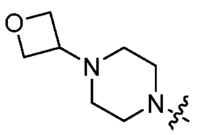, 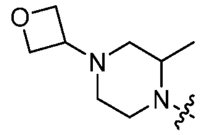,

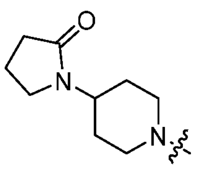, 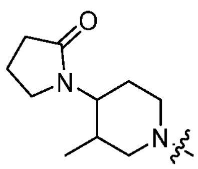, 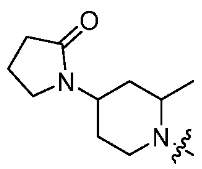, 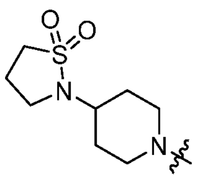, 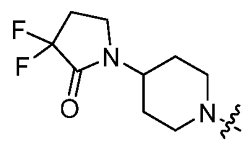,

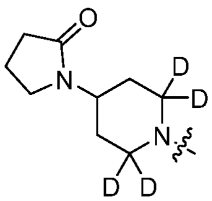, 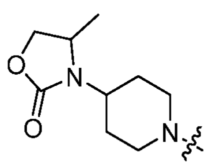, 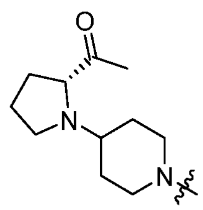, 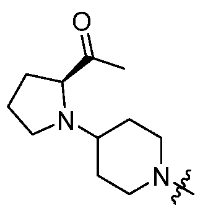, 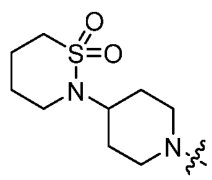,

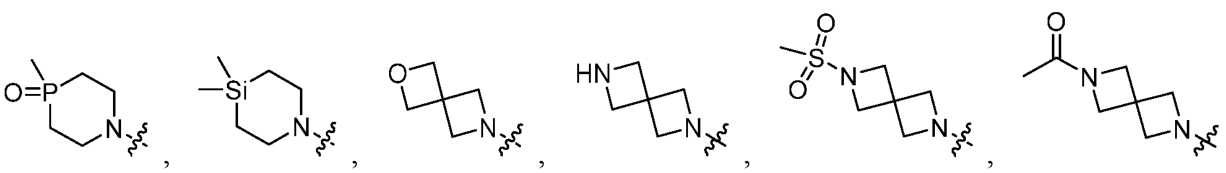, , , , , ,

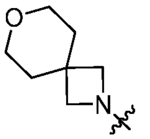, 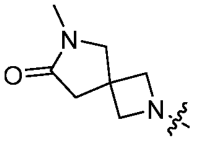, 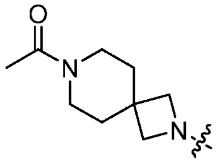, 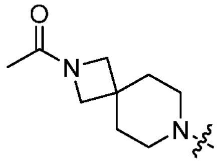, 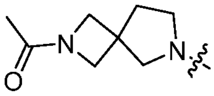,

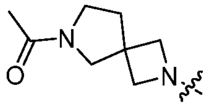, 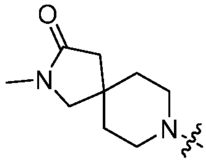, 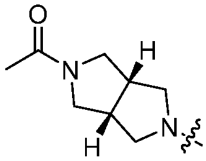, 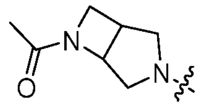, 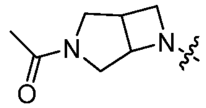,

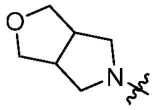, 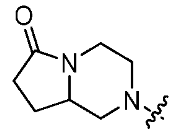, 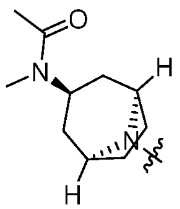, 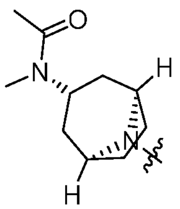, 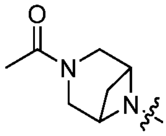, 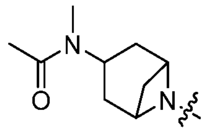,

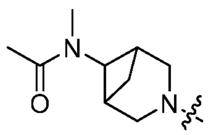, 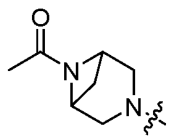, 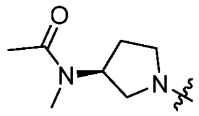, 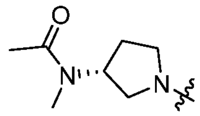, 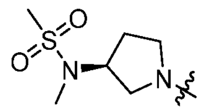,

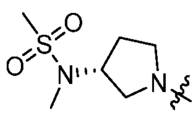, 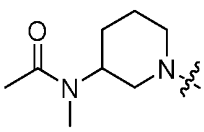, 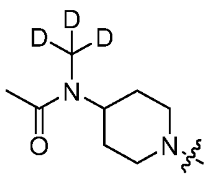, 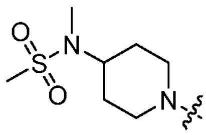, 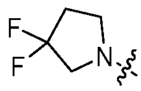 and 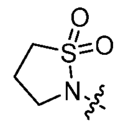,

or $R^3$ is connected to $R^2$ or $R^{2'}$, such that the structural unit

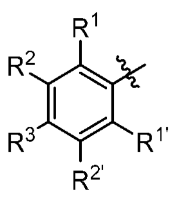 is 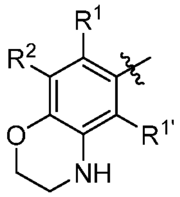

or

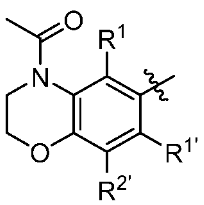,

and $R^2$, $R^{2'}$ and $R^3$ are not hydrogen at the same time.

In some embodiments, one of $R^2$ and $R^{2'}$ is hydrogen and the other is selected from the group consisting of

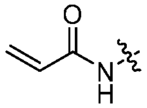, 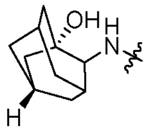, 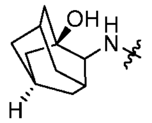, 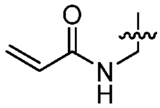, 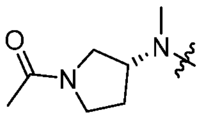,

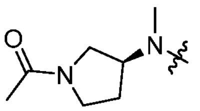, 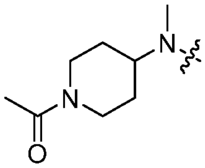, 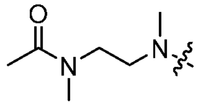, 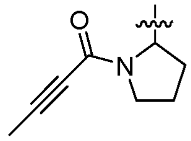, 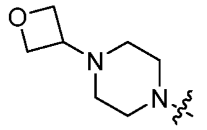,

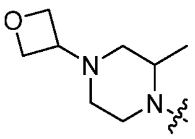, 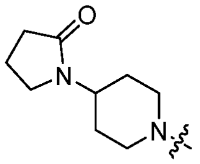, 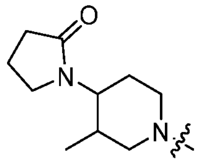, 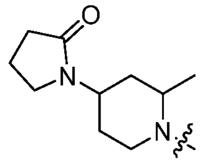, 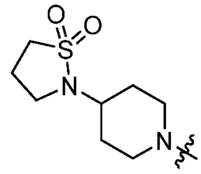,

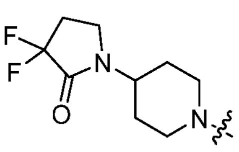, 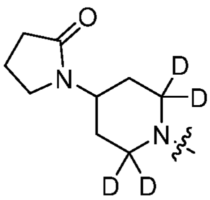, 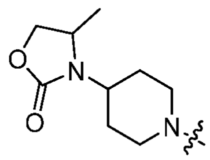, 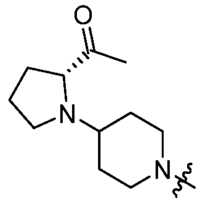, 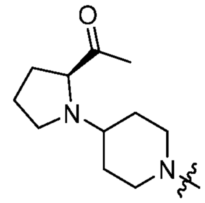,

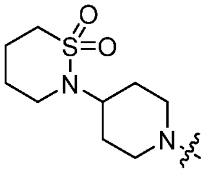, 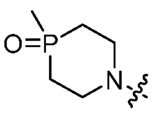, 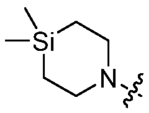, 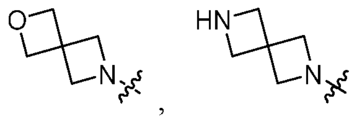, 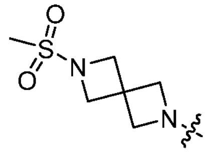,

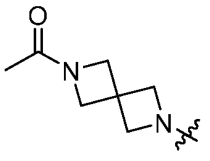, 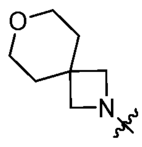, 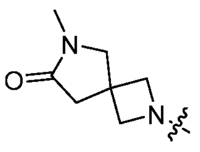, 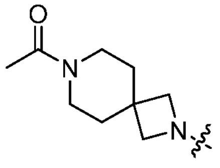, 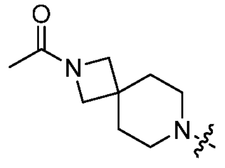,

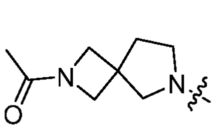, 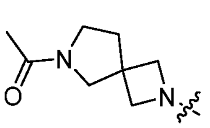, 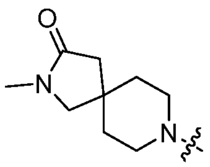, 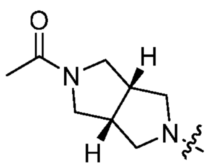, 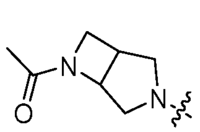,

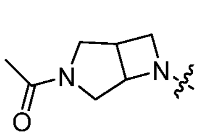, 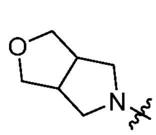, 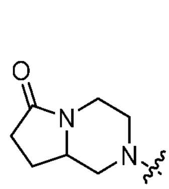, 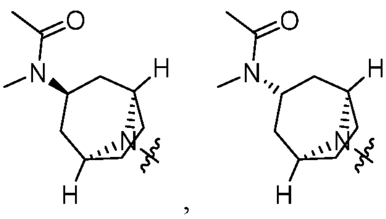, 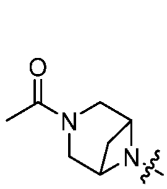,

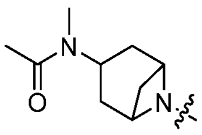 , 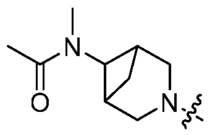 , 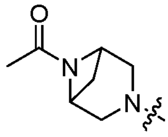 , 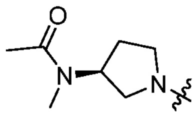 , 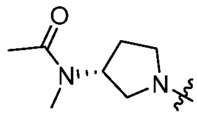 ,

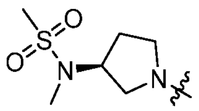 , 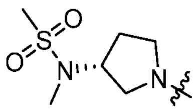 , 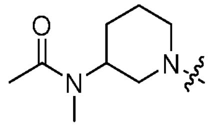 , 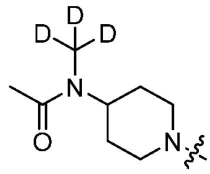 , 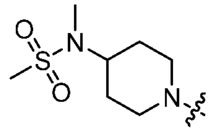 ,

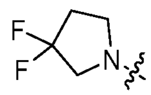 and 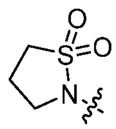 ;

and $R^3$ is hydrogen. In some embodiments, when $R^2$, $R^{2'}$ or $R^3$ are each independently selected from the group consisting of hydrogen and

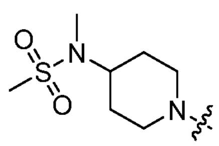 ,

$R^1$ or $R^{1'}$ is fluorine.

In some embodiments, 2 of $R^2$, $R^{2'}$ or $R^3$ are hydrogen.

In some embodiments, $R^2$ and $R^{2'}$ are hydrogen, and $R^3$ is not hydrogen.

In some embodiments, $R^2$ is not hydrogen, and $R^{2'}$ and $R^3$ are hydrogen.

In some embodiments, $R^{2'}$ is not hydrogen, and $R^2$ and $R^3$ are hydrogen.

In some embodiments, the present application encompasses the variables defined above and embodiments thereof, as well as any combination thereof.

In another aspect, the present application provides a compound of the following formulas or a pharmaceutically acceptable salt thereof,

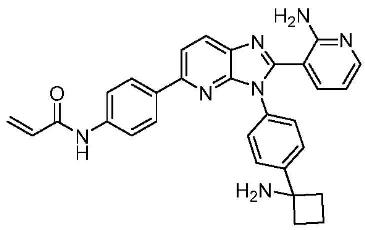 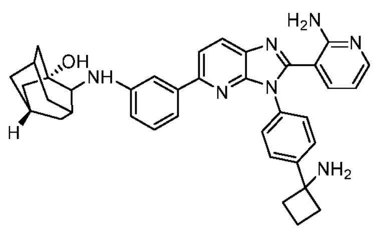 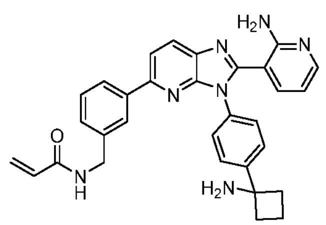

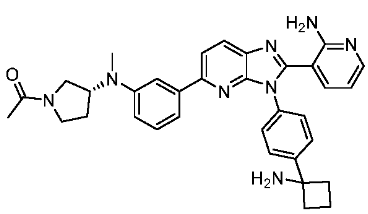 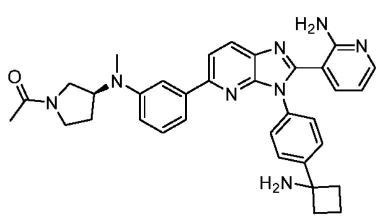 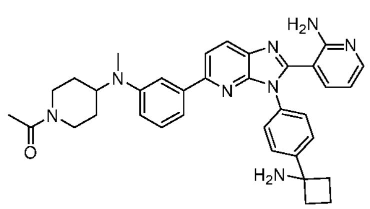

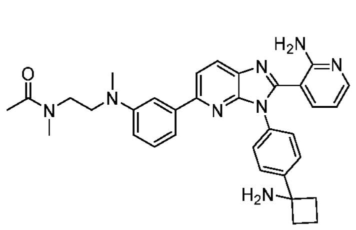
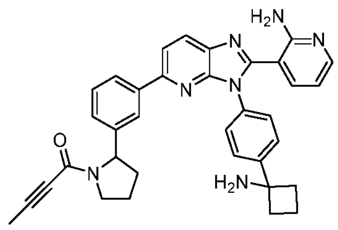
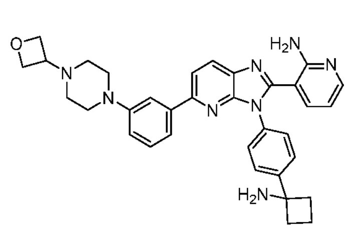
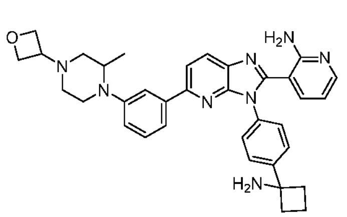
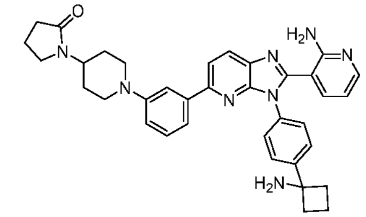
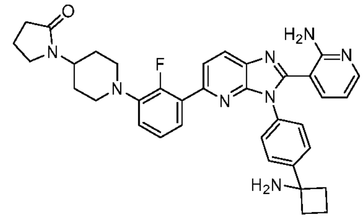
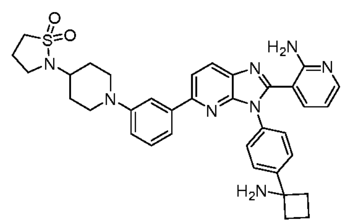
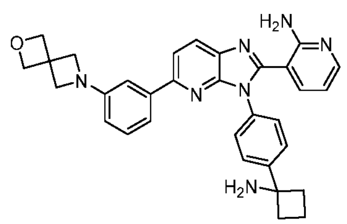
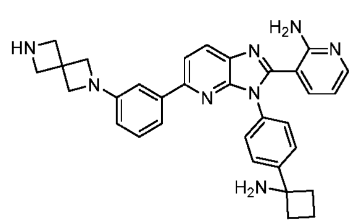
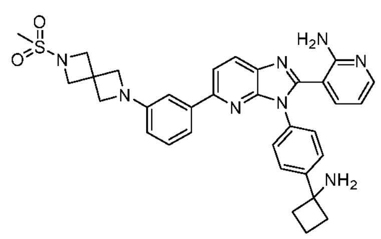
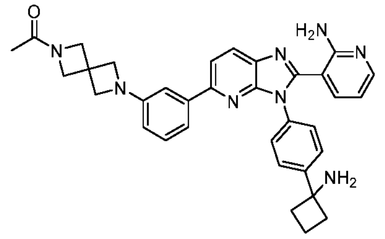
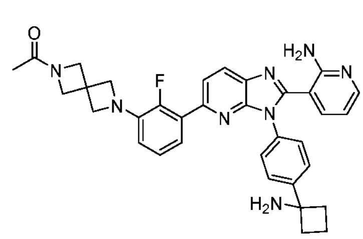
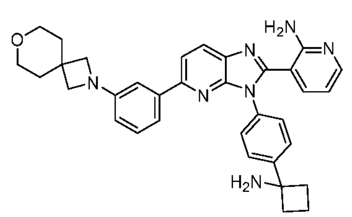
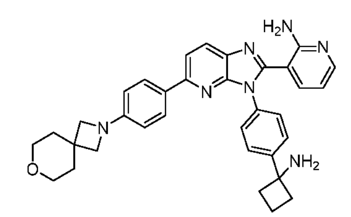
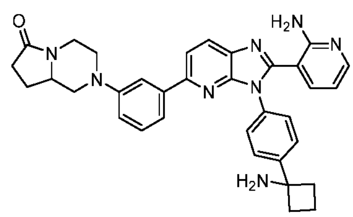
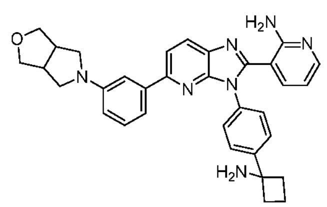
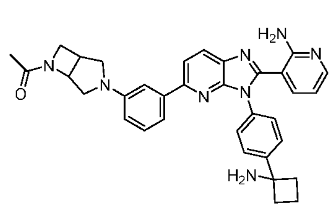
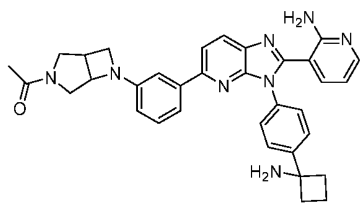

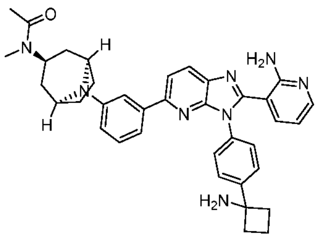
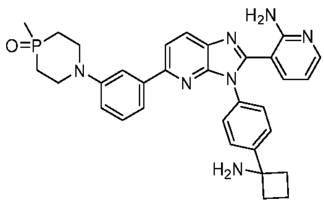
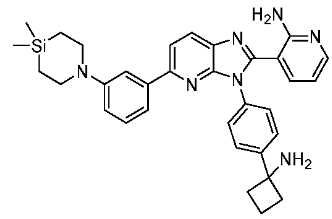

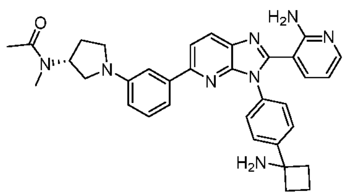
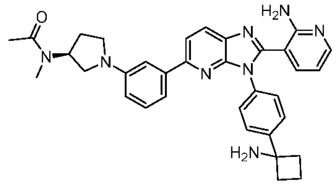
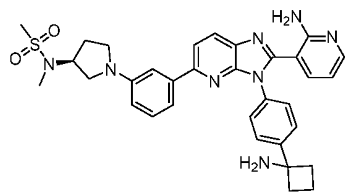

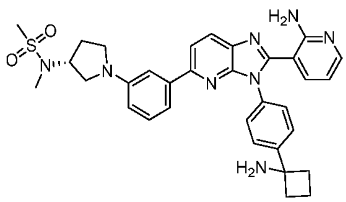
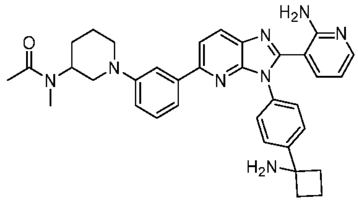
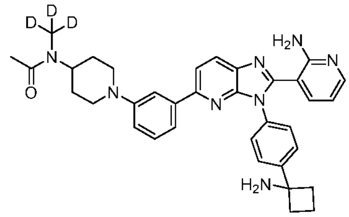

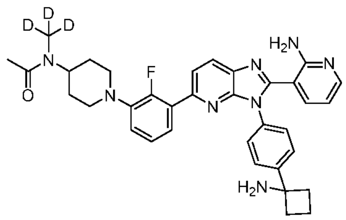
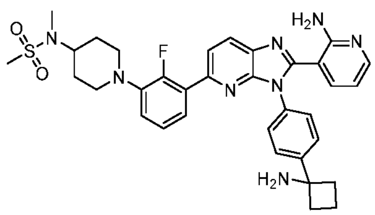
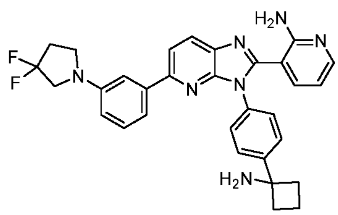

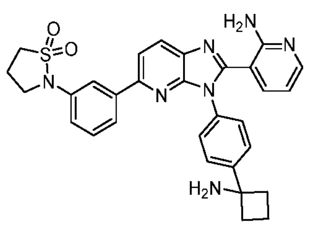
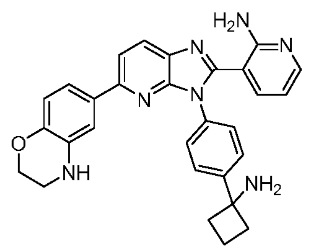
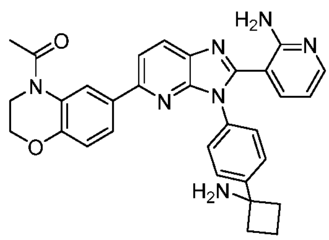

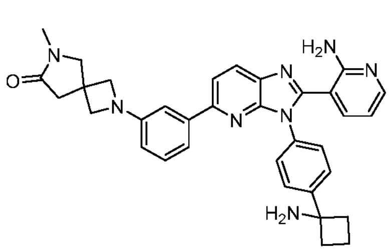
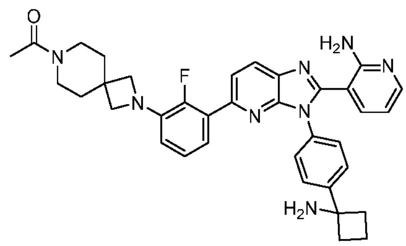

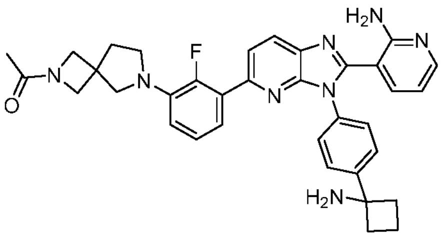
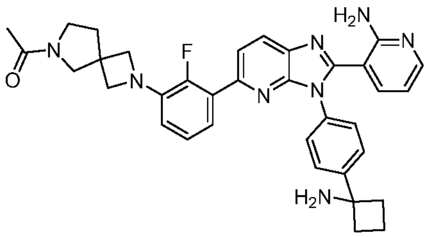

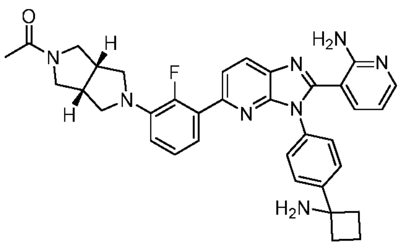
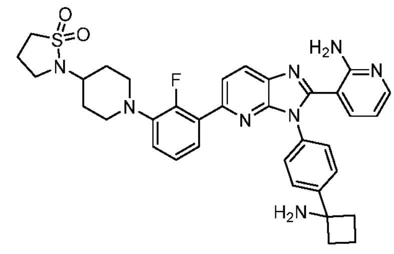
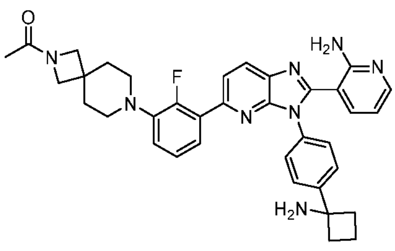

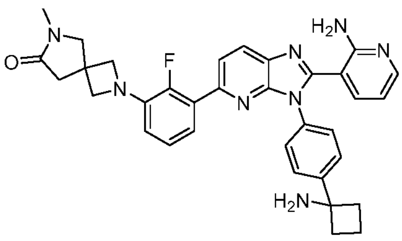
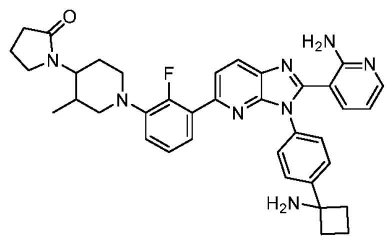
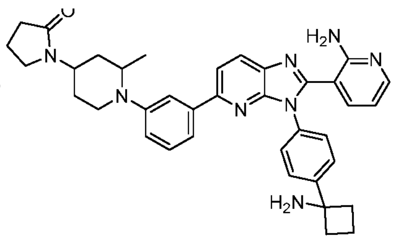

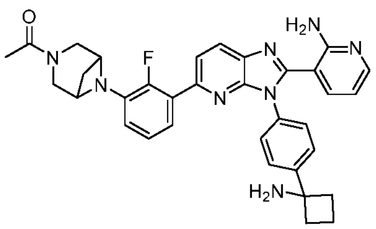
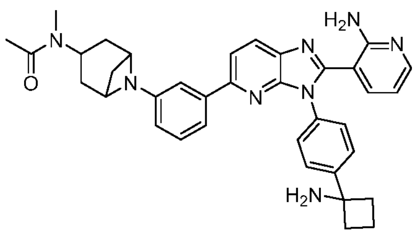
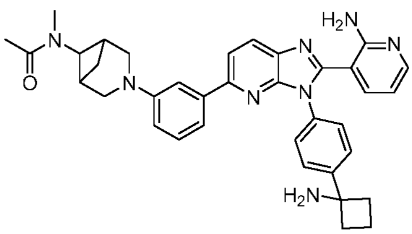

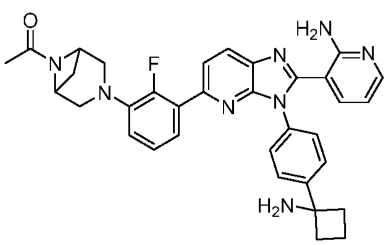
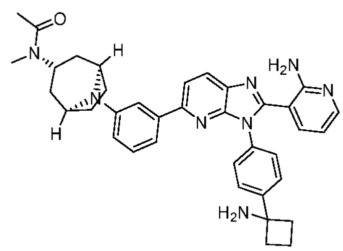
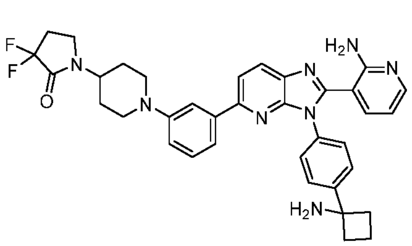

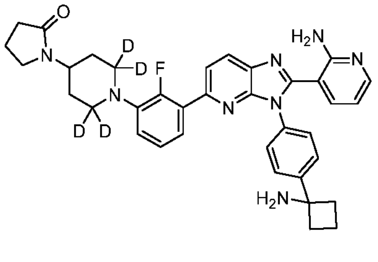
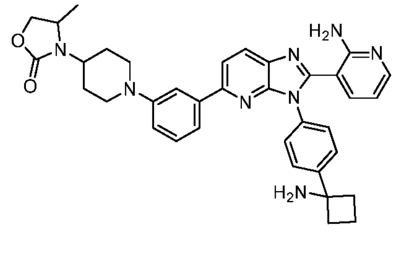
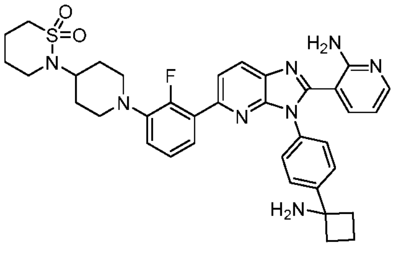

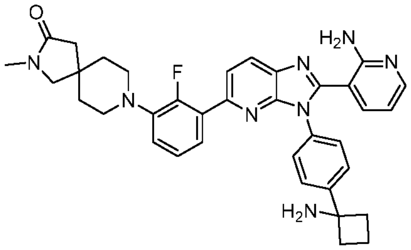 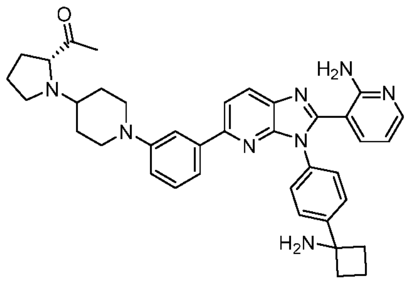 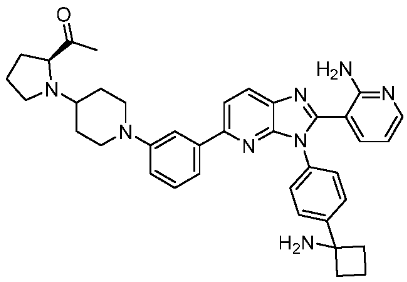 .

In another aspect, the present application further provides a pharmaceutical composition comprising the compound or the pharmaceutically acceptable salt thereof of the present application described above. In some embodiments, the pharmaceutical composition of the present application further comprises a pharmaceutically acceptable excipient.

In another aspect, the present application further provides a method for treating a disease mediated by Akt kinase in a mammal, comprising administering to a mammal, preferably a human, in need of such treatment a therapeutically effective amount of the compound, the pharmaceutically acceptable salt thereof or the pharmaceutical composition thereof described above.

In another aspect, the present application further provides use of the compound, the pharmaceutically acceptable salt thereof or the pharmaceutical composition thereof described above in preparing a medicament for treating a disease mediated by Akt kinase.

In another aspect, the present application further provides use of the compound, the pharmaceutically acceptable salt thereof or the pharmaceutical composition thereof described above in treating a disease mediated by Akt kinase.

In another aspect, the present application further provides the compound, the pharmaceutically acceptable salt thereof or the pharmaceutical composition thereof of the present application described above for use in treating a disease mediated by Akt kinase.

In some embodiments, the disease mediated by Akt kinase is selected from a cancer, e.g., prostate cancer and endometrial cancer.

The compound of the present application has good inhibition effect on phosphorylation of LNcap cells and LNcap cells AKT1(S473), and has good cell activity, low toxicity, good pharmacokinetic property, strong *in vivo* tumor inhibition effect and the like.

**Definitions**

Unless otherwise stated, the following terms used herein shall have the following meanings. A certain term, unless otherwise specifically defined, should not be considered uncertain or unclear, but construed according to its common meaning in the field. When referring to a trade name herein, it is intended to refer to its corresponding commercial product or its active ingredient.

The term "substituted" means that any one or more hydrogen atoms on a specific atom are substituted by substituents, as long as the valence of the specific atom is normal and the resulting compound is stable. When the substituent is oxo (namely =O), it means that two hydrogen atoms are substituted, and oxo is not available on an aromatic group. There is no keto substitution on the aromatic moiety. The term "optional" or "optionally" means that the subsequently described event or circumstance may, but not necessarily, occur. The description includes instances where the event or circumstance occurs and instances where the event or circumstance does not occur. For example, an ethyl "optionally" substituted by halogen, means that the ethyl may be unsubstituted ($CH_2CH_3$), monosubstituted (for example, $CH_2CH_2F$), polysubstituted (for example, $CHFCH_2F$, $CH_2CHF_2$ and the like) or fully substituted ($CF_2CF_3$). It will be understood by those skilled in the art that for any groups comprising one or more substituents, any substitutions or substituting patterns which may not exist or cannot be synthesized spatially are not introduced.

$C_{m-n}$ used herein means that the portion has an integer number of carbon atoms in the given range. For example, "$C_{1-6}$" means that the group may have 1 carbon atom, 2 carbon atoms, 3 carbon atoms, 4 carbon atoms, 5 carbon atoms, or 6 carbon atoms.

When any variable (e.g., R) occurs more than once in the constitution or structure of a compound, the variable is independently defined in each case. Therefore, for example, if a group is substituted with 2 R, the definition of each R is independent.

When a bond of a substituent is cross-linked to two atoms on a ring, the substituent can be bonded to any atom on the ring. For example, structural unit

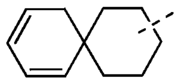 or 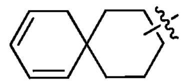

represents that substitution may occur in any one position of cyclohexyl or cyclohexadienyl.

The term "halo-" or "halogen" refers to fluorine, chlorine, bromine and iodine.

The term "hydroxy" refers to -OH group.

The term "amino" refers to $-NH_2$ group.

The term "alkyl" refers to hydrocarbyl with a general formula of $C_nH_{2n+1}$, n being an integer from 1 to 20. The alkyl can be linear or branched. The alkyl herein may contain 1 to 10 carbon atoms ($C_1$-$C_{10}$ alkyl), 1 to 6 carbon atoms ($C_1$-$C_6$ alkyl), 1 to 4 carbon atoms ($C_1$-$C_4$ alkyl), or 1 to 3 carbon atoms ($C_1$-$C_3$ alkyl). For example, the term "$C_{1-6}$ alkyl" refers to alkyl containing 1 to 6 carbon atoms (for example, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl and 2-methylpentyl). The alkyl moieties (namely alkyl) of alkoxy, alkylamino, dialkylamino, alkylsulfonyl and alkylthio are similarly defined as above.

The term "alkoxyl" refers to -O-alkyl.

The term "aminoalkyl" refers to $-alkyl-NH_2$.

The term "alkylacyl" refers to -C(O)-alkyl.

The term "alkylsulfonyl" refers to-$SO_2$-alkyl.

The term "alkenyl" refers to linear or branched unsaturated aliphatic hydrocarbyl consisting of carbon atoms and hydrogen atoms with at least one double bond. The alkenyl herein may contain 2-10 carbon atoms ($C_2$-$C_{10}$ alkenyl), 2-6 carbon atoms ($C_2$-$C_6$ alkenyl), or 2-4 carbon atoms ($C_2$-$C_4$ alkenyl). Non-limiting examples of alkenyl include, but are not limited to, ethenyl, 1-propenyl, 2-propenyl, 1-butenyl, isobutenyl, 1,3-butadienyl, and the like.

The term "alkynyl" refers to linear or branched unsaturated aliphatic hydrocarbyl consisting of carbon atoms and hydrogen atoms with at least one triple bond. The alkynyl herein may contain 2-10 carbon atoms ($C_2$-$C_{10}$ alkynyl), 2-6 carbon atoms ($C_2$-$C_6$ alkynyl), or 2-4 carbon atoms ($C_2$-$C_4$ alkynyl). Non-limiting examples of alkynyl include, but are not limited to, ethynyl, 1-propinyl, 2-propinyl, 1,3-butadiynyl (-C=C-C=CH), and the like.

The term "cycloalkyl" refers to a carbon ring that is fully saturated and may exist as a monocyclic, bridged cyclic or spiro cyclic structure. The cycloalkyl herein may be 3-12 membered cycloalkyl, 3-10 membered cycloalkyl, 5-10 membered cycloalkyl, 3-8 membered cycloalkyl or 3-6 membered cycloalkyl. Non-limiting examples of cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "heterocyclyl" refers to a fully saturated or partially unsaturated (but not fully unsaturated heteroaromatic group) nonaromatic ring which may exist in the form of a monocyclic, bridged cyclic, or spiro cyclic structure. The heterocyclyl herein may be a 3-7 membered ring containing 1 to 3 heteroatoms (preferably 1 or 2 heteroatoms) independently selected from the group consisting of sulfur, silicon, phosphorus, oxygen and/or nitrogen. The heterocyclyl herein may be 4-12 membered heterocyclyl, 4-8 membered heterocyclyl, 5-6 membered heterocyclyl, 4-5 membered heterocyclyl or 6 membered heterocyclyl containing 1 to 3 heteroatoms (preferably 1 or 2 heteroatoms) independently selected from the group consisting of sulfur, oxygen and/or nitrogen. The "5-6 membered monoheterocyclyl whose ring atoms consist of a nitrogen atom and a carbon atom" herein may contain 1 or 2 nitrogen atoms. The "5-6 membered monoheterocyclyl whose ring atoms contain a silicon atom or a phosphorus atom" herein may contain 1 silicon atom or 1 phosphorus atom and 1 or 2 heteroatoms selected from the group consisting of N, O and S. Non-limiting examples of heterocyclyl include, but are not limited to, oxiranyl, tetrahydrofuranyl, dihydrofuranyl, pyrrolidinyl, *N*-methylpyrrolidinyl, dihydropyrrolyl, piperidinyl, piperazinyl, pyrazolidinyl, 4H-pyranyl, morpholinyl, sulfomorpholinyl, tetrahydrothienyl,

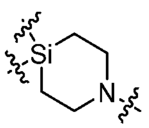, 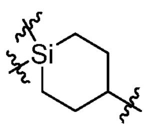, 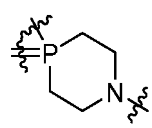,

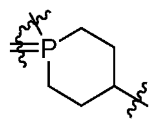

and the like.

The term "heterocycloalkyl" refers to a fully saturated cyclic group that may exist in the form of a monocyclic, bridged cyclic, or spiro cyclic structure. The heterocycloalkyl herein may be a 3-7 membered ring containing 1 to 3

heteroatoms (preferably 1 or 2 heteroatoms) independently selected from the group consisting of sulfur, silicon, phosphorus, oxygen and/or nitrogen. The heterocycloalkyl herein may be 4-12 membered heterocycloalkyl, 4-8 membered heterocycloalkyl, 5-6 membered heterocycloalkyl, 4-5 membered heterocycloalkyl or 6 membered heterocycloalkyl containing 1 to 3 heteroatoms (preferably 1 or 2 heteroatoms) independently selected from the group consisting of sulfur, oxygen and/or nitrogen. Examples of 3 membered heterocycloalkyl include, but are not limited to, oxiranyl, thiiranyl, and aziranyl; non-limiting examples of 4 membered heterocycloalkyl include, but are not limited to, azetidinyl, oxetanyl, and thietanyl; examples of 5 membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6 membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, 1,4-dithianyl,

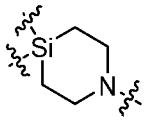, 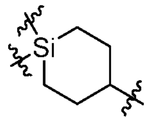, 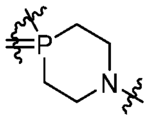,

and

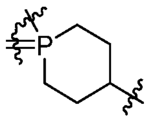;

examples of 7 membered heterocycloalkyl include, but are not limited to, azacycloheptanyl, oxacycloheptanyl and thiocycloheptanyl. Preferably, the heterocycloalkyl is a monocyclic heterocycloalkyl having 5 or 6 ring atoms.

The term "monoheterocyclyl" refers to a monocyclic ring that is fully saturated or partially unsaturated (but not fully saturated aromatic) and has 3 to 10 ring atoms, or 4 to 6 ring atoms. The monoheterocyclyl herein may be a 3-10 membered ring containing 1 to 3 heteroatoms (preferably 1 or 2 heteroatoms) independently selected from the group consisting of sulfur, silicon, phosphorus, oxygen and/or nitrogen. The monoheterocyclyl herein may be a 3-10 membered ring, a 3-8 membered ring, a 3-7 membered ring or a 4-6 membered ring containing 1 to 3 heteroatoms (preferably 1 or 2 heteroatoms) independently selected from the group consisting of sulfur, oxygen and/or nitrogen. Non-limiting examples of the mono-heterocyclic ring include an oxirane ring, a tetrahydrofuran ring, a dihydrofuran ring, a 3,4-dihydrofuran ring, a 3,6-dihydrofuran ring, a tetrahydropyrrole ring, a dihydropyrrole ring, a piperidine ring, a piperazine ring, a morpholine ring, a tetrahydropyrazole ring, a tetrahydrothiophene ring, and the like.

The term "monoheterocycloalkyl" refers to a fully saturated monocyclic ring having 3 to 10 ring atoms, or 4 to 6 ring atoms. The monoheterocycloalkyl herein may be a 3-10 membered ring containing 1 to 3 heteroatoms (preferably 1 or 2 heteroatoms) independently selected from the group consisting of sulfur, silicon, phosphorus, oxygen and/or nitrogen. The monoheterocycloalkyl herein may be a 3-10 membered, 3-8 membered, 3-7 membered or 4-6 membered ring containing 1 to 3 heteroatoms (preferably 1 or 2 heteroatoms) independently selected from the group consisting of sulfur, oxygen and/or nitrogen. Non-limiting examples of monoheterocycloalkyl include an oxirane ring, a tetrahydrofuran ring, a tetrahydropyrrole ring, a piperidine ring, a piperazine ring, a morpholine ring, a tetrahydropyrazole ring, a tetrahydrothiophene ring, and the like.

The term "spirocyclyl" refers to a polycyclic ring that is fully saturated or partially unsaturated (but not fully saturated aromatic) and shares a common carbon atom (referred to as a spiro atom) between 5-20 membered monocyclic rings. The spirocyclyl is preferably 6-14 membered, more preferably 6-10 membered, 7-9 membered or 10 membered. According to the number of spiro atoms shared among the rings, the spiro cyclic ring may be a monospiro cyclic ring, a bispiro cyclic ring or a polyspiro cyclic ring, preferably a monospiro cyclic ring or a bispiro cyclic ring, and more preferably a 4 membered/4 membered, 4 membered/5 membered, 4 membered/6 membered, 5 membered/5 membered or 5 membered/6 membered monospiro cyclic ring. Non-limiting examples of spiro cyclic ring include

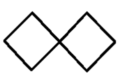, 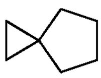, 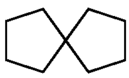, 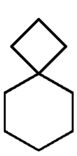, 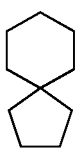, 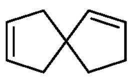 and 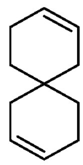.

The term "spiro-cycloalkyl" refers to fully saturated spiro-cyclyl.

The term "spiro-heterocyclyl" refers to a spiro ring whose one or more ring atoms are heteroatoms selected from the group consisting of sulfur, silicon, phosphorus, oxygen and/or nitrogen (preferably 1 or 2 heteroatoms, preferably selected from the group consisting of N, O and/or S), the remaining ring atoms being carbon. The spiro-heterocyclyl is preferably 6-14 membered, more preferably 6-10 membered, or 7-9 membered or 10 membered. According to the number of spiro atoms shared among the rings, the spiro heterocyclic ring may be a monospiro heterocyclic ring, a bispiro heterocyclic ring or a polyspiro heterocyclic ring, preferably a monospiro heterocyclic ring or a bispiro heterocyclic ring, and more preferably a 4 membered/4 membered, 4 membered/5 membered, 4 membered/6 membered, 5 membered/5 membered or 5 membered/6 membered monospiro heterocyclic ring, preferably each ring contains 1 heteroatom selected from the group consisting of N, O and/or S. Non-limiting examples of spiro heterocyclic ring include

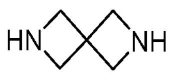, 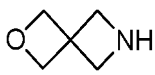, 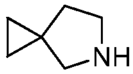,

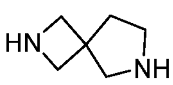, 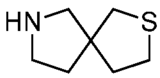, 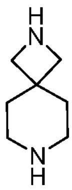, 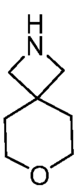, 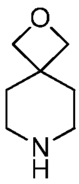, 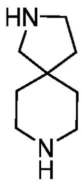, 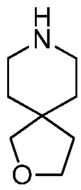 and 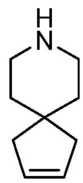.

The term "spiro-heterocycloalkyl" refers to fully saturated spiro-heterocyclyl.

The term "bridged cyclyl" refers to an all-carbon polycyclic ring that is fully saturated or partially unsaturated (but not fully saturated aromatic) and has 5 to 20 ring atoms with the two rings sharing 3 or more ring atoms. The bridged cyclyl is preferably a 6-14 membered polycyclic ring, and more preferably a 6-10 membered polycyclic ring. According to the number of the formed rings, the bridged ring may be bicyclic, tricyclic, tetracyclic or polycyclic bridged ring, preferably bicyclic or tricyclic bridged ring, and more preferably bicyclic bridged ring. Non-limiting examples of the bridged ring include

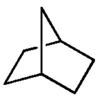, 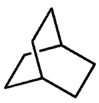, 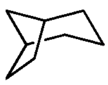, 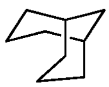, 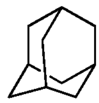, 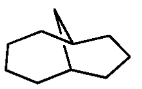 and 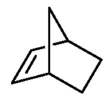.

The term "bridged cycloalkyl" refers to a fully saturated bridged cyclyl.

The term "bridged heterocyclyl" refers to a bridged ring in which one or more ring atoms are heteroatoms selected from the group consisting of sulfur, silicon, phosphorus, oxygen and/or nitrogen (preferably 1 or 2 heteroatoms, preferably selected from the group consisting of N, O and/or S), the remaining ring atoms being carbon. The bridged heterocyclyl is preferably 6-14 membered, and more preferably 6-10 membered, 7-9 membered, 7 membered or 8 membered. According to the number of the formed rings, the bridged heterocyclic ring may be bicyclic, tricyclic, tetracyclic or polycyclic bridged heterocyclic ring, preferably bicyclic or tricyclic bridged heterocyclic ring, and more preferably bicyclic bridged heterocyclic ring. Non-limiting examples of bridged heterocyclic ring include

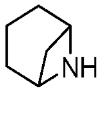, 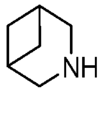, 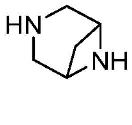, 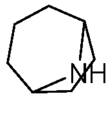, 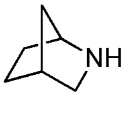, 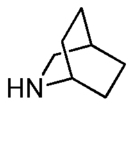, 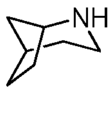, 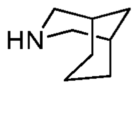,

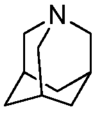, 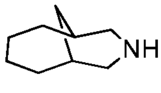 and 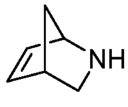.

The term "bridged heterocycloalkyl" refers to a fully saturated bridged heterocyclyl.

The term "fused cyclyl" refers to an all-carbon polycyclic ring that is fully saturated or partially unsaturated (but not fully saturated aromatic) and has 5 to 20 ring atoms with the two rings sharing 2 ring atoms. The fused cyclyl is

preferably a 6-14 membered polycyclic ring, and more preferably a 6-10 membered polycyclic ring. According to the number of the formed rings, the fused ring may be bicyclic, tricyclic, tetracyclic or polycyclic fused ring, preferably bicyclic or tricyclic fused ring, and more preferably bicyclic fused ring. Non-limiting examples of fused ring include

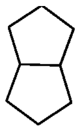, 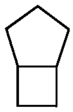,

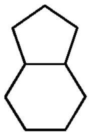, 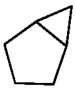, 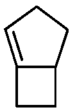, 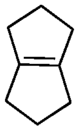, 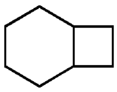, 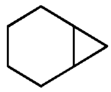 and 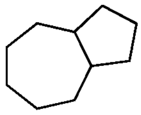.

The term "fused cycloalkyl" refers to a fully saturated fused cyclyl.

The term "fused-heterocyclyl" refers to a fused ring in which one or more ring atoms are heteroatoms selected from the group consisting of sulfur, silicon, phosphorus, oxygen and/or nitrogen (preferably 1 or 2 heteroatoms, preferably selected from the group consisting of N, O and/or S), the remaining ring atoms being carbon. The fused-heterocyclyl is preferably 6-14 membered, and more preferably 6-10 membered, 7-10 membered, 7 membered, 8 membered or 9 membered. According to the number of the formed rings, the bridged heterocyclic ring may be bicyclic, tricyclic or polycyclic bridged heterocyclic ring, preferably bicyclic bridged heterocyclic ring. Non-limiting examples of fused ring include

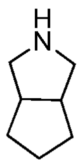, 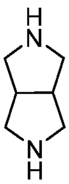, 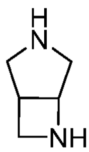, 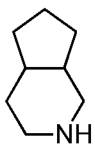, 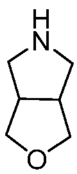, 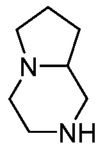, 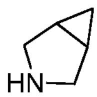, 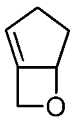, 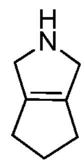,

and

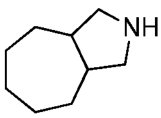.

The term "fused heterocycloalkyl" refers to a fully saturated fused-heterocyclyl.

The groups described above each may be optionally substituted with one or more (e.g., 1, 2, or 3) substituents including, but not limited to, deuterium, hydroxy, halogen (F, Cl, Br, or I), 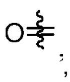, $C_1$-$C_6$ alkyl, $C_1$-$C_6$ alkylsulfonyl, $C_1$-$C_6$ alkylacyl, and $C_1$-$C_6$ alkylacyl-N ($C_1$-$C_6$ alkyl)-.

The term "treating" or "treatment" means administering the compound or formulation described herein to ameliorate or eliminate a disease or one or more symptoms associated with the disease, and includes:

(i) inhibiting a disease or disease state, i.e., arresting its progression; and
(ii) alleviating a disease or disease state, i.e., causing its regression.

The term "therapeutically effective amount" refers to an amount of the compound disclosed herein for (i) treating a specific disease, condition, or disorder, or (ii) alleviating, ameliorating, or eliminating one or more symptoms of a specific disease, condition, or disorder. The amount of the compound of the present application composing the "therapeutically effective amount" varies dependently on the compound, the disease state and its severity, the route of administration, and the age of the mammal to be treated, but can be determined routinely by those skilled in the art in accordance

with their knowledge and the present disclosure.

Therapeutic dosages of the compounds disclosed herein may be determined by, for example, the specific use of a treatment, the route of administration of the compound, the health and condition of a patient, and the judgment of a prescribing physician. The proportion or concentration of the compound disclosed herein in a pharmaceutical composition may not be constant and depends on a variety of factors including dosages, chemical properties (e.g., hydrophobicity), and routes of administration. For example, the compound of the present application may be provided for parenteral administration by a physiological buffered aqueous solution containing about 0.1-10%w/v of the compound. Certain typical dosage ranges from about 1 μg/kg body weight/day to about 1 g/body weight/day. In certain embodiments, the dosage ranges from about 0.01 mg/kg body weight/day to about 100 mg/kg body weight/day. The dosage is likely to depend on such variables as the type and degree of progression of the disease or disorder, the general health condition of the particular patient, the relative biological potency of the compound selected, the excipient formulation and its route of administration. Effective doses can be extrapolated from dose-response curves derived from *in vitro* or animal model test systems.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio. A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof disclosed herein and a pharmaceutically acceptable excipient. The pharmaceutical composition is intended to facilitate the administration of the compound to an organic entity. The pharmaceutical compositions of the present invention may be prepared by conventional methods in the art, for example, by mixing the compound of the present application or a salt thereof with one or more pharmaceutically acceptable excipients.

The term "pharmaceutically acceptable excipients" refers to those which do not have a significant irritating effect on an organic entity and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent and water.

The word "comprise" and variations thereof such as "comprises" or "comprising" will be understood in an open, non-exclusive sense, i.e., "including but not limited to".

The compounds and intermediates disclosed herein may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerism. A specific example of a proton tautomer is an imidazole moiety where a proton can transfer between two ring nitrogens. A valence tautomer includes the interconversion via recombination of some bonding electrons.

The present application also comprises isotopically labeled compounds which are identical to those recited herein but have one or more atoms replaced by an atom having an atomic mass or mass number different from the atomic mass or mass number usually found in nature. Examples of isotopes that can be incorporated into the compounds disclosed herein include isotopes of hydrogen, carbon, nitrogen, oxygen, phosphorus, sulfur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$.

Certain isotopically labeled compounds disclosed herein (e.g., those labeled with $^{3}H$ and $^{14}C$) can be used to analyze compounds and/or substrate tissue distribution. Tritiated (*i.e.,* $^{3}H$) and carbon-14 (*i.e.,* $^{14}C$) isotopes are particularly preferred for their ease of preparation and detectability. Positron emitting isotopes, such as $^{15}O$, $^{13}N$, $^{11}C$ and $^{18}F$ can be used in positron emission tomography (PET) studies to determine substrate occupancy. Isotopically labeled compounds disclosed herein can generally be prepared by following procedures analogous to those disclosed in the schemes and/or examples below while substituting a non-isotopically labeled reagent with an isotopically-labeled reagent.

Furthermore, substitution with heavier isotopes such as deuterium (i.e., $^{2}H$) may provide certain therapeutic advantages (e.g., increased *in vivo* half-life or reduced dosage) resulting from greater metabolic stability and hence may be preferred in some circumstances in which deuterium substitution may be partial or complete, wherein partial deuterium substitution refers to substitution of at least one hydrogen with at least one deuterium. The hydrogens in the exemplary methyl are all deuterated to form methyl-$d_3$, but not limited thereto.

The compound disclosed herein can be asymmetrical, for example, has one or more stereoisomers. Unless otherwise stated, all stereoisomers are included, for example, enantiomers and diastereoisomers. The compound with asymmetric carbon atoms disclosed herein can be separated in an optically pure form or in a racemic form. The optically pure form can be separated from a racemic mixture or can be synthesized using a chiral raw material or a chiral reagent. For example, the

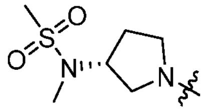 and 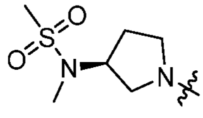

contained in the structure of the compound is the (*R*)-and (*S*)-enantiomers.

Unless otherwise stated, the absolute configuration of a stereogenic center is represented by a wedged solid bond ( ) and a wedged dashed bond ( ), and the relative configuration of a stereogenic center is represented by a straight solid bond ( ) and a straight dashed bond ( ). A wavy line ( ) represents a wedged solid bond ( ) or a wedged dashed bond ( ), or a wavy line ( ) represents a straight solid bond ( ) and a straight dashed bond ( ).

Unless otherwise stated, when a double bond structure such as a carbon-carbon double bond, a carbon-nitrogen double bond, and a nitrogen-nitrogen double bond is present in the compound, and each atom on the double bond is linked to two different substituents (in the double bond including a nitrogen atom, a lone pair of electrons on the nitrogen atom is regarded as a substituent to which the nitrogen atom is linked), if the atom on the double bond of the compound and its substituents are linked using a wavy line ( ), it means that the compound exists in the form of a (Z)-type isomer, an (E)-type isomer, or a mixture of the two isomers.

The pharmaceutical composition disclosed herein can be prepared by combining the compound disclosed herein with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous and intravenous administration.

The pharmaceutical composition disclosed herein can be manufactured using methods well known in the art, such as conventional mixing, dissolving, granulating, dragee-making, levigating, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compound disclosed herein to be formulated into tablets, pills, pastilles, dragees, capsules, liquids, gels, slurries, suspensions, etc. for oral administration to a patient.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners or flavoring agents and the like.

The pharmaceutical compositions may also be suitable for parenteral administration, such as sterile solutions, suspensions or lyophilized products in suitable unit dosage forms.

The compounds of the present application can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the embodiments disclosed herein are carried out in a proper solvent that must be suitable for the chemical changes in the present application and the reagents and materials required. In order to acquire the compounds disclosed herein, it is sometimes necessary for those skilled in the art to modify or select a synthesis procedure or a reaction scheme based on the existing embodiments.

An important consideration in synthesis route planning in the art is the selection of suitable protecting groups for reactive functional groups (e.g., amino in the present application). For example, reference may be made to Greene 's Protective Groups in Organic Synthesis (4th Ed.) Hoboken, New Jersey: John Wiley & Sons, Inc. All references cited herein are incorporated by reference in their entirety.

In some embodiments, the compound of formula (I) disclosed herein may be prepared by one skilled in the art of organic synthesis using standard methods in the art by the following routes:

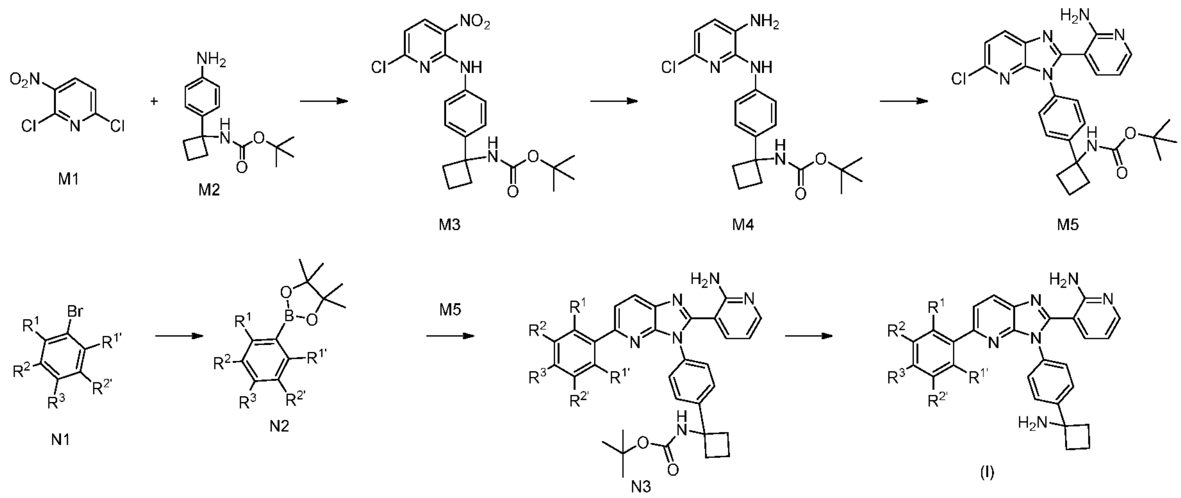

M1 M2 M3 M4 M5

N1 N2 N3 (I)

wherein,
$R^1$, $R^{1'}$, $R^2$, $R^{2'}$ and $R^3$ are defined as above.

## DETAILED DESCRIPTION

For clarity, the present application is further described with the following examples, which are, however, not intended to limit the scope of the invention. It will be apparent to those skilled in the art that various changes and modifications can be made to the specific examples of the present invention without departing from the spirit and scope of the present invention. All reagents are commercially available and can be used without further purification.

### Example 1: Preparation of Compound 1

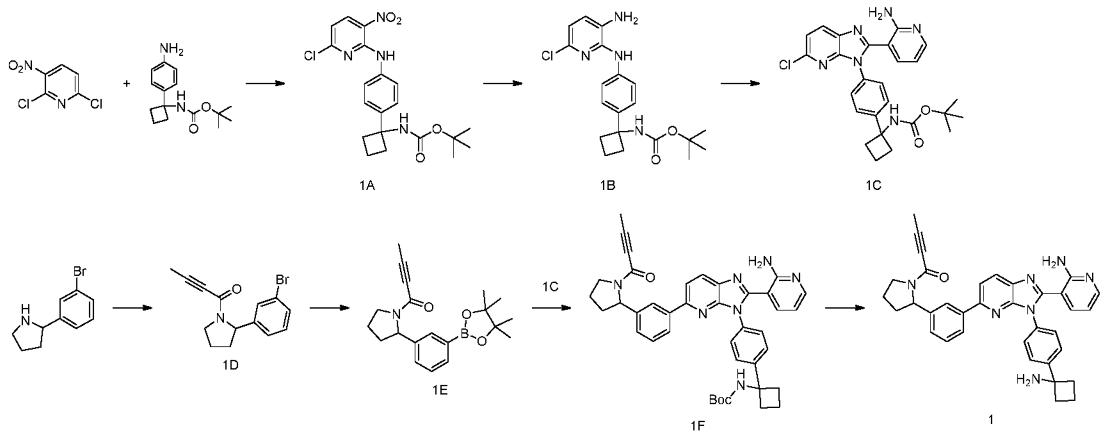

1A 1B 1C

1D 1E 1F 1

### Step A: Preparation of compound 1A

*N,N*-diisopropylethylamine (13.4 g) was added slowly dropwise to a solution of 2,6-dichloro-3-nitropyridine (20.0 g) and *tert*-butyl (1-(4-aminophenyl)cyclobutyl)carbamate (25.5 g) in tetrahydrofuran at -30 °C under nitrogen atmosphere. After the addition was completed, the reaction solution was stirred at room temperature. After the reaction was completed, the reaction solution was concentrated to give compound 1A (43.4 g).
MS (ESI, $[M+Na]^+$) *m/z*:441.5.

### Step B: Preparation of compound 1B

To a reaction flask were added compound 1A (40.0 g), zinc powder (31.2 g), ammonium chloride (5.1 g), ethanol (400 mL) and water (40 mL) successively at room temperature. After the addition was completed, the reaction solution

was reacted at 90 °C. After the reaction was completed, the reaction solution was filtered, the filtrate was concentrated, and the residue was dissolved in dichloromethane, washed with water, dried over anhydrous sodium sulfate, filtered, and concentrated to give compound 1B (41.0 g).

MS (ESI, $[M+H]^+$) *m/z*:389.3.

**Step C: Preparation of compound 1C**

To a reaction flask were added compound 1B (34.0 g), 2-aminonicotinaldehyde (11.2 g), sodium perborate (14.3 g), acetic acid (250 mL) and methanol (30 mL) successively at room temperature. After the addition was completed, the reaction solution was stirred at 55 °C. After the reaction was completed, the reaction solution was concentrated, and added with ethyl acetate and water; then the pH of the reaction solution was adjusted to 11-12 with an aqueous sodium hydroxide solution. The reaction solution was separated, and the organic phase was dried over anhydrous sodium sulfate, filtered, concentrated, and subjected to column chromatography (dichloromethane/methanol = 50/1) to give compound 1C (16.0 g).

MS (ESI, $[M+H]^+$) *m/z*:491.3.

**Step D: Preparation of compound 1D**

2-butynoic acid (1.00 g), 2-(7-azabenzotriazole)-*N,N,N,N*-tetramethyluronium hexafluorophosphate (4.97 g) and dichloromethane (50 mL) were added to a reaction flask successively, and a solution of N,N-diisopropylethylamine (3.07 g) and 2-(3-bromophenyl)-pyrrolidine (2.96 g) in dichloromethane (30 mL) was slowly added dropwise to the reaction flask at 0 °C. After the addition was completed, the reaction solution was stirred for reaction at room temperature. After the reaction was completed, a saturated aqueous ammonium chloride solution was added to the reaction solution, the phases were separated, the aqueous phase was extracted with dichloromethane, the organic phases were combined, washed with saturated brine, and dried over anhydrous sodium sulfate. The organic phase was filtered, concentrated, and subjected to column chromatography (petroleum ether/ethyl acetate = 1/1) to give compound 1D (3.92 g).

MS (ESI, $[M+H]^+$) *m/z:* 292.0.

**Step E: Preparation of compound 1E**

To a reaction flask were added compound 1D (0.92 g), bis(pinacolato)diboron (1.20 g), potassium acetate (0.93 g), [1,1'-bis(diphenylphosphino)ferrocene]palladium dichloride dichloromethane complex (0.13 g) and 1,4-dioxane (15 mL) successively. The reaction solution was stirred for reaction at 100 °C under nitrogen atmosphere. After the reaction was completed, the reaction solution was filtered and concentrated. The reaction solution was subjected to column chromatography (petroleum ether/ethyl acetate = 2/1) to give compound 1E (0.38 g).

MS (ESI, $[M+H]^+$) *m/z:* 340.2.

**Step F: Preparation of compound 1F**

To a microwave tube were added compound 1E (0.17 g), compound 1C (0.12 g), potassium carbonate (0.11 g), tetrakis(triphenylphosphine)palladium (0.03 g), 1,4-dioxane (3 mL) and water (0.5 mL) successively. After the addition was completed, the reaction solution was purged with nitrogen gas, and reacted at 100 °C in microwave reactor for 2 h. After the reaction was completed, the reaction solution was filtered and concentrated. The reaction solution was subjected to column chromatography (dichloromethane/methanol = 20/1) to give compound 1F (68 mg).

MS (ESI, $[M+Na]^+$) *m/z:* 690.6.

**Step G: Preparation of compound 1**

Compound 1F (68 mg), methanesulfonic acid (0.1 mL) and dichloromethane (4 mL) were added successively to a reaction flask, and the mixture was stirred at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 1 (18 mg).

HRMS (ESI, $[M+H]^+$) *m/z*: 568.2823.

$^1H$ NMR (500MHz, DMSO-$d_6$) $\delta$ 8.28 (d, J = 8.3 Hz, 1H), 8.05 - 7.95 (m, 2H), 7.94 - 7.79 (m, 2H), 7.71 - 7.63 (m, 2H), 7.58 - 7.50 (m, 2H), 7.46 - 7.38 (m, 1H), 7.31 - 7.16 (m, 2H), 6.93 (d, J = 8.5Hz, 2H), 6.46 - 6.42 (m, 1H), 5.24 - 5.02 (m, 1H), 3.83 (t, J = 6.9 Hz, 1H), 3.65 - 3.54 (m, 1H), 2.62 - 2.52 (m, 2H), 2.45 - 2.30 (m, 3H), 2.18 - 2.09 (m, 1H),

1.94 - 1.73 (m, 4H), 1.87 (s, 3H).

**Example 2: Preparation of Compound 2**

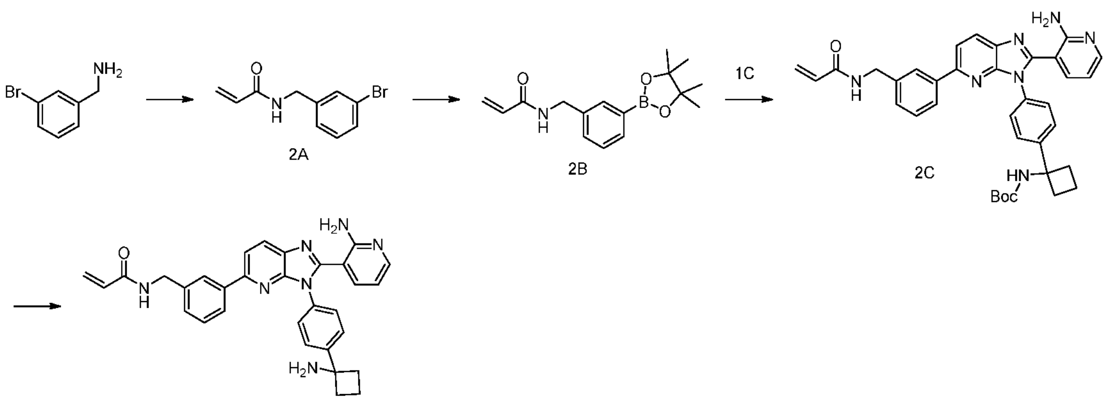


2A 2B 2C

2

**Step A: Preparation of compound 2A**

A solution of acryloyl chloride (1.08 g) in tetrahydrofuran (10 mL) was slowly added to a reaction solution of 3-bromobenzylamine (2.00 g) and *N,N* diisopropylethylamine (1.63 g) in tetrahydrofuran (90 mL) in a reaction flask at -10 °C under nitrogen atmosphere. After the addition was completed, the reaction solution was stirred at room temperature until the reaction was completed. The reaction solution was added with a saturated aqueous ammonium chloride solution and extracted with ethyl acetate; the organic phases were combined, washed with saturated brine, and dried over anhydrous sodium sulfate. The reaction solution was filtered and concentrated to give compound 2A (2.58 g).

MS (ESI, $[M+H]^+$) *m/z:* 240.1.

**Step B: Preparation of compound 2B**

To a reaction flask were added compound 2A (2.00 g), bis(pinacolato)diboron (3.17 g), potassium acetate (2.45 g), [1,1'-bis(diphenylphosphino)ferrocene]palladium dichloride dichloromethane complex (0.68 g) and 1,4-dioxane (30 mL) successively. The reaction solution was stirred at 100 °C under nitrogen atmosphere. After the reaction was completed, the reaction solution was filtered and concentrated. The reaction solution was subjected to column chromatography (petroleum ether/ethyl acetate = 1/1) to give compound 2B (1.89 g).

MS (ESI, $[M+H]^+$) *m/z:* 288.1.

**Step C: Preparation of compound 2C**

To a microwave tube were added compound 2B (0.14 g), compound 1C (0.12 g), potassium carbonate (0.11 g), tetrakis(triphenylphosphine)palladium (0.028 g), 1,4-dioxane (3 mL) and water (0.5 mL) successively. After the addition was completed, the reaction solution was purged with nitrogen gas, and reacted at 100 °C in microwave reactor. After the reaction was completed, the reaction solution was filtered and concentrated. The reaction solution was subjected to column chromatography (dichloromethane/methanol = 20/1) to give compound 2C (85 mg).

MS (ESI, $[M+H]^+$) *m/z:* 616.5.

$^1H$ NMR (500 MHz, DMSO-$d_6$) δ 8.28 (d, *J* = 8.3 Hz, 1H), 8.05 - 7.95 (m, 2H), 7.94 - 7.79 (m, 2H), 7.71 - 7.63 (m, 2H), 7.58 - 7.50 (m, 2H), 7.42 (dt, *J* = 26.0, 7.7 Hz, 1H), 7.31 - 7.16 (m, 2H), 6.93 (d, *J* = 8.5 Hz, 2H), 6.46 - 6.42 (m, 1H),5.24 - 5.02 (m, 1H), 3.83 (t, *J* = 6.9 Hz, 1H), 3.65 - 3.54 (m, 1H), 2.62 - 2.52 (m, 2H), 2.45 - 2.30 (m, 3H), 2.18 - 2.09 (m, 1H), 1.94 - 1.73 (m, 4H), 1.87 (s, 3H).

**Step D: Preparation of compound 2**

Compound 2C (80 mg), methanesulfonic acid (0.1 mL) and dichloromethane (4 mL) were added successively to a reaction flask. The mixture was stirred at room temperature. After the reaction was completed, 10% sodium hydroxide solution was added to the reaction solution to adjust the pH to 13, water and dichloromethane were added to extract the

reaction solution, and the reaction solution was washed with saturated brine and dried over anhydrous sodium sulfate. The reaction solution was filtered, concentrated, and subjected to column chromatography (dichloromethane/methanol = 10/1) to give compound 2 (45 mg).

HRMS (ESI, $[M+H]^+$) *m/z*: 516.2516.

$^1H$ NMR (500 MHz, DMSO-$d_6$) $\delta$ 8.67 (t, *J* = 6.0 Hz, 1H), 8.27 (d, *J* = 8.4 Hz, 1H), 8.01 (dd, *J* = 4.8, 1.8 Hz, 1H), 7.98 - 7.88 (m, 3H), 7.67 - 7.59 (m, 2H), 7.50 - 7.40 (m, 3H), 7.30 (dt, *J* = 7.6, 1.3 Hz, 1H), 7.21 (dd, *J* = 7.6, 1.9 Hz, 1H), 6.94 (s, 2H), 6.42 (dd, *J* = 7.7, 4.9 Hz, 1H), 6.31 (dd, *J* = 17.1, 10.2 Hz, 1H), 6.15 (dd, *J* = 17.1, 2.2 Hz, 1H), 5.65 (dd, *J* = 10.2, 2.2 Hz, 1H), 4.43 (d, *J* = 5.9 Hz, 2H), 2.49 - 2.43 (m, 2H), 2.23 - 2.13 (m, 2H), 2.11 - 2.00 (m, 1H), 1.77 - 1.69 (m, 1H).

### Example 3: Preparation of Compound 3

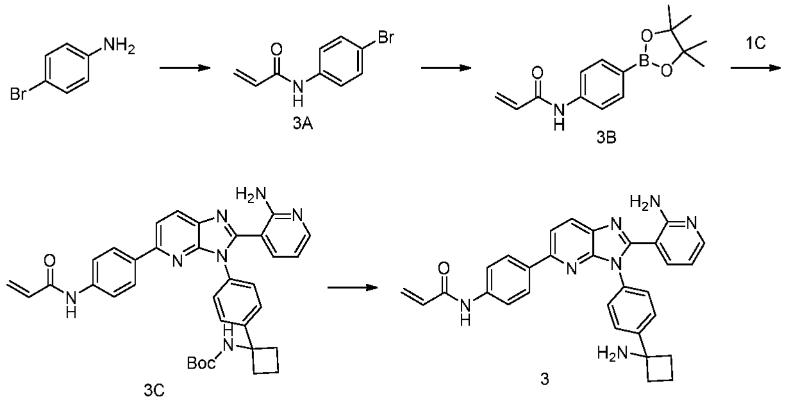


### Step A: Preparation of compound 3A

A solution of acryloyl chloride (2.98 g) in tetrahydrofuran (10 mL) was slowly added to a stirred solution ofp-bromophenylamine (5.00 g) and *N*,*N* diisopropylethylamine (4.51 g) in tetrahydrofuran (90 mL) in a reaction flask at -10 °C under nitrogen atmosphere. After the addition was completed, the reaction solution was stirred at room temperature until the reaction was completed. The reaction solution was added with a saturated aqueous ammonium chloride solution and extracted with ethyl acetate; the organic phases were combined, washed with saturated brine, and dried over anhydrous sodium sulfate. The reaction solution was filtered and concentrated to give compound 3A (5.90 g).

MS (ESI, $[M+H]^+$) *m/z:* 226.1.

### Step B: Preparation of compound 3B

To a reaction flask were added compound 3A (0.5 g), bis(pinacolato)diboron (0.84 g), potassium acetate (0.65 g), [1,1'-bis(diphenylphosphino)ferrocene]palladium dichloride dichloromethane complex (0.18 g) and 1,4-dioxane (20 mL) successively. The reaction solution was stirred at 100 °C under nitrogen atmosphere. After the reaction was completed, the reaction solution was filtered and concentrated. The reaction solution was subjected to column chromatography (petroleum ether/ethyl acetate = 2/1) to give compound 3B (0.26 g).

MS (ESI, $[M+H]^+$) *m/z:* 274.5.

### Step C: Preparation of compound 3C

To a microwave tube were added compound 3B (0.13 g), compound 1C (0.12 g), potassium carbonate (0.11 g), tetrakis(triphenylphosphine)palladium (0.028 g), 1,4-dioxane (3 mL) and water (0.5 mL) successively. After the addition was completed, the reaction solution was purged with nitrogen gas, and reacted at 100 °C in microwave reactor for 2 h. After the reaction was completed, the reaction solution was filtered and concentrated. The reaction solution was subjected to column chromatography (dichloromethane/methanol = 20/1) to give compound 3C (78 mg).

MS (ESI, $[M+H]^+$) *m/z:* 602.4.

**Step D: Preparation of compound 3**

Compound 3C (70 mg), methanesulfonic acid (0.1 mL) and dichloromethane (4 mL) were added successively to a reaction flask, and the mixture was stirred at room temperature. After the reaction was completed, 10% sodium hydroxide solution was added to the reaction solution to adjust the pH to 13, dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 3 (34 mg).
HRMS (ESI, $[M+H]^+$) *m/z*: 502.2343.
$^1H$ NMR (500 MHz, DMSO-$d_6$) $\delta$ 10.26 (s, 1H), 8.17 (d, *J* = 8.3 Hz, 1H), 7.99 - 7.91 (m, 3H), 7.88 (d, *J* = 8.4 Hz, 1H), 7.70 (d, *J*= 8.5 Hz, 2H), 7.57 (d, *J* = 8.4 Hz, 2H), 7.39 (d, *J* = 8.4 Hz, 2H), 7.12 (dd, *J* = 7.7, 1.8 Hz, 1H), 6.90 (s, 2H), 6.45 - 6.30 (m, 2H), 6.21 (dd, *J* = 16.9, 2.0 Hz, 1H), 5.70 (dd, *J* = 10.0, 2.0 Hz, 1H), 2.42 - 2.36 (m, 2H), 2.15 - 2.07 (m, 2H), 2.05 - 1.95 (m, 1H), 1.71 - 1.62 (m, 1H).

**Example 4: Preparation of Compound 4**

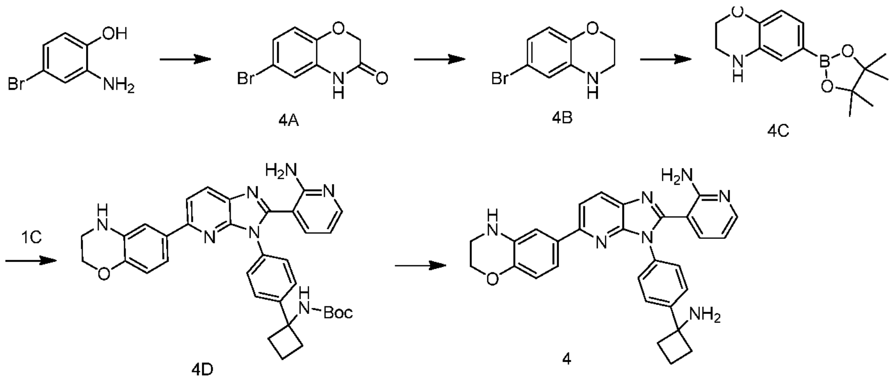

4A 4B 4C 4D 4

**Step A: Preparation of compound 4A**

2-Chloroacetyl chloride (3.24 g) was added dropwise to a reaction solution of 2-amino-4-bromophenol (3.60 g) and sodium carbonate (3.22 g) in tetrahydrofuran (60 mL) in an ice-water bath under nitrogen atmosphere. After the addition was completed, the reaction solution was stirred at room temperature. After the reaction was completed, the reaction solution was concentrated and subj ected to column chromatography (petroleum ether/ethyl acetate = 3/1) to give compound 4A (3.60 g).
MS (ESI, $[M-H]^-$) *m/z:* 226.1; MS (ESI, $[M+H]^+$) *m/z:* 228.0.

**Step B: Preparation of compound 4B**

A solution of 1 M borane in tetrahydrofuran (516 mg) was added slowly to a solution of compound 4A (452 mg) in tetrahydrofuran (10 mL) in an ice-water bath under nitrogen atmosphere. After the addition was completed, the reaction solution was stirred for reaction at 70 °C. After the reaction was completed, the reaction was quenched with saturated sodium bicarbonate, and the reaction solution was extracted with ethyl acetate, and the organic phases were combined, washed with saturated brine, dried over anhydrous sodium sulfate, filtered, concentrated, and subjected to column chromatography (petroleum ether/ethyl acetate = 4/1) to give compound 4B (380 mg).
MS (ESI, $[M+H]^+$) *m/z:* 214.3.

**Step C: Preparation of compound 4C**

To a reaction flask were added [1,1'-bis(diphenylphosphino)ferrocene]palladium dichloride (123 mg), compound 4B (320 mg), bis(pinacolato)diboron (508 mg), potassium carbonate (294 mg) and dioxane (5 mL) successively. The reaction solution was reacted at 100 °C. After the reaction was completed, the reaction solution was concentrated and subjected to column chromatography (petroleum ether/ethyl acetate = 3/1) to give compound 4C (242 mg).
MS (ESI, $[M+H]^+$) *m/z:* 262.4.

**Step D: Preparation of compound 4D**

To a reaction flask were added compound 4C (200 mg), potassium carbonate (270 mg), tetrakis(triphenylphosphine)palladium (47 mg), compound 1C (320 mg), water (0.5 mL), and dioxane (5 mL) successively. After the addition was completed, the reaction solution was reacted at 110 °C. After the reaction was completed, the reaction solution was concentrated and subjected to column chromatography (dichloromethane/methanol = 98/2) to give compound 4D (260 mg).

**Step E: Preparation of compound 4**

To the reaction flask were added compound 4D (260 mg), methanesulfonic acid (0.5 mL), and dichloromethane (5 mL) successively. After the addition was completed, the reaction solution was stirred at room temperature. After the reaction was completed, the pH of the reaction solution was adjusted to 7 to 9 with saturated aqueous sodium bicarbonate, the reaction solution was extracted with dichloromethane, the organic phases were combined, washed with saturated brine, dried over anhydrous sodium sulfate, filtered, concentrated, and subjected to column chromatography (dichloromethane/methanol = 95/5) to give compound 4 (135 mg).
HRMS (ESI, $[M+H]^+$) *m/z:* 490.2360.
$^1H$ NMR (500 MHz, DMSO-$d_6$): δ 8.16(d, *J* = 5.0 Hz,1H),7.99 (dd, *J* = 5.0 Hz,10.0Hz, 1H), 7.75(d, *J* = 10.0 Hz,1H), 7.61(d, *J* = 10.0 Hz,1H), 7.41(d, *J* = 10.0 Hz,1H), 7.23(d, *J* = 5.0 Hz,1H), 7.16 - 7.14(m,2H), 6.94(s, 2H), 6.71(d, *J* = 5.0 Hz,1H), 6.39(q, *J* = 5.0 Hz,1H), 5.88(s, 1H), 4.14(t, *J* = 5.0Hz, 2H), 3.30(d, *J* = 10.0Hz, 2H), 2.46 - 2.14(m, 2H), 2.13(br, 2H), 2.12 - 2.03(m, 3H), 1.78 - 1.72(m, 1H).

**Example 5: Preparation of Compound 5**

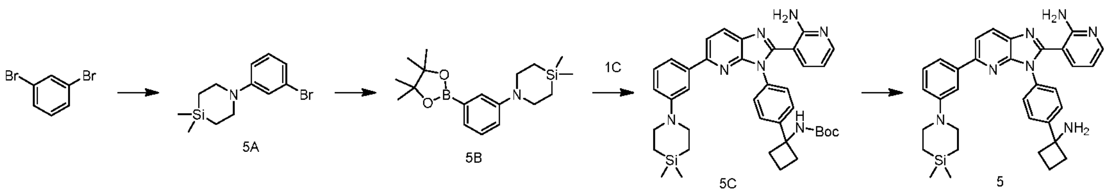


**Step A: Preparation of compound 5A**

To a reaction flask were added 1,1'-binaphthyl-2,2'-diphemyl phosphine (279 mg), tris(dibenzylideneacetone)dipalladium (274 mg), 1,3-dibromobenzene (705 mg), 4,4-dimethyl-1,4-silapiperidine (386 mg), sodium *tert*-butoxide (431 mg) and toluene (10 mL) successively under nitrogen atmosphere. After the addition was completed, the reaction solution was reacted at 115 °C. After the reaction was completed, the reaction solution was concentrated and subjected to column chromatography (petroleum ether/ethyl acetate = 95/5) to give compound 5A (475 mg). The compound was directly used in the next step without purification.

**Step B: Preparation of compound 5B**

To a reaction flask were added [1,1'-bis(diphenylphosphino)ferrocene]palladium dichloride (63.9 mg), compound 5A (475 mg), bis(pinacolato)diboron (795 mg), potassium acetate (461 mg) and dioxane (8 mL) successively under nitrogen atmosphere. After the addition was completed, the reaction solution was reacted at 110 °C. After the reaction was completed, the reaction solution was concentrated and subjected to column chromatography (petroleum ether/ethyl acetate = 9/1) to give compound 5B (450 mg).
MS (ESI, $[M+H]^+$) *m/z:* 332.5.

**Step C: Preparation of compound 5C**

To a reaction flask were added compound 5B (311 mg), potassium carbonate (287 mg), compound 1C (340 mg), water (1 mL), and dioxane (10 mL) successively under nitrogen atmosphere. After the addition was completed, the reaction solution was reacted at 110 °C. After the reaction was completed, the reaction solution was concentrated and subjected to column chromatography (dichloromethane/methanol = 96/4) to give compound 5C (280 mg).

MS (ESI, $[M+H]^+$) *m/z:* 660.7.

**Step D: Preparation of compound 5**

In a reaction flask, methanesulfonic acid (200 mg) was slowly added to a solution of compound 5C (280 mg) in dichloromethane (5 mL). After the addition was completed, the reaction solution was reacted at room temperature. After the reaction was completed, the pH of the reaction solution was adjusted to 7 to 9 with saturated aqueous sodium bicarbonate, the reaction solution was extracted with dichloromethane, the organic phases were combined, washed with saturated brine, dried over anhydrous sodium sulfate, filtered, concentrated, and subjected to column chromatography (dichloromethane/methanol = 95/5) to give compound 5 (120 mg).

MS (ESI, $[M+H]^+$) *m/z:* 560.6.

$^1$HNMR (500 MHz, DMSO-$d_6$): δ 8.16 (d, *J* = 10.0Hz, 1H), 7.96-7.95(m, 1H),7.85 (d, *J* =5.0Hz,1H), 7.54 (d, *J* =10.0Hz, 1H), 7.47(s, 1H), 7.38(d, *J* =5.0Hz, 1H), 7.21-7.17(m,3H), 6.97(br, 2H), 6.82(q, *J* =5.0Hz, 1H), 6.37(q, *J* =5.0Hz, 1H), 3.63-3.61(m, 4H), 2.38 - 2.32(m, 2H), 2.17(br, 2H), 2.09 - 2.03(m,2H), 2.00 - 1.93(m, 1H), 1.64 - 1.61(m, 1H), 0.67 - 0.65(m,4H), 0.001(s, 6H).

**Example 6: Preparation of Compound 6**

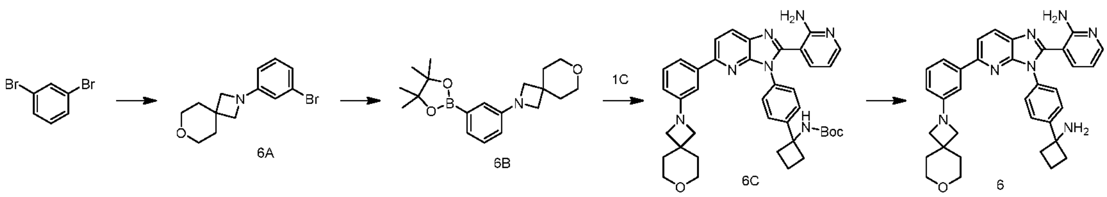


**Step A: Preparation of compound 6A**

To a reaction flask were added 1,1'-binaphthyl-2,2'-diphemyl phosphine (0.475 g), tris(dibenzylideneacetone)dipalladium (0.466 g), 1,3-dibromobenzene (1.200 g), 7-oxa-2-azaspiro[3.5]nonane (0.647 g), sodium tert-butoxide (0.733 g) and toluene (10 mL) successively under nitrogen atmosphere. After the addition was completed, the reaction solution was reacted at 115 °C. After the reaction was completed, the reaction solution was concentrated and subjected to column chromatography (petroleum ether/ethyl acetate = 85/15) to give compound 6A (780 mg).

**Step B: Preparation of compound 6B**

To a reaction flask were added [1,1'-bis(diphenylphosphino)ferrocene]palladium dichloride (339 mg), compound 6A(780 mg), bis(pinacolato)diboron (1404 mg), potassium acetate (814 mg) and dioxane (8 mL) successively under nitrogen atmosphere. After the addition was completed, the reaction solution was reacted at 110 °C. After the reaction was completed, the reaction solution was concentrated and subjected to column chromatography (petroleum ether/ethyl acetate = 9/1) to give compound 6B (520 mg).

MS (ESI, $[M+H]^+$) *m/z:* 330.5.

**Step C: Preparation of compound 6C**

To a reaction flask were added compound 6B (520 mg), potassium carbonate (220 mg), tetratriphenylphosphine (36 mg), compound 1C (260 mg), water (0.50 mL), and dioxane (10 mL) successively under nitrogen atmosphere. After the addition was completed, the reaction solution was reacted at 110 °C. After the reaction was completed, the reaction solution was concentrated and subjected to column chromatography (dichloromethane/methanol = 98/2) to give compound 6C (240 mg).

MS (ESI, $[M+H]^+$) *m/z:* 658.7.

**Step D: Preparation of compound 6**

To a reaction flask were added methanesulfonic acid (331 mg), compound 6C (240 mg), and dichloromethane (5 mL) successively, and after the addition was completed, the mixture was stirred at room temperature. After the reaction

was completed, the pH of the reaction solution was adjusted to 7 to 9 with saturated aqueous sodium bicarbonate, the reaction solution was extracted with dichloromethane, the organic phases were combined, washed with saturated brine, dried over anhydrous sodium sulfate, filtered, concentrated, and subjected to column chromatography (dichloromethane/methanol = 95/5) to give compound 6 (140 mg).

MS (ESI, $[M+H]^+$) *m/z:* 558.6.

$^1$HNMR (500 MHz, DMSO-$d_6$): δ 8.45 (d, *J* = 5.0Hz, 1H), 8.22 (t, *J* = 5.0Hz, 2H), 8.11-8.10(m,1H), 8.08(d, *J* = 10.0Hz, 1H), 8.01(dd, J=5.0Hz, 10.0Hz, 1H), 7.82-7.78(m,2H), 7.72 - 7.70(m,2H), 7.65(t, *J* = 10.0Hz, 1H), 7.55(dd, *J* =5.0Hz, 10.0Hz, 1H), 6.98(t, *J* = 10.0Hz, 1H), 4.34(s, 4H), 3.59-3.57(m, 4H), 2.66(t, *J* = 5.0Hz,4H), 2.55(t, *J* = 5.0Hz, 4H), 1.97(t, *J* = 5.0 Hz, 4H).

### Example 7: Preparation of Compound 7

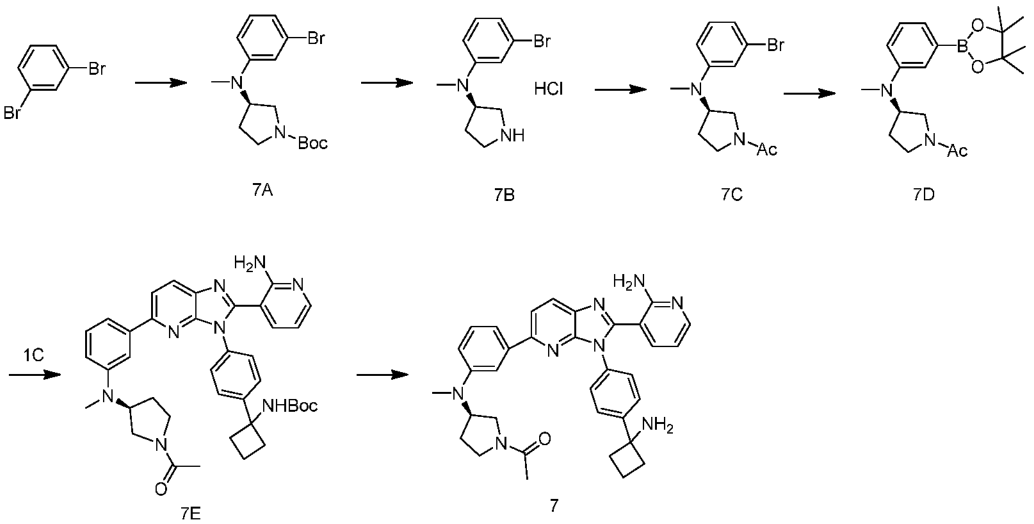


7A 7B 7C 7D 7E 7

### Step A: Preparation of compound 7A

1,1'-binaphthyl-2,2'-diphemyl phosphine (367 mg), bis(dibenzylideneacetone)palladium (270 mg), 1,3-dibromobenzene (695 mg), *tert*-butyl (*R*)-3-(methylamine)pyrrole-1-carboxylate (590 mg), sodium *tert*-butoxide (566 mg) and dioxane (15 mL) were successively added to a reaction flask under nitrogen atmosphere. After the addition was completed, the reaction solution was reacted at 120 °C. After the reaction was completed, the reaction solution was concentrated and subjected to column chromatography (petroleum ether/ethyl acetate = 95/5) to give compound 7A (642 mg).

MS (ESI, $[M-55+H]^+$) *m/z:* 299.1.

### Step B: Preparation of compound 7B

To a reaction flask were added compound 7A (640 mg), dichloromethane (5 mL) and a solution of 4 M hydrogen chloride in dioxane (3 mL) successively under nitrogen atmosphere. After the addition was completed, the reaction solution was stirred at room temperature until the reaction was completed, and the solvent was removed by distillation under the reduced pressure to give compound 7B (680 mg).

### Step C: Preparation of compound 7C

To a reaction flask were added compound 7B (680 mg), tetrahydrofuran (20 mL), N-diisopropylethylamine (581 mg), and acetyl chloride (300 mg) separately. After the addition was completed, the reaction solution was reacted at room temperature. After the reaction was completed, the reaction solution was concentrated and subjected to column chromatography (dichloromethane/methanol = 20/1) to give compound 7C (430 mg).

MS (ESI, $[M+H]^+$) *m/z:* 297.4.

**Step D: Preparation of compound 7D**

To a reaction flask were added [1,1'-bis(diphenylphosphino)ferrocene]palladium dichloride (154 mg), compound 7C (360 mg), bis(pinacolato)diboron (478 mg), potassium acetate (370 mg) and dioxane (8 mL) successively under nitrogen atmosphere. After the addition was completed, the reaction solution was reacted at 110 °C. The reaction was completed, the reaction was quenched by saturated brine, the reaction solution was extracted by ethyl acetate, organic phases were combined, the reaction solution was washed with saturated brine and dried over anhydrous sodium sulfate. The organic phase was filtered, concentrated, and subjected to column chromatography (petroleum ether/ethyl acetate = 90/10) to give compound 7D (440 mg).

MS (ESI, $[M+H]^+$) *m/z:* 345.6.

**Step E: Preparation of compound 7E**

To a reaction flask were added tetrakis(triphenylphosphine)palladium (99 mg), compound 1C (420 mg), compound 7D (294 mg), potassium carbonate (236 mg), dioxane (5 mL), and water (0.5 mL) successively. After the addition was completed, the reaction solution was heated and reacted at 120 °C. After the starting materials were reacted completely, the reaction solution was concentrated and subjected to column chromatography (dichloromethane/methanol = 98/2) to give compound 7E (540 mg).

MS (ESI, $[M+H]^+$) *m/z:* 673.7.

**Step F: Preparation of compound 7**

To the reaction flask were added compound 7E (4901 mg), dichloromethane (6 mL) and methanesulfonic acid (420 mg) successively. After the addition was completed, the reaction solution was reacted at room temperature. After the reaction was completed, the pH of the reaction solution was adjusted to be neutral by using sodium bicarbonate, the reaction solution was extracted with dichloromethane, and the organic phase were combined, washed with saturated brine and dried over anhydrous sodium sulfate. The reaction solution was filtered, concentrated, and subjected to column chromatography (dichloromethane/methanol = 96/4) to give compound 7 (30 mg).

MS (ESI, $[M+H]^+$) *m/z:* 573.4.

$^1H$ NMR (500 MHz, DMSO-$d_6$): δ 8.23 (d, *J* =3.5Hz, 1H), 8.01 - 7.96(m, 2H), 7.62 - 7.60 (m, 2H),7.55(d, *J*=9.0Hz, 1H), 7.45(d, *J*=6.5Hz, 1H), 7.29 - 7.22 (m, 1H), 7.01(d, *J*=7.5Hz, 1H), 6.92(d, *J*=7.5Hz, 1H), 6.42(t, *J*=6.5Hz, 1H), 4.58 - 4.47(m, 1H), 3.69 - 3.40(m,3H), 3.28 - 3.25(m, 2H), 2.83 - 2.80(m, 3H), 2.50(s, 3H), 2.15 - 2.08(m, 3H), 2.06 - 2.00(m, 2H), 1.96 - 1.85(m, 3H).

**Example 8: Preparation of Compound 8**

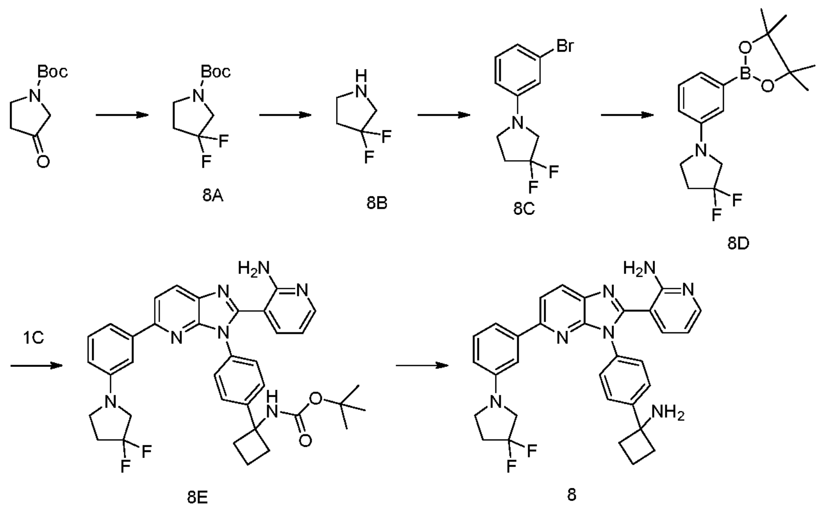

8A
8B
8C
8D
8E
8

**Step** A: **Preparation of compound 8A**

A solution of *N,N*-diethyl-1,1,1-trifluoro-14-thiamine (20.890 g) in 1,2-dichloroethane (25 mL) was slowly added dropwise to a reaction flask containing *tert*-butyl 3-oxopyrrolidine-1-carboxylate (4.000 g) under nitrogen atmosphere. After the addition was completed, the reaction solution was stirred at room temperature. After the reaction was completed, the reaction solution was concentrated and subjected to column chromatography (petroleum ether/ethyl acetate = 4/1) to give compound 8A (2.510 g).

MS (ESI, $[M+H]^+$) *m/z:* 208.0.

**Step B: Preparation of compound 8B**

To a reaction flask were added compound 8A (1.000 g), a solution of 1,4-dioxane (2 mL), and a solution of 4 M hydrochloric acid in 1,4-dioxane (9.50 mL) successively. After the addition was completed, the reaction solution was stirred for reaction at room temperature. After the reaction was completed, the reaction solution was concentrated to give compound 8B (0.512 g).

**Step C: Preparation of compound 8C**

To a reaction flask were added sodium tert-butoxide (0.928 g), tris(dibenzylideneacetone)dipalladium (0.442 g), 3,3-difluoropyrrolidine (0.517 g), 1,3-dibromobenzene (2.279 g), 1,1'-binaphthyl-2,2'-diphemyl phosphine (0.451 g) and toluene (30 mL) successively under nitrogen atmosphere. After the addition was completed, the reaction solution was stirred at 110 °C. After the reaction was completed, the reaction solution was concentrated and subjected to column chromatography (petroleum ether/ethyl acetate = 95/5) to give compound 8C (1.199 g).

**Step D: Preparation of compound 8D**

To a reaction flask were added compound 8C (1.150 g), potassium carbonate (1.227 g), bis(pinacolato)diboron (1.587 g), [1,1'-bis(diphenylphosphino)ferrocene]palladium dichloride (0.170 g), 1,1'-binaphthyl-2,2'-diphemyl phosphine (0.451 g), and *N,N*-dimethylformamide (30 mL) successively under nitrogen atmosphere. After the addition was completed, the reaction solution was stirred at 110 °C. After the reaction was completed, the reaction solution was concentrated and subjected to column chromatography (petroleum ether/ethyl acetate = 90/10) to give compound 8D (0.779 g).

MS (ESI, $[M+H]^+$) *m/z:* 310.2.

**Step E: Preparation of compound 8E**

To a reaction flask were added compound 8D (0.307 g), potassium carbonate (0.115 g), compound 1C (0.210 g), tetrakis(triphenylphosphine)palladium (0.048 g), 1,4-dioxane (16.00 mL), and water (4.00 mL) successively under nitrogen atmosphere. After the addition was completed, the reaction solution was stirred for reaction at 140 °C. After the reaction was completed, the reaction solution was concentrated and subjected to column chromatography (dichloromethane/methanol = 98/3) to give compound 8E (0.140 g).

MS (ESI, $[M+H]^+$) *m/z:* 638.4.

**Step F: Preparation of compound 8**

To a reaction flask were added compound 8E (0.140 g), dichloromethane (10 mL) and methanesulfonic acid (0.160 g) successively. After the addition was completed, the reaction solution was stirred for reaction at room temperature. After the reaction was completed, the reaction solution was concentrated and subjected to column chromatography (dichloromethane/methanol = 93/7) to give compound 8 (98 mg).

MS (ESI, $[M+H]^+$) *m/z:* 538.6.

$^1$H NMR (500 MHz, DMSO-$d_6$) δ 8.24 (d, *J* = 8.4 Hz, 1H), 8.00 (t, *J* = 7.6 Hz, 2H), 7.64 (d, *J* = 8.0 Hz, 2H), 7.46 (d, *J* = 8.0 Hz, 2H), 7.40 (d, *J* = 7.7 Hz, 1H), 7.27 (dq, *J* = 13.3, 7.8 Hz, 3H), 7.02 (s, 2H), 6.66 (d, *J* = 8.1 Hz, 1H), 6.43 (dd, *J* = 7.7, 4.7 Hz, 1H), 3.74 (t, *J* = 13.3 Hz, 2H), 3.53 (t, *J* = 7.2 Hz, 2H), 2.55 (dd, *J* = 1.6, 10.3 Hz, 2H), 2.44 (t, *J*= 8.6 Hz, 2H), 2.24 - 2.10 (m, 2H), 2.11 - 2.00 (m, 1H), 1.72 (dd, *J* = 11.4, 6.5 Hz, 1H).

**Example 9: Preparation of Compound 9**

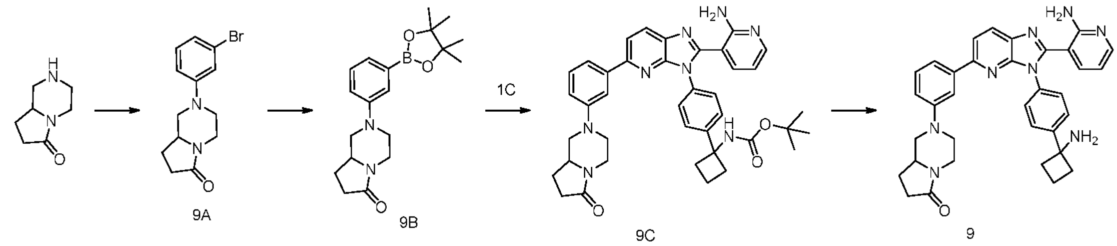


9A 9B 9C 9

### Step A: Preparation of compound 9A

Hexahydropyrrolo[1,2-A]pyrazin-6-one hydrochloride (0.230 g), 1,3-dibromobenzene (0.774 g), tris(dibenzylideneacetone)dipalladium (0.150 g), 1,1'-binaphthyl-2,2'-diphemyl phosphine (0.153 g), sodium *tert*-butoxide (0.552 g) and toluene (7.5 mL) were added successively to a reaction flask under nitrogen atmosphere. After the addition was completed, the reaction solution was stirred at 100 °C. After the reaction was completed, the reaction solution was concentrated and subjected to column chromatography (petroleum ether/ethyl acetate = 4/6) to give compound 9A (0.100 g).

MS (ESI, $[M+H]^+$) *m/z:* 295.4296.3.

### Step B: Preparation of compound 9B

To a reaction flask were added potassium carbonate (0.249 g), bis(pinacolato)diboron (0.332 g), [1,1'-bis(diphenylphosphino)ferrocene]palladium dichloride (0.027 g), compound 9A (0.260 g), and *N,N*-dimethylformamide (20.00 mL) successively under nitrogen atmosphere. After the addition was completed, the reaction solution was stirred at 70 °C. After the reaction was completed, the reaction solution was concentrated and subjected to column chromatography (petroleum ether/ethyl acetate = 1/1) to give compound 9B (0.112 g).

MS (ESI, $[M+H]^+$) *m/z:* 343.5.

### Step C: Preparation of compound 9C

To a reaction flask were added compound 9B (0.268 g), compound 1C (0.145 g), potassium carbonate (0.079 g), tetrakis(triphenylphosphine)palladium (0.033 g), 1,4-dioxane (4 mL), and water (7.5 mL) successively under nitrogen atmosphere. After the addition was completed, the reaction solution was stirred at 100 °C. After the reaction was completed, the reaction solution was concentrated and subjected to column chromatography (dichloromethane/methanol = 93/7) to give compound 9C (0.130 g).

MS (ESI, $[M+H]^+$) *m/z:* 671.7.

### Step D: Preparation of compound 9

To the reaction flask were added compound 9C (0.130 g), dichloromethane (10 mL) and methanesulfonic acid (0.149 g) successively. After the addition was completed, the reaction solution was stirred at room temperature. After the reaction was completed, the reaction solution was concentrated and subjected to column chromatography (dichloromethane/methanol = 93/7) to give compound 9 (0.047 g).

MS (ESI, $[M+H]^+$) *m/z*: 571.5.

$^1H$ NMR (500 MHz, DMSO-$d_6$)δ 8.25 (d, *J* = 8.4 Hz, 1H), 8.09 - 7.95 (m, 2H), 7.74 - 7.60 (m, 3H), 7.53 - 7.41 (m, 3H), 7.31 (t, *J* = 7.9 Hz, 1H), 7.24 (dd, *J* = 7.6, 1.9 Hz, 1H), 7.14 - 7.02 (m, 1H), 6.98 (s, 2H), 6.43 (dd, *J* = 7.7, 4.8 Hz, 1H), 4.55 (s, 2H), 3.95 - 3.85 (m, 2H), 3.81 - 3.73 (m, 1H), 3.69 (dtd, *J* = 10.8, 7.1, 3.7 Hz, 1H), 2.93 (td, *J* = 12.6, 3.8 Hz, 1H), 2.61 (td, *J* = 12.2, 3.6 Hz, 1H), 2.49 - 2.42 (m, 2H), 2.35 - 2.21 (m, 4H), 2.16 (tdd, *J* = 13.9, 7.7, 3.8 Hz, 1H), 2.09 (ddd, *J* = 11.1, 5.2, 2.4 Hz, 1H), 1.75 (dtt, *J* = 11.0, 9.0, 6.2 Hz, 1H), 1.69 - 1.59 (m, 1H), 0.90 - 0.80 (m, 1H).

### Example 10: Preparation of Compound 10

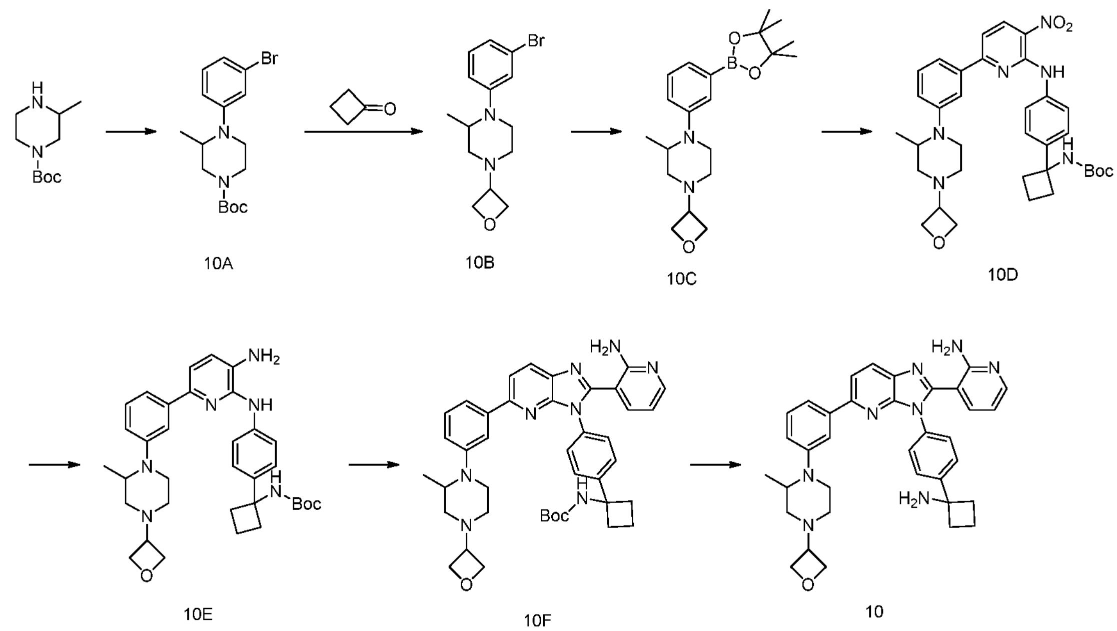

10A 10B 10C 10D

10E 10F 10

**Step** A: **Preparation of compound 10A**

To a reaction flask were added 1,3-dibromobenzene (2.356 g), *tert*-butyl 3-methylpiperazine-1-carboxylate (1.000 g), 2,2'-bis(diphenylphosphino)-1,1'-binaphthyl (0.280 g), tris(dibenzylideneacetone)dipalladium (0.320 g), sodium *tert*-butoxide (1.440 g) and toluene (30 mL) successively under nitrogen atmosphere. After the addition was completed, the reaction solution was stirred at 70 °C. After the reaction was completed, the reaction solution was concentrated and subjected to column chromatography (petroleum ether/ethyl acetate = 10/1) to give compound 10A (0.927 g).

MS (ESI, $[M+H]^+$) *m/z:* 355.3.

**Step B: Preparation of compound 10B**

To a reaction flask were added compound 10A (0.494 g), trifluoroacetic acid (6.440 g), 3-oxetanone (0.150 g), sodium cyanoborohydride (0.175 g), dichloromethane (2 mL) and methanol (2 mL) successively at room temperature. After the addition was completed, the reaction solution was stirred for reaction. After the reaction was completed, the reaction solution was concentrated and subjected to column chromatography (dichloromethane/methanol = 9/1) to give compound 10B (0.300 g).

**Step C: Preparation of compound 10C**

To a reaction flask were added compound 10B (0.300 g), potassium carbonate (0.275 g), bis(pinacolato)diboron (0.356 g), [1,1'-bis(diphenylphosphino)ferrocene]palladium dichloride (0.035 g) and *N,N*-dimethylformamide (34.00 mL) successively under nitrogen atmosphere. After the addition was completed, the reaction solution was stirred at 70 °C. After the reaction was completed, the reaction solution was concentrated and subjected to column chromatography (petroleum ether/ethyl acetate = 1/1) to give compound 10C (0.403 g).

MS (ESI, $[M+H]^+$) *m/z*:359.5.

**Step D: Preparation of compound 10D**

To a reaction flask were added compound 10C (0.394 g), *tert*-butyl (1-(4-(((6-chloro-3-nitropyridin-2-yl)amino)phenyl)cyclobutyl)carbamate (0.522 g), tetrakis(triphenylphosphine)palladium (0.059 g), saturated sodium bicarbonate solution (2.50 mL), ethanol (20 mL) and toluene (20 mL) successively under nitrogen atmosphere. After the addition was completed, the reaction solution was stirred at 100 °C. After the reaction was completed, the reaction solution was concentrated and subjected to column chromatography (petroleum ether/ethyl acetate = 1/1) to give compound 10D (0.579 g).

MS (ESI, $[M+H]^+$) *m/z*:615.6.

**Step E: Preparation of compound 10E**

To a reaction flask were added compound 10D (0.579 g), palladium/carbon (0.022 g), and tetrahydrofuran (15 mL) successively under hydrogen protection. After the addition was completed, the reaction solution was stirred for reaction at 30 °C. After the reaction was completed, the reaction solution was concentrated and subjected to column chromatography (petroleum ether/ethyl acetate = 1/2) to give compound 10E (0.401 g).

MS (ESI, $[M+H]^+$) *m/z:* 585.6.

**Step F: Preparation of compound 10F**

To a reaction flask were added compound 10E (0.100 g), 2-aminonicotine (0.021 g), sodium perborate tetrahydrate (0.014 g), methanol (5 mL) and acetic acid (2 mL) successively under nitrogen atmosphere. After the addition was completed, the reaction solution was stirred at 50 °C. After the reaction was completed, the reaction solution was concentrated and subjected to column chromatography (dichloromethane/methanol = 96/4) to give compound 10F (0.078 g).

MS (ESI, $[M+H]^+$) *m/z:* 687.5.

**Step G: Preparation of compound 10**

To a reaction flask were added compound 10F (0.050 g), methanesulfonic acid (0.035 g), and dichloromethane (5 mL) successively at room temperature. After the addition was completed, the reaction solution was stirred for reaction at room temperature. After the reaction was completed, the reaction solution was concentrated and subjected to column chromatography (dichloromethane/methanol = 99/1) to give compound 10 (33 mg).

MS (ESI, $[M+H]^+$) *m/z:* 587.5.

$^1$H NMR (500 MHz, $CDCl_3$) δ 8.07 (dd, *J* = 32.5, 6.3 Hz, 2H), 7.76 (d, *J* = 8.5 Hz, 1H), 7.64 - 7.47 (m, 4H), 7.38 - 7.28 (m, 2H), 7.20 (d, *J* = 7.8 Hz, 1H), 6.96 (d, *J* = 8.3 Hz, 2H), 6.63 (s, 2H), 6.41 (t, *J* = 6.5 Hz, 1H), 4.70 (dd, *J* = 15.0, 7.8 Hz, 2H), 4.62 (d, *J* = 6.7 Hz, 1H), 3.86 (s, 2H), 3.60 - 3.41 (m, 2H), 3.28 (d, *J* = 11.6 Hz, 1H), 3.18 (t, *J* = 10.8 Hz, 1H), 2.63 (dt, *J* = 18.5, 8.5 Hz, 3H), 2.43 (s, 2H), 2.30 (d, *J* = 10.9 Hz, 3H), 2.15 (p, *J* =9.1, 8.7 Hz, 1H), 1.88 - 1.78 (m, 1H), 1.11 (d, *J* = 6.4 Hz, 4H).

**Example 11: Preparation of Compound 11**

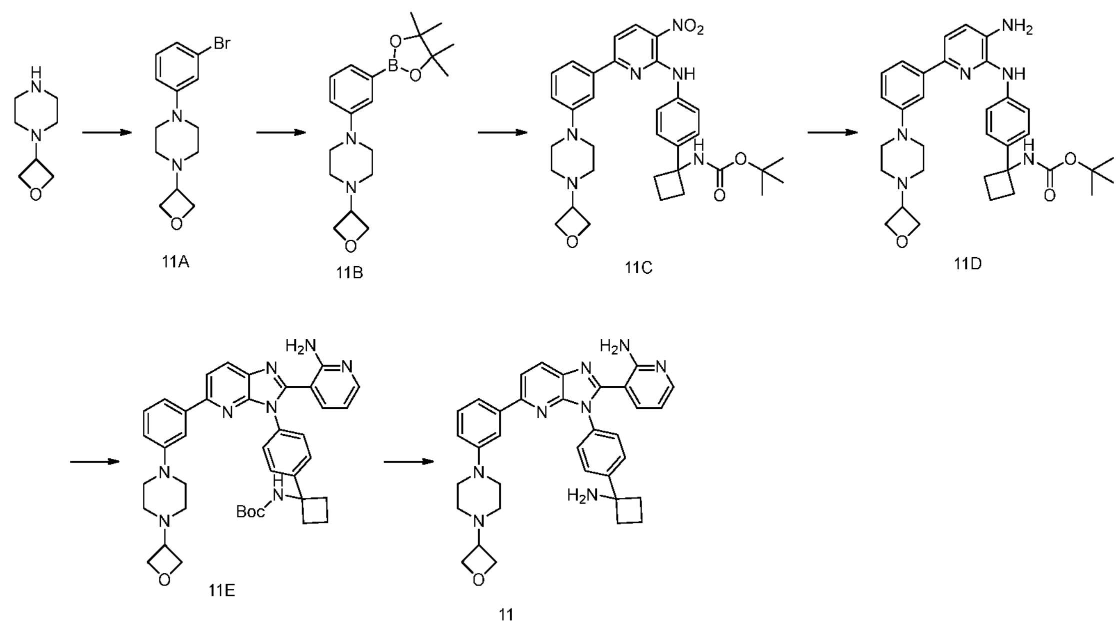

11A 11B 11C 11D

11E 11

Step A: **Preparation of compound 11A**

To a reaction flask were added 1,3-dibromobenzene (4.240 g), 1-(oxetan-3-yl)piperazine (1.278 g), 1,1'-binaphthyl-2,2'-bis-diphenylphosphine (0.420 g), sodium tert-butoxide (2.590 g), tris(dibenzylideneacetone)dipalladium (0.411 g) and toluene (22 mL) successively under nitrogen atmosphere. After the addition was completed, the reaction solution was stirred for reaction at 70 °C. After the reaction was completed, the reaction solution was concentrated and subjected to column chromatography (petroleum ether/ethyl acetate = 1/1) to give compound 11A (2.032 g).

MS (ESI, $[M+K]^+$) *m/z:* 335.4.

**Step B: Preparation of compound 11B**

To a reaction flask were added compound 11A (2.032 g), potassium *tert*-butoxide (2.010 g), bis(pinacolato)diboron (2.600 g), [1,1'-bis(diphenylphosphino)ferrocene]palladium dichloride (0.223 g) and *N,N*-dimethylformamide (34 mL) successively under nitrogen atmosphere. After the addition was completed, the reaction solution was stirred for reaction at 70 °C. After the reaction was completed, the reaction solution was concentrated and subjected to column chromatography (petroleum ether/ethyl acetate = 1/1) to give compound 11B (2.016 g).

MS (ESI, $[M+H]^+$) *m/z:* 345.4.

**Step C: Preparation of compound 11C**

To a reaction flask were added *tert*-butyl (1-(4-(((6-chloro-3-nitropyridin-2-yl)amino)phenyl)cyclobutyl)carbamate (0.568 g), compound 11B (0.576 g), tetrakis(triphenylphosphine)palladium (0.078 g), ethanol (15 mL), saturated sodium bicarbonate (3 mL) and toluene (15 mL) successively under nitrogen atmosphere. After the addition was completed, the reaction solution was stirred at 100 °C. After the reaction was completed, the reaction solution was concentrated and subjected to column chromatography (petroleum ether/ethyl acetate = 1/1) to give compound 11C (0.732 g).

MS (ESI, $[M+H]^+$) *m/z:* 601.3.

**Step D: Preparation of compound 11D**

Compound 11C (0.037 g) and palladium/carbon (0.001 g) were added to the reaction flask successively under hydrogen protection. After the addition was completed, the reaction solution was stirred for reaction at 30 °C. After the reaction was completed, the reaction solution was concentrated and subj ected to column chromatography (petroleum ether/ethyl acetate = 2/1) to give compound 11D (0.013 g).

MS (ESI, $[M+H]^+$) *m/z*: 571.5.

**Step E: Preparation of compound 11E**

To a reaction flask were added compound 11D (0.100 g), 2-aminonicotine (0.022 g), sodium perborate tetrahydrate (0.014 g), methanol (5 mL) and acetic acid (2 mL) successively under nitrogen atmosphere. After the addition was completed, the reaction solution was stirred for reaction at 50 °C. After the reaction was completed, the reaction solution was concentrated and subjected to column chromatography (dichloromethane/methanol = 99/1) to give compound 11E (50 mg).

MS (ESI, $[M+H]^+$) *m/z:* 673.6.

**Step F: Preparation of compound 11**

To a reaction flask were added compound 11E (50 mg), methanesulfonic acid (35 mg), and dichloromethane (3 mL) successively at room temperature, and the mixture was stirred for reaction. After the reaction was completed, the reaction solution was concentrated and subjected to column chromatography (dichloromethane/methanol = 99/1) to give compound 11 (28 mg).

MS (ESI, $[M+H]^+$) *m/z:* 573.5.

$^1$H NMR (500 MHz, $CDCl_3$) δ 8.11 (d, *J* = 8.3 Hz, 1H), 8.07 (tt, *J* = 5.9, 3.2 Hz, 1H), 7.77 (d, *J*= 8.3 Hz, 1H), 7.61 (t, *J* = 2.0 Hz, 1H), 7.59 - 7.54 (m, 2H), 7.54 - 7.46 (m, 2H), 7.45 - 7.41 (m, 2H), 7.34 (t, *J* = 7.8 Hz, 2H), 7.20 - 7.13 (m, 1H), 6.96 (dd, *J* = 8.3, 2.5 Hz, 1H), 6.63 (s, 2H), 6.45 - 6.36 (m, 1H), 4.69 (dt, *J* = 19.8, 6.4 Hz, 4H), 3.57 (p, *J* = 6.5 Hz, 1H), 3.29 (t, *J* = 5.0 Hz, 4H), 2.62 (ddd, *J* = 11.9, 8.9, 6.2 Hz, 2H), 2.52 (t, *J* = 4.9 Hz, 4H), 2.25 (q, *J* = 10.5, 9.1 Hz, 2H), 2.18 - 2.00 (m, 2H).

**Example 12: Preparation of Compound 12**

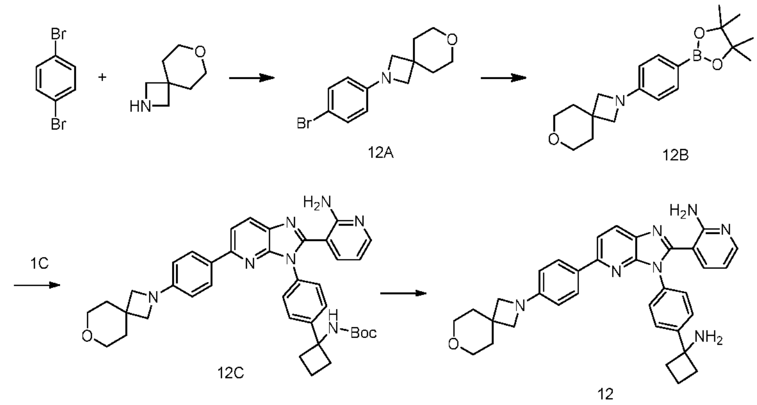


12A 12B 12C 12

### Step A: Preparation of compound 12A

7-Oxa-2-azaspiro[3.5]nonane (0.809 g), 1,4-dibromobenzene (1.500 g), tris(dibenzylideneacetone)dipalladium (0.582 g), 1,1'-binaphthyl-2,2'-diphemyl phosphine (0.594 g), sodium *tert*-butoxide (0.917 g) and toluene (20 mL) were added to a reaction flask successively under nitrogen atmosphere. After the addition was completed, the reaction solution was stirred at 120 °C. After the reaction was completed, the reaction solution was concentrated and subjected to column chromatography (petroleum ether/ethyl acetate = 17/3) to give compound 12A (1.257 g).

$^1$H NMR (500 MHz, DMSO-$d_6$) δ 7.27 - 7.29(m, 2H), 6.35 - 6.37(m, 2H), 3.56(s, 4H,), 3.53(t, *J* = 5.2Hz, 4H), 1.71(t, *J* = 5.2Hz, 4H).

### Step B: Preparation of compound 12B

To a reaction flask were added potassium acetate (1.263 g), bis(pinacolato)diboron (2.178 g), [1,1'-bis (diphenylphosphino)ferrocene]palladium dichloride (0.525 g), compound 12A (0.452 g) and dioxane (15 mL) successively under nitrogen atmosphere. After the addition was completed, the reaction solution was stirred at 110 °C. After the reaction was completed, the reaction solution was concentrated and subjected to column chromatography (petroleum ether/ethyl acetate = 9/1) to give compound 12B (1.500 g).

MS (ESI, $[M+H]^+$) *m/z:* 330.5.

### Step C: Preparation of compound 12C

To a reaction flask were added compound 12B (0.419 g), compound 1C (0.500 g), potassium carbonate (0.422 g), tetrakis(triphenylphosphine)palladium (0.058 g), 1,4-dioxane (10 mL), and water (5 mL) successively under nitrogen atmosphere. After the addition was completed, the reaction solution was stirred at 110 °C. After the reaction was completed, the reaction solution was concentrated and subjected to column chromatography (dichloromethane/methanol = 98:2) to give compound 12C (0.114 g).

MS (ESI, $[M+H]^+$) *m/z:* 658.7.

### Step D: Preparation of compound 12

To a reaction flask were added compound 12C (0.114 g), dichloromethane (10 mL) and methanesulfonic acid (0.081 g) successively. After the addition was completed, the reaction solution was stirred for reaction at room temperature. After the reaction was completed, 20% aqueous sodium hydroxide solution was added, and the reaction solution was stirred under an ice bath. The pH of the reaction solution was adjusted to 12, the phases were separated, the aqueous phase was extracted by dichloromethane, and the organic phases were combined, washed with saturated brine and dried over anhydrous sodium sulfate. The reaction solution was filtered, concentrated, and subjected to column chromatography (dichloromethane/methanol = 90/10) to give compound 12 (28 mg).

MS (ESI, $[M+H]^+$) *m/z:* 558.6.

$^1$H NMR (500 MHz, DMSO-$d_6$) δ7.96 - 7.98(m, 2H), 7.83-7.85(m, 2H), 7.61 -7.62(m, 1H), 7.47 - 7.48(m, 2H), 7.33 - 7.35(m, 2H), 7.02 - 7.04(m, 1H), 6.49(s, 2H), 6.40 - 6.42(m, 2H), 6.30 - 6.32(m, 1H), 3.61(s, 4H), 3.59(t, *J* = 5.2Hz, 4H), 3.59(q, *J* =9.25Hz, 2H), 2.18(q, J = 10.5Hz, 2H), 2.03 - 2.11(m, 2H), 1.76(t, *J* = 5.2Hz, 4H).

**Example 13: Preparation of Compound 13**

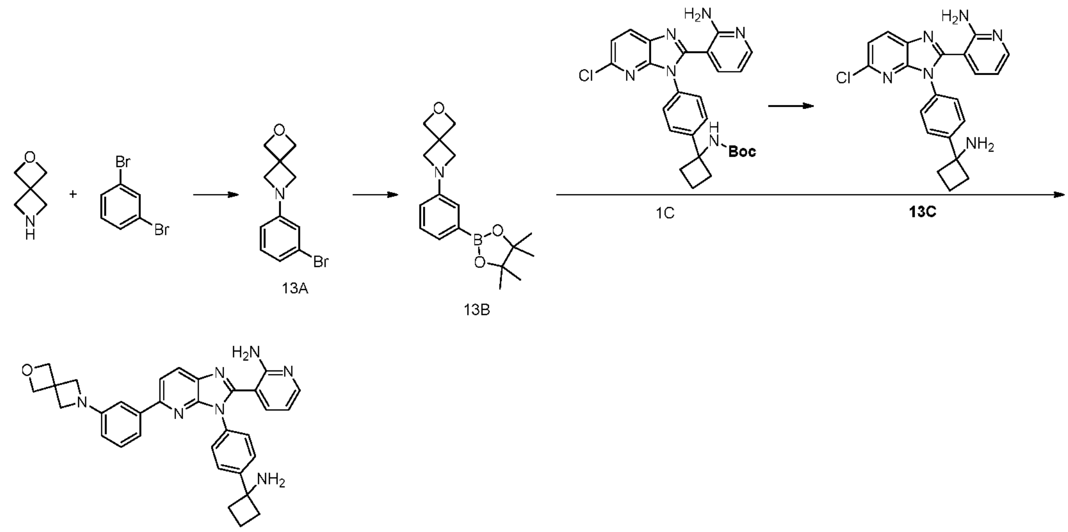


13A

13B

13

**Step A: Preparation of compound 13A**

To a reaction flask were added 2-oxa-6-azaspiro[3,3]heptane (1.00 g), *m*-dibromobenzene (2.49 g), (tris(dibenzylideneacetone)dipalladium (0.24), 1,1'-binaphthyl-2,2'-diphemyl phosphine (0.16 g), tetrahydrofuran (50 mL) and sodium *tert*-butoxide (2.03 g) successively, and the reaction solution was stirred at 80 °C under nitrogen atmosphere. After the reaction was completed, the reaction solution was filtered, concentrated, and subjected to column chromatography (petroleum ether/ethyl acetate = 10/1) to give compound 13A (1.24 g).
MS (ESI, $[M+H]^+$) *m/z*: 254.3.
$^1H$ NMR (500 MHz, DMSO-$d_6$): δ 7.09 (t, *J* = 8.0 Hz, 1H), 6.81 (dd, *J* = 1.0, 8.0 Hz, 1H), 6.56 (t, *J* = 2.0 Hz, 1H), 6.40 (dd, *J* = 2.0, 8.0 Hz, 1H), 4.70 (s, 4H), 3.97 (m, 4H).

**Step B: Preparation of compound 13B**

To a reaction flask containing compound 13A (1.13 g) were added bis(pinacolato)diboron (1.69 g), [1,1'-bis(diphenylphosphino)ferrocene]palladium dichloride dichloromethane complex (0.18 g), 1,4-dioxane (50 mL) and potassium acetate (1.30 g) successively, and after the addition was completed, the reaction solution was stirred for reaction at 120 °C under nitrogen atmosphere. After the reaction was completed, the reaction solution was filtered, concentrated and subjected to column chromatography (petroleum ether/ethyl acetate = 9/1) to give compound 13B (1.22 g).
MS (ESI, $[M+H]^+$) *m/z:* 302.2.
$^1H$ NMR (500 MHz, DMSO-$d_6$): δ 7.18 (t, *J* = 7.5 Hz, 1H), 7.01 (d, *J* = 7.0 Hz, 1H), 6.69 (d, *J* = 2.5 Hz, 1H), 6.55 (d, *J* = 2.0, 8.0 Hz, 1H), 4.70 (s, 4H), 3.95 (s, 4H), 1.28 (s, 12H).
$^{13}C$ NMR (125 MHz, DMSO-$d_6$): δ 151.34, 128.76, 123.98, 117.41, 115.17, 83.97, 80.38, 61.48, 38.96, 25.12.

**Step C: Preparation of compound 13C**

To a reaction flask containing compound 1C (0.495 g) were added methylene chloride (20 mL) and methanesulfonic acid (1.480 g) successively, and after the addition was completed, the reaction solution was stirred for reaction at room temperature. After the reaction was completed, the reaction solution was poured into water, the pH of the reaction solution was adjusted to 9 with an aqueous sodium hydroxide solution (20% w/w), and the reaction solution was extracted with dichloromethane, dried over anhydrous sodium sulfate, filtered and concentrated to give compound 13C (0.393 g).
MS (ESI, $[M+H]^+$) m/z: 391.2.

**Step D: Preparation of compound 13**

To a microwave tube were added compound 13B (0.20 g), compound 13C (0.30 g), potassium carbonate (0.28

g), tetrakis(triphenylphosphine)palladium (0.16 g), 1,4-dioxane (10 mL), and water (1 mL) successively. After the addition was completed, the reaction solution was purged with nitrogen gas, and reacted at 120 °C in microwave reactor for 2 h. After the reaction was completed, the reaction solution was filtered and concentrated. The reaction solution was subjected to column chromatography (dichloromethane/methanol = 20/1) to give compound 13 (135 mg).

MS (ESI, $[M+H]^+$) *m/z:* 530.7.

$^1H$ NMR (500 MHz, DMSO-$d_6$): δ 8.09 (d, *J* = 8.0 Hz, 1H), 7.96 (d, *J* = 4.5 Hz, 1H), 7.73 (d, *J* = 8.0 Hz, 1H), 7.55 (d, *J* = 8.0 Hz, 2H), 7.42 (d, *J* = 8.0 Hz, 2H), 7.36 (d, *J* = 7.5 Hz, 1H), 7.34 (t, *J* = 8.0 Hz, 1H), 7.12 (d, *J* = 7.5 Hz, 1H), 7.01 (s, 1H), 6.46-6.44 (m, 1H), 6.39 - 6.36 (m, 1H), 4.81 (s, 4H), 4.02 (s, 4H), 3.43-3.33 (m, 4H), 2.66 - 2.60 (m, 2H), 2.41 - 2.36 (m, 2H), 2.22 - 2.13 (m, 1H), 1.88 - 1.80 (m, 1H).

**Example 14: Preparation of Compound 14**

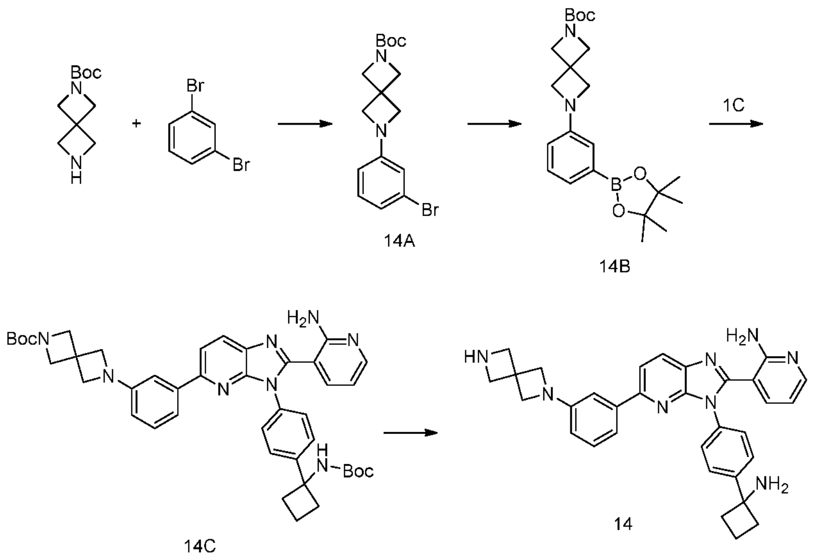

14A
14B
14C
14

**Step A: Preparation of compound 14A**

To a reaction flask were added *tert*-butyl 2,6-diazaspiro[3.3]heptane-2-carboxylate (0.10 g), *m*-dibromobenzene (0.45 g), tris(dibenzylideneacetone)dipalladium (32 mg), 1,1'-binaphthyl-2,2'-diphemyl phosphine (21 mg), tetrahydrofuran (20 mL) and sodium *tert*-butoxide (0.20 g) successively, and the reaction solution was stirred for reaction at 80 °C under nitrogen atmosphere. After the reaction was completed, the reaction solution was filtered, concentrated, and subjected to column chromatography (petroleum ether/ethyl acetate = 10/1) to give compound 14A (108 mg).

$^1H$ NMR (500 MHz, DMSO-$d_6$): δ 7.09 (t, *J* = 8.0 Hz, 1H), 6.81 (dd, *J* = 1.0, 8.0 Hz, 1H), 6.55 (t, *J* = 2.0 Hz, 1H), 6.39 (dd, *J* = 2.0, 8.0 Hz, 1H), 4.01 (s, 4H), 3.93 (s, 4H), 1.38 (s, 9H).

**Step B: Preparation of compound 14B**

To a reaction flask containing compound 14A(152 mg) were added bis(pinacolato)diboron (164 mg), [1,1'-bis(diphenylphosphino)ferrocene]palladium dichloride dichloromethane complex (50 mg), 1,4-dioxane (5 mL) and potassium acetate (127 mg) successively, and after the addition was completed, the reaction solution was stirred for reaction at 120 °C under nitrogen atmosphere. After the reaction was completed, the reaction solution was filtered, concentrated and subjected to column chromatography (petroleum ether/ethyl acetate = 9/1) to give compound 14B (53 mg).

MS (ESI, $[M+H]^+$) *m/z:* 401.5.

**Step C: Preparation of compound 14C**

To a microwave tube were added compound 14B (50 mg), compound 1C (67 mg), potassium carbonate (70 mg), tetrakis(triphenylphosphine)palladium (40 mg), 1,4-dioxane (4 mL), and water (0.5 mL) successively. After the addition was completed, the reaction solution was purged with nitrogen gas, and reacted at 120 °C in microwave reactor

for 2 h. After the reaction was completed, the reaction solution was filtered and concentrated. The reaction solution was subjected to column chromatography (dichloromethane/methanol = 10/1) to give compound 14C (80 mg).

MS (ESI, $[M+H]^+$) *m/z:* 729.7.

$^1$H NMR (500 MHz, DMSO-$d_6$): 6 8.23 (d, *J* = 8.5 Hz, 1H), 8.00 (dd, *J* = 1.5, 5.0 Hz, 1H), 7.92 (d, *J* = 8.5 Hz, 1H), 7.69 - 7.54 (m, 3H), 7.46-7.34 (m, 3H), 7.24 - 7.17 (m, 2H), 7.10 - 7.07 (m, 3H), 6.46 (dd, *J* = 1.5, 7.5 Hz, 1H), 6.33 (dd, *J* = 5.0, 7.5 Hz, 1H), 4.03 (s, 4H), 3.95 (s, 4H), 2.46 - 2.41 (m, 4H), 2.02 (brs, 1H), 1.83 (brs, 1H), 1.39 (s, 9H), 1.36 (s, 9H).

**Step D: Preparation of compound 14**

To a reaction flask were added compound 14C (69 mg), methanesulfonic acid (0.1 mL) and dichloromethane (10 mL) successively, and the reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 14 (25 mg).

HRMS (ESI, $[M+H]^+$) *m/z*: 529.2814.

$^1$H NMR (500 MHz, DMSO-$d_6$): δ 8.23 (d, *J* = 8.0 Hz, 1H), 8.01 (dd, *J* = 2.0, 5.0 Hz, 1H), 7.91 (d, *J* = 8.5 Hz, 1H), 7.64 (d, *J* = 8.5 Hz, 2H), 7.43 (d, *J* = 8.5 Hz, 2H), 7.34 (d, *J* = 8.0 Hz, 1H), 7.25 - 7.21 (m, 2H), 7.06 - 7.02 (m, 3H), 6.46 - 6.44 (m, 1H), 6.43 - 6.41 (m, 1H), 3.90 (s, 4H), 3.64 (s, 4H), 3.43 - 3.33 (m, 3H), 2.46 - 2.41 (m, 2H), 2.16 - 2.11 (m, 2H), 2.07 - 2.01 (m, 1H), 1.76 - 1.67 (m, 1H).

**Example 15: Preparation of Compound 15**

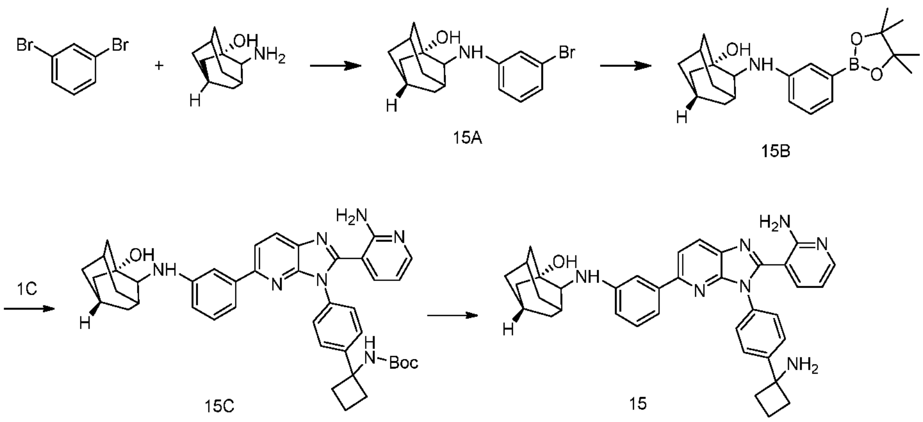


15A 15B 15C 15

**Step A: Preparation of compound 15A**

To a reaction flask were added (5*S*,7*S*)-2-aminoadamantan-1-ol (1.17 g), 1,3-dibromobenzene (1.65 g), tris(dibenzylideneacetone)dipalladium (0.64 g), 1,1'-binaphthyl-2,2'-diphemyl phosphine (0.65 g), sodium *tert*-butoxide (1.00 g) and toluene (10 mL) successively under nitrogen atmosphere. After the addition was completed, the reaction solution was stirred for reaction at 120 °C. After the reaction was completed, the reaction solution was concentrated and subjected to column chromatography (petroleum ether/ethyl acetate = 4/1) to give compound 15A (1.15 g).

MS (ESI, $[M+H]^+$) *m/z:* 322.2.

**Step B: Preparation of compound 15B**

To a reaction flask were added potassium acetate (0.795 g), bis(pinacolato)diboron (1.372 g), [1,1'-bis (diphenylphosphino)ferrocene]palladium dichloride (0.331 g), compound 15A (1.154 g) and dioxane (30 mL) successively under nitrogen atmosphere. After the addition was completed, the reaction solution was stirred at 110 °C. After the reaction was completed, the reaction solution was concentrated and subjected to column chromatography (petroleum ether/ethyl acetate = 4/1) to give compound 15B (0.750 g).

MS (ESI, $[M+H]^+$) *m/z:* 370.6.

**Step C: Preparation of compound 15C**

To a reaction flask were added compound 15B (0.750 g), compound 1C (0.612 g), potassium carbonate (0.495 g), tetrakis(triphenylphosphine)palladium (0.069 g), 1,4-dioxane (20 mL), and water (10 mL) successively under nitrogen atmosphere. After the addition was completed, the reaction solution was stirred for reaction at 110 °C. After the reaction was completed, the reaction solution was concentrated and subjected to column chromatography (dichloromethane/methanol = 3/2) to give compound 15C (1.215 g).

MS (ESI, $[M+H]^+$) *m/z:* 698.8.

**Step D: Preparation of compound 15**

To a reaction flask were added compound 15C (1.215 g), dichloromethane (20 mL) and methanesulfonic acid (0.694 g) successively. After the addition was completed, the reaction solution was stirred for reaction at room temperature. After the reaction was completed, 20% aqueous sodium hydroxide solution was added, and the reaction solution was stirred under an ice bath. The pH of the reaction solution was adjusted to 12, the phases were separated, the aqueous phase was extracted by dichloromethane, and the organic phases were combined, washed with saturated brine and dried over anhydrous sodium sulfate. The reaction solution was filtered, concentrated, and subjected to column chromatography (dichloromethane/methanol = 4/1) to give compound 15 (0.388 g).

MS (ESI, $[M+H]^+$) *m/z:* 598.7.

$^1H$ NMR (500 MHz, DMSO-$d_6$) δ 8.21(d, 1H), 7.99 - 8.00(m, 1H), 7.83(d, 1H), 7.62(d, 2H), 7.43(d, 2H), 7.28(s, 1H), 7.19 - 7.20(m, 1H), 7.12 - 7. 13(m, 2H), 6.96(s, 2H), 6.69(d, 1H), 6.39 - 6.42(s, 1H), 5.61(d, 1H), 2.41 - 2.46(m, 3H), 1.93 - 2.16(m, 9H), 1.63 - 1.77(m, 7H), 1.30(d, 2H).

**Example 16: Preparation of Compound 16**

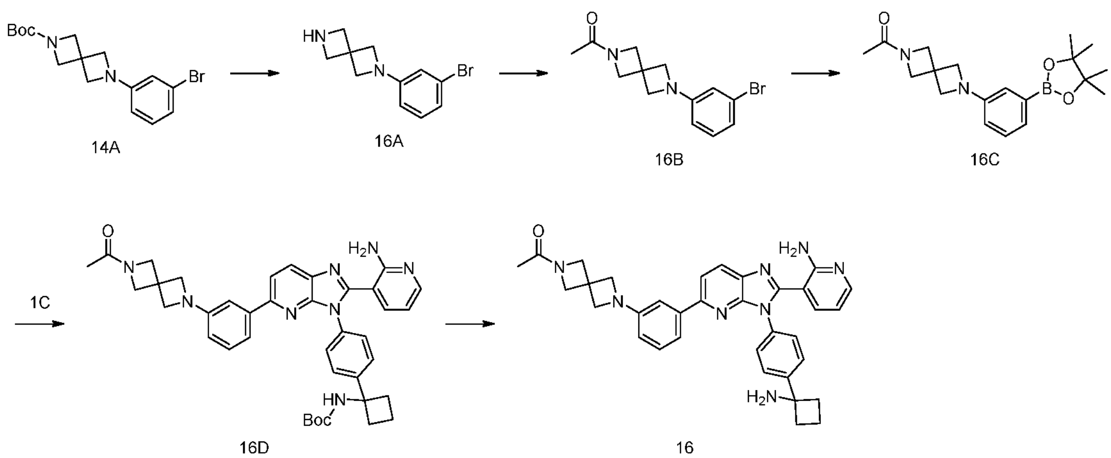

14A
16A
16B
16C
16D
16

**Step A: Preparation of compound 16A**

Compound 14A(1.00 g) was weighed out and added to a reaction flask, methylene chloride (100 mL) was added, the mixture was stirred and dissolved, followed by addition of methanesulfonic acid (0.82 g), and after the addition was completed, the reaction solution was stirred for reaction at room temperature. After the reaction was completed, triethylamine (2.5 mL) was added to terminate the reaction. Dichloromethane was added to adjust the volume of the reaction solution to 120 mL to obtain a solution of compound 16A. The reaction solution was not concentrated and was directly fed for the next step.

**Step B: Preparation of compound 16B**

A solution of compound 16A (35 mL) was added to the acetic anhydride (169 mg), and after the addition was completed, the reaction solution was stirred for reaction at room temperature. After the reaction was completed, the reaction solution was concentrated and subjected to column chromatography (dichloromethane/methanol = 96/4) to give compound 16B (219 mg).

MS (ESI, $[M+H]^+$) *m/z:* 295.1.

$^1$H NMR (500 MHz, $CDCl_3$): δ 7.06 (t, *J*= 8.0 Hz, 1H), 6.90 - 6.88 (m, 1H), 6.57 (d, *J*= 4.0 Hz, 1H), 6.37 - 6.34 (m, 1H), 4.29 (s, 2H), 4.16 (s, 2H), 3.99 (s, 4H), 1.88 (s, 3H).

### Step C: Preparation of compound 16C

To a reaction flask containing compound 16B (210 mg) were added bis(pinacolato)diboron (271 mg), [1,1'-bis(diphenylphosphino)ferrocene]palladium dichloride dichloromethane complex (29 mg), 1,4-dioxane (20 mL) and potassium acetate (209 mg) successively, and after the addition was completed, the reaction solution was stirred for reaction at 120 °C under nitrogen atmosphere. After the reaction was completed, the reaction solution was filtered, concentrated and subjected to column chromatography (dichloromethane/methanol = 98/2) to give compound 16C (185 mg).

MS (ESI, $[M+H]^+$) *m/z:* 343.3.

### Step D: Preparation of compound 16D

To a microwave tube were added compound 16C (140 mg), compound 1C (276 mg), potassium carbonate (239 mg), tetrakis(triphenylphosphine)palladium (75 mg), 1,4-dioxane (10 mL), and water (1 mL) successively. After the addition was completed, the reaction solution was purged with nitrogen gas, and reacted at 120 °C in microwave reactor for 2 h. After the reaction was completed, the reaction solution was filtered and concentrated. The reaction solution was subjected to column chromatography (dichloromethane/methanol = 96/4) to give compound 16D (280 mg).

MS (ESI, $[M+H]^+$) *m/z:* 671.7.

### Step E: Preparation of compound 16

To a reaction flask were added compound 16D (260 mg), methanesulfonic acid (0.25 mL) and dichloromethane (20 mL) successively, and the reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 16 (125 mg).

MS (ESI, $[M+H]^+$) *m/z:* 571.6.

$^1$H NMR (500 MHz, $CDCl_3$): 8.11-8.04 (d, *J* = 8.5 Hz, 1H), 8.06 - 7.96 (dd, *J* = 1.0, 4.5 Hz, 1H), 7.76 - 7.67 (d, *J* = 8.5 Hz, 1H), 7.65 - 7.48 (d, *J* = 8.5 Hz, 2H), 7.43 - 7.35 (m, 3H), 7.26 - 7.25 (m, 1H), 7.15-6.98 (m, 2H), 6.61 - 6.57 (m, 2H), 6.44-6.32 (m, 1H), 6.39-6.25 (m, 1H), 4.29-4.26 (m, 2H), 4.20-4.16 (m, 2H), 4.05 - 3.92 (m, 4H), 2.69 - 2.64 (m, 2H), 2.58 - 2.52 (m, 2H), 2.46 - 2.26 (m, 3H), 1.90 - 1.86 (m, 4H).

### Example 17: Preparation of Compound 17

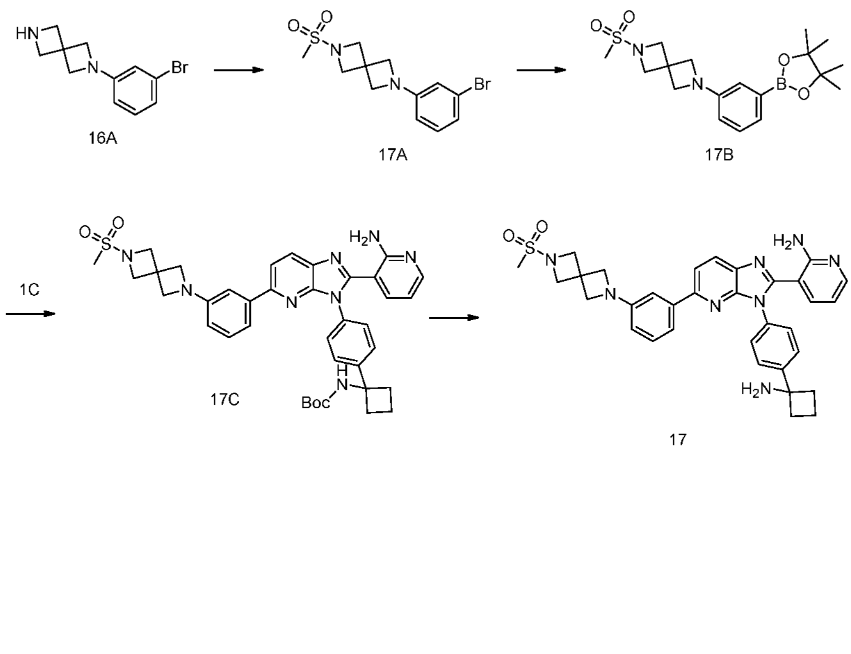


16A 17A 17B 17C 17

**Step** A: **Preparation of compound** 17A

A solution of compound 16A (35 mL) was added to the methanesulfonyl chloride (189 mg), after the addition was completed, the reaction solution was stirred for reaction at room temperature. After the reaction was completed, the reaction solution was concentrated and subjected to column chromatography (petroleum ether/ethyl acetate = 3/1) to give compound 17A (227 mg).

MS (ESI, $[M+H]^+$) *m*/*z*: 331.1.

$^1$H NMR (500 MHz, $CDCl_3$): δ 7.06 (t, *J*= 3.0 Hz, 1H), 6.90 - 6.88 (m, 1H), 6.57 (t, J = 2.0 Hz, 1H), 6.36 - 6.34 (m, 1H), 4.10 (s, 4H), 3.98 (s, 4H), 2.88 (s, 3H).

**Step B: Preparation of compound 17B**

To a reaction flask containing compound 17A (220 mg) were added bis(pinacolato)diboron (253 mg), [1,1'-bis(diphenylphosphino)ferrocene]palladium dichloride dichloromethane complex (27 mg), 1,4-dioxane (20 mL) and potassium acetate (196 mg) successively, and after the addition was completed, the reaction solution was stirred for reaction at 120 °C under nitrogen atmosphere. After the reaction was completed, the reaction solution was filtered, concentrated and subjected to column chromatography (petroleum ether/ethyl acetate = 1/1) to give compound 17B (157 mg).

MS (ESI, $[M+H]^+$) *m*/*z:* 379.3.

**Step C: Preparation of compound 17C**

To a microwave tube were added compound 17B (132 mg), compound 1C (223 mg), potassium carbonate (193 mg), tetrakis(triphenylphosphine)palladium (81 mg), 1,4-dioxane (10 mL), and water (1 mL) successively. After the addition was completed, the reaction solution was purged with nitrogen gas, and reacted at 120 °C in microwave reactor for 2 h. After the reaction was completed, the reaction solution was filtered, concentrated and subjected to column chromatography (dichloromethane/methanol = 98/2) to give compound 17C (146 mg).

MS (ESI, $[M+H]^+$) *m*/*z:* 707.7.

**Step D: Preparation of compound 17**

To a reaction flask were added compound 17C (150 mg), methanesulfonic acid (0.15 mL) and dichloromethane (15 mL) successively, and the reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 95/5) to give compound 17 (47 mg).

HRMS (ESI, $[M+H]^+$) *m*/*z:* 607.2606.

$^1$H NMR (500 MHz, $CDCl_3$): 8.05 - 7.91 (m, 2H), 7.69 - 7.60 (m, 1H), 7.52 - 7.41 (m, 2H), 7.37 - 7.26 (m, 3H), 7.23 - 7.20 (m, 1H), 7.09 - 6.93 (m, 2H), 6.53, 6.44 (s, 2H), 6.42 - 6.18 (m, 2H), 4.04, 4.02 (s, 4H), 3.96, 3.85 (s, 4H), 2.81, 2.80 (s, 3H), 2.58 - 2.36 (m, 3H), 2.22 - 2.02 (m, 3H).

**Example 18: Preparation of Compound 18**

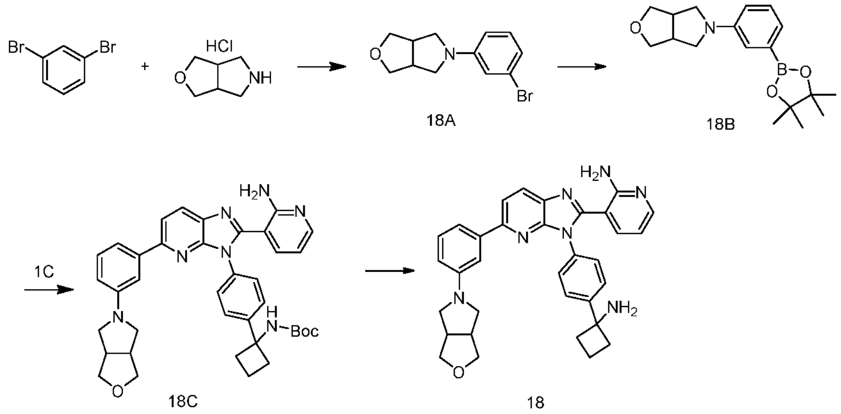


18A 18B 18C 18

**Step** A: **Preparation of compound 18A**

To a reaction flask were added hexahydro-1*H*-furo[3,4-c]pyrrole hydrochloride (0.824 g), 1,3-dibromobenzene (1.300 g), tris(dibenzylideneacetone)dipalladium (0.505 g), 1,1'-binaphthyl-2,2'-diphemyl phosphine (0.515 g), sodium *tert*-butoxide (0.794 g) and toluene (10 mL) successively under nitrogen atmosphere. After the addition was completed, the reaction solution was stirred for reaction at 120 °C. After the reaction was completed, the reaction solution was concentrated and subjected to column chromatography (petroleum ether/ethyl acetate = 19/1) to give compound 18A (0.452 g).

MS (ESI, $[M+H]^+$) *m/z:* 268.4.

**Step B: Preparation of compound 18B**

To a reaction flask were added potassium acetate (0.496 g), bis(pinacolato)diboron (0.809 g), [1,1'-bis (diphenylphosphino)ferrocene]palladium dichloride (0.206 g), compound 18A (0.452 g) and dioxane (20 mL) successively under nitrogen atmosphere. After the addition was completed, the reaction solution was stirred for reaction at 110 °C. After the reaction was completed, the reaction solution was concentrated and subjected to column chromatography (petroleum ether/ethyl acetate = 19/1) to give compound 18B (0.490 g).

MS (ESI, $[M+H]^+$) *m/z:* 316.5.

**Step C: Preparation of compound 18C**

To a reaction flask were added compound 18B (0.210 g), compound 1C (0.327 g), potassium carbonate (0.276 g), tetrakis(triphenylphosphine)palladium (0.038 g), 1,4-dioxane (15 mL), and water (7.5 mL) successively under nitrogen atmosphere. After the addition was completed, the reaction solution was stirred for reaction at 110 °C. After the reaction was completed, the reaction solution was concentrated and subjected to column chromatography (dichloromethane/methanol = 1/1) to give compound 18C (0.400 g).

MS (ESI, $[M+H]^+$) *m/z:* 644.8.

**Step D: Preparation of compound 18**

To the reaction flask were added compound 18C (0.400 g), dichloromethane (10 mL) and methanesulfonic acid (0.149 g) successively. After the addition was completed, the reaction solution was stirred for reaction at room temperature. After the reaction was completed, 20% aqueous sodium hydroxide solution was added, and the reaction solution was stirred under an ice bath. The pH of the reaction solution was adjusted to 12, the phases were separated, the aqueous phase was extracted by dichloromethane, and the organic phases were combined, washed with saturated brine and dried over anhydrous sodium sulfate. The reaction solution was filtered, concentrated, and subjected to column chromatography (dichloromethane/methanol = 7/3) to give compound 18 (0.285 g).

MS (ESI, $[M+H]^+$) *m/z:* 544.7.

$^1H$ NMR (500 MHz, DMSO-$d_6$) $\delta$ 8.43(d, 1H), 8.11 - 8.21(m, 3H), 8.00(d, 1H), 7.92(d, 1H), 7.80(d, 2H), 7.71(d, 2H), 7.48 - 7.58(m, 2H), 6.98(t, 1H), 3.70 - 3.88(m, 6H,), 3.21(s, 2H), 2.65 - 2.68(m, 4H), 2.21 - 2.28(m, 1H), 1.82 - 1.90(m, 1H), 1.17 - 1.29 (m, 2H).

**Example 19: Preparation of Compound 19**

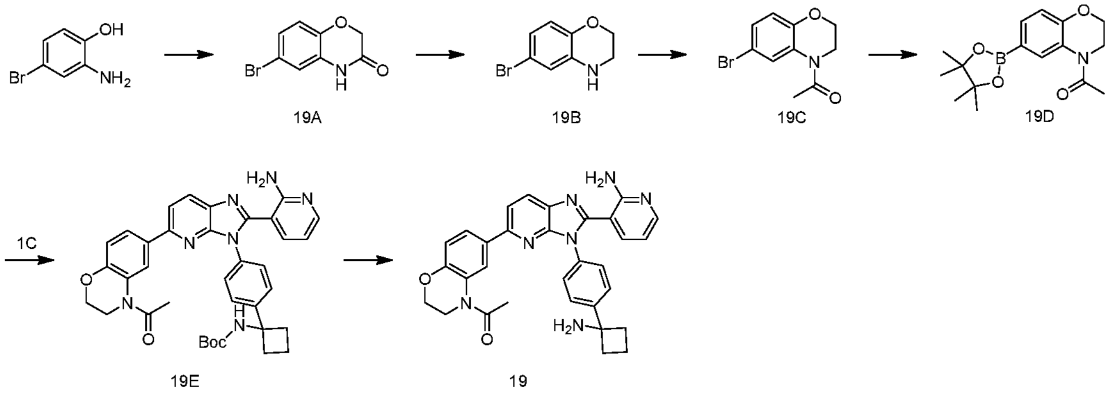


19A 19B 19C 19D

19E 19

**Step A: Preparation of compound 19A**

In a reaction flask, a solution of 2-chloroacetyl chloride (3.24 g) in tetrahydrofuran (10 mL) was slowly added dropwise to a reaction solution of 2-amino-4-bromophenol (3.60 g) and sodium carbonate (3.22 g) in tetrahydrofuran (60 mL) under nitrogen atmosphere at 0 °C. After the addition was completed, the reaction solution was stirred at room temperature. Potassium carbonate (5.29 g) was added to the reaction solution, and the reaction solution was stirred for reaction at 66 °C, filtered, concentrated, and subjected to column chromatography (petroleum ether/ethyl acetate = 3/1) to give compound 19A (3.4 g).
MS (ESI, $[M-H]^-$) *m/z*: 226.4.

**Step B: Preparation of compound 19B**

In a reaction flask, a solution (15 mL) of 2 M borane in toluene-dimethylsulfide was added slowly under nitrogen atmosphere at 0 °C to a solution of compound 19A (2.28 g) in tetrahydrofuran (10 mL). After the addition was completed, the mixture was stirred at room temperature and reacted at 66 °C. After the reaction was completed, the reaction was quenched with a saturated aqueous sodium hydroxide solution, the reaction solution was extracted with ethyl acetate, and the organic phases were combined, washed with saturated brine, dried over anhydrous sodium sulfate, filtered, concentrated, and subjected to column chromatography (petroleum ether/ethyl acetate = 4/1) to give compound 19B (2.10 g). The reaction product from this step was used directly in the next step.

**Step C: Preparation of compound 19C**

In a reaction flask, acetyl chloride (0.733 g) was slowly added to a solution of compound 19B (2.000 g), *N,N*-diisopropylethylamine (1.208 g) in tetrahydrofuran (12 mL) under nitrogen atmosphere a 0 °C. After the addition was completed, the reaction solution was stirred at room temperature. After the reaction was completed, the reaction was quenched with saturated sodium carbonate solution, the reaction solution was extracted with ethyl acetate, and the organic phases were combined, washed with saturated brine, dried over anhydrous sodium sulfate, filtered, concentrated, and subjected to column chromatography (petroleum ether/ethyl acetate = 95/5) to give compound 19C (2.200 g). The reaction product from this step was used directly in the next step.

**Step D: Preparation of compound 19D**

To a reaction flask were added [1,1'-bis(diphenylphosphino)ferrocene]palladium dichloride (0.702 g), compound 19C (2.200 g), bis(pinacolato)diboron (4.360 g), potassium acetate (1.686 g) and dioxane (8 mL) successively under nitrogen atmosphere; after the addition was completed, the reaction solution was reacted at 110 °C. After the reaction was completed, the reaction solution was extracted with ethyl acetate, and organic phases were combined, washed with saturated brine, dried over anhydrous sodium sulfate, filtered, concentrated, and subjected to column chromatography (petroleum ether/ethyl acetate = 90/10) to give compound 19D (2.410 g).
MS (ESI, $[M+H]^+$) *m/z:* 304.5.

**Step E: Preparation of compound 19E**

To a reaction flask were added compound 19D (324 mg), compound 1C (350 mg), potassium carbonate (197 mg), tetrakis(triphenylphosphine)palladium (82 mg), and dioxane (10 mL) successively under nitrogen atmosphere, and after the addition was completed, the reaction solution was reacted at 110 °C. After the reaction was completed, the reaction solution was concentrated and subjected to column chromatography (petroleum ether/ethyl acetate = 95/5) to give compound 19E (320 mg).

MS (ESI, $[M+H]^+$) *m/z:* 632.7.

**Step F: Preparation of compound 19**

To the reaction flask were added compound 19E (320 mg), dichloromethane (10 mL) and methanesulfonic acid (48.7 mg) successively, and the reaction solution was stirred for reaction at room temperature. The raw materials were reacted completely. The reaction was quenched with saturated aqueous sodium carbonate (30 mL), the reaction solution was extracted with dichloromethane, and the organic phases were combined, washed with saturated brine, dried over anhydrous sodium sulfate, filtered, concentrated, and subjected to column chromatography (dichloromethane/methanol = 94/6) to give compound 19 (120 mg).

MS (ESI, $[M+H]^+$) *m/z:* 532.7.

$^1H$ NMR (500 MHz, DMSO-$d_6$): $\delta$ 8.22 (d, *J* =8.0Hz, 1H), 8.00 (s, 1H), 7.87 (br, 1H), 7.63 - 7.62 (m, 2H), 7.45 - 7.44 (m, 2H), 7.21(d, *J*=7.5Hz, 1H), 7.00 - 7.69 (m, 3H), 6.41 (t, *J*=5.0Hz, 1H), 4.29 (s, 2H), 3.88 (s, 2H), 2.50 - 2.43 (m, 2H), 2.24 (s, 3H), 2.19 - 2.15 (m.2H), 2.06 - 2.04 (m, 1H), 1.75 - 1.72 (m, 1H).

**Example 20: Preparation of Compound 20**

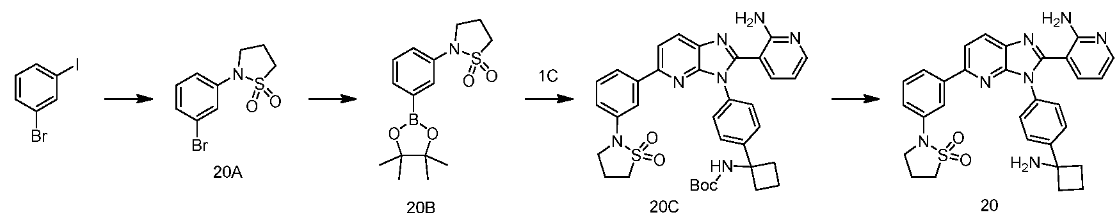


20A 20B 20C 20

**Step A: Preparation of compound 20A**

To a reaction flask were added 1-bromo-3-iodobenzene (1.180 g), isothiazole-1,1-dioxide (0.720 g), cuprous iodide (0.159 g), potassium carbonate (1.153 g), (1*S*,2*S*)-*N*1,*N*2-dimethylcyclohexyl-1,2-diamine (0.119 g), and dimethyl sulfoxide (10 mL) successively under nitrogen atmosphere, and the reaction solution was reacted at 120 °C. After the reaction was completed, the reaction solution was concentrated and subjected to column chromatography (petroleum ether/ethyl acetate = 90/10) to give compound 20A (640 mg).

MS(ESI, $[M+H]^+$) *m/z:* 276.2.

**Step B: Preparation of compound 20B**

To a reaction flask were added [1,1'-bis(diphenylphosphino)ferrocene]palladium dichloride (151 mg), compound 20A(640 mg), bis(pinacolato)diboron (1177 mg), potassium acetate (455 mg) and dioxane (8 mL) successively under nitrogen atmosphere; after the addition was completed, the reaction solution was reacted at 110 °C. After the reaction was completed, the reaction solution was concentrated and subjected to column chromatography (petroleum ether/ethyl acetate = 80/20) to give compound 20B (620 mg).

MS(ESI, $[M+H]^+$)*m/z*: 324.5.

**Step C: Preparation of compound 20C**

To a reaction flask were added tetrakis(triphenylphosphine)palladium (99 mg), compound 1C (420 mg), compound 20B (415 mg), potassium carbonate (236 mg), dioxane (5 mL) and water (0.5 mL) successively, and the reaction solution was heated at 120 °C. After the reaction was completed, the reaction solution was concentrated and subjected to column chromatography (petroleum ether/ethyl acetate = 60/40) to give compound 20C (380 mg).

MS(ESI, $[M+H]^+$) *m/z*: 652.5.

**Step D: Preparation of compound 20**

To the reaction flask were added compound 20C (150 mg), dichloromethane (6 mL) and methanesulfonic acid (133 mg) successively. After the addition was completed, the reaction solution was reacted at room temperature. After the reaction was completed, the pH of the reaction solution was adjusted to be neutral with saturated aqueous sodium bicarbonate, the reaction solution was extracted with dichloromethane, the organic phases were combined, washed with saturated brine, dried over anhydrous sodium sulfate, filtered, concentrated, and subjected to column chromatography (dichloromethane/methanol = 97/3) to give compound 20 (27 mg).

MS(ESI, $[M+H]^+$)*m/z*: 552.5.

$^1$H NMR (500 MHz, DMSO-$d_6$): δ 8.29 (d, *J* = 8.0 Hz, 1H), 8.01 (dd, *J* = 3.0 Hz, 6.0Hz, 1H), 7.97 (d, *J*= 8.5 Hz, 1H), 7.88 (s, 1H), 7.77(d, *J* = 7.5 Hz, 1H), 7.64(d, *J* =9.0 Hz, 1H), 7.50-7.45(m, 3H), 7.28 - 7.23(m, 2H), 6.96(br, 2H), 6.44(q, *J*= 7.5 Hz, 1H), 3.81(t, *J*= 7.5 Hz, 2H), 3.54(t, *J*= 7.5 Hz, 2H), 2.43(t, *J*= 7.5 Hz, 1H), 2.34(s, 2H), 2.26 - 2.21(m, 2H), 2.09(m, 1H), 1.77 - 1.75(m, 1H).

**Example 21: Preparation of Compound 21**

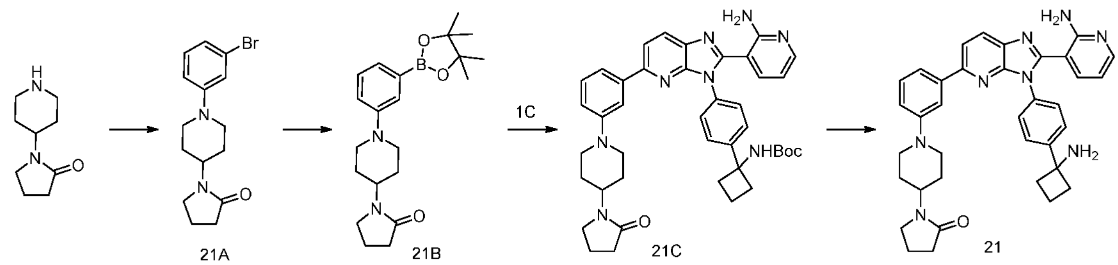


21A 21B 21C 21

**Step** A: **Preparation of compound 21A**

To a reaction flask were added sodium *tert*-butoxide (0.179 g), 1,1'-binaphthyl-2,2'-diphemyl phosphine (0.040 g), tris(dibenzylideneacetone)dipalladium (0.039 g), 1,3-dibromobenzene (0.200 g), 1-(piperidin-4-yl)pyrrolidin-2-one hydrochloride (0.174 g) and toluene (10 mL) successively, and the reaction solution was stirred for reaction at 80 °C under nitrogen atmosphere. After the reaction was completed, the reaction solution was concentrated, added with 5 mL of saturated brine and extracted with ethyl acetate (10 mL × 3), and the organic phases were combined and dried over anhydrous sodium sulfate. The reaction solution was filtered and concentrated. The reaction solution was subjected to column chromatography (dichloromethane/methanol = 60/1) to give compound 21A (0.270 g).

MS (ESI, $[M+H]^+$) *m/z:* 323.4.

**Step B: Preparation of compound 21B**

To a reaction flask were added compound 21A (0.270 g), potassium acetate (0.270 g), [1,1'-bis(diphenylphosphino)ferrocene]palladium dichloride dichloromethane complex (0.037 g), bis(pinacolato)diboron (0.280 g), 1,4-dioxane (25 mL) and water (5 mL) successively, and the reaction solution was stirred for reaction at 100 °C under nitrogen atmosphere. After the reaction was completed, the reaction solution was concentrated, added with saturated brine and extracted with ethyl acetate, and the organic phases were combined and dried over anhydrous sodium sulfate. The reaction solution was filtered, concentrated, and subjected to column chromatography (dichloromethane/methanol = 60/1) to give compound 21B (0.300 g).

MS (ESI, $[M+H]^+$) *m/z:* 371.6.

**Step C: Preparation of compound 21C**

To a reaction flask were added compound 1C (2.40 g), compound 21B (0.30 g), potassium carbonate (0.17 g), tetrakis(triphenylphosphine)palladium (0.057 g), 1,4-dioxane (25 mL) and water (5 mL) successively, and the reaction solution was stirred for reaction at 100 °C under nitrogen atmosphere. After the reaction was completed, the reaction solution was concentrated, added with saturated brine and extracted with ethyl acetate, and the organic phases were

combined and dried over anhydrous sodium sulfate. The reaction solution was filtered, concentrated, and subjected to column chromatography (dichloromethane/methanol = 65/1) to give compound 21C (0.25 g).
MS (ESI, M+H]$^+$) *m/z*: 699.8.

**Step D: Preparation of compound 21**

To a reaction flask were added compound 21C (0.25 g), dichloromethane (25 mL) and methanesulfonic acid (0.30 g) successively. The reaction solution was stirred at 30 °C for reaction; after the reaction was completed, the pH of the reaction solution was adjusted to be alkaline, the reaction solution was added with saturated brine and extracted with dichloromethane, and the organic phases were combined, and dried over anhydrous sodium sulfate. The reaction solution was filtered, concentrated, and subjected to column chromatography (dichloromethane/methanol = 10/1) to give compound 21 (0.19 g).
HRMS (ESI, [M+H]$^+$) *m/z:* 599.3237.
$^1$H NMR (500 MHz, $CDCl_3$) δ 7.99 (dd, *J* = 11.1, 8.5 Hz, 1H), 7.90 (t, *J* = 4.6 Hz, 1H), 7.64 (t, *J* = 8.2 Hz, 1H), 7.57 (d, *J* = 8.2 Hz, 2H), 7.48 (s, 1H), 7.47 - 7.22 (m, 4H), 7.01 (t, *J* = 7.6 Hz, 1H), 6.80 (dd, *J* = 28.6, 9.7 Hz, 1H), 6.55 (d, *J* = 15.2 Hz, 2H), 6.27 (ddd, *J* = 58.5, 7.7, 5.0 Hz, 1H), 4.05 (ddd, *J* = 16.5, 10.7, 3.8 Hz, 1H), 3.65 (t, *J* = 16.0 Hz, 2H), 3.28 (dt, *J* = 14.1, 6.9 Hz, 2H), 2.76 (dt, *J* = 24.5, 12.3 Hz, 2H), 2.62 - 2.46 (m, 4H), 2.44 - 2.22 (m, 4H), 1.99- 1.86 (m, 3H), 1.83 - 1.65 (m, 5H).

**Example 22: Preparation of Compound 22**

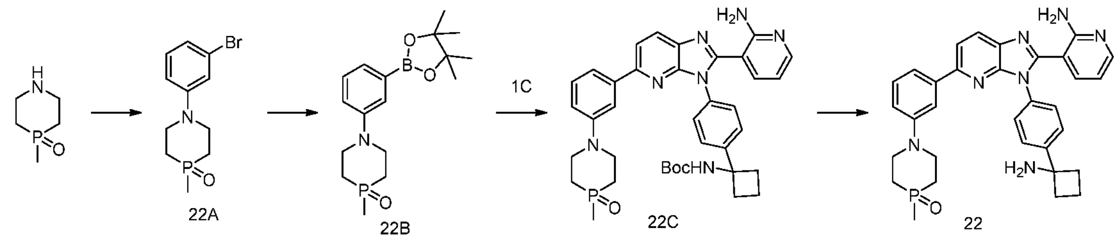

22A 22B 22C 22

**Step A: Preparation of compound 22A**

To a reaction flask were added sodium *tert*-butoxide (0.180 g), 1,1'-binaphthyl-2,2'-diphemyl phosphine (0.040 g), tris(dibenzylideneacetone)dipalladium (0.039 g), 1,3-dibromobenzene (0.200 g), 4-methyl-4-oxo-1,4-azaphosphine (0.11 g) and toluene (10 mL) successively. The reaction solution was stirred for reaction at 80 °C under nitrogen atmosphere. After the reaction was completed, the reaction solution was concentrated, added with saturated brine and extracted with ethyl acetate, and the organic phases were combined and dried over anhydrous sodium sulfate. The reaction solution was filtered, concentrated, and subjected to column chromatography (dichloromethane/methanol = 60/1) to give compound 22A (0.230 g).
MS (ESI, [M+H]$^+$) *m/z:* 288.4.

**Step B: Preparation of compound 22B**

To a reaction flask were added compound 22A (0.23 g), potassium acetate (0.28 g), [1,1'-bis(diphenylphosphino)ferrocene]palladium dichloride dichloromethane complex (39 mg), bis(pinacolato)diboron (0.29 g), 1,4-dioxane (25 mL) and water (5 mL) successively. The reaction solution was stirred for reaction at 100 °C under nitrogen atmosphere. After the reaction was completed, the reaction solution was concentrated, condition with saturated brine and extracted with ethyl acetate, and the organic phases were combined and dried over anhydrous sodium sulfate. The reaction solution was filtered, concentrated, and subjected to column chromatography (dichloromethane/methanol = 60/1) to give compound 22B (0.28 g).
MS (ESI, [M+H]$^+$) *m/z:* 336.6.

**Step C: Preparation of compound 22C**

To a reaction flask were added compound 1C (2.30 g), compound 22B (0.28 g), potassium carbonate (0.16 g), tetrakis(triphenylphosphine)palladium (55 mg), 1,4-dioxane (25 mL) and water (5 mL) successively, and the reaction

solution was stirred for reaction at 100 °C under nitrogen atmosphere. After the reaction was completed, the reaction solution was concentrated, added with saturated brine and extracted with ethyl acetate, and the organic phases were combined and dried over anhydrous sodium sulfate. The reaction solution was filtered, concentrated, and subjected to column chromatography (dichloromethane/methanol = 65/1) to give compound 22C (0.24 g).

MS (ESI, $[M+H]^+$) *m/z:* 664.7.

**Step D: Preparation of compound 22**

To a reaction flask were added compound 22C (0.24 g), dichloromethane (25 mL) and methanesulfonic acid (0.30 g) successively. The reaction solution was stirred at 30 °C for reaction; after the reaction was completed, the pH of the reaction solution was adjusted to be alkaline, the reaction solution was added with saturated brine and extracted with dichloromethane, and the organic phases were combined, and dried over anhydrous sodium sulfate. The reaction solution was filtered, concentrated, and subjected to column chromatography (dichloromethane/methanol = 10/1) to give compound 22 (0.16 g).

HRMS (ESI, $[M+H]^+$) *m/z*: 564.2634.

$^1$H NMR (500 MHz, DMSO-$d_6$) δ 8.24 (d, *J* = 8.3 Hz, 1H), 8.02 (dd, *J* = 4.7, 1.6 Hz, 1H), 7.97 (d, *J* = 8.4 Hz, 1H), 7.64 (d, *J* = 8.4 Hz, 2H), 7.61 (s, 1H), 7.49 (d, *J* = 8.5 Hz, 2H), 7.38 (d, *J* = 7.6 Hz, 1H), 7.30 (t, *J* = 7.9 Hz, 1H), 7.26 (dd, *J* = 7.6, 1.6 Hz, 1H), 7.06 - 6.96 (m, 3H), 6.43 (dd, *J* = 7.6, 4.8 Hz, 1H), 4.48 (s, 2H), 4.03 - 3.85 (m, 2H), 3.57 - 3.46 (m, 2H), 2.49 - 2.43 (m, 2H), 2.28 - 2.20 (m, 2H), 2.13 - 2.03 (m, 1H), 1.88 (t, *J* = 13.6 Hz, 2H), 1.75 (ddt, *J* = 20.2, 14.2, 6.4 Hz, 3H), 1.53 (d, *J* = 13.0 Hz, 3H).

**Example 23: Preparation of Compound 23**

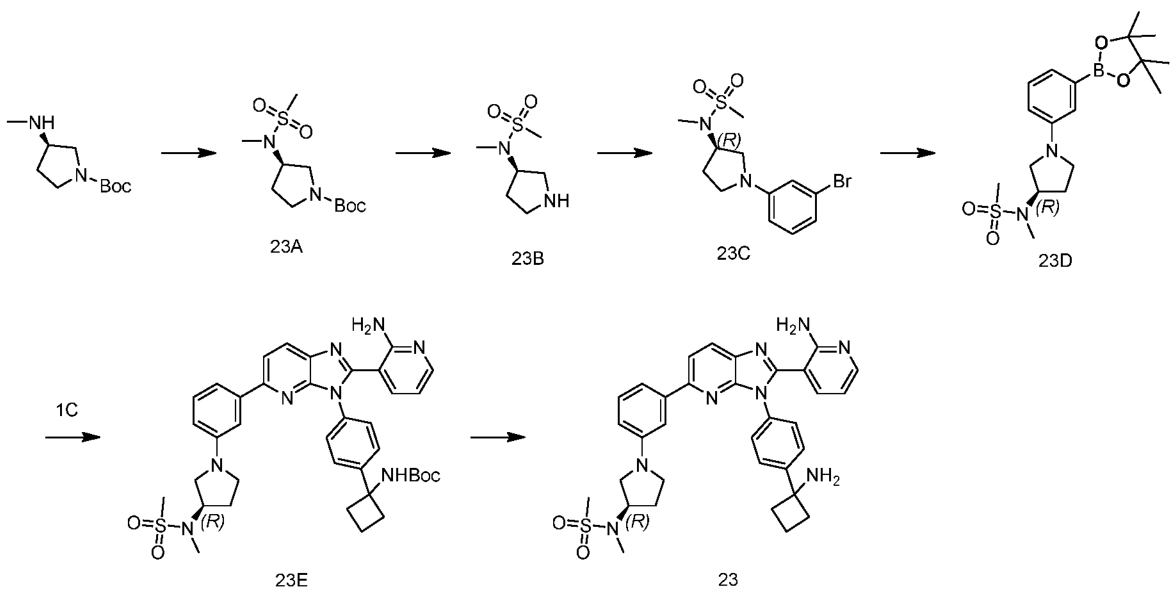

23A
23B
23C
23D
23E
23

**Step A: Preparation of compound 23A**

In a reaction flask, methanesulfonyl chloride (0.572 g) was added at 0 °C to a solution of *tert*-butyl (*R*)-3-(methylamine)pyrrole-1-carboxylate (1.000 g) and *N*,*N*-diisopropylethylamine (0.645 g) in dichloromethane (15 mL) under nitrogen atmosphere, and the reaction solution was stirred at room temperature overnight. After the reaction was completed, the reaction was quenched by saturated aqueous sodium carbonate solution, the reaction solution was extracted with dichloromethane, and the organic phases were combined, washed with saturated brine, dried over anhydrous sodium sulfate, filtered, concentrated, and subjected to column chromatography (petroleum ether/ethyl acetate = 1/1) to give compound 23A (1.160 g).

**Step B: Preparation of compound 23B**

In a reaction flask, a solution of 4 M hydrogen chloride in dioxane (4 mL) was added slowly to a solution of

compound 23A (590 mg) in dichloromethane (10 mL) under an ice-water bath. After the addition was completed, the reaction solution was stirred for reaction at room temperature. After the reaction was completed, the reaction solution was directly distilled under reduced pressure to remove the solvent to obtain compound 23B, which was used directly in the next step.

**Step C: Preparation of compound 23C**

1,1'-binaphthyl-2,2'-diphemyl phosphine (1320 mg), sodium tert-butoxide (204 mg), 1,3-dibromobenzene (500 mg) and dioxane (10 mL) were added successively to a reaction flask containing compound 23B. After the addition was completed, the reaction solution was stirred for reaction at 120 °C. After the reaction was completed, the reaction solution was concentrated and subjected to column chromatography (petroleum ether/ethyl acetate = 80/20) to give compound 23C (380 mg).

MS (ESI, $[M+H]^+$) *m/z:* 333.3.

**Step D: Preparation of compound 23D**

To a reaction flask were added [1,1'-bis(diphenylphosphino)ferrocene]palladium dichloride (93 mg), compound 23C (380 mg), bis(pinacolato)diboron (434 mg), potassium acetate (336 mg) and dioxane (8 mL) successively under nitrogen atmosphere. After the addition was completed, the reaction solution was reacted at 110 °C. After the reaction was completed, the reaction solution was concentrated and subjected to column chromatography (petroleum ether/ethyl acetate = 80/20) to give compound 23D (280 mg).

MS (ESI, $[M+H]^+$) *m/z:* 381.3.

**Step E: Preparation of compound 23E**

To a reaction flask were added tetrakis(triphenylphosphine)palladium (99 mg), compound 1C (420 mg), compound 23D (325 mg), potassium acetate (236 mg), dioxane (5 mL), and water (0.5 mL) successively. After the addition was completed, the reaction solution was stirred for reaction at 120 °C. After the reaction was completed, the reaction solution was concentrated and subjected to column chromatography (petroleum ether/ethyl acetate = 60/40) to give compound 23E (154 mg).

MS (ESI, $[M+H]^+$) *m/z:* 709.8.

**Step F: Preparation of compound 23**

To the reaction flask were added compound 23E (100 mg), dichloromethane (6 mL) and methanesulfonic acid (600 mg) successively. After the addition was completed, the reaction solution was stirred for reaction at room temperature. After the reaction was completed, the pH of the reaction solution was adjusted to be neutral by sodium bicarbonate, the reaction solution was extracted with dichloromethane, and the organic phase were combined, washed with saturated brine and dried over anhydrous sodium sulfate. The reaction solution was filtered, concentrated, and subjected to column chromatography (dichloromethane/methanol = 97/3) to give compound 23 (60 mg).

MS (ESI, $[M+H]^+$) *m/z:* 609.6.

$^1H$ NMR (500 MHz, DMSO-$d_6$): δ 8.23 (d, *J* =3.5Hz, 1H), 8.01 - 7.96(m, 2H), 7.62 (d, *J*=7.5Hz, 1H),7.44(d, *J*=7.5Hz, 2H), 7.32(d, *J*=7.0Hz, 2H), 7.27 - 7.22 (m, 3H), 7.01(s, 1H), 6.43(d, *J*=7.0Hz, 2H), 6.42-6.41(m, 1H),4.54(t, *J*=7.5Hz, 1H), 3.47(q, *J*=7.5Hz, 1H), 3.24(t, *J*=7.5Hz, 1H), 2.99(s, 3H), 2.78(s, 3H), 2.44 - 2.42(m, 2H), 2.22 - 2.20(s, 6H).

**Example 24: Preparation of Compound 24**

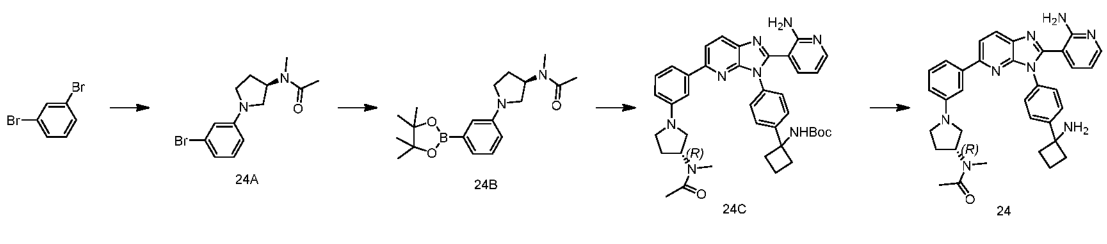

**Step A: Preparation of compound 24A**

To a reaction flask were added 1,1'-binaphthyl-2,2'-diphemyl phosphine (349 mg), bis(dibenzylideneacetone)palladium (256 mg), (*R*)-*N*-methyl-*N*-(pyrrol-3-yl)acetamide hydrochloride (500 mg), 1,3-dibromobenzene (660 mg), sodium *tert*-butoxide (807 mg) and dioxane (15 mL) successively under nitrogen atmosphere. After the addition was completed, the reaction solution was heated and reacted at 120 °C. After the reaction was completed, the reaction solution was concentrated and subjected to column chromatography (petroleum ether/ethyl acetate = 60/40) to give compound 24A (740 mg).

MS (ESI, $[M+H]^+$) *m/z:* 297.4.

**Step B: Preparation of compound 24B**

To a reaction flask [1,1'-bis(diphenylphosphino)ferrocene]palladium dichloride (179 mg), compound 24A (650 mg), bis(pinacolato)diboron (1111 mg), potassium acetate (644 mg) and dioxane (15 mL) successively under nitrogen atmosphere; after the addition was completed, the reaction solution was reacted at 110 °C. After the reaction was completed, the reaction solution was concentrated and subjected to column chromatography (petroleum ether/ethyl acetate = 80/20) to give compound 24B (740 mg).

MS (ESI, $[M+H]^+$) *m/z:* 345.6.

**Step C: Preparation of compound 24C**

To a reaction flask were added tetrakis(triphenylphosphine)palladium (208 mg), compound 1C, compound 24B (620 mg), potassium carbonate (498 mg), dioxane (15 mL), and water (0.5 mL) successively. After the addition was completed, the reaction solution was heated and reacted at 120 °C. After the reaction was completed, the reaction solution was concentrated and subjected to column chromatography (dichloromethane/methanol = 98/2) to give compound 24C (470 mg).

MS (ESI, $[M+H]^+$) *m/z:* 673.6.

**Step D: Preparation of compound 24**

To the reaction flask were added compound 24C (420 mg), dichloromethane (6 mL) and methanesulfonic acid (360 mg) successively. After the addition was completed, the reaction solution was stirred for reaction at room temperature. After the reaction was completed, the pH of the reaction solution was adjusted to be neutral with saturated aqueous sodium bicarbonate, the reaction solution was extracted with dichloromethane, the organic phases were combined, washed with saturated brine, dried over anhydrous sodium sulfate, filtered, concentrated, and subjected to column chromatography (dichloromethane/methanol = 96/4) to give compound 24 (140 mg).

MS (ESI, $[M+H]^+$) *m/z:* 573.7.

$^1$H NMR (500 MHz, DMSO-$d_6$): δ 8.22 (d, *J*=8.0Hz,1H), 8.00(dd, *J*=3.5Hz, 7.0Hz, 1H), 7.62(d, *J*=7.5Hz,1H), 7.44(d, *J*=7.5Hz,1H), 7.30 - 7.14 (m, 4H), 7.02(s, 2H), 6.61(br, 1H), 6.43 - 6.41(m, 1H), 5.20(t, *J*=7.5Hz,1H), 4.68(t, *J*=7.5Hz,1H), 3.49 - 3.48(m, 1H), 3.37 - 3.23(m, 6H), 2.89(s, 2H), 2.43 - 2.42(m, 2H), 2.15 - 2.12(m, 4H), 2.03(s, 2H).

**Example 25: Preparation of Compound 25**

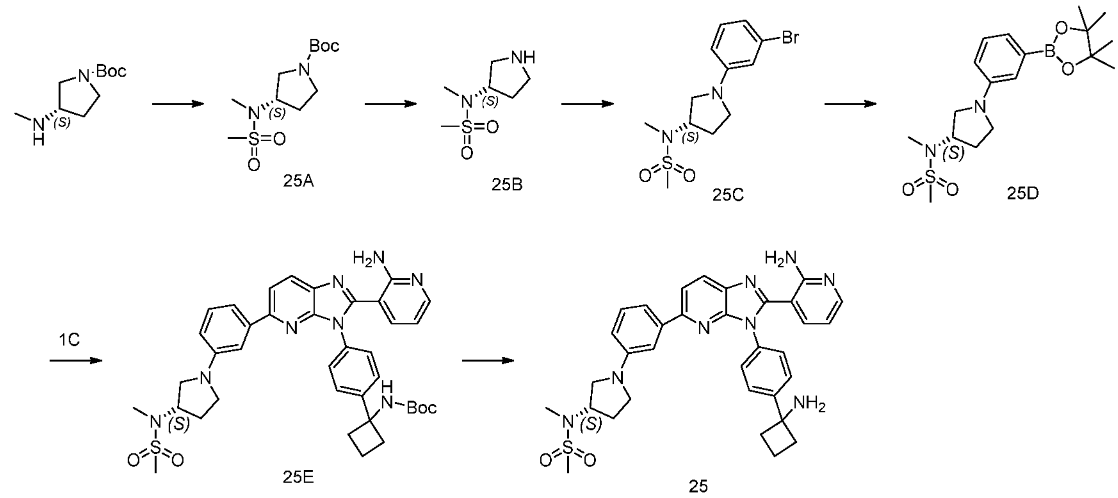


25A 25B 25C 25D 25E 25

**Step A: Preparation of compound 25A**

To a reaction flask were added *tert*-butyl (*S*)-3-(methylamino)pyrrolidine-1-carboxylate (1.60 g), *N*-diisopropylethylamine (1.24 g), and methanesulfonyl chloride (0.91 g) successively. After the addition was completed, the reaction solution was stirred for reaction at room temperature. After the reaction was completed, the reaction solution was concentrated to give compound 25A (2.53 g).

**Step B: Preparation of compound 25B**

To a reaction flask were added a solution (6 mL) of hydrochloric acid in dioxane, compound 25A (2.53 g), and dichloromethane (20 mL) successively. The reaction solution was reacted at room temperature; after the reaction was completed, the reaction solution was concentrated to give compound 25B (1.58 g).

**Step C: Preparation of compound 25C**

To a reaction flask were added compound 25B (0.700 g), 1,3-dibromobenzene (0.949 g), tris(dibenzylideneacetone)dipalladium (0.368 g), 1,1'-binaphthyl-2,2'-diphemyl phosphine (0.500 g), sodium *tert*-butoxide (1.500 g), and toluene (20 mL) successively under nitrogen atmosphere. After the addition was completed, the reaction solution was stirred for reaction at 120 °C. After the reaction was completed, the reaction solution was concentrated and subjected to column chromatography (petroleum ether/ethyl acetate = 4/1) to give compound 25C (0.456 g).

MS (ESI, $[M+H]^+$) *m/z:* 333.3.

**Step D: Preparation of compound 25D**

To a reaction flask were added potassium acetate (0.385 g), bis(pinacolato)diboron (0.627 g), [1,1'-bis (diphenylphosphino)ferrocene]palladium dichloride (0.160 g), compound 25C (0.430 g) and dioxane (20 mL) successively under nitrogen atmosphere. After the addition was completed, the reaction solution was stirred for reaction at 110 °C. After the starting materials were reacted completely, the reaction solution was concentrated and subjected to column chromatography (petroleum ether/ethyl acetate = 7/3) to give compound 25D (0.166 g).

MS (ESI, $[M+H]^+$) *m/z:* 381.6.

**Step E: Preparation of compound 25E**

To a reaction flask were added compound 25D (0.165 g), compound 1C (0.213 g), potassium carbonate (0.180 g), tetrakis(triphenylphosphine)palladium (0.025 g), 1,4-dioxane (10 mL), and water (5 mL) successively under nitrogen atmosphere. After the addition was completed, the reaction solution was stirred for reaction at 110 °C. After the reaction was completed, the reaction solution was concentrated and subjected to column chromatography (dichloromethane/methanol = 17/3) to give compound 25E (0.075 g).

MS (ESI, $[M+H]^+$) *m/z:* 709.7.

**Step F: Preparation of compound 25**

To a reaction flask were added compound 25E (0.075 g), dichloromethane (10 mL) and methanesulfonic acid (0.050 g) successively. After the addition was completed, the reaction solution was stirred for reaction at room temperature. After the reaction was completed, 20% aqueous sodium hydroxide solution was added, and the reaction solution was stirred under an ice bath. The pH of the reaction solution was adjusted to 12, the phases were separated, the aqueous phase was extracted by dichloromethane, and the organic phases were combined, washed with saturated brine and dried over anhydrous sodium sulfate. The reaction solution was filtered, concentrated, and subjected to column chromatography (dichloromethane/methanol = 3/7) to give compound 25 (0.040 g).

MS (ESI, $[M+H]^+$) *m/z:* 609.7.

$^1$H NMR (500 MHz, DMSO-$d_6$) $\delta$8.41(d, 1H), 8.20(d, 1H), 7.96(d, 1H), 7.84(d, 1H), 7.75(d, 2H), 7.67(d, 2H), 7.41 - 7.45(m, 2H), 7.29(m, 1H), 6.96(m, 1H), 4.26(s, 4H), 4.00(m, 1H), 3.53(d, 2H), 3.08(t, 2H), 2.63(s, 4H), 2.25(t, 3H), 1.71 - 2.01(m, 7H).

**Example 26: Preparation of Compound 26**

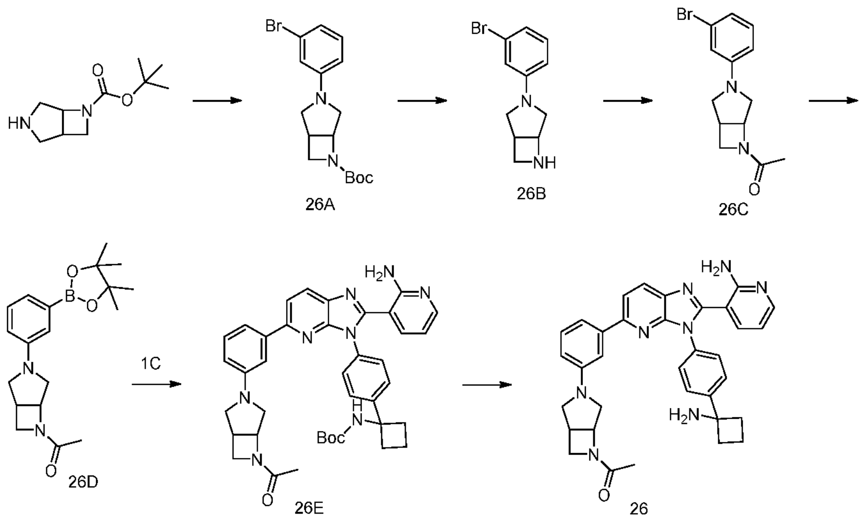


26A 26B 26C 26D 26E 26

**Step A: Preparation of compound 26A**

To a reaction flask were added 1,3-dibromobenzene (0.54 g), tert-butyl 3,6-diazabicyclo[3.2.0]heptane-6-carboxylate (0.30 g), sodium tert-butoxide (0.29 g), tris(dibenzylideneacetone)dipalladium (0.14 g), 1,1'-binaphthyl-2,2'-diphemyl phosphine (0.09 g) and 1,4-dioxane (12 mL) successively. The reaction solution was stirred for reaction at 100 °C under nitrogen atmosphere. After the reaction was completed, the reaction solution was filtered, concentrated, and subjected to column chromatography (petroleum ether/ethyl acetate = 5/1) to give compound 26A (0.51 g).

MS (ESI, $[M\text{-}Boc+H]^+$) *m/z*: 253.4.

**Step B: Preparation of compound 26B**

To a reaction flask were added compound 26A (0.46 g) and a solution of 4 M hydrogen chloride in dioxane (8 mL) successively, and the reaction solution was stirred for reaction at room temperature. After the reaction was completed, the reaction solution was concentrated to give compound 26B (0.43 g).

MS (ESI, $[M+H]^+$) *m/z:* 253.4.

**Step C: Preparation of compound 26C**

Acetyl chloride (2.98 g) was slowly added to a solution of compound 26B (0.43 g) and triethylamine (0.69 g) in dichloromethane (20 mL) in a reaction flask at 0 °C under nitrogen atmosphere. After the addition was completed, the reaction solution was stirred for reaction at room temperature. After the reaction was completed, the reaction solution was added with a saturated aqueous sodium bicarbonate solution (75 mL) and extracted with dichloromethane (50 mL

× 3), and the organic phases were combined, washed with saturated brine (100 mL), and dried over anhydrous sodium sulfate. The organic phase was filtered, concentrated, and subjected to column chromatography (petroleum ether/ethyl acetate = 1/4) to give compound 26C (0.25 g).

MS (ESI, $[M+H]^+$) *m/z:* 295.4.

**Step D: Preparation of compound 26D**

To a 100 mL reaction flask were added compound 26C (0.25 g), bis(pinacolato)diboron (0.32 g), potassium acetate (0.25 g), [1,1'-bis(diphenylphosphino)ferrocene]palladium dichloride dichloromethane complex (0.069 g) and 1,4-dioxane (15 mL) successively. The reaction solution was stirred for reaction at 100 °C under nitrogen atmosphere. After the reaction was completed, the reaction solution was filtered, concentrated, and subjected to column chromatography (petroleum ether/ethyl acetate = 1/4) to give compound 26D (0.22 g).

MS (ESI, $[M+H]^+$) *m/z:* 343.6.

**Step E: Preparation of compound 26E**

To a 25 mL microwave tube were added compound 26D (0.21 g), compound 1C (0.20 g), potassium carbonate (0.17 g), tetrakis(triphenylphosphine)palladium (0.047 g), 1,4-dioxane (10 mL) and water (1.5 mL) successively. After the addition was completed, the reaction solution was purged with nitrogen gas, and reacted at 140 °C in microwave reactor for 2 h. After the reaction was completed, the reaction solution was filtered, concentrated, and subjected to column chromatography (dichloromethane/methanol = 20/1) to give compound 26E (0.22 g).

MS (ESI, $[M+H]^+$) *m/z:* 671.7.

**Step F: Preparation of compound 26**

To a 50 mL reaction flask were added compound 26E (183 mg), methanesulfonic acid (0.2 mL), and dichloromethane (10 mL) successively. The reaction solution was stirred for reaction at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 26 (106 mg).

HRMS (ESI, $[M+H]^+$) *m/z*: 571.2924.

$^1H$ NMR (500 MHz, $CDCl_3$) δ 8.11 (dd, *J* = 8.3, 5.2 Hz, 1H), 8.07 (dt, *J* = 4.9, 1.7 Hz, 1H), 7.79 (dd, *J* = 8.4, 3.6 Hz, 1H), 7.60 - 7.54 (m, 2H), 7.53 - 7.41 (m, 4H), 7.36 - 7.30 (m, 1H), 7.16 (ddd, *J* = 14.3, 7.8, 1.8 Hz, 1H), 6.82 - 6.76 (m, 1H), 6.62 (s, 2H), 6.40 (dt, *J* = 7.8, 5.3 Hz, 1H), 4.94 (dd, *J* = 6.9, 4.5 Hz, 1H), 4.30 - 4.10 (m, 2H), 3.98 - 3.83 (m, 1H), 3.76 (dd, *J* = 22.2, 10.2 Hz, 1H), 3.29 - 3.18 (m, 1H), 3.08 - 2.88 (m, 2H), 2.68 - 2.56 (m, 2H), 2.32 - 2.05 (m,4H), 1.84 (s, 3H).

**Example 27: Preparation of Compound 27**

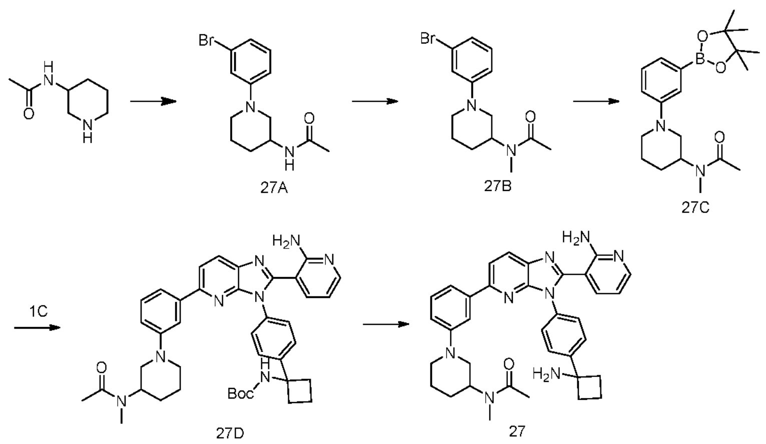


27A 27B 27C 27D 27

**Step** A: **Preparation of compound 27A**

To a reaction flask were added 1,3-dibromobenzene (4.98 g), *tert*-butyl 3,6-diazabicyclo[3.2.0]heptane-6-carboxylate (2.00 g), sodium *tert*-butoxide (2.70 g), tris(dibenzylideneacetone)dipalladium (0.64 g), 1,1'-binaphthyl-2,2'-diphemyl phosphine (0.66 g) and 1,4-dioxane (100 mL) successively. The reaction solution was stirred for reaction at 100 °C under nitrogen atmosphere; after the reaction was completed, the reaction solution was filtered, concentrated, and subjected to column chromatography (petroleum ether/ethyl acetate = 1/1) to give compound 27A (2.78 g).
MS (ESI, $[M+H]^+$) *m/z:* 297.4.

**Step B: Preparation of compound 27B**

Sodium hydride (1.87 g) was slowly added to a solution of compound 27A (2.78 g) in tetrahydrofuran (60 mL) at 0 °C under nitrogen atmosphere. After the addition was completed, the mixture was stirred at room temperature for 0.5 h, and methyl iodide (6.64 g) was added thereto for reaction. After the reaction was completed, a saturated aqueous ammonium chloride solution was added to the reaction solution in an ice bath, followed by extraction with ethyl acetate, and the organic phases were combined, washed with saturated brine, and dried over anhydrous sodium sulfate. The organic phase was filtered, concentrated, and subjected to column chromatography (petroleum ether/ethyl acetate = 1/2) to give compound 27B (1.91 g).
MS (ESI, $[M+H]^+$) *m*/*z*: 311.4.

**Step C: Preparation of compound 27C**

To a reaction flask were added compound 27B (1.91 g), bis(pinacolato)diboron (2.34 g), potassium acetate (1.81 g), [1,1'-bis(diphenylphosphino)ferrocene]palladium dichloride dichloromethane complex (0.50 g) and 1,4-dioxane (120 mL) successively. The reaction solution was stirred for reaction at 100 °C under nitrogen atmosphere. After the reaction was completed, the reaction solution was filtered, concentrated, and subjected to column chromatography (petroleum ether/ethyl acetate = 1/1) to give compound 27C (2.11 g).
MS (ESI, $[M+H]^+$) *m*/*z:* 359.6.

**Step D: Preparation of compound 27D**

To a microwave tube were added compound 27C (0.33 g), compound 1C (0.30 g), potassium carbonate (0.25 g), tetrakis(triphenylphosphine)palladium (71 mg), 1,4-dioxane (14 mL), and water (2 mL) successively. After the addition was completed, the reaction solution was purged with nitrogen gas, and reacted at 140 °C in microwave reactor. After the reaction was completed, the reaction solution was filtered, concentrated, and subjected to column chromatography (dichloromethane/methanol = 20/1) to give compound 27D (0.38 g).
MS (ESI, $[M+H]^+$) *m*/*z*: 687.8.

**Step E: Preparation of compound 27**

Compound 27D (370 mg), methanesulfonic acid (0.3 mL) and dichloromethane (16 mL) were added successively to a reaction flask. The reaction solution was stirred for reaction at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 27 (157 mg).
HRMS (ESI, $[M+H]^+$) *m*/*z*: 587.3278.
$^1$H NMR (500 MHz, $CDCl_3$) δ 8.10 (dd, *J* =9.5, 8.3 Hz, 1H), 8.06 (dd, *J* = 4.8, 1.8 Hz, 1H), 7.79 (dd, *J* = 8.4, 1.3 Hz, 1H), 7.67 - 7.57 (m, 3H), 7.48 (dt, *J* = 7.8, 1.0 Hz, 1H), 7.46 - 7.41 (m, 2H), 7.31 (dt, *J* = 18.8, 7.9 Hz, 1H), 7.12 (ddd, *J* = 29.3, 7.8, 1.8 Hz, 1H), 6.93 (td, *J* = 7.6, 2.4 Hz, 1H), 6.60 (s, 2H), 6.39 (ddd, *J* = 7.8, 5.9, 4.8 Hz, 1H), 4.77 - 4.68 (m, 1H), 3.86 - 3.72 (m, 1H), 3.72 - 3.56 (m, 1H), 2.91 (d, *J* = 18.3 Hz, 3H), 2.75 - 2.56 (m, 4H), 2.47 - 2.35 (m, 2H), 2.26 - 2.18 (m, 1H), 2.15 (s, 3H), 1.92 - 1.70 (m, 5H).

**Example 28: Preparation of Compound 28**

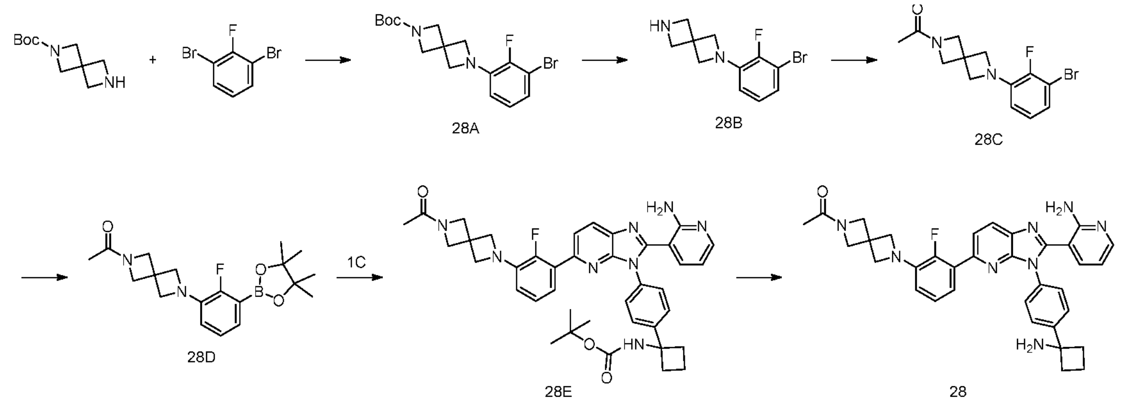

28A 28B 28C 28D 28E 28

**Step A: Preparation of compound 28A**

To a reaction flask were added *tert*-butyl 2,6-diazaspiro[3.3]heptane-2-carboxylate (0.50 g), 1,3-dibromofluorobenzene (0.66 g), tris(dibenzylideneacetone)dipalladium (80 mg), 1,1'-binaphthyl-2,2'-diphemyl phosphine (60 mg), tetrahydrofuran (50 mL) and sodium *tert*-butoxide (1.00 g) successively. After the addition was completed, the reaction solution was stirred for reaction at 80 °C under nitrogen atmosphere. After the reaction was completed, the reaction solution was filtered, concentrated, and subjected to column chromatography (petroleum ether/ethyl acetate = 10/1) to give compound 28A (0.51 g).

**Step B: Preparation of compound 28B**

Compound 28A (0.50 g) was weighed out and added to a reaction flask, methylene chloride (10 mL) was added, the mixture was stirred and dissolved, followed by addition of methanesulfonic acid (0.39 g), and after the addition was completed, the reaction solution was stirred for reaction at room temperature. After the reaction was completed, triethylamine (2 mL) was added to terminate the reaction to give a solution of compound 28B. The reaction solution was not concentrated and was directly fed for the next step.

**Step C: Preparation of compound 28C**

Acetic anhydride (206 mg) was added to the above solution of compound 28B, and the mixture was stirred at room temperature for reaction. After the reaction was completed, the reaction solution was concentrated and subjected to column chromatography (dichloromethane/methanol = 96:4) to give compound 28C (313 mg).
MS (ESI, $[M+H]^+$) *m/z*: 313.79.

**Step D: Preparation of compound 28D**

To a reaction flask containing compound 28C (300 mg) were added bis(pinacolato)diboronate (365 mg), [1,1'-bis(diphenylphosphino)ferrocene]palladium dichloride dichloromethane complex (39 mg), 1,4-dioxane (30 ml) and potassium acetate (282 mg) successively. After the addition was completed, the reaction solution was stirred for reaction at 120 °C under nitrogen atmosphere. After the reaction was completed, the reaction solution was filtered, concentrated and subjected to column chromatography (dichloromethane/methanol = 98/2) to give compound 28D (278 mg).
MS (ESI, $[M+H]^+$) *m/z:* 361.04.

**Step E: Preparation of compound 28E**

To a microwave tube were added compound 28D (300 mg), compound 1C (409 mg), potassium carbonate (460 mg), tetrakis(triphenylphosphine)palladium (48 mg), 1,4-dioxane (10 mL), and water (1 mL) successively. After the addition was completed, the reaction solution was purged with nitrogen gas, and reacted at 120 °C in microwave reactor. After the reaction was completed, the reaction solution was filtered, concentrated and subjected to column chromatography (dichloromethane/methanol = 96/4) to give compound 28E (252 mg).
MS (ESI, $[M+H]^+$) *m/z:* 689.5.

**Step F: Preparation of compound 28**

Compound 28E (250 mg), methanesulfonic acid (349 mg) and dichloromethane (30 mL) were added successively to a reaction flask. The reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 28 (70 mg).

MS (ESI, $[M+H]^+$) *m/z:* 589.5.

$^1H$ NMR (500 MHz, $CDCl_3$): 8.25 (d, *J* = 8.5 Hz, 1H), 8.00 (dd, *J* = 2.0, 5.0 Hz, 1H), 7.68 (dd, *J* = 2.0, 8.0 Hz, 1H), 7.60 (d, *J* = 8.5 Hz, 2H), 7.42 (d, *J* = 8.5 Hz, 2H), 7.20 (dd, *J* = 2.0, 8.0 Hz, 1H), 7.10 - 7.44 (m, 2H), 6.94 (m, 2H), 6.61 - 6.57 (m, 1H), 6.42 - 6.40 (m, 1H), 4.29 (s, 2H), 4.08 (s, 4H), 4.02 (s, 2H), 2.44 - 2.37 (m, 2H), 2.13 - 2.06 (m, 2H), 2.06 - 1.98 (m, 1H), 1.75 (s, 3H), 1.73 - 1.65 (m, 1H).

**Example 29: Preparation of Compound 29**

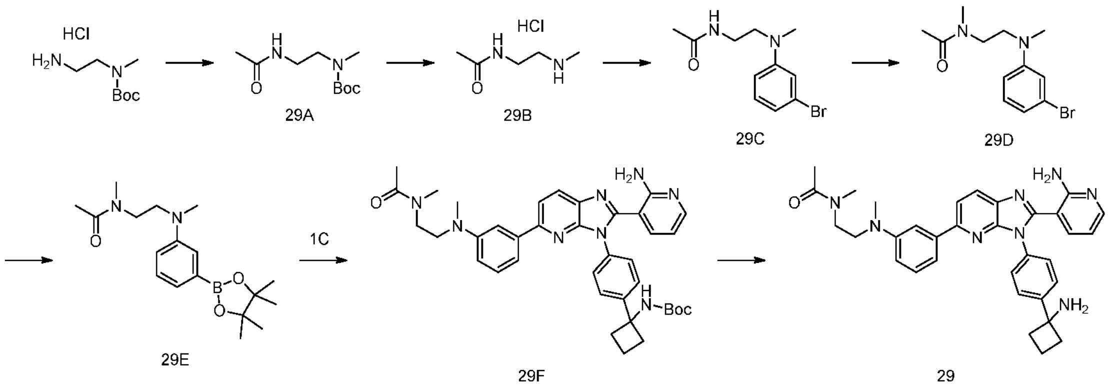


29A 29B 29C 29D 29E 29F 29

**Step A: Preparation of compound 29A**

*N-tert*-butoxycarbonyl-*N*-methylethylenediamine (1.00 g), methylene chloride (30 mL), anhydrous potassium carbonate (1.59 g) and acetic anhydride (0.64 g) were added to the reaction flask successively. After the addition was completed, the reaction solution was stirred for reaction at room temperature. After the reaction was completed, The reaction solution was washed with water and saturated brine successively, and dried over anhydrous sodium sulfate. The reaction solution was filtered and concentrated to give compound 29A (1.13 g).

**Step B: Preparation of compound 29B**

To a reaction flask containing compound 29A (1.13 g) was added a solution of hydrogen chloride (4 M) in 1,4-dioxane (20 mL), and after the addition was completed, the reaction solution was stirred for reaction at room temperature. After the reaction was completed, the reaction solution was concentrated to give compound 29B (0.87 g).

**Step C: Preparation of compound 29C**

To a reaction flask were added compound 29B (0.87 g), 1,3-dibromofluorobenzene (5.42 g), tris(dibenzylideneacetone)dipalladium (263 mg), 1,1'-binaphthyl-2,2'-diphemyl phosphine (179 mg), tetrahydrofuran (50 mL) and sodium tert-butoxide (3.31 g) successively. After the addition was completed, the reaction solution was stirred for reaction at 80 °C under nitrogen atmosphere. After the reaction was completed, the reaction solution was filtered, concentrated, and subjected to column chromatography (ethyl acetate) to give compound 29C (0.48 g).

MS (ESI, $[M+H]^+$) *m/z:* 271.1.

**Step D: Preparation of compound 29D**

Tetrahydrofuran (10 mL) was added to a reaction flask containing compound 29C (0.48 g), and after the mixture

was dissolved with stirring, sodium hydride (0.28 g) was added after the reaction solution was cooled with ice/ethanol. After the addition was completed, the reaction solution was stirred for 10 min, methyl iodide (0.76 g) was added, and after the addition was completed, the reaction solution was stirred for reaction at room temperature. The reaction was completed. The reaction solution was poured into aqueous saturated ammonium chloride solution, stirred, and extracted with dichloromethane, and the organic phases were combined. The reaction solution was dried over anhydrous sodium sulfate. The reaction solution was filtered and concentrated to give compound 29D (0.64 g).

MS (ESI, $[M+H]^+$) *m/z:* 285.1.

**Step E: Preparation of compound 29E**

To a reaction flask containing compound 29D (509 mg) were added bis(pinacolato)diboron (680 mg), [1,1'-bis(diphenylphosphino)ferrocene]palladium dichloride dichloromethane complex (73 mg), 1,4-dioxane (20 mL) and potassium acetate (525 mg) successively. After the addition was completed, the reaction solution was stirred for reaction at 120 °C under nitrogen atmosphere. After the reaction was completed, the reaction solution was filtered, concentrated and subjected to column chromatography (petroleum ether/ethyl acetate = 1/2) to give compound 29E (441 mg).

MS (ESI, $[M+H]^+$) *m/z:* 333.3.

**Step E: Preparation of compound 29F**

To a microwave tube were added compound 29E (440 mg), compound 1C (716 mg), potassium carbonate (550 mg), tetrakis(triphenylphosphine)palladium (77 mg), 1,4-dioxane (10 mL), and water (1 mL) successively. After the addition was completed, the reaction solution was purged with nitrogen gas, and reacted at 120 °C in microwave reactor for 2 h. After the reaction was completed, the reaction solution was filtered, concentrated and subjected to column chromatography (dichloromethane/methanol = 96/4) to give compound 29F (400 mg).

MS (ESI, $[M+H]^+$) *m/z:* 661.39.

**Step G: Preparation of compound 29**

To a reaction flask were added compound 29F (400 mg), methanesulfonic acid (582 mg) and dichloromethane (30 mL) successively, and the reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 29 (387 mg).

MS (ESI, $[M+H]^+$) *m/z*: 561.34.

$^1$H NMR (500 MHz, $CDCl_3$): 8.29 (d, *J* = 8.5 Hz, 1H), 8.01 (dd, *J* = 1.5, 5.0 Hz, 1H), 7.96 (t, J=8.5 Hz, 1H), 7.62 (d, *J* = 8.0 Hz, 2H), 7.47 - 7.40 (m, 3H), 7.31 - 7.22 (m, 2H), 7.01 (s, 2H), 6.79 - 6.74 (m, 1H), 6.43 - 6.41 (m, 1H), 3.56 - 3.54 (m, 1H), 3.49 - 3.46 (m, 1H), 3.43 - 3.33 (m, 6H), 2.94 (s, 2H), 2.90 (s, 1H), 2.78 (s, 1H), 2.46 - 2.41 (m, 2H), 2.18 - 2.13 (m, 2H), 2.08 - 2.02 (m, 1H), 1.88 (s, 2H), 1.79 (s, 2H), 1.75 - 1.66 (m, 1H).

**Example 30: Preparation of Compound 30**

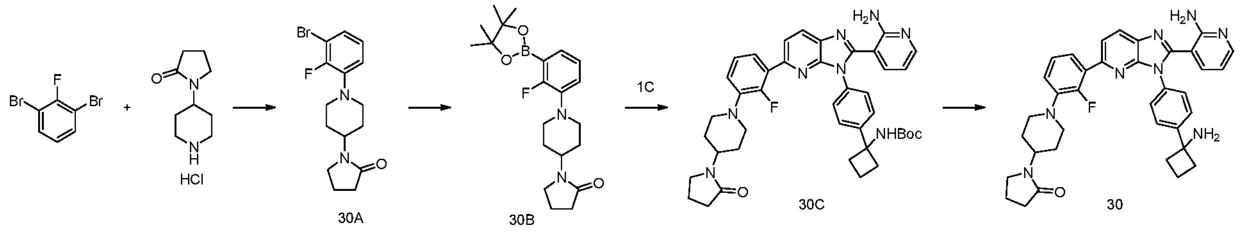


30A 30B 30C 30

**Step A: Preparation of compound 30A**

1-(Piperidin-4-yl)pyrrolidin-2-one hydrochloride (0.32 g), 1,3-dibromofluorobenzene (0.40 g), tris(dibenzylideneacetone)dipalladium (0.07 g), 1,1'-binaphthyl-2,2'-diphemyl phosphine (0.10 g), sodium *tert*-butoxide (0.33 g) and toluene (20 mL) were added to a reaction flask successively under nitrogen atmosphere. After the addition was completed,

the reaction solution was stirred for reaction at 120 °C. After the starting materials were reacted completely, the reaction solution was concentrated and subjected to column chromatography (dichloromethane/methanol = 65/1) to give compound 30A (0.20 g).

**Step B: Preparation of compound 30B**

To a reaction flask were added potassium acetate (0.06 g), bis(pinacolato)diboron (0.10 g), [1,1'-bis (diphenylphosphino)ferrocene]palladium dichloride (25 mg), compound 30A (0.20 g) and dioxane (20 mL) successively under nitrogen atmosphere. After the addition was completed, the reaction solution was stirred for reaction at 110 °C. After the starting materials were reacted completely, the reaction solution was concentrated and subjected to column chromatography (dichloromethane/methanol = 65/1) to give compound 30B (0.21 g).

**Step C: Preparation of compound 30C**

To a reaction flask were added compound 30B (0.21 g), compound 1C (0.11 g), potassium carbonate (0.06 g), tetrakis(triphenylphosphine)palladium (13 mg), 1,4-dioxane (15 mL), and water (7.5 mL) successively under nitrogen atmosphere. After the addition was completed, the reaction solution was stirred for reaction at 110 °C. After the reaction was completed, the reaction solution was concentrated and subjected to column chromatography (dichloromethane/methanol = 3/7) to give compound 30C (0.16 g).

MS (ESI, $[M+H]^+$) *m/z:* 717.8.

**Step D: Preparation of compound 30**

To a reaction flask were added compound 30C (0.16 g), dichloromethane (10 mL) and methanesulfonic acid (0.21 g) successively. After the addition was completed, the reaction solution was stirred for reaction at room temperature. After the reaction was completed, 20% aqueous sodium hydroxide solution was added, and the reaction solution was stirred under an ice bath. The pH of the reaction solution was adjusted to 12, the phases were separated, the aqueous phase was extracted by dichloromethane, and the organic phases were combined, washed with saturated brine and dried over anhydrous sodium sulfate. The reaction solution was filtered, concentrated, and subjected to column chromatography (dichloromethane/methanol = 2/3) to give compound 30 (0.08 g).

MS (ESI, $[M+H]^+$) *m/z:* 617.7.

$^1H$ NMR (500 MHz, DMSO-$d_6$) $\delta$ 8.23 (d, 1H), 7.96 - 8.02 (m, 2H), 7.63 (d, 2H), 7.47 (d, 2H), 7.22 - 7.33 (m, 3H), 6.98 (s, 2H), 6.62 - 6.64 (m, 1H), 6.41 - 6.43 (m, 1H), 4.51 - 4.57 (m, 1H), 3.47 (q, 2H), 3.24 (q, 2H), 2.99 (s, 3H), 2.78 (s, 3H), 2.44 - 2.48 (m, 2H), 2.02 - 2.25 (m, 5H), 1.69 - 1.77 (m, 1H), 1.23 (s, 2H).

**Example 31: Preparation of Compound 31**

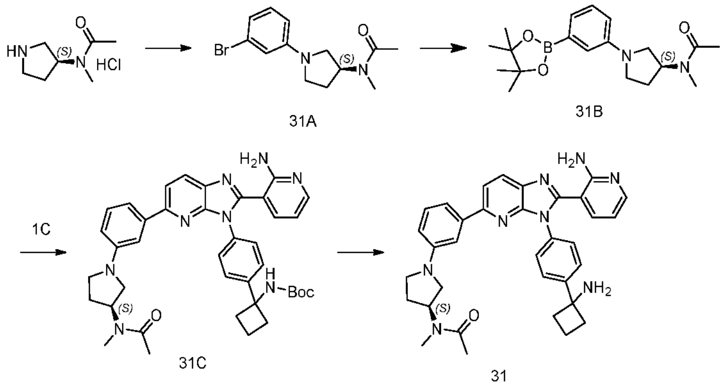

31A 31B 31C 31

**Step A: Preparation of compound 31A**

1-(Piperidin-4-yl)pyrrolidin-2-one hydrochloride (0.61 g), 1,3-dibromobenzene (0.80 g), tris(dibenzylideneacetone)dipalladium (0.31 g), 1,1'-binaphthyl-2,2'-diphemyl phosphine (0.32 g), sodium *tert*-butoxide (0.49 g) and toluene (10 mL) were added to a reaction flask successively under nitrogen atmosphere. After the addition was completed, the

reaction solution was stirred for reaction at 120 °C. After the starting materials were reacted completely, the reaction solution was concentrated and subjected to column chromatography (petroleum ether/ethyl acetate = 2/3) to give compound 31A (0.50 g).

MS (ESI, $[M+H]^+$) *m/z:* 297.1.

**Step B: Preparation of compound 31B**

To a reaction flask were added potassium acetate (0.489 g), bis(pinacolato)diboron (0.811 g), [1,1'-bis (diphenylphosphino)ferrocene]palladium dichloride (0.207 g), compound 31A (0.502 g) and dioxane (20 mL) successively under nitrogen atmosphere. After the addition was completed, the reaction solution was stirred for reaction at 110 °C. After the starting materials were reacted completely, the reaction solution was concentrated and subjected to column chromatography (petroleum ether/ethyl acetate = 7/3) to give compound 31B (0.293 g).

MS (ESI, $[M+H]^+$) *m/z:* 345.6.

**Step C: Preparation of compound 31C**

To a reaction flask were added compound 31B (0.293 g), compound 1C (0.320 g), potassium carbonate (0.270 g), tetrakis(triphenylphosphine)palladium (0.040 g), 1,4-dioxane (15 mL), and water (7.5 mL) successively under nitrogen atmosphere. After the addition was completed, the reaction solution was stirred for reaction at 110 °C. After the reaction was completed, the reaction solution was concentrated and subjected to column chromatography (dichloromethane/methanol = 7/3) to give compound 31C (0.294 g).

MS (ESI, $[M+H]^+$) *m/z:* 673.4.

**Step D: Preparation of compound 31**

To a reaction flask were added compound 31C (0.294 g), dichloromethane (10 mL) and methanesulfonic acid (0.210 g) successively. After the addition was completed, the reaction solution was stirred for reaction at room temperature. After the reaction was completed, 20% aqueous sodium hydroxide solution was added, and the reaction solution was stirred under an ice bath. The pH of the reaction solution was adjusted to 12, the phases were separated, the aqueous phase was extracted by dichloromethane, and the organic phases were combined, washed with saturated brine and dried over anhydrous sodium sulfate. The reaction solution was filtered, concentrated, and subjected to column chromatography (dichloromethane/methanol = 2/3) to give compound 31 (0.183 g).

MS (ESI, $[M+H]^+$) *m/z:* 573.7.

$^1H$ NMR (500 MHz, DMSO-$d_6$) $\delta$ 8.21(d, 1H), 7.95 - 8.01(m, 2H), 7.62(d, 2H), 7.44(d, 2H), 7.23 - 7.32(m, 4H), 7.02(s, 2H), 6.60(d, 1H), 6.41 - 6.43(m, 1H), 3.36 - 3.49(m, 1H), 3.21 - 3.35(m, 4H), 2.89(s, 3H), 2.42 - 2.43(m, 2H), 2.03 - 2.20(m, 8H), 1.7(m, 1H).

**Example 32: Preparation of Compound 32**

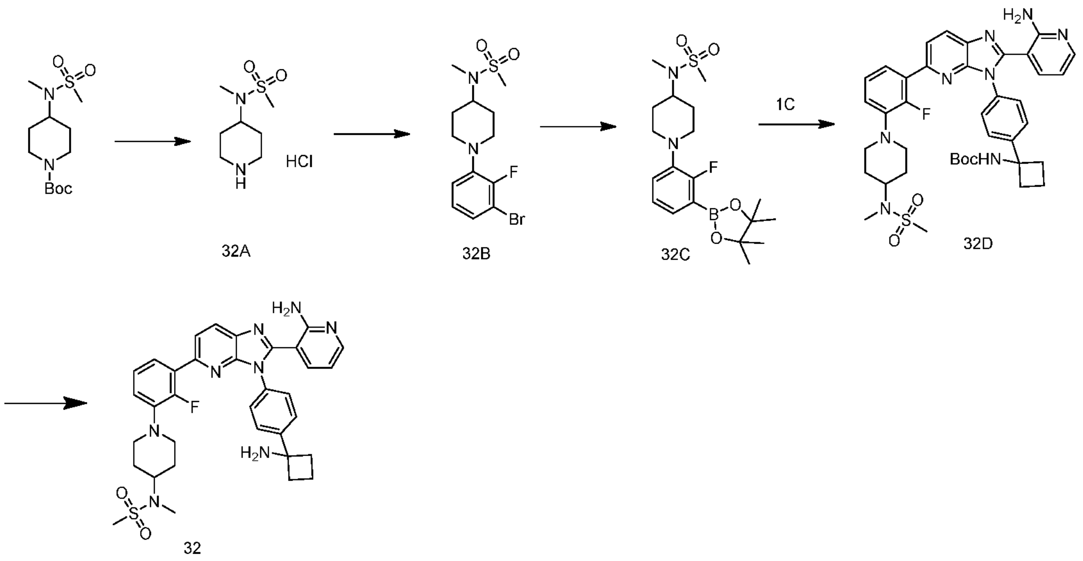

32A 32B 32C 32D

32

**Step A: Preparation of compound 32A**

To a 100 mL single-neck flask were added *tert*-butyl 4-(*N*-methylmethanesulfonamide)piperidine-1-carbamate (1.8 g), methylene chloride (5 mL), and a solution of 4 M hydrogen chloride (2 mL) in dioxane successively, and after the addition was completed, the reaction solution was stirred for reaction at room temperature. After the reaction was completed, the reaction solution was distilled under reduced pressure to give compound 32A (1.26 g), which was used directly in the next step.

**Step B: Preparation of compound 32B**

To a 100 mL single-neck flask were added 2,2'-(3,3'-dichloro-1,1'-biphenyl-4,4'-bisazo)bis(*N*-phenyl-3-oxobutyramide) (0.491 g), bis(dibenzylideneacetone)palladium (0.361 g), 1,3-dibromo-2-fluorobenzene (1 g), compound 32A (1.2 g), sodium *tert*-butoxide (0.757 g), and dioxane (15 mL) successively under nitrogen atmosphere, and the mixture was heated to 120 °C for reaction. After the reaction was completed, the reaction solution was distilled under reduced pressure to remove the solvent, concentrated, and then subjected to column chromatography (petroleum ether:ethyl acetate = 85:15) to give compound 32B (680 mg).

MS (ESI, $[M+H]^+$) *m/z:* 365.1.

**Step C: Preparation of compound 32C**

To a 100 mL one-neck flask were added [1,1'-bis(diphenylphosphino)ferrocene]palladium dichloride dichloromethane complex (139 mg), compound 32B (620mg), bis(pinacolato)diboron (862 mg), potassium acetate (333 mg), and dioxane (15 mL) successively, and the mixture was heated to 120 °C under nitrogen atmosphere for reaction. After the reaction was completed, the reaction solution was concentrated and subjected to column chromatography (petroleum ether: ethyl acetate = 75:25) to give compound 32C (420 mg).

MS (ESI, $[M+H]^+$) *m/z:* 413.3.

**Step D: Preparation of compound 32D**

To a 100 mL single-neck flask were added tetrakis(triphenylphosphine)palladium (51.8 mg), compound 32C (220 mg), compound 1C (220 mg), potassium carbonate (124 mg), dioxane (5 mL) and water (0.5 mL) successively, and the mixture was transferred to a 100 °C oil bath and heated for reaction. After the reaction was completed, the reaction solution was concentrated and subjected to column chromatography (dichloromethane:methanol = 94:6) to give 32D (180 mg).

MS (ESI, $[M+H]^+$) *m/z:* 741.4.

**Step E: Preparation of compound 32**

To a 50 mL single-necked flask were added compound 32D (180 mg), dichloromethane (10 mL) and methanesulfonic acid (46.7 mg) successively, and after the addition was completed, the reaction solution was stirred for reaction at room temperature. After the reaction was completed, the pH of the reaction solution was adjusted to be neutral with a saturated aqueous sodium carbonate solution, extracted with dichloromethane, washed with saturated brine, dried over anhydrous sodium sulfate, filtered, concentrated, and subjected to column chromatography (dichloromethane:methanol = 96:4) to give compound 32 (120 mg).

MS (ESI, $[M+H]^+$) *m/z:* 641.7.

$^1H$ NMR (500 MHz, $CDCl_3$): δ 8.12 (d, *J* = 7.5 Hz, 1H), 8.07 (dd, *J* = 5.0, 10.0 Hz, 1H), 7.80 (dd, *J* = 5.0, 10.0 Hz, 1H), 7.56 - 7.51 (m, 3H), 7.40 (d, *J* = 10.0 Hz, 1H), 7.12 (q, *J* = 10.0 Hz, 1H), 6.99 (t, *J* = 7.5 Hz, 1H), 6.59 (br, 2H), 6.39 (q, *J* = 8.0 Hz, 1H), 3.93 - 3.91 (m, 1H), 3.51 (d, *J* = 10.0 Hz, 2H), 2.88 - 2.87 (m, 6H), 2.81 (t, *J* = 10.0 Hz, 2H), 2.63 - 2.58(m, 2H), 2.24 - 2.19 (m, 1H), 2.12 - 2.06 (m, 2H), 1.83 (br, 6H).

**Example 33: Preparation of Compound 33**

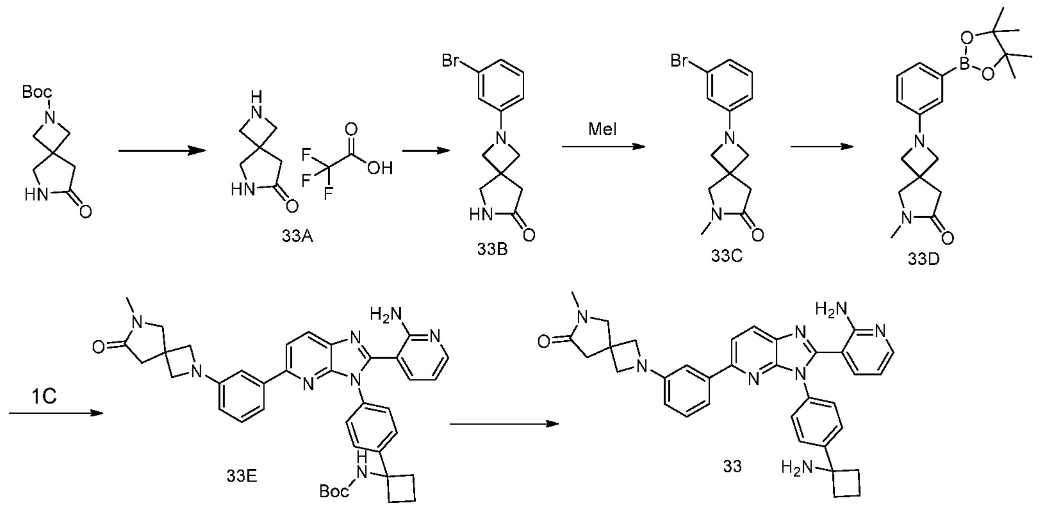


33A 33B 33C 33D 33E 33

**Step A: Preparation of compound 33A**

Trifluoroacetic acid (2 mL) was slowly added dropwise to a reaction solution of *tert*-butyl 7-oxo-2,6-diazaspiro[3,4]octane-2-carboxylate (0.5 g) in dichloromethane (10 mL) at 0 °C. After the addition was completed, the reaction solution was stirred at room temperature. After the reaction was completed, the reaction solution was concentrated to give compound 33A (0.78 g).

MS (ESI, $[M+H]^+$) *m*/z: 127.4.

**Step B: Preparation of compound 33B**

To a reaction flask were added compound 33A (0.5 g), 1,3-dibromobenzene (0.74 g), (tris(dibenzylideneacetone)dipalladium (0.19 g), 1,1'-binaphthyl-2,2'-diphemyl phosphine (0.13 g), sodium *tert*-butoxide (1 g) and 1,4-dioxane (30 mL) successively. After the addition was completed, the reaction solution was stirred for reaction at 100 °C under nitrogen atmosphere. After the reaction was completed, the reaction solution was filtered, concentrated, and subjected to column chromatography (dichloromethane/methanol = 50/1) to give compound 33B (0.54 g).

MS (ESI, $[M+H]^+$) *m/z:* 281.4.

**Step C: Preparation of compound 33C**

Sodium hydride (0.37 g) was slowly added to a solution of compound 33B (0.52 g) in tetrahydrofuran (30 mL) at 0 °C under nitrogen atmosphere, and after the addition was completed, the reaction solution was transferred to the room temperature condition and stirred for 0.5 h, and further added with methyl iodide (1.32 g) for reaction. After the reaction was completed, a saturated aqueous ammonium chloride solution (75 mL) was added to the reaction solution in an ice bath, and the mixture was extracted with ethyl acetate (50 mL × 3), and the organic phases were combined, washed with a saturated aqueous sodium chloride solution (100 mL), dried, filtered, and concentrated to give compound

33C (0.50 g).

MS (ESI, $[M+H]^+$) *m/z:* 295.4.

**Step D: Preparation of compound 33D**

To a reaction flask were added compound 33C (0.50 g), bis(pinacolato)diboron (0.65 g), potassium acetate (0.50 g), [1,1'-bis(diphenylphosphino)ferrocene]palladium dichloride dichloromethane complex (0.14 g) and 1,4-dioxane (30 mL) successively. The reaction solution was stirred for reaction at 100 °C under nitrogen atmosphere. After the reaction was completed, the reaction solution was filtered and concentrated. The reaction solution was subjected to column chromatography (dichloromethane/methanol = 20/1) to give compound 33D (0.12 g).

MS (ESI, $[M+H]^+$) *m/z:* 343.5.

**Step E: Preparation of compound 33E**

To a microwave tube were added compound 33D (0.12 g), compound 1C (0.14 g), potassium carbonate (0.12 g), tetrakis(triphenylphosphine)palladium (0.03 g), 1,4-dioxane (4 mL) and water (0.5 mL) successively. After the addition was completed, the reaction solution was purged with nitrogen gas, and reacted at 100 °C in microwave reactor. After the reaction was completed, the reaction solution was filtered and concentrated. The reaction solution was subjected to column chromatography (dichloromethane/methanol = 20/1) to give compound 33E (0.18 g).

MS (ESI, $[M+H]^+$) *m/z:* 671.4.

**Step F: Preparation of compound 33**

Compound 33E (176 mg), methanesulfonic acid (0.15 mL) and dichloromethane (10 mL) were added successively to a reaction flask, and the mixture was stirred at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 33 (69 mg).

MS (ESI, $[M+H]^+$) *m/z:* 571.6.

$^1H$ NMR (500 MHz, $CDCl_3$) δ 8.14 (dd, *J* = 8.4, 2.8 Hz, 1H), 8.02 (dd, *J* = 5.0, 1.9 Hz, 1H), 7.78 (dd, *J* = 8.5, 2.7 Hz, 1H), 7.60 - 7.55 (m, 2H), 7.48 - 7.42 (m, 3H), 7.30 (t, *J* = 7.9 Hz, 1H), 7.17 (dt, *J* = 7.8, 1.8 Hz, 1H), 7.09 (d, *J* = 2.2 Hz, 1H), 6.52 (dd, *J* = 7.9, 2.4 Hz, 1H), 6.42 (dd, *J* = 7.7, 4.8 Hz, 1H), 3.91 (s, 4H), 3.69 (d, *J* = 2.6 Hz, 2H), 2.89 (s, 3H), 2.72 (s, 2H), 2.68 - 2.61 (m, 2H), 2.35 - 2.28 (m, 2H), 2.20 - 2.02 (m,1H), 1.90 - 1.80 (m, 1H).

**Example 34: Preparation of Compound 34**

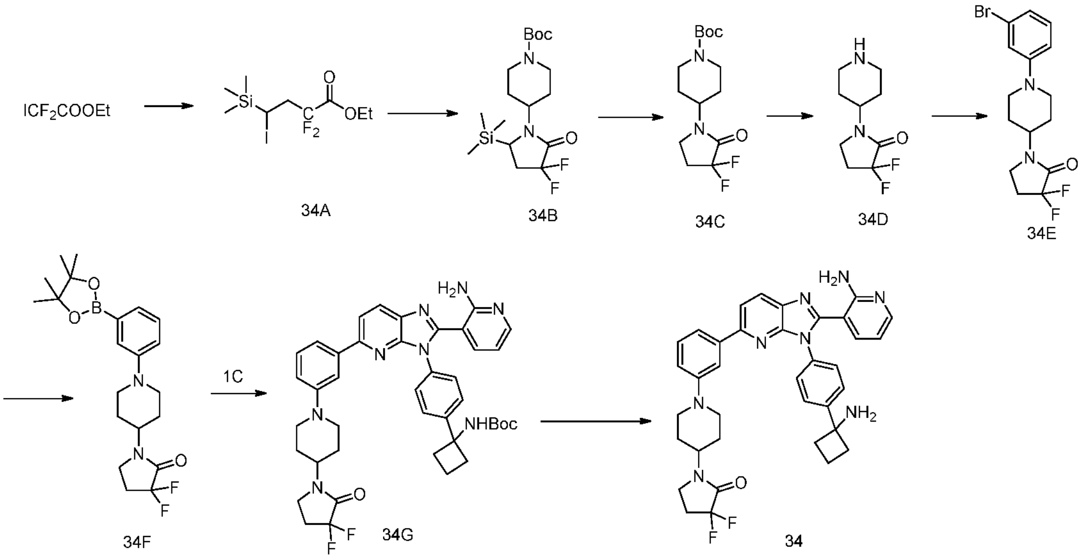


34A 34B 34C 34D 34E 34F 34G 34

**Step A: Preparation of compound 34A**

To the reaction flask were added ethyl 2,2-difluoro-2-iodoacetate (10 g), vinyltrimethylsilane (8.9 g), copper powder (0.13 g) and acetonitrile (50 mL) successively. After the addition was completed, the reaction solution was stirred at 65 °C. After the reaction was completed, the reaction solution was distilled under reduced pressure to remove the solvent and concentrated to give compound 34A (8.34 g).
MS (ESI, $[M+H]^+$) *m*/*z:* 351.0.

**Step B: Preparation of compound 34B**

To a reaction flask were added compound 34A (3.0 g), *tert*-butyl 4-aminopiperidine-1-carboxylate (1.1 g) and ethanol (20 mL) successively. The mixture was stirred at room temperature. After the reaction was completed, the reaction solution was concentrated and subjected to column chromatography (petroleum ether/ethyl acetate = 8/2) to give compound 34B (1.0 g).
MS (ESI, $[M+K]^+$) *m*/*z*: 415.4.

**Step C: Preparation of compound 34C**

To a reaction flask were added compound 34B (1.0 g), potassium fluoride (1.0 g) and dimethyl sulfoxide (20 mL) successively. The mixture was stirred at room temperature. After the reaction was completed, the reaction solution was concentrated to give compound 34C (0.76 g).
MS (ESI, $[M+H]^+$) *m*/*z:* 305.4.

**Step D: Preparation of compound 34D**

To the reaction flask were added compound 34C (3.0 g), a solution of 4 M hydrochloric acid in dioxane (6.2 mL), and dichloromethane (20 mL) successively. The mixture was stirred at room temperature. After the reaction was completed, the reaction solution was concentrated to give compound 34D (0.63 g).
MS (ESI, $[M+H]^+$) *m*/*z:* 205.4.

**Step E: Preparation of compound 34E**

To a reaction flask were added 1,1'-binaphthyl-2,2'-diphemyl phosphine (0.33 g), tris(dibenzylideneacetone)dipalladium (0.37 g), 1,3-dibromo-2-fluorobenzene (4.5 g), compound 34D (0.63 g), sodium *tert*-butoxide (0.28 g) and dioxane (20 mL) successively under nitrogen atmosphere. After the addition was completed, the reaction solution was stirred at 100 °C. After the reaction was completed, the reaction solution was concentrated and subjected to column chromatography (petroleum ether/ethyl acetate = 9/1) to give compound 34E (0.23 g).
MS (ESI, $[M+H]^+$) *m*/*z:* 359.0.

**Step F: Preparation of compound 34F**

To a reaction flask were added compound 34E (0.43 g), bis(pinacolato)diboron (0.25 g), potassium acetate (0.19 g), [1,1'-bis(diphenylphosphino)ferrocene]palladium dichloride dichloromethane complex (0.10 g) and 1,4-dioxane (10 mL) successively. The reaction solution was stirred at 100 °C under nitrogen atmosphere. After the reaction was completed, the reaction solution was filtered and concentrated. The reaction solution was subjected to column chromatography (dichloromethane/methanol = 20/1) to give compound 34F (0.17 g).
MS (ESI, $[M+H]^+$) *m*/*z:* 407.12.

**Step G: Preparation of compound 34G**

To a reaction flask were added compound 34F (0.17 g), compound 1C (0.12 g), potassium carbonate (0.090 g), tetrakis(triphenylphosphine)palladium (0.077 g), 1,4-dioxane (10 mL), and water (10 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 34G (0.20 g).
MS (ESI, $[M+H]^+$) *m*/*z:* 735.7.

**Step H: Preparation of compound 34**

Compound 34G (0.21 g), methanesulfonic acid (0.13 g) and dichloromethane (15 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 34 (0.041 g).

MS (ESI, $[M+H]^+$) *m/z:* 635.5.

$^1H$ NMR (500 MHz, $CDCl_3$) $\delta$ 8.48 (d, *J* = 8.6 Hz, 1H), 8.31 (d, *J* = 5.1 Hz, 1H), 8.14 (d, *J* = 8.5 Hz, 1H), 7.97 (d, *J* = 8.2 Hz, 2H), 7.92 - 7.83 (m, 3H), 7.80 (d, *J* = 7.8 Hz, 1H), 7.64 (t, *J* = 8.0 Hz, 1H), 7.53 (d, *J* = 7.7 Hz, 1H), 7.33 (d, *J* = 8.4 Hz, 1H), 6.77 (t, *J* = 6.4 Hz, 1H), 4.46 - 4.33 (m, 1H), 4.14 (d, *J* = 12.3 Hz, 2H), 3.82 (t, *J* = 6.7 Hz, 2H), 3.21 (t, *J* = 12.2 Hz, 2H), 3.13 - 3.02 (m, 2H), 2.94 (q, *J* = 10.3, 9.6 Hz, 2H), 2.86 (dd, *J* = 14.7, 7.7 Hz, 2H), 2.32 - 2.24 (m, 2H), 2.18 (d, *J* = 13.6 Hz, 2H), 1.59 (d, *J* = 17.5 Hz, 2H).

**Example 35: Preparation of Compound 35**

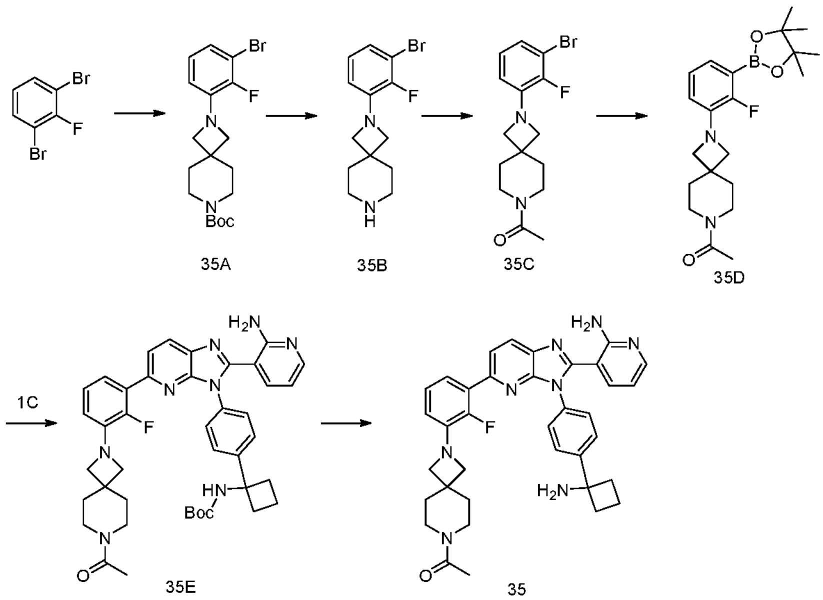


35A 35B 35C 35D

35E 35

**Step A: Preparation of compound 35A**

To a reaction flask were added 1,1'-binaphthyl-2,2'-bis-diphenylphosphine (0.21 g), tris(dibenzylideneacetone)dipalladium (0.20 g), 1,3-dibromo-2-fluorobenzene (1.68 g), tert-butyl 2,7-diazaspiro[3.5]nonane-7-carbonate (1.00 g), sodium *tert*-butoxide (1.70 g), and tetrahydrofuran (25 mL) successively under nitrogen atmosphere. After the addition was completed, the reaction solution was stirred at 85 °C. After the reaction was completed, the reaction solution was concentrated and subjected to column chromatography (petroleum ether/ethyl acetate = 5/1) to give compound 35A (1.51 g).

MS (ESI, $[M-100+H]^+$) *m/z*: 299.4.

**Step B: Preparation of compound 35B**

Compound 35A (1.51 g), methanesulfonic acid (3.62 g) and dichloromethane (60 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered and concentrated to give compound 35B (1.32 g).

MS (ESI, $[M+H]^+$) *m/z:* 299.4.

**Step C: Preparation of compound 35C**

To a reaction flask were added compound 35B (1.32 g), acetic anhydride (0.67 g), triethylamine (1.32 g) and dichloromethane (50 mL) successively. The mixture was stirred at room temperature. After the reaction was completed, water and dichloromethane were added to extract the reaction solution, and the reaction solution was washed with saturated brine and dried over anhydrous sodium sulfate. The reaction solution was filtered and concentrated to give compound 35C (1.32 g).
MS (ESI, $[M+H]^+$) *m/z:* 341.4.

**Step D: Preparation of compound 35D**

To a reaction flask were added compound 35C (1.32 g), bis(pinacolato)diboron (1.47 g), potassium acetate (1.14 g), [1,1'-bis(diphenylphosphino)ferrocene]palladium dichloride dichloromethane complex (0.32 g) and 1,4-dioxane (50 mL) successively. The reaction solution was stirred at 100 °C under nitrogen atmosphere. After the reaction was completed, the reaction solution was filtered and concentrated. The reaction solution was subjected to column chromatography (dichloromethane/methanol = 20/1) to give compound 35D (1.27 g).
MS (ESI, $[M+H]^+$) *m/z:* 389.6.

**Step E: Preparation of compound 35E**

To a reaction flask were added compound 35D (0.59 g), compound 1C (0.5 g), potassium carbonate (0.42 g), tetrakis(triphenylphosphine)palladium (0.12 g), 1,4-dioxane (25 mL), and water (5 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 35E (0.64 g).
MS (ESI, $[M+H]^+$) *m/z:* 717.8.

**Step F: Preparation of compound 35**

Compound 35E (0.63 g), methanesulfonic acid (0.84 g) and dichloromethane (30 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 35 (0.32 g).
MS (ESI, $[M+H]^+$) *m/z:* 617.7.
$^1H$ NMR (500 MHz, $CDCl_3$) δ 8.09 (d, *J* = 8.3 Hz, 1H), 8.05 (dd, *J* = 4.8, 1.5 Hz, 1H), 7.76 (dd, *J*= 8.3, 2.0 Hz, 1H), 7.53 (d, *J* = 8.4 Hz, 2H), 7.38 (d, *J* = 8.4 Hz, 2H), 7.24 (t, *J* = 6.7 Hz, 1H), 7.09 (dd, *J* = 7.8, 1.6 Hz, 1H), 7.03 (t, *J* = 7.8 Hz, 1H), 6.59 (s, 2H), 6.46 (t, *J* = 8.1 Hz, 1H), 6.36 (dd, *J* = 7.8, 4.9 Hz, 1H), 3.77 (s, 4H), 3.60 - 3.54 (m, 2H), 3.44 - 3.38 (m, 2H), 2.59 (ddd, *J* = 11.8, 8.9, 6.6 Hz, 2H), 2.19 (ddd, *J* = 11.5, 9.1, 6.0 Hz, 2H), 2.09 (s, 4H), 2.03 (s, 1H), 1.92 (s, 2H), 1.87 - 1.83 (m, 2H), 1.81 - 1.77 (m, 2H).

**Example 36: Preparation of Compound 36**

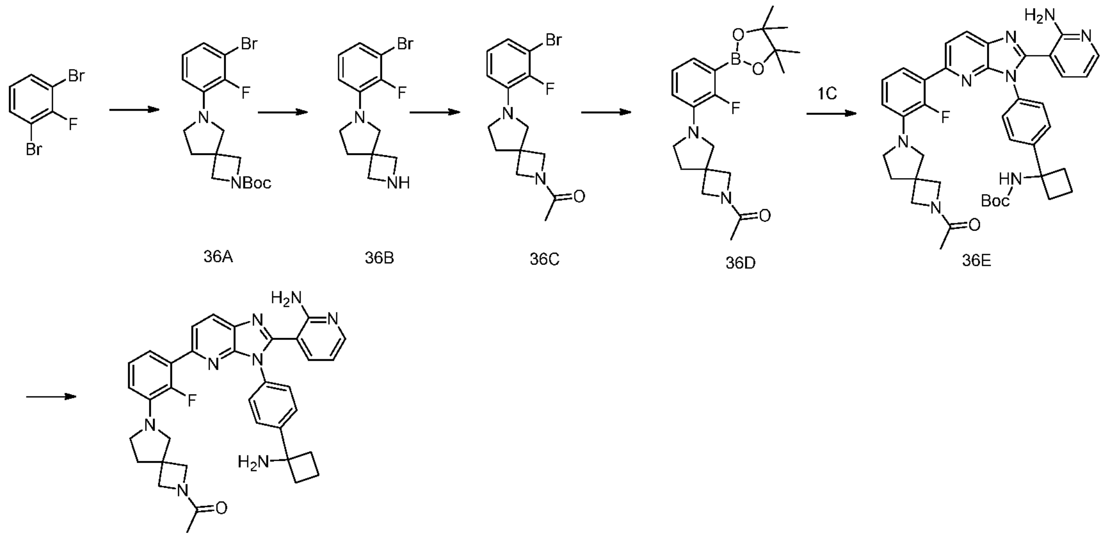


36A 36B 36C 36D 36E

36

**Step A: Preparation of compound 36A**

To a reaction flask were added 1,1'-binaphthyl-2,2'-bis-diphenylphosphine (0.088 g), tris(dibenzylideneacetone)dipalladium (0.086 g), 1,3-dibromo-2-fluorobenzene (0.72 g), tert-butyl 2,6-diaza-spiro[3.4]octane-2-carbonate (0.40 g), sodium tert-butoxide (0.72 g), and tetrahydrofuran (25 mL) successively under nitrogen atmosphere. After the addition was completed, the reaction solution was stirred at 85 °C. After the reaction was completed, the reaction solution was concentrated and subjected to column chromatography (petroleum ether/ethyl acetate = 9/1) to give compound 36A (620 mg).

MS (ESI, $[M+H]^+$) *m/z:* 385.4.

**Step B: Preparation of compound 36B**

Compound 36A (600 mg), methanesulfonic acid (1.55 g) and dichloromethane (30 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered and concentrated to give compound 36B (420 mg).

MS (ESI, $[M+H]^+$) *m/z:* 285.3.

Step C: **Preparation of compound 36C**

To a reaction flask were added compound 36B (0.42 g), acetic anhydride (0.23 g), triethylamine (0.45 g) and dichloromethane (20 mL) successively. The mixture was stirred at room temperature. After the reaction was completed, water and dichloromethane were added to extract the reaction solution, and the reaction solution was washed with saturated brine and dried over anhydrous sodium sulfate. The reaction solution was filtered and concentrated to give compound 36C (440 mg).

MS (ESI, $[M+H]^+$) *m/z*: 327.4.

**Step D: Preparation of compound 36D**

To a reaction flask were added compound 36C (0.44 g), bis(pinacolato)diboron (0.51 g), potassium acetate (0.40 g), [1,1'-bis(diphenylphosphino)ferrocene]palladium dichloride dichloromethane complex (0.11 g) and 1,4-dioxane (30 mL) successively. The reaction solution was stirred at 100 °C under nitrogen atmosphere. After the reaction was completed, the reaction solution was filtered and concentrated. The reaction solution was subjected to column chromatography (dichloromethane/methanol = 40/1) to give compound 36D (0.63 g).

MS (ESI, $[M+H]^+$) *m/z:* 375.5.

**Step E: Preparation of compound 36E**

To a reaction flask were added compound 36D (0.74 g), compound 1C (0.5 g), potassium carbonate (0.42 g), tetrakis(triphenylphosphine)palladium (0.12 g), 1,4-dioxane (25 mL), and water (5 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 36E (500 mg).

MS (ESI, $[M+H]^+$) *m/z*: 703.4.

**Step F: Preparation of compound 36**

Compound 36E (500 mg), methanesulfonic acid (0.7 g) and dichloromethane (20 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 36 (190 mg).

MS (ESI, $[M+H]^+$) *m/z:* 603.6.

$^1H$ NMR (500 MHz, $CDCl_3$) $\delta$ 8.12 (d, *J* = 8.3 Hz, 1H), 8.06 (dd, *J* = 4.8, 1.7 Hz, 1H), 7.76 (dd, *J* = 8.3, 2.4 Hz, 1H), 7.54 (d, *J* = 8.4 Hz, 2H), 7.40 (d, *J* = 8.4 Hz, 2H), 7.27 - 7.18 (m, 1H), 7.10 (dd, *J* = 7.8, 1.6 Hz, 1H), 7.06 (t, *J* = 7.9 Hz, 1H), 6.78 - 6.64 (m, 1H), 6.59 (s, 2H), 6.38 (dd, *J* = 7.8, 4.9 Hz, 1H), 4.11 (d, *J* = 8.4 Hz, 1H), 4.05 (d, *J* = 8.4 Hz, 1H), 4.01 (d, *J* = 9.9 Hz, 1H), 3.96 (d, *J* = 9.9 Hz, 1H), 3.60 (qd, *J* = 9.8, 2.0 Hz, 2H), 3.49 (ddq, *J* = 14.4, 9.5, 7.3 Hz, 2H), 2.66 - 2.54 (m, 2H), 2.27 - 2.17 (m, 4H), 2.16 - 1.94 (m, 4H), 1.89 (s, 3H).

**Example 37: Preparation of Compound 37**

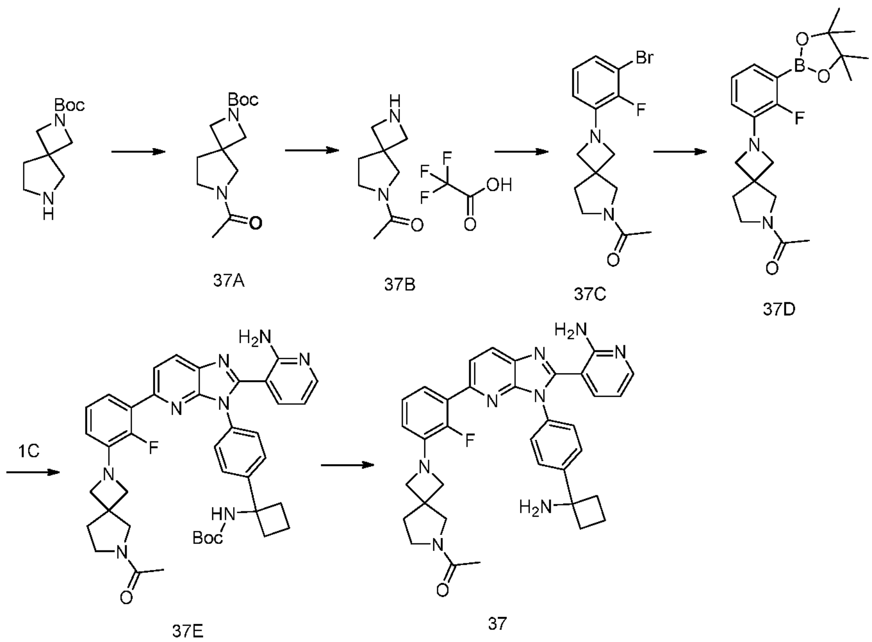


37A 37B 37C 37D 37E 37

**Step A: Preparation of compound 37A**

To a reaction flask were added *tert*-butyl 2,6-diaza-spiro[3.4]octane-2-carbonate (0.60 g), acetic anhydride (0.44 g), triethylamine (0.86 g) and methylene chloride (40 mL) successively. The mixture was stirred at room temperature. After the reaction was completed, water and dichloromethane were added to extract the reaction solution, and the reaction solution was washed with saturated brine and dried over anhydrous sodium sulfate. The reaction solution was filtered and concentrated to give compound 37A (0.82 g).

MS (ESI, $[M+H]^+$) *m/z:* 255.4.

**Step B: Preparation of compound 37B**

To a reaction flask were added compound 37A (0.82 g), trifluoroacetic acid (6.14 g), and dichloromethane (50 mL) successively. The mixture was stirred at room temperature. After the reaction was completed, the reaction solution was concentrated to give compound 37B (1.68 g).

MS (ESI, $[M+H]^+$) *m*/*z*: 155.4.

**Step C: Preparation of compound 37C**

To a reaction flask were added 1,1'-binaphthyl-2,2'-diphemyl phosphine (0.22 g), tris(dibenzylideneacetone)dipalladium (0.22 g), 1,3-dibromo-2-fluorobenzene (1.68 g), compound 37B (1.20 g), sodium *tert*-butoxide (0.88 g) and tetrahydrofuran (100 mL) successively under nitrogen atmosphere. After the addition was completed, the reaction solution was stirred at 85 °C. After the reaction was completed, the reaction solution was concentrated and subjected to column chromatography (petroleum ether/ethyl acetate = 9/1) to give compound 37C (960 mg).

MS (ESI, $[M+H]^+$) *m*/*z*: 327.4.

**Step D: Preparation of compound 37D**

To a reaction flask were added compound 37C (0.78 g), bis(pinacolato)diboron (0.92 g), potassium acetate (0.80 g), [1,1'-bis(diphenylphosphino)ferrocene]palladium dichloride dichloromethane complex (0.19 g) and 1,4-dioxane (60 mL) successively. The reaction solution was stirred at 100 °C under nitrogen atmosphere. After the reaction was completed, the reaction solution was filtered and concentrated. The reaction solution was subjected to column chromatography (dichloromethane/methanol = 20/1) to give compound 37D (0.64 g).

MS (ESI, $[M+H]^+$) *m*/*z:* 375.5.

**Step E: Preparation of compound 37E**

To a reaction flask were added compound 37D (0.53 g), compound 1C (0.32 g), potassium carbonate (0.27 g), tetrakis(triphenylphosphine)palladium (0.075 g), 1,4-dioxane (25 mL), and water (5 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 37E (0.43 g).

MS (ESI, $[M+H]^+$) *m*/*z:* 703.7.

**Step F: Preparation of compound 37**

Compound 37E (0.43 g), methanesulfonic acid (0.58 g) and dichloromethane (25 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 37 (0.10 g).

MS (ESI, $[M+H]^+$) *m*/*z:* 603.6.

$^1$H NMR (500 MHz, $CDCl_3$) δ 8.26 -7.99 (m, 2H), 7.76 (d, *J* = 8.1 Hz, 1H), 7.54 (d, *J* = 6.2 Hz, 2H), 7.39 (d, *J* = 7.5 Hz, 2H), 7.33 - 7.26 (m, 1H), 7.22 - 6.93 (m, 2H), 6.59 (s, 2H), 6.48 (dd, *J* = 13.6, 6.9 Hz, 1H), 6.38 (dd, *J* = 7.1, 5.1 Hz, 1H), 3.93 (s, 3H), 3.66 (d, *J* = 10.6 Hz, 2H), 3.54 (dt, *J* = 10.4, 6.9 Hz, 2H), 2.60 (dd, *J* = 17.4, 8.9 Hz, 2H), 2.27 (t, *J* = 6.7 Hz, 1H), 2.21 (dd, *J* = 15.6, 10.0 Hz, 2H), 2.12 (dd, *J* = 14.1, 7.5 Hz, 2H), 2.05 (t, *J* = 6.6 Hz, 3H), 1.98 (s, 3H), 1.87 - 1.75 (m, 1H).

**Example 38: Preparation of Compound 38**

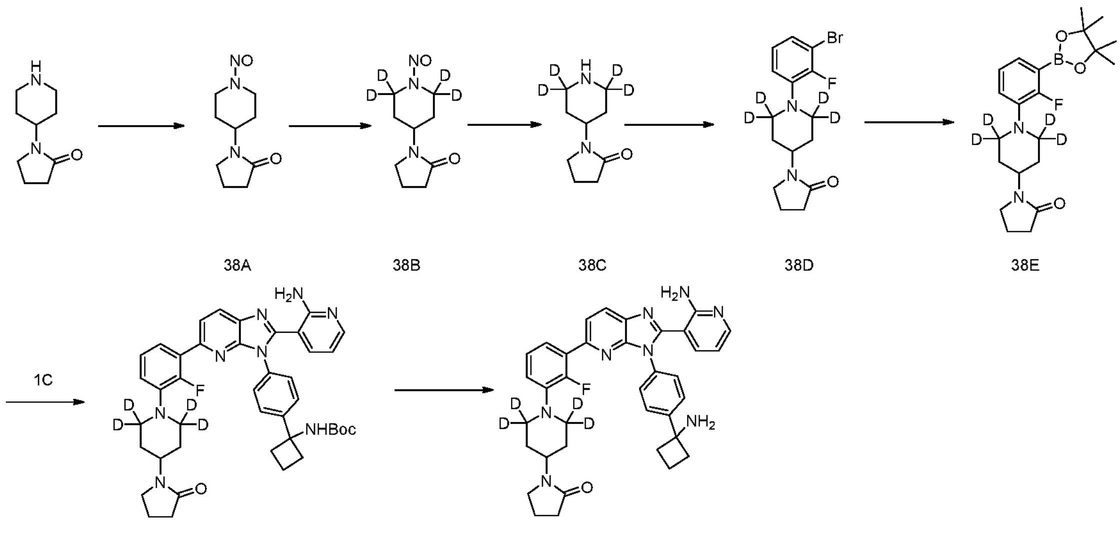


38A 38B 38C 38D 38E

38F 38

**Step A: Preparation of compound 38A**

Diluted hydrochloric acid (0.433 g) was added dropwise to 1-(piperidin-4-yl)pyrrolidin-2-one (1 g) and water (10 mL) at 0 °C, and after the addition was completed, an aqueous sodium nitrite solution (0.533 g) was slowly added dropwise and stirred at room temperature. After the reaction was completed, the reaction solution was extracted with dichloromethane, dried over anhydrous sodium sulfate, filtered and concentrated to give compound 38A (0.7 g).
MS (ESI, $[M+H]^+$) *m*/*z*: 198.4.

**Step B: Preparation of compound 38B**

Heavy water (8 mL) was slowly added to compound 38A (0.7 g) and sodium methoxide (0.553 g) under nitrogen atmosphere, and after the addition was completed, the mixture was stirred at 80 °C. Deuterated ethanol (5 mL) was added slowly to the reaction flask, and then the reaction solution was stirred at 80 °C. After the reaction was completed, the reaction solution was extracted with dichloromethane, dried over anhydrous sodium sulfate, filtered under vacuum and concentrated to give compound 38B (0.45 g).
$^1H$ NMR (500 MHz, $CDCl_3$) δ 4.40 - 4.29 (m, 1H), 3.33 - 3.21 (m, 2H), 2.00 (t, *J* = 7.0 Hz, 2H), 1.97 - 1.92 (m, 1H), 1.82 - 1.71 (m, 2H), 1.44 (tt, *J* = 14.8, 7.3 Hz, 1H), 1.23 (s, 1H), 0.92 - 0.75 (m, 1H).

**Step C: Preparation of compound 38C**

Sodium methoxide (0.362 g) was added to a solution of compound 38B (0.45 g) in deuterated ethanol (2.5 mL) and heavy water (5.00 mL) at room temperature, and after the addition was completed, nickel-aluminum alloy (1.35 g) was added to the reaction flask, and the temperature was controlled at 30-40 °C. After the addition was completed, the reaction solution was stirred at 35 °C. After the reaction was completed, the reaction solution was filtered and concentrated, and the residue was extracted with methylene chloride, dried over anhydrous sodium sulfate, filtered and concentrated to give compound 38C (0.23 g).
MS (ESI, $[M+H]^+$) *m*/*z*: 173.5.

**Step D: Preparation of compound 38D**

To a microwave tube were added sodium *tert*-butoxide (0.352 g), 1,1'-binaphthyl-2,2'-bis-diphenylphosphine (0.152 g), tris(dibenzylideneacetone)dipalladium (0.224 g), 1,3-dibromo-2-fluorobenzene (0.31 g), compound 38C and 1,4-dioxane (10 mL) successively, and the reaction solution was stirred for reaction under nitrogen atmosphere at 100°C. After the reaction solution was completed, the reaction solution was filtered and concentrated. The reaction solution was subjected to column chromatography (dichloromethane/methanol = 40/1) to give compound 38D (0.14 g).
MS (ESI, $[M+H]^+$) *m/z:* 345.3.

**Step E: Preparation of compound 38E**

To a reaction flask were added compound 38D (0.14 g), bis(pinacolato)diboron (0.21 g), potassium acetate (0.12 g), [1,1'-bis(diphenylphosphino)ferrocene]palladium dichloride dichloromethane complex (0.066 g) and 1,4-dioxane (15 mL) successively. The reaction solution was stirred for reaction at 100 °C under nitrogen atmosphere. After the reaction was completed, the reaction solution was filtered and concentrated. The reaction solution was subjected to column chromatography (dichloromethane/methanol = 40/1) to give compound 38E (0.13 g).

MS (ESI, $[M+H]^+$) *m/z:* 393.4.

**Step F: Preparation of compound 38F**

To a reaction flask were added compound 38E (0.13 g), compound 1C (0.1 g), potassium carbonate (0.056 g), tetrakis(triphenylphosphine)palladium (0.047 g), 1,4-dioxane (10 mL), and water (2 mL) successively. After the addition was completed, the reaction solution was purged with nitrogen gas, and stirred for reaction at 100 °C. After the reaction was completed, the reaction solution was filtered and concentrated. The reaction solution was subjected to column chromatography (dichloromethane/methanol = 30/1) to give compound 38F (0.03 g).

MS (ESI, $[M+H]^+$) *m/z:* 721.4.

**Step G: Preparation of compound 38**

To a reaction flask were added compound 38F (0.028 g), methanesulfonic acid (0.019 g) and dichloromethane (20 mL) successively, and the reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 38 (0.013 g).

MS (ESI, $[M+H]^+$) *m/z:* 621.5.

$^1$HNMR (500 MHz, $CDCl_3$) δ 8.11 (d, *J* = 8.3 Hz, 1H), 8.06 (d, *J* = 3.6 Hz, 1H), 7.81 (d, *J* = 6.9 Hz, 1H), 7.55 (d, *J* = 8.2 Hz, 2H), 7.51 (t, *J* = 7.1 Hz, 1H), 7.40 (d, *J* = 8.2 Hz, 2H), 7.12 (dd, *J* = 12.9, 7.2 Hz, 2H), 7.00 (t, *J* = 7.6 Hz, 1H), 6.57 (s, 2H), 6.39 (dd, *J* = 7.5, 4.9 Hz, 1H), 4.15 (t, *J* = 12.1 Hz, 1H), 3.48 (s, 1H), 3.41 (t, *J* = 6.9 Hz, 2H), 2.65 - 2.57 (m, 2H), 2.41 (dd, *J* = 16.7, 8.4 Hz, 1H), 2.28 - 2.21 (m, 2H), 2.20 - 2.09 (m, 2H), 2.03 (dd, *J* = 13.9, 7.0 Hz, 2H), 1.93 (dd, *J* = 16.1, 7.5 Hz, 2H), 1.78 (d, *J* = 12.3 Hz, 2H).

**Example 39: Preparation of Compound 39**

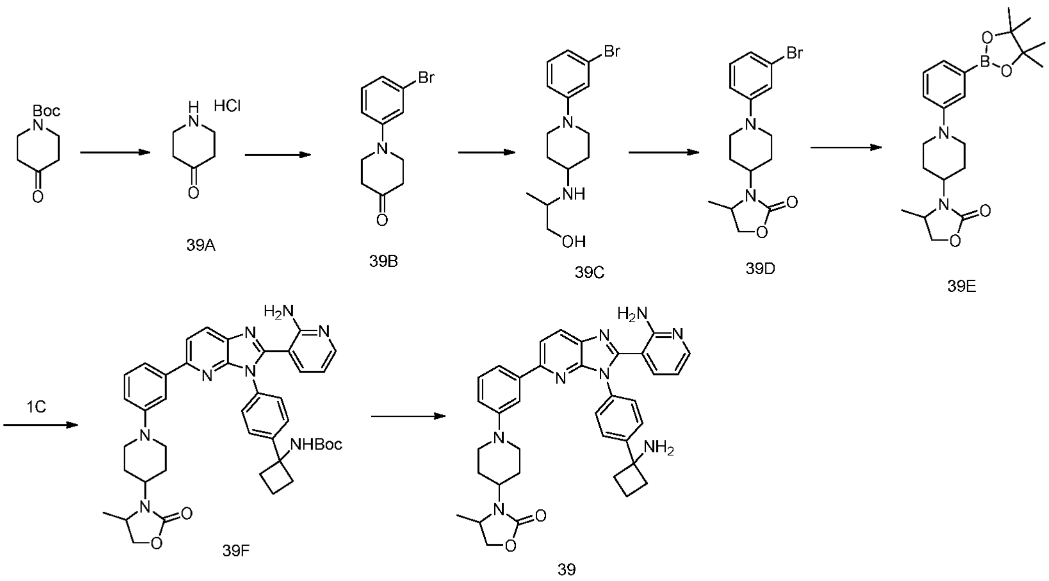


39A 39B 39C 39D 39E

39F 39

**Step A: Preparation of compound 39A**

Diluted hydrochloric acid (0.914 g) was slowly added to a solution of *tert*-butyl 4-oxopiperidine-1-carboxylate (1 g) in methylene chloride (20 mL) at room temperature, and after the addition was completed, the mixture was stirred at room temperature. After the reaction was completed, the reaction solution was concentrated to give compound 39A (0.7 g).
MS (ESI, $[M+H]^+$) *m/z*: 100.1.

**Step B: Preparation of compound 39B**

To a reaction flask were added compound 39A (0.7 g), 1-bromo-3-iodobenzene (1.461 g), potassium carbonate (2.85 g), cuprous iodide (0.098 g), *L*-alanine (0.119 g), and dimethyl sulfoxide (10 mL) successively, and the reaction solution was stirred for reaction at 100 °C under nitrogen atmosphere. After the reaction was completed, the reaction solution was filtered, concentrated, and subjected to column chromatography (petroleum ether/ethyl acetate = 5/1) to give compound 39B (0.088 g).
MS (ESI, $[M+H]^+$) *m/z:* 254.1.

**Step C: Preparation of compound 39C**

To a reaction flask were added compound 39B (0.3 g), 2-aminopropan-1-ol (0.177 g), acetic acid (0.142 g) and methanol (10 mL) successively, and the reaction solution was stirred for 1 h at room temperature under nitrogen atmosphere. Sodium cyanoborohydride (0.148 g) was added to the reaction flask, and the reaction solution was stirred at room temperature. After the reaction was completed, the reaction solution was concentrated and subjected to column chromatography (dichloromethane/methanol = 25/1) to give compound 39C (0.23 g)
MS (ESI, $[M+H]^+$) *m/z:* 313.4.

**Step D: Preparation of compound 39D**

To a reaction flask were added *N*,*N*-carbonyldiimidazole (0.179 g), compound 39C (0.23 g), toluene (10 mL) successively, and the reaction solution was stirred at room temperature for 1 h under nitrogen atmosphere and then stirred at 110 °C. After the reaction was completed, the reaction solution was concentrated, and subjected to column chromatography (petroleum ether/ethyl acetate = 5/1) to give compound 39D (0.16 g).
MS (ESI, $[M+H]^+$) *m/z:* 339.1.

**Step E: Preparation of compound 39E**

To a reaction flask were added compound 39D (0.16 g), bis(pinacolato)diboron (0.24 g), potassium acetate (0.14 g), [1,1'-bis(diphenylphosphino)ferrocene]palladium dichloride dichloromethane complex (0.039 g) and 1,4-dioxane (20 mL) successively. The reaction solution was stirred for reaction at 100 °C under nitrogen atmosphere. After the reaction was completed, the reaction solution was filtered and concentrated. The reaction solution was subjected to column chromatography (dichloromethane/methanol = 50/1) to give compound 39E (0.06 g).
MS (ESI, $[M+H]^+$) *m/z:* 387.6.

**Step F: Preparation of compound 39F**

To a reaction flask were added compound 39E (0.06 g), compound 1C (0.08 g), potassium carbonate (0.045 g), tetrakis(triphenylphosphine)palladium (0.038 g), 1,4-dioxane (10 mL), and water (2 mL) successively. After the addition was completed, the reaction solution was purged with nitrogen gas, and stirred for reaction at 100 °C. After the reaction was completed, the reaction solution was filtered and concentrated. The reaction solution was subjected to column chromatography (dichloromethane/methanol = 30/1) to give compound 39F (65 mg).
MS (ESI, $[M+H]^+$) *m/z:* 715.5.

**Step G: Preparation of compound 39**

To a reaction flask were added compound 39F (0.065 g), methanesulfonic acid (0.051 g) and dichloromethane (20 mL) successively, and the reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated

brine and dried over anhydrous sodium sulfate. The reaction solution was filtered, concentrated, and subjected to column chromatography (dichloromethane/methanol = 20/1) to give compound 39 (0.032 g).

MS (ESI, $[M+H]^+$) *m/z:* 615.7.

$^1H$ NMR (500 MHz, $CDCl_3$) δ 8.05 (d, *J* = 8.3 Hz, 1H), 7.91 (d, *J* = 4.0 Hz, 1H), 7.71 (d, *J* = 8.4 Hz, 1H), 7.66 (d, *J* = 8.2 Hz, 2H), 7.49 (s, 1H), 7.45 (d, *J* = 7.6 Hz, 1H), 7.40 (d, *J* = 8.2 Hz, 2H), 7.24 (d, *J* = 7.9 Hz, 1H), 7.09 (d, *J* = 7.5 Hz, 1H), 6.88 (d, *J* = 7.9 Hz, 3H), 6.41 (dd, *J* = 7.2, 5.3 Hz, 1H), 4.35 (t, *J* = 8.3 Hz, 1H), 4.00 - 3.90 (m, 1H), 3.82 (dd, *J* = 8.2, 5.6 Hz, 1H), 3.77 - 3.65 (m, 3H), 2.86 - 2.72 (m, 4H), 2.68 (s, 4H), 2.37 (d, *J* = 7.5 Hz, 1H), 2.07 (qd, *J* = 12.1, 3.7 Hz, 1H), 1.99 - 1.92 (m, 2H), 1.88 (d, *J* = 7.1 Hz, 1H), 1.81 (d, *J* = 10.8 Hz, 1H), 1.32 (d, *J* = 6.1 Hz, 3H).

**Example 40: Preparation of Compound 40**

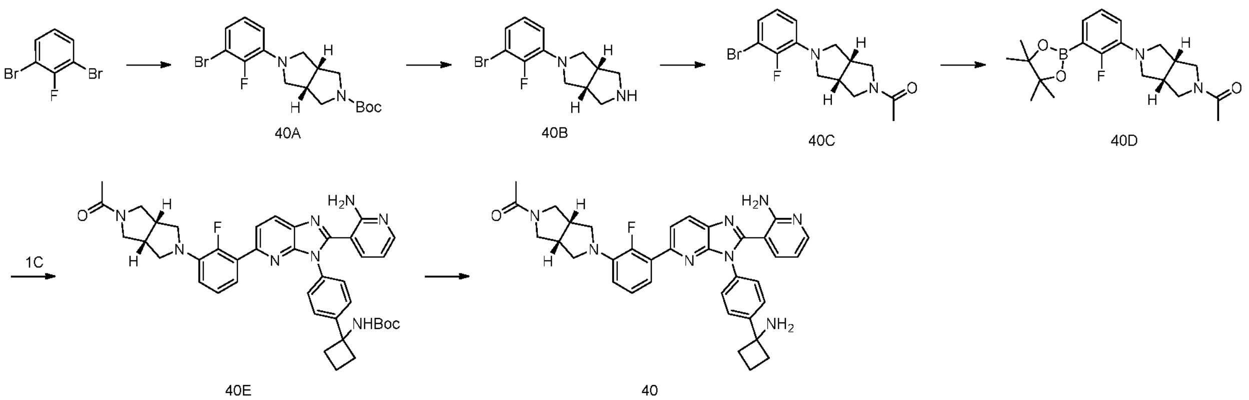


40A 40B 40C 40D

40E 40

**Step A: Preparation of compound 40A**

To a reaction flask were added *Cis*-2-Boc-hexahydropyrrolo[3,4-c]pyrrole (250 mg), 1,3-dibromo-2-fluorobenzene (448 mg), tris(dibenzylideneacetone)dipalladium (55 mg), 1,1'-binaphthyl-2,2'-diphemyl phosphine (40 mg), tetrahydrofuran (30 mL) and sodium tert-butoxide (509 mg) successively, and the reaction solution was stirred for reaction at 80 °C under nitrogen atmosphere. After the reaction was completed, the reaction solution was concentrated and subjected to column chromatography (petroleum ether/ethyl acetate = 5/1) to give compound 40A (164 mg).

MS (ESI, $[M+H]^+$) *m/z:* 385.0.

**Step B: Preparation of compound 40B**

To a reaction flask containing compound 40A (164 mg) were added dichloromethane (15 mL) and methanesulfonic acid (130 mg) successively, and after the addition was completed, the reaction solution was stirred for reaction at room temperature. After the reaction was completed, and the reaction solution was directly used in the next step without treatment.

**Step C: Preparation of compound 40C**

To the reaction solution containing compound 40B were added triethylamine (42.9 mg) and acetic anhydride (43.3 mg), and after the addition was completed, the reaction solution was stirred for reaction at room temperature. After the reaction was completed, the reaction solution was washed with water 2 times and dried over anhydrous sodium sulfate. The reaction solution was filtered, and the filtrate was concentrated to give compound 40C (142 mg).

MS (ESI, $[M+H]^+$) *m/z*: 327.4.

**Step D: Preparation of compound 40D**

To a reaction flask were added compound 40C (142 mg), bis(pinacolato)diboron (164 mg), potassium acetate (127 mg), [1,1'-bis(diphenylphosphino)ferrocene]palladium dichloride dichloromethane complex (50 mg) and 1,4-dioxane (10 mL) successively. The reaction solution was stirred for reaction at 100 °C under nitrogen atmosphere. After the reaction was completed, the reaction solution was filtered and concentrated. The reaction solution was subjected to column chromatography (petroleum ether/ethyl acetate = 5/1) to give compound 40D (101 mg).

MS (ESI, $[M+H]^+$) *m/z:* 375.4.

**Step E: Preparation of compound 40E**

To a microwave tube were added compound 40D (100 mg), compound 1C (135 mg), potassium carbonate (150 mg), tetrakis(triphenylphosphine)palladium (20 mg), 1,4-dioxane (3 mL) and water (0.3 mL) successively. After the addition was completed, the reaction solution was purged with nitrogen gas, and reacted at 120 °C in microwave reactor. After the reaction was completed, the reaction solution was filtered and concentrated. The reaction solution was subjected to column chromatography (dichloromethane/methanol = 20/1) to give compound 40E (101 mg).
MS (ESI, $[M+H]^+$) *m/z:* 703.5.

**Step F: Preparation of compound 40**

To a reaction flask were added compound 40E (101 mg), methanesulfonic acid (138 mg) and dichloromethane (10 mL) successively, and after the addition was completed, the reaction solution was stirred for reaction at room temperature. After the reaction was completed, a saturated aqueous sodium carbonate solution was added to the reaction solution to adjust the pH to 9, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 20/1) to give compound 40 (42 mg).
MS (ESI, $[M+H]^+$) *m/z:* 603.7.
$^1H$ NMR (500 MHz, $CDCl_3$) $\delta$ 8.12 (d, *J* = 8.5 Hz, 1H), 8.05 (d, *J* = 1.0 Hz, 1H), 7.76 (d, *J* = 7.0 Hz, 1H), 7.54 (d, *J* = 8.0 Hz, 2H), 7.40 (d, *J* = 8.0 Hz, 2H), 7.26-7.24 (m, 1H), 7.10 (d, *J* = 7.5 Hz, 1H), 7.06 (t, *J* = 8.0 Hz, 1H), 6.71 (t, *J* = 8.0 Hz, 1H), 6.58 (s, 2H), 6.39 (dd, *J* = 5.0, 7.5 Hz, 1H), 3.80-3.75 (m, 2H), 3.61-3.56 (m, 2H), 3.54-3.51 (m, 1H), 3.43-3.40 (m, 1H), 3.37-3.32 (m, 2H), 3.10-3.04 (m, 1H), 3.02-2.96 (m, 1H), 2.63-2.58 (m, 2H), 2.27-2.24 (m, 2H), 2.17-2.12 (m, 2H), 2.06 (s, 3H), 2.06-2.04 (m, 1H), 1.86-1.78 (m, 1H).

**Example 41: Preparation of Compound 41**

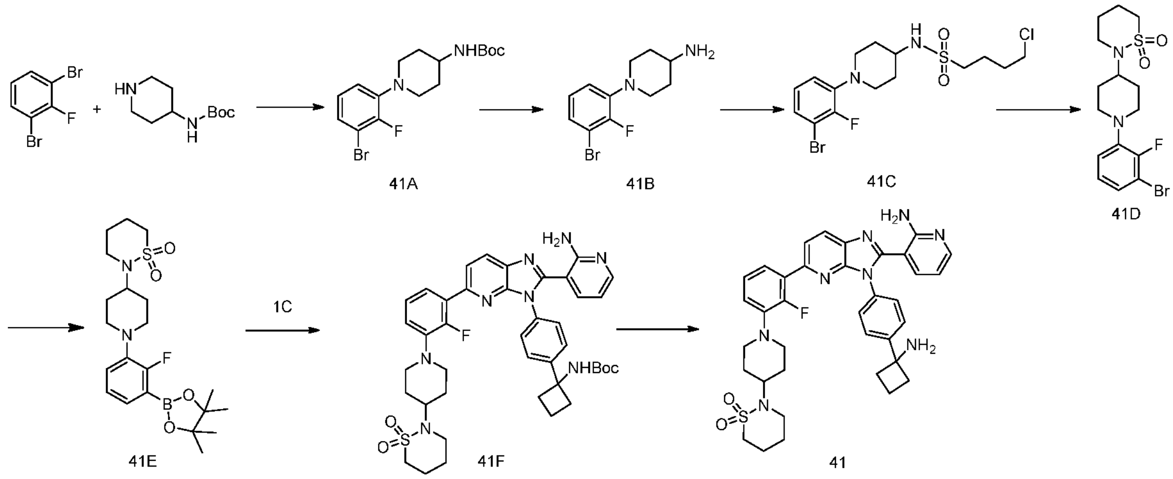


41A 41B 41C 41D 41E 41F 41

**Step A: Preparation of compound 41A**

To a reaction flask were added 2,2'-bis(diphenylphosphino)-1,1'-binaphthyl (2.94 g), bis(benzhydrylacetylene)palladium (2.164 g), 1,3-dibromo-2-fluorobenzene (6 g), *tert*-butylpiperidin-4-ylcarbamate (4.73 g), sodium *tert*-butoxide (4.54 g), dioxane (20 mL) successively, and the mixture was stirred overnight at 100 °C under nitrogen atmosphere. After the reaction was completed, the reaction solution was filtered, concentrated, and subjected to column chromatography (dichloromethane/methanol = 50/1) to give compound 41A (2.6 g).
MS (ESI, $[M+H]^+$) *m/z:* 373.2.

**Step B: Preparation of compound 41B**

To a reaction flask were added compound 41A (2.6 g), dichloromethane (30 mL) and a solution (7.37 mL) of hydrochloric acid in dioxane successively, and the mixture was stirred at room temperature for 1 h. 10% aqueous sodium hydroxide solution was added to the reaction solution to adjust the pH to 13, water and dichloromethane were added to extract the reaction solution the reaction solution was washed with saturated brine and dried over anhydrous sodium sulfate. The reaction solution was filtered and concentrated to give compound 41B (2 g).
MS (ESI, $[M+H]^+$) *m/z:* 273.3.

**Step C: Preparation of compound 41C**

In an ice-water bath, 4-chlorobutane-1-sulfonyl chloride (0.611 g) was slowly added to a solution of compound 41B (0.873 g) in *N*,*N*-dimethylformamide (15 mL), and after the addition was completed, the reaction solution was slowly warmed to room temperature and stirred. After the reaction was completed, the reaction solution was added to 30 mL of ice water, extracted with ethyl acetate, washed with saturated brine, dried over anhydrous sodium sulfate, filtered, concentrated, and subjected to column chromatography (petroleum ether/ethyl acetate = 2/1) to give compound 41C (0.826 g).
MS (ESI, $[M+H]^+$) *m/z:* 427.4.

**Step D: Preparation of compound 41D**

After compound 41C (0.826 g) was dissolved in *N*,*N*-dimethylformamide (15 mL), sodium hydrogen (153 mg) was added under an ice-bath condition, and after the addition was completed, the reaction solution was refluxed at 80 °C overnight. After the reaction was completed, the reaction solution was poured into 30 mL of ice water, extracted with ethyl acetate, washed with saturated brine, dried over anhydrous sodium sulfate, filtered, concentrated, and subjected to column chromatography (petroleum ether/ethyl acetate = 2/3) to give compound 41D (0.3 g).
MS (ESI, $[M+H]^+$) *m/z:* 391.6.

**Step E: Preparation of compound 41E**

To a reaction flask were added compound 41D (0.3 g), bis(pinacolato)diboron (0.389 g), potassium acetate (0.15 g), [1,1'-bis(diphenylphosphino)ferrocene]palladium dichloride dichloromethane complex (0.125 g) and 1,4-dioxane (15 mL) successively. The reaction solution was stirred for reaction at 100 °C under nitrogen atmosphere. After the reaction was completed, the reaction solution was filtered and concentrated. The reaction solution was subjected to column chromatography (petroleum ether/ethyl acetate = 7/3) to give compound 41E (0.265 g).
MS (ESI, $[M+H]^+$) *m/z:* 439.5.

**Step F: Preparation of compound 41F**

To a reaction flask were added compound 41E (0.265 g), compound 1C (0.187 g), potassium carbonate (0.105 g), tetrakis(triphenylphosphine)palladium (0.088 g), 1,4-dioxane (10 mL), and water (2 mL) successively. After the addition was completed, the reaction solution was purged with nitrogen gas, and stirred for reaction at 110 °C. After the reaction was completed, the reaction solution was filtered and concentrated. The reaction solution was subjected to column chromatography (dichloromethane/10% methanol = 4/1) to give compound 41F (184 mg).
MS (ESI, $[M+H]^+$) *m/z:* 767.7.

**Step G: Preparation of compound 41**

Compound 41F (171 mg), methanesulfonic acid (0.142 mL) and dichloromethane (10 mL) were added successively to a reaction flask, and the mixture was stirred at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/10% methanol = 1/1) to give compound 41 (80 mg).
MS (ESI, $[M+H]^+$) *m/z:* 667.6.
$^1$H NMR (500 MHz, DMSO-$d_6$) δ 8.26 (d, 1H), 8.00 (s, 1H), 7.73 (s, 1H), 7.60 (s, 2H), 7.44 (s, 2H), 7.29 (s, 1H), 7.09 - 7.19 (m, 3H), 6.94 (s, 2H), 6.41 (s, 1H), 3.86 (s, 1H), 3.56 (s, 2H), 3.42 (s, 2H), 3.06 (s, 2H), 2.77 (s, 2H), 2.43 (s, 2H), 2.17 (s, 2H), 2.04 (s, 3H), 1.89 (d, 2H), 1.72 (s, 2H), 1.60 (s, 2H).

**Example 42: Preparation of Compound 42**

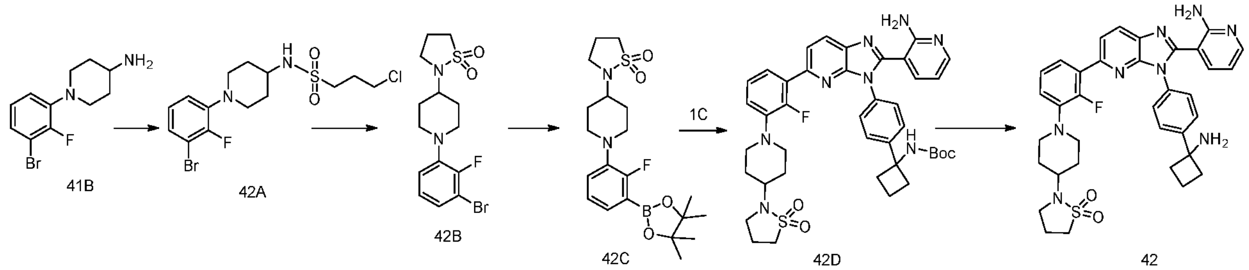


**Step A: Preparation of compound 42A**

In an ice-water bath, 3-chloropropane-1-sulfonyl chloride (0.538 g) was slowly added to a solution of compound 41B (0.83 g) in *N*,*N*-dimethylformamide (15 mL), and after the addition was completed, the reaction solution was slowly warmed to room temperature and stirred. After the reaction was completed, the reaction solution was added to 30 mL of ice water, extracted with ethyl acetate, washed with saturated brine, dried over anhydrous sodium sulfate, filtered, concentrated, and subjected to column chromatography (petroleum ether/ethyl acetate = 13/7) to give compound 42A (0.212 g).
MS (ESI, $[M+H]^+$) *m/z:* 413.4.

**Step B: Preparation of compound 42B**

After compound 42A (0.212 g) was dissolved in *N*,*N*-dimethylformamide (15 mL), sodium hydrogen (146 mg) was added under an ice-bath condition, and after the addition was completed, the reaction solution was refluxed at 80 °C overnight. After the reaction was completed, the reaction solution was poured into 30 mL of ice water, extracted with ethyl acetate, washed with saturated brine, dried over anhydrous sodium sulfate, filtered, concentrated, and subjected to column chromatography (petroleum ether/ethyl acetate = 2/3) to give compound 42B (0.249 g).
MS (ESI, $[M+H]^+$) *m/z:* 377.3.

**Step C: Preparation of compound 42C**

To a reaction flask were added compound 42B (0.249 g), bis(pinacolato)diboron (0.335 g), potassium acetate (0.13 g), [1,1'-bis(diphenylphosphino)ferrocene]palladium dichloride dichloromethane complex (0.054 g) and 1,4-dioxane (15 mL) successively. The reaction solution was stirred for reaction at 100 °C under nitrogen atmosphere. After the reaction was completed, the reaction solution was filtered and concentrated. The reaction solution was subjected to column chromatography (petroleum ether/ethyl acetate = 1/1) to give compound 42C (0.21 g).
MS (ESI, $[M+H]^+$) *m/z:* 425.3.

**Step D: Preparation of compound 42D**

To a reaction flask were added compound 42C (0.21 g), compound 1C (0.243 g), potassium carbonate (0.137 g), tetrakis(triphenylphosphine)palladium (0.057 g), 1,4-dioxane (10 mL), and water (2 mL) successively. After the addition was completed, the reaction solution was purged with nitrogen gas, and stirred for reaction at 110 °C. After the reaction was completed, the reaction solution was filtered and concentrated. The reaction solution was subjected to column chromatography (dichloromethane/10% methanol = 3/7) to give compound 42D (185 mg).
MS (ESI) *m/z:* 753.7 $[M+H]^+$.

**Step E: Preparation of compound 42**

Compound 42D (185 mg), methanesulfonic acid (0.16 mL) and dichloromethane (10 mL) were added successively to a reaction flask, and the mixture was stirred at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography

(dichloromethane/10% methanol = 2/3) to give compound 42 (40 mg).

MS (ESI, [M+H]$^+$) *m/z:* 653.6.

$^1$H NMR (500 MHz, DMSO-$d_6$) δ 8.26 (d, 1H), 8.00 (d, 1H), 7.7 (d, 1H), 7.59 (d, 2H), 7.42 (d, 2H), 7.28 (t, 1H), 7.08 - 7.20 (m, 3H), 6.90 (s, 2H), 6.41 (t, 1H), 3.4 (d, 2H), 3.28 (t, 3H), 3.19 (t, 2H), 2.8 (m, 2H), 2.42 (q, 2H), 2.2 (m, 2H), 2.02 - 2.13 (m,3H), 1.88 (s, 4H), 1.70 (m, 1H).

**Example 43: Preparation of Compound 43**

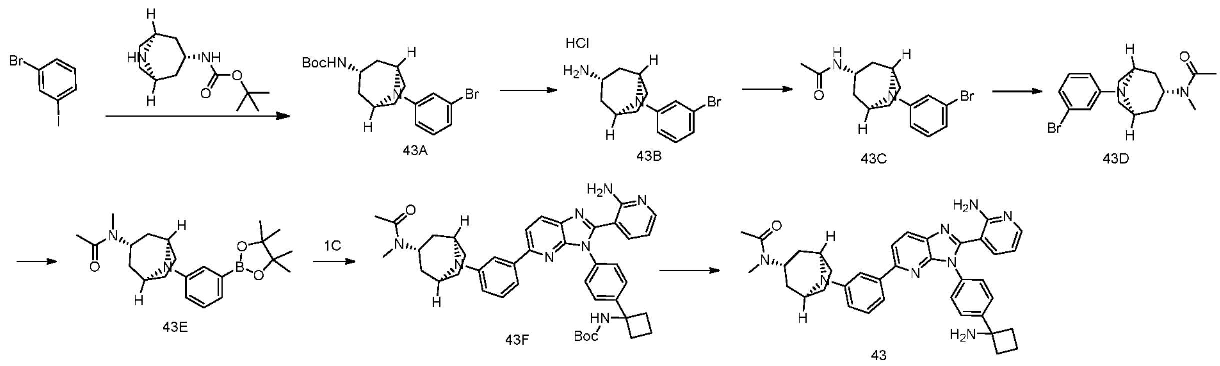

43A 43B 43C 43D 43E 43F 43

**Step A: Preparation of compound 43A**

To a reaction flask were added *tert*-butyl exo-8-azabicyclo[3.2.1]octan-3-carbamate (0.5 g), 1-bromo-3-iodobenzene (0.75 g), potassium carbonate (0.92 g), cuprous iodide (0.05 g), *L*-proline (0.02 g), and dimethyl sulfoxide (10 mL) successively. After the addition was completed, the reaction solution was stirred for reaction at 100 °C under nitrogen atmosphere. After the reaction was completed, the reaction solution was filtered, concentrated, and subjected to column chromatography (petroleum ether/ethyl acetate = 5/1) to give compound 43A (0.14 g).

MS (ESI, [M+H]$^+$) *m/z:* 381.5.

**Step B: Preparation of compound 43B**

To a reaction flask were added compound 43A (0.14 g) and a solution of 4 M dioxane in hydrogen chloride (4 mL) successively, and the mixture was stirred at room temperature. After the reaction was completed, the reaction solution was concentrated to give compound 43B (0.15 g).

MS (ESI, [M+H]$^+$) *m/z:* 281.8.

**Step C: Preparation of compound 43C**

Acetic anhydride (0.07 g) was slowly added to a reaction solution of compound 43B (0.15 g) and triethylamine (0.24 g) in dichloromethane (6 mL) at 0 °C, and after the addition was completed, the reaction solution was transferred to the room temperature condition and stirred for reaction. After the reaction was completed, a saturated aqueous sodium bicarbonate solution (50 mL) was added to the reaction solution, and the reaction solution was extracted with methylene chloride (30 mL × 3), and the organic phases were combined, washed with a saturated aqueous sodium chloride solution (50 mL) and dried. The reaction solution was filtered and concentrated to give compound 43C (0.11 g). MS (ESI, [M+H]$^+$) *m/z:* 323.4.

**Step D: Preparation of compound 43D**

Sodium hydride (0.07 g) was slowly added to a solution of compound 43C (0.22 g) in tetrahydrofuran (12 mL) at 0 °C under nitrogen atmosphere, and after the addition was completed, the reaction solution was transferred to the room temperature condition and stirred for 0.5 h, and further added with methyl iodide (0.48 g) for reaction. After the reaction was completed, a saturated aqueous ammonium chloride solution (50 mL) was added to the reaction solution in an ice bath, and the reaction solution was extracted with ethyl acetate (50 mL × 3), and the organic phases were combined, washed with a saturated aqueous sodium chloride solution (100 mL), and dried. The reaction solution was filtered and concentrated to give compound 43D (0.22 g).

MS (ESI, $[M+H]^+$) *m/z:* 336.8.

**Step E: Preparation of compound 43E**

To a reaction flask were added compound 43D (0.22 g), bis(pinacolato)diboron (0.25 g), potassium acetate (0.20 g), [1,1'-bis(diphenylphosphino)ferrocene]palladium dichloride dichloromethane complex (0.05 g) and 1,4-dioxane (10 mL) successively. The reaction solution was stirred for reaction at 100 °C under nitrogen atmosphere. After the reaction was completed, the reaction solution was filtered and concentrated. The reaction solution was subjected to column chromatography (dichloromethane/methanol = 20/1) to give compound 43E (0.1 g).

MS (ESI, $[M+H]^+$) *m/z:* 385.6.

**Step F: Preparation of compound 43F**

To a microwave tube were added compound 43E (0.10 g), compound 1C (0.11 g), potassium carbonate (0.09 g), tetrakis(triphenylphosphine)palladium (0.03 g), 1,4-dioxane (4 mL) and water (0.5 mL) successively. After the addition was completed, the reaction solution was purged with nitrogen gas, and reacted at 100 °C in microwave reactor. After the reaction was completed, the reaction solution was filtered and concentrated. The reaction solution was subjected to column chromatography (dichloromethane/methanol = 20/1) to give compound 43F (0.10 g).

MS (ESI, $[M+H]^+$) *m/z:* 713.7.

**Step F: Preparation of compound 43**

Compound 43F (100 mg), methanesulfonic acid (0.10 mL) and dichloromethane (6 mL) were added successively to a reaction flask, and the mixture was stirred at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 43 (26 mg).

MS (ESI, $[M+H]^+$) *m/z:* 613.7.

$^1H$ NMR (500 MHz, $CDCl_3$) δ 8.06 (dt, *J* = 36.6, 6.5 Hz, 2H), 7.76 (q, *J* = 6.6, 5.8 Hz, 1H), 7.58 (t, *J* = 7.4 Hz, 2H), 7.53 - 7.39 (m, 3H), 7.39 - 7.24 (m, 2H), 7.15 (q, *J* = 6.9 Hz, 1H), 6.79 (t, *J* = 7.5 Hz, 1H), 6.61 (s, 2H), 6.39 (q, *J* = 6.4 Hz, 1H), 5.18 (dq, *J* = 12.7, 6.5 Hz, 1H), 4.35 (d, *J* = 14.0 Hz, 2H), 2.61 (q, *J* = 9.4, 8.6 Hz, 3H), 2.55 - 2.30 (m, 6H), 2.25 - 2.05 (m, 4H), 2.05 - 1.77 (m, 6H), 1.53 - 1.20 (m, 3H).

**Example 44: Preparation of Compound 44**

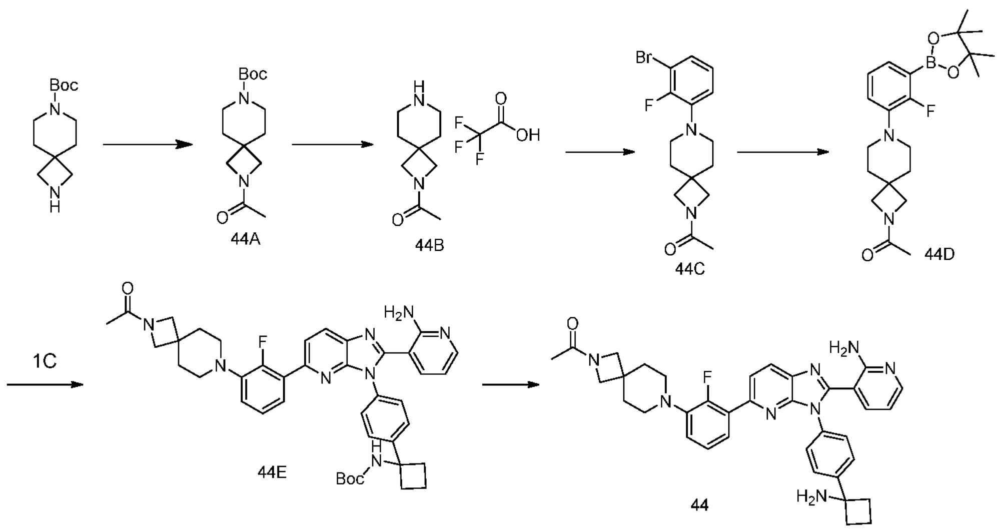

**Step A: Preparation of compound 44A**

Acetic anhydride (0.68 g) was slowly added to a reaction solution of *tert*-butyl 2,7-diazaspiro[3.5]nonane-7-carboxylate (1 g) and triethylamine (2.24 g) in methylene chloride (20 mL) at 0 °C, and after the addition was completed, the reaction solution was transferred to the room temperature condition and stirred for reaction. After the reaction was completed, the reaction solution was extracted with saturated aqueous sodium bicarbonate and dichloromethane, washed with saturated brine, and dried over anhydrous sodium sulfate. The reaction solution was filtered and concentrated to give compound 44A (1.18 g).
MS (ESI, $[M+H]^+$) *m/z:* 269.5.

**Step B: Preparation of compound 44B**

Trifluoroacetic acid (2 mL) was added dropwise slowly to a reaction solution of compound 44A (0.5 g) in dichloromethane (10 mL) at 0 °C. After the addition was completed, the reaction solution was stirred at room temperature. After the reaction was completed, the reaction solution was concentrated to give compound 44B (0.85 g).
MS (ESI, $[M+H]^+$) *m/z*: 169.4.

**Step C: Preparation of compound 44C**

To a reaction flask were added compound 44B (0.5 g), 1,3-dibromofluorobenzene (0.68 g), (tris(dibenzylideneacetone)dipalladium (0.16 g), 1,1'-binaphthyl-2,2'-diphemyl phosphine (0.11 g), sodium *tert*-butoxide (0.85 g) and 1,4-dioxane (25 mL) successively. After the addition was completed, the reaction solution was stirred for reaction at 100 °C under nitrogen atmosphere. After the reaction was completed, the reaction solution was filtered, concentrated, and subjected to column chromatography (dichloromethane/methanol = 50/1) to give compound 44C (0.22 g).
MS (ESI, $[M+H]^+$) *m/z:* 341.4.

**Step D: Preparation of compound 44D**

To a reaction flask were added compound 44C (0.22 g), bis(pinacolato)diboron (0.24 g), potassium acetate (0.19 g), [1,1'-bis(diphenylphosphino)ferrocene]palladium dichloride dichloromethane complex (0.05 g) and 1,4-dioxane (20 mL) successively. The reaction solution was stirred for reaction at 100 °C under nitrogen atmosphere. After the reaction was completed, the reaction solution was filtered and concentrated. The reaction solution was subjected to column chromatography (dichloromethane/methanol = 20/1) to give compound 44D (98 mg).
MS (ESI, $[M+H]^+$) *m/z:* 389.5.

**Step E: Preparation of compound 44E**

To a microwave tube were added compound 44D (98 mg), compound 1C (103 mg), potassium carbonate (87 mg), tetrakis(triphenylphosphine)palladium (24 mg), 1,4-dioxane (4 mL) and water (0.5 mL) successively. After the addition was completed, the reaction solution was purged with nitrogen gas, and reacted at 100 °C in microwave reactor. After the reaction was completed, the reaction solution was filtered and concentrated. The reaction solution was subjected to column chromatography (dichloromethane/methanol = 20/1) to give compound 44E (84 mg).
MS (ESI, $[M+H]^+$) *m/z:* 717.4.

**Step F: Preparation of compound 44**

Compound 44E (84 mg), methanesulfonic acid (0.10 mL) and dichloromethane (6 mL) were added successively to a reaction flask, and the mixture was stirred at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 44 (37 mg).
MS (ESI, $[M+H]^+$) *m/z:* 617.6.
$^1$H NMR (500 MHz, $CDCl_3$) δ 8.11 (d, *J* = 8.3 Hz, 1H), 8.05 - 7.99 (m, 1H), 7.79 (dd, *J* = 8.4, 2.1 Hz, 1H), 7.56 (d, *J* = 8.0 Hz, 2H), 7.49 (t, *J* = 7.3 Hz, 1H), 7.38 (d, *J* = 8.0 Hz, 2H), 7.14 - 7.06 (m, 2H), 6.95 (t, *J* = 7.9 Hz, 1H), 6.59 (s, 2H), 6.39 (dd, *J* = 7.7, 4.8 Hz, 1H), 3.80 (d, *J* = 49.5 Hz, 4H), 3.06 - 2.92 (m, 4H), 2.64 - 2.58 (m,2H), 2.40 - 2.32 (m, 2H), 2.24 - 2.14 (m, 1H), 1.98 -1.92 (m, 4H), 1.89 (s, 3H), 1.85 - 1.80 (m, 1H).

## Example 45: Preparation of Compound 45

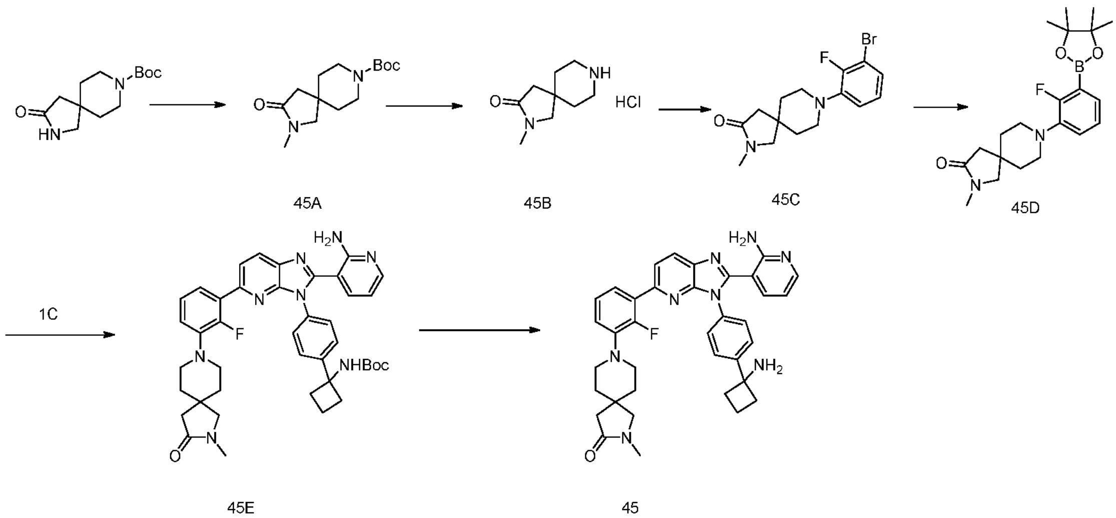


45A 45B 45C 45D 45E 45

### Step A: Preparation of compound 45A

Sodium hydride (7.9 g) was slowly added to a solution of tert-butyl 3-oxo-2,8-diazospiro[4.5]decane-8-carboxylate (10 g) in tetrahydrofuran at 0 °C under nitrogen atmosphere, and the mixture was stirred at room temperature for 1 h. Methyl iodide (27.9 g) was added dropwise to the reaction flask at 0 °C and then stirred at room temperature. After the reaction was completed, the reaction was quenched with aqueous ammonium chloride solution, and the reaction solution was extracted with ethyl acetate, dried over anhydrous sodium sulfate, filtered, and concentrated to give compound 45A (13.0 g).

MS (ESI, $[M+H]^+$) *m/z:* 269.5.

### Step B: Preparation of compound 45B

To a reaction flask were added compound 45A (13.0 g), dichloromethane (260 mL) and diluted hydrochloric acid (12.4 g) successively, and the mixture was stirred at room temperature. After the reaction was completed, the reaction solution was filtered and dried to give compound 45B (8.9 g). MS (ESI, $[M+H]^+$) *m/z*: 169.4.

### Step C: Preparation of compound 45C

To a reaction flask were added sodium *tert*-butoxide (11.3 g), 1,1'-binaphthyl-2,2'-bis-diphenylphosphine (1.8 g), tris(dibenzylideneacetone)dipalladium (2.7 g), 1,3-dibromo-2-fluorobenzene (11.2 g), compound 45B (8.9 g) and 1,4-dioxane (100 mL) successively, and the reaction solution was stirred for reaction under nitrogen atmosphere at 100 °C. After the reaction solution was completed, the reaction solution was filtered and concentrated. The reaction solution was subjected to column chromatography (dichloromethane/methanol = 100/1) to give compound 45C (4.8 g).

MS (ESI, $[M+H]^+$) *m/z:* 341.4.

### Step D: Preparation of compound 45D

To a reaction flask were added compound 45C (3.0 g), bis(pinacolato)diboron (3.4 g), potassium acetate (2.6 g), [1,1'-bis(diphenylphosphino)ferrocene]palladium dichloride dichloromethane complex (1.4 g) and 1,4-dioxane (100 mL) successively. The reaction solution was stirred for reaction at 100 °C under nitrogen atmosphere. After the reaction was completed, the reaction solution was filtered and concentrated. The reaction solution was subjected to column chromatography (dichloromethane/methanol = 100/1) to give compound 45D (3.0 g).

MS (ESI, $[M+H]^+$) *m/z:* 389.5.

**Step E: Preparation of compound 45E**

To a reaction flask were added compound 45D (2.8 g), compound 1C (2.3 g), potassium carbonate (1.3 g), tetrakis(triphenylphosphine)palladium (1.1 g), 1,4-dioxane (50 mL), and water (10 mL) successively. After the addition was completed, the reaction solution was purged with nitrogen gas, and stirred for reaction at 100 °C. After the reaction was completed, the reaction solution was filtered and concentrated. The reaction solution was subjected to column chromatography (dichloromethane/methanol = 20/1) to give compound 45E (2.1 g).

MS (ESI, $[M+H]^+$) *m/z:* 717.5.

**Step F: Preparation of compound 45**

To a reaction flask were added compound 45E (2.1 g), methanesulfonic acid (2.3 g) and dichloromethane (80 mL) successively, and the reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 20/1) to give compound 45 (0.43 g).

MS (ESI, $[M+H]^+$) *m/z:* 617.6.

$^1H$ NMR (500 MHz,$CDCl_3$) δ 8.19 - 7.88 (m, 2H), 7.73 (d, *J* = 8.5 Hz, 1H), 7.60 - 7.26 (m, 5H), 7.05 (s, 2H), 6.94 (d, *J* = 8.8 Hz, 1H), 6.53 (d, *J* = 10.6 Hz, 1H), 6.33 (s, 1H), 3.19 (s, 2H), 3.02 (s, 4H), 2.94 (d, *J* = 9.5 Hz, 2H), 2.79 (s, 3H), 2.60 - 2.52 (m, 1H), 2.27 (d, *J* = 14.2 Hz, 3H), 2.10 (s, 1H), 1.75 (s, 4H), 1.19 (d, *J* = 14.4 Hz, 1H).

**Example 46: Preparation of Compound 46**

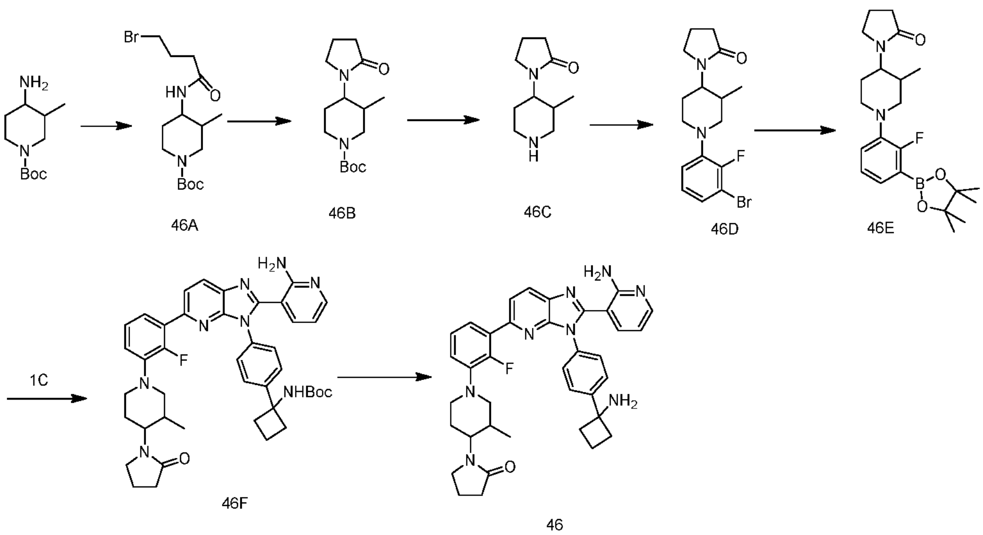


**Step A: Preparation of compound 46A**

To a reaction flask were added *tert*-butyl 4-amino-3-methylpiperidine-1-carboxylate oxalate (1.4 g), triethylamine (0.93 g), 4-bromobutyryl chloride (0.93 g) and tetrahydrofuran (25 mL) successively. After the addition was completed, the reaction solution was stirred at room temperature. After the reaction was completed, the reaction solution was distilled under reduced pressure to remove the solvent and concentrated to give compound 46A (0.84 g).

MS (ESI, $[M +H]^+$) *m/z:* 363.1.

**Step B: Preparation of compound 46B**

To a reaction flask were added compound 46A (3.0 g), sodium hydride (0.088 g), and tetrahydrofuran (25 mL) successively. The mixture was stirred at room temperature. After the reaction was completed, the reaction solution was concentrated to give compound 46B (0.18 g).

MS (ESI, $[M+H]^+$) *m/z:* 283.5.

**Step C: Preparation of compound 46C**

To a reaction flask were added compound 46B (1.0 g), a solution of 4 M hydrochloric acid in 1,4-dioxane (3.7 mL), and a solution of 1,4-dioxane (20 mL) successively. The mixture was stirred at room temperature. After the reaction was completed, the reaction solution was concentrated to give compound 46C (0.83 g).
MS (ESI, $[M+H]^+$) *m*/*z*: 183.5.

**Step D: Preparation of compound 46D**

To a reaction flask were added compound 46C (3.0 g), cesium carbonate (2.3 g), tris(dibenzylideneacetone)dipalladium-chloroform adduct (0.21 g), 1-(3-methylpiperidin-4-yl)pyrrolidin-2-one hydrochloride (0.51 g), 1,3-dibromom-fluorobenzene (0.89 g), 1,1'-binaphthyl-2,2'-diphemyl phosphine (0.14 g), and dioxane (10 mL) successively. The reaction solution was stirred at 100 °C. After the reaction was completed, the reaction solution was concentrated and subjected to column chromatography (petroleum ether/ethyl acetate = 9/1) to give compound 46D (0.38 g).
MS (ESI, $[M+H]^+$) *m*/*z:* 355.4.

**Step E: Preparation of compound 46E**

To a reaction flask were added 46D (0.38 g), potassium carbonate (0.21 g), bis(pinacolato)diboron (0.41 g), [1,1'-bis(diphenylphosphino)ferrocene]palladium dichloride (0.17 g) and dioxane (20 mL) successively under nitrogen atmosphere. After the addition was completed, the reaction solution was stirred at 100 °C. After the reaction was completed, the reaction solution was concentrated and subjected to column chromatography (petroleum ether/ethyl acetate = 9/1) to give compound 46E (0.16 g).
MS (ESI, $[M+H]^+$) *m*/*z:* 403.6.

**Step F: Preparation of compound 46F**

To a reaction flask were added compound 46E (0.16 g), compound 1C (0.18 g), potassium carbonate (0.087 g), tetrakis(triphenylphosphine)palladium (0.075 g), 1,4-dioxane (10 mL), and water (2.0 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 46F (0.20 g).
MS (ESI, $[M+H]^+$) *m*/*z:* 731.8.

**Step G: Preparation of compound 46**

Compound 46F (0.20 g), methanesulfonic acid (0.16 g) and dichloromethane (15 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 46 (0.078 g).
MS (ESI, $[M+H]^+$) *m*/*z:* 631.7.
$^1$H NMR (500 MHz, $CDCl_3$) δ 8.10 - 8.04 (m, 1H), 7.94 (dd, *J* = 5.0, 2.3 Hz, 1H), 7.74 (dq, *J* = 8.1, 2.7 Hz, 1H), 7.56 - 7.47 (m, 2H), 7.43 - 7.33 (m, 3H), 7.05 (tt, *J* = 11.9, 3.0 Hz, 2H), 6.95 (t, *J* = 8.0 Hz, 1H), 6.34 (ddd, *J* = 7.4, 4.9, 2.1 Hz, 1H), 3.71 (td, *J* = 12.1, 4.2 Hz, 1H), 3.46 - 3.32 (m, 3H), 3.32 - 3.26 (m, 1H), 2.76 (td, *J* = 11.9, 2.9 Hz, 1H), 2.59 (dddd, *J* = 11.9, 9.0, 6.3, 2.3 Hz, 2H), 2.47 - 2.41 (m, 1H), 2.37 (dtt, *J* = 11.7, 5.8, 2.6 Hz, 4H), 2.15 (ddtt, *J* = 15.9, 9.6, 6.7, 3.4 Hz, 1H), 1.98 (dp, *J* = 12.3, 5.2, 4.0 Hz, 3H), 1.81 (ddtq, *J* = 12.4, 9.3, 6.3, 3.7, 2.9 Hz, 1H), 1.72 - 1.60 (m, 1H), 1.34 (d, *J* = 2.3 Hz, 1H), 1.26 - 1.14 (m, 3H).

**Example 47: Preparation of Compound 47**

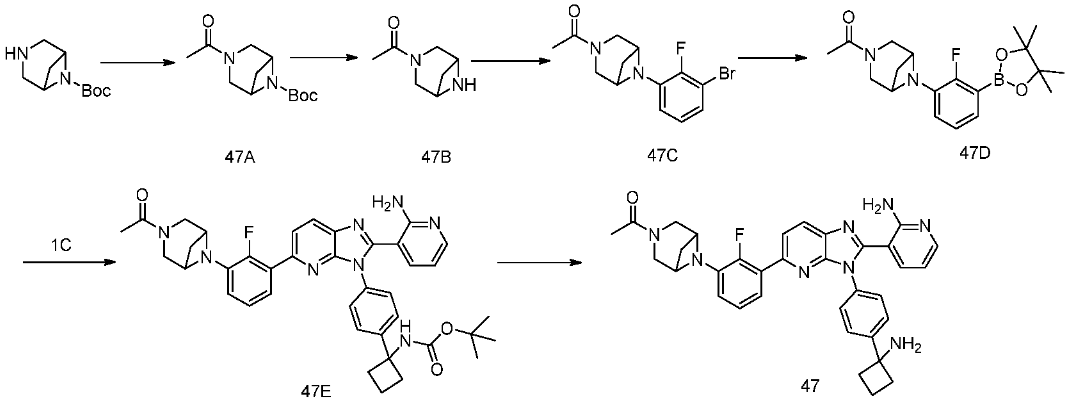


47A 47B 47C 47D

47E 47

**Step A: Preparation of compound 47A**

To a reaction flask were added *tert*-butyl 3,6-diazabicyclo[3.1.1]heptane-6-carboxylate (2.5 g), acetic anhydride (1.93 g), triethylamine (6.3 g), and dichloromethane (40 mL) successively. The mixture was stirred at room temperature. After the reaction was completed, water and dichloromethane were added to extract the reaction solution, and the reaction solution was washed with saturated brine and dried over anhydrous sodium sulfate. The reaction solution was filtered and concentrated to give compound 47A (3.0 g).

MS (ESI, $[M+Na]^+$) *m/z:* 241.1.

**Step B: Preparation of compound 47B**

To a reaction flask were added compound 47A (3.01 g), trifluoroacetic acid (2.2 g), and dichloromethane (50 mL) successively. The mixture was stirred at room temperature. After the reaction was completed, the reaction solution was concentrated to give compound 47B (2.3 g).

MS (ESI, $[M+H]^+$) *m/z*: 141.1.

**Step C: Preparation of compound 47C**

To a reaction flask were added 1,1'-binaphthyl-2,2'-diphemyl phosphine (0.73 g), tris(dibenzylideneacetone)dipalladium (0.26 g), 1,3-dibromo-2-fluorobenzene (4.5 g), compound 47B (1.6 g), sodium *tert*-butoxide (11 g) and dioxane (20 mL) successively under nitrogen atmosphere. After the addition was completed, the reaction solution was stirred at 85 °C. After the reaction was completed, the reaction solution was concentrated and subjected to column chromatography (petroleum ether/ethyl acetate = 9/1) to give compound 47C (0.43 g).

MS (ESI, $[M+H]^+$) *m/z:* 312.9.

**Step D: Preparation of compound 47D**

To a reaction flask were added compound 47C (0.43 g), bis(pinacolato)diboron (0.52 g), potassium acetate (0.22 g), [1,1'-bis(diphenylphosphino)ferrocene]palladium dichloride dichloromethane complex (0.270 g) and 1,4-dioxane (60 mL) successively. The reaction solution was stirred at 100 °C under nitrogen atmosphere. After the reaction was completed, the reaction solution was filtered concentrated. The reaction solution was subjected to column chromatography (dichloromethane/methanol = 20/1) to give compound 47D (0.13 g).

MS (ESI, $[M+H]^+$) *m/z:* 361.6.

**Step E: Preparation of compound 47E**

To a reaction flask were added compound 47D (0.13 g), compound 1C (0.12 g), potassium carbonate (0.068 g), tetrakis(triphenylphosphine)palladium (0.057 g), 1,4-dioxane (25 mL), and water (5 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 47E (0.21 g).

MS (ESI, $[M+H]^+$) *m/z:* 689.44.

**Step F: Preparation of compound 47**

Compound 47E (0.21 g), methanesulfonic acid (0.58 g) and dichloromethane (25 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 47 (0.11 g).

MS (ESI, $[M+H]^+$) *m/z*: 589.6.

$^1$H NMR (500 MHz, $CDCl_3$) δ 8.07 - 7.89 (m, 2H), 7.61 (d, *J* = 8.4 Hz, 1H), 7.46 (dd, *J* = 17.7, 8.1 Hz, 2H), 7.28 (t, *J* = 12.7 Hz, 2H), 7.15 (t, *J* = 7.4 Hz, 1H), 7.05 (d, *J* = 7.8 Hz, 1H), 6.97 (q, *J* = 7.7 Hz, 1H), 6.48 (dt, *J* = 26.1, 8.2 Hz, 2H), 6.33 (dd, *J* = 7.8, 4.8 Hz, 1H), 4.46 - 4.25 (m, 2H), 3.86 (dd, *J* = 18.6, 12.8 Hz, 2H), 3.53 (d, *J* = 13.7 Hz, 1H), 3.47 - 3.28 (m, 1H), 2.78 (q, *J* = 6.9 Hz, 1H), 2.48 (dq, *J* = 53.9, 11.6, 10.3 Hz, 4H), 2.28 - 2.09 (m, 1H), 1.91 - 1.79 (m, 3H), 1.54 (d, *J* = 8.6 Hz, 1H), 1.35 (s, 1H).

**Example 48: Preparation of Compound 48**

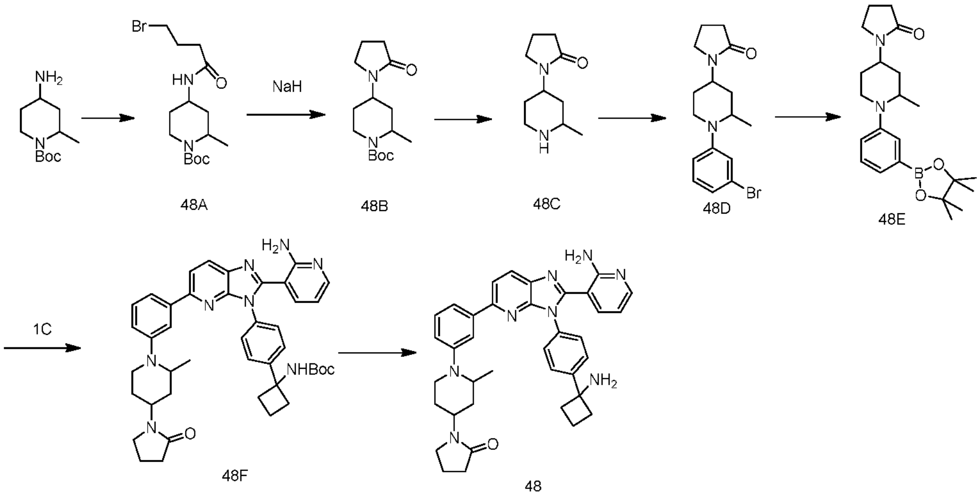

48A 48B 48C 48D 48E
48F 48

**Step A: Preparation of compound 48A**

To a reaction flask were added *tert*-butyl 4-amino-3-methylpiperidine-1-carboxylate oxalate (3.5 g), triethylamine (2.9 g), 4-bromobutyryl chloride (3.3 g) and tetrahydrofuran (25 mL) successively. After the addition was completed, the reaction solution was stirred at room temperature. After the reaction was completed, the reaction solution was distilled under reduced pressure to remove the solvent and concentrated to give compound 48A (0.78 g).

MS (ESI, $[M+H]^+$) *m/z:* 363.1.

**Step B: Preparation of compound 48B**

To a reaction flask were added compound 48A (1.4 g), sodium hydride (0.088 g), and tetrahydrofuran (25 mL) successively. The mixture was stirred at room temperature. After the reaction was completed, the reaction solution was concentrated to give compound 48B (0.52 g).

MS (ESI, $[M+H]^+$) *m/z*: 283.5.

**Step C: Preparation of compound 48C**

To a reaction flask were added compound 48B (0.52 g), a solution of 4 M hydrochloric acid in 1,4-dioxane (0.34 mL), and a solution of 1,4-dioxane (20 mL) successively. The mixture was stirred at room temperature. After the reaction was completed, the reaction solution was concentrated to give compound 48C (0.41 g).

MS (ESI, $[M +H]^+$) *m/z*: 183.5.

**Step D: Preparation of compound 48D**

To a reaction flask were added compound 48C (0.41 g), cesium carbonate (2.2 g), tris(dibenzylideneacetone)dipalladium-chloroform adduct (0.21 g), 1-(3-methylpiperidin-4-yl)pyrrolidin-2-one hydrochloride (0.51 g), 1,3-dibromom-fluorobenzene (0.89 g), 1,1'-binaphthyl-2,2'-diphemyl phosphine (0.14 g), and dioxane (10 mL) successively. The reaction solution was stirred at 100 °C. After the reaction was completed, the reaction solution was concentrated and subjected to column chromatography (petroleum ether/ethyl acetate = 9/1) to give compound 48D (0.28 g).

MS (ESI, $[M+H]^+$) *m/z:* 337.1.

**Step E: Preparation of compound 48E**

To a reaction flask were added 48D (0.28 g), potassium carbonate (0.24 g), bis(pinacolato)diboron (0.32 g), [1,1'-bis(diphenylphosphino)ferrocene]palladium dichloride (0.138 g) and dioxane (20 mL) successively under nitrogen atmosphere. After the addition was completed, the reaction solution was stirred at 100 °C. After the reaction was completed, the reaction solution was concentrated and subjected to column chromatography (petroleum ether/ethyl acetate = 9/1) to give compound 48E (0.087 g).

MS (ESI, $[M+H]^+$) *m/z:* 385.3.

**Step F: Preparation of compound 48F**

To a reaction flask were added compound 48E (0.087 g), compound 1C (0.075 g), potassium carbonate (0.087 g), tetrakis(triphenylphosphine)palladium (0.075 g), 1,4-dioxane (10 mL), and water (2 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 48F (0.080 g).

MS (ESI, $[M+H]^+$) *m/z*: 713.1.

**Step G: Preparation of compound 48**

Compound 48F (0.080 g), methanesulfonic acid (0.16 g) and dichloromethane (15 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 48 (0.021 g).

MS (ESI, $[M+H]^+$) *m/z*: 613.1.

$^1H$ NMR (500 MHz, $CDCl_3$) δ 8.09 - 7.94 (m, 2H), 7.70 (d, *J* = 8.5 Hz, 1H), 7.48 (h, *J* = 9.0, 8.5 Hz, 3H), 7.36 (d, *J* = 7.8 Hz, 2H), 7.23 (t, *J* = 7.9 Hz, 1H), 7.09 (d, *J* = 7.7 Hz, 1H), 6.84 (dd, *J* = 8.2, 2.4 Hz, 1H), 6.59 (d, *J* = 20.1 Hz, 2H), 6.33 (dd, *J* = 7.8, 4.8 Hz, 1H), 4.39 (ddt, *J* = 12.9, 9.3, 4.6 Hz, 1H), 4.26 (dt, *J* = 22.0, 11.0 Hz, 1H), 3.47 (dt, *J* = 12.6, 3.7 Hz, 1H), 3.29 (t, *J* = 7.0 Hz, 2H), 3.16 - 3.03 (m, 1H), 2.54 (dt, *J* = 12.0, 8.5 Hz, 3H), 2.35 (t, *J* = 8.1 Hz, 3H), 2.16 (tt, *J* = 9.0, 4.5 Hz, 3H), 1.72 (dtd, *J* = 24.1, 12.1, 11.6, 6.7 Hz, 4H), 1.60 (d, *J* = 12.3 Hz, 2H), 1.07 (d, *J* = 6.9 Hz, 3H).

**Example 49: Preparation of Compound 49**

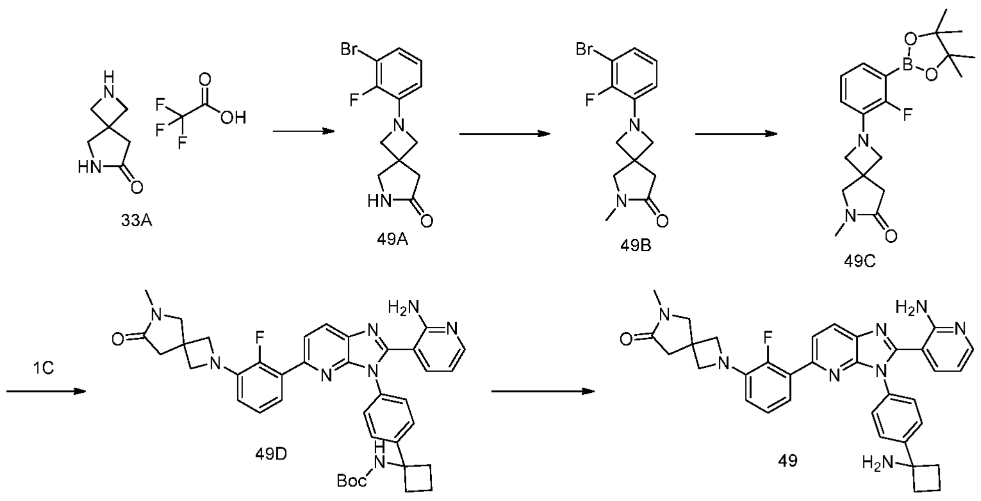


33A 49A 49B 49C 49D 49

**Step A: Preparation of compound 49A**

To a reaction flask were added 33A (0.52 g), 1,3-dibromofluorobenzene (0.83 g), (tris(dibenzylideneacetone)dipalladium (0.20 g), 1,1'-binaphthyl-2,2'-diphemyl phosphine (0.14 g), sodium *tert*-butoxide (1.04 g) and 1,4-dioxane (30 mL) successively. After the addition was completed, the reaction solution was stirred for reaction at 100 °C under nitrogen atmosphere. After the reaction was completed, the reaction solution was filtered, concentrated, and subjected to column chromatography (dichloromethane/methanol = 50/1) to give compound 49A (0.22 g).

MS (ESI, $[M+H]^+$) *m/z:* 299.3.

**Step B: Preparation of compound 49B**

Sodium hydride (0.11 g) was slowly added to a solution of compound 49A (0.16 g) in tetrahydrofuran (10 mL) at 0 °C under nitrogen atmosphere, and after the addition was completed, the reaction solution was transferred to the room temperature condition and stirred for 0.5 h, and further added with methyl iodide (0.23 g) for reaction. After the reaction was completed, a saturated aqueous ammonium chloride solution was added to the reaction solution in an ice bath, followed by extraction with ethyl acetate, and the reaction solution was washed with saturated brine, and dried over anhydrous sodium sulfate. The reaction solution was filtered and concentrated to give compound 49B (0.18 g).

MS (ESI, $[M+H]^+$) *m/z*: 313.3.

**Step C: Preparation of compound 49C**

To a reaction flask were added compound 49B (0.17 g), bis(pinacolato)diboron (0.20 g), potassium acetate (0.16 g), [1,1'-bis(diphenylphosphino)ferrocene]palladium dichloride dichloromethane complex (0.04 g) and 1,4-dioxane (10 mL) successively. The reaction solution was stirred for reaction at 100 °C under nitrogen atmosphere. After the reaction was completed, the reaction solution was filtered and concentrated. The reaction solution was subjected to column chromatography (dichloromethane/methanol = 20/1) to give compound 49C (0.19 g).

MS (ESI, $[M+H]^+$) *m/z:* 361.5.

**Step D: Preparation of compound 49D**

To a microwave tube were added compound 49C (0.14 g), compound 1C (0.16 g), potassium carbonate (0.14 g), tetrakis(triphenylphosphine)palladium (0.04 g), 1,4-dioxane (4 mL) and water (0.5 mL) successively. After the addition was completed, the reaction solution was purged with nitrogen gas, and reacted at 100 °C in microwave reactor. After the reaction was completed, the reaction solution was filtered and concentrated. The reaction solution was subjected to column chromatography (dichloromethane/methanol = 20/1) to give compound 49D (0.14 g).

MS (ESI, $[M+H]^+$) *m/z:* 689.7.

**Step E: Preparation of compound 49**

Compound 49D (135 mg), methanesulfonic acid (0.10 mL) and dichloromethane (6 mL) were added successively to a reaction flask, and the mixture was stirred at room temperature. After the reaction was completed, 10% aqueous

sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 49 (49 mg).

MS (ESI, $[M+H]^+$) *m/z:* 589.4.

$^1H$ NMR (500 MHz, $CDCl_3$) δ 8.14 (dd, *J* = 8.5, 3.4 Hz, 1H), 8.02 (dt, *J* = 4.9, 2.5 Hz, 1H), 7.83 - 7.75 (m, 1H), 7.55 (dt, *J* = 8.4, 2.4 Hz, 2H), 7.42 (dt, *J* = 8.3, 2.4 Hz, 2H), 7.32 - 7.26 (m, 1H), 7.17 - 7.13 (m, 1H), 7.07 (dd, *J* = 9.5, 6.0 Hz, 1H), 6.57 - 6.49 (m, 1H), 6.45 - 6.39 (m, 1H), 4.10 - 3.96 (m, 4H), 3.71 (d, *J* = 3.3 Hz, 2H), 2.89 (d, *J* = 3.5 Hz, 3H), 2.72 (d, *J* = 3.5 Hz, 2H), 2.70 - 2.60 (m, 2H), 2.37 - 2.22 (m, 2H), 2.20 - 2.08 (m, 1H), 1.90 - 1.80 (m, 1H).

**Example 50: Preparation of Compound 50**

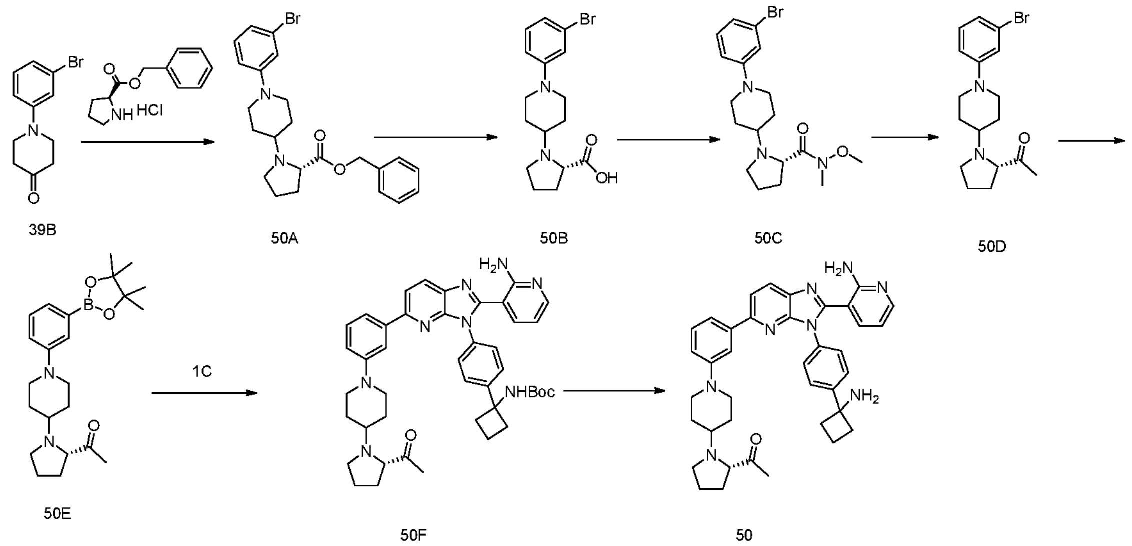


39B 50A 50B 50C 50D

50E 50F 50

**Step A: Preparation of compound 50A**

To a reaction flask were added compound 39B (1.7 g), *L*-proline benzyl hydrochloride (2.4 g), acetic acid (0.8 g) and methanol (20 mL) successively, and the mixture was stirred at room temperature under nitrogen atmosphere. Sodium cyanoborohydride (0.84 g) was added thereto, and after the addition was completed, the mixture was stirred at room temperature. After the reaction was completed, a saturated aqueous sodium hydrogencarbonate solution (50 mL) was added to the reaction solution, and the mixture was concentrated; the residue was extracted with dichloromethane, dried over anhydrous sodium sulfate, filtered, and concentrated to give compound 50A (1.3 g).

MS (ESI, $[M+H]^+$) *m/z:* 443.4.

**Step B: Preparation of compound 50B**

To a reaction flask were added compound 50A (1.3 g), sodium hydroxide (0.35 g), tetrahydrofuran (30 mL) and water (10 mL) successively, and the reaction solution was stirred for reaction at 70 °C. After the reaction was completed, the reaction solution was concentrated, the residue was extracted with ethyl acetate, and the pH of the aqueous phase was adjusted to 4-5 with 1 M diluted hydrochloric acid. The aqueous phase was then concentrated, dissolved in methanol (30 mL), filtered through an organic membrane, and further concentrated to give compound 50B (1.0 g).

**Step C: Preparation of compound 50C**

To a reaction flask were added compound 50B (1.0 g), 2-(7-azabenzotriazole)-*N*,*N*,*N'*,*N'*-tetramethyluronium hexafluorophosphate (2.2 g) and dichloromethane (20 mL) successively, and the mixture was stirred at room temperature under nitrogen atmosphere. *N*,*N*-diisopropylethylamine (1.5 g) and *N,O*-dimethylhydroxylamine hydrochloride (0.4 g) were added to the reaction solution, and after the addition was completed, the reaction solution was stirred at room temperature. After the reaction was completed, the reaction solution was concentrated and subjected to column chromatography (dichloromethane/methanol = 100/1) to give compound 50C (1.0 g).

MS (ESI, $[M+H]^+$) *m/z:* 396.2.

**Step D: Preparation of compound 50D**

Methylmagnesium bromide (1.5 g) was added dropwise to a solution of compound 50C (1.0 g) in tetrahydrofuran (20 mL) at -15 °C under nitrogen atmosphere, and after the addition was completed, the mixture was stirred at room temperature. After the reaction was completed, 50 mL of a saturated aqueous ammonium chloride solution was added to the reaction flask, and the reaction solution was extracted with ethyl acetate and concentrated. The reaction solution was subjected to column chromatography (dichloromethane/methanol = 40/1) to give compound 50D (0.23 g).

MS (ESI, $[M+H]^+$) *m/z:* 351.1.

**Step E: Preparation of compound 50E**

To a reaction flask were added compound 50D (0.23 g), bis(pinacolato)diboron (0.33 g), potassium acetate (0.2 g), [1,1'-bis(diphenylphosphino)ferrocene]palladium dichloride dichloromethane complex (0.053 g) and 1,4-dioxane (20 mL) successively. The reaction solution was stirred for reaction at 100 °C under nitrogen atmosphere. After the reaction was completed, the reaction solution was filtered, concentrated, and subjected to column chromatography (dichloromethane/methanol = 40/1) to give compound 50E (0.054 g).

MS (ESI, $[M+H]^+$) *m/z:* 399.4.

**Step F: Preparation of compound 50F**

To a reaction flask were added compound 50E (0.054 g), compound 1C (0.06 g), potassium carbonate (0.051 g), tetrakis(triphenylphosphine)palladium (0.014 g), 1,4-dioxane (5 mL), and water (1 mL) successively. After the addition was completed, the reaction solution was stirred for reaction at 100 °C under nitrogen atmosphere. After the reaction was completed, the reaction solution was filtered, concentrated, and subjected to column chromatography (dichloromethane/methanol = 20/1) to give compound 50F (0.050 g).

MS (ESI, $[M+H]^+$) *m/z:* 727.4.

**Step F: Preparation of compound 50**

To a reaction flask were added compound 50F (0.05 g), methanesulfonic acid (0.066 g) and dichloromethane (6 mL) successively, and the reaction solution was stirred for reaction at room temperature. After the reaction was completed, 10% aqueous sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 20/1) to give compound 50 (0.0050 g).

MS (ESI, $[M+H]^+$) *m/z:* 627.7.

$^1$H NMR (500 MHz, DMSO) δ 9.36 (d, *J* = 41.5 Hz, 1H), 8.99 (s, 1H), 8.34 - 8.26 (m, 1H), 8.08 (d, *J* = 6.1 Hz, 1H), 8.03 (d, *J* = 8.4 Hz, 1H), 7.74 (dd, *J* = 33.2, 8.5 Hz, 2H), 7.66 - 7.57 (m, 3H), 7.48 (d, *J* = 7.1 Hz, 3H), 7.31 (t, *J* = 7.9 Hz, 1H), 7.02 (d, *J* = 6.5 Hz, 1H), 6.61 (s, 1H), 4.88 (d, *J* = 26.5 Hz, 1H), 3.90 (dd, *J* = 20.9, 9.0 Hz, 2H), 3.55 (d, *J* = 34.7 Hz, 2H), 2.72 (s, 3H), 2.63 (t, *J* = 7.8 Hz, 4H), 2.37 (d, *J* = 9.4 Hz, 3H), 2.28 - 2.09 (m, 3H), 2.07 - 1.96 (m, 3H), 1.88 - 1.80 (m, 2H), 1.62 (dd, *J* = 71.1, 14.4 Hz, 3H).

**Example 51: Preparation of Compound 51**

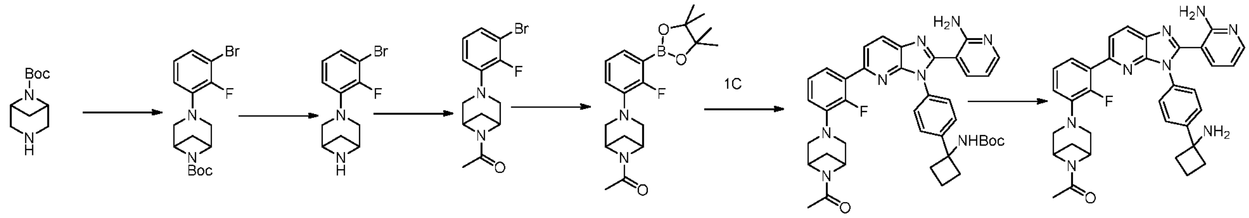


51A 51B 51C 51D 51E 51

**Step A: Preparation of compound 51A**

To a reaction flask were added 1,1'-binaphthyl-2,2'-bis-diphenylphosphine (0.22 g), tris(dibenzylideneacetone)dipalladium (0.32 g), 1,3-dibromo-2-fluorobenzene (0.89 g), *tert*-butyl 2,7-diazaspiro[3.5]nonane-7-carbonate (0.70 g), sodium *tert*-butoxide (1.0 g), and tetrahydrofuran (25 mL) successively under nitrogen atmosphere. After the addition was completed, the reaction solution was stirred at 85 °C. After the reaction was completed, the reaction solution was concentrated and subjected to column chromatography (petroleum ether/ethyl acetate = 5/1) to give compound 51A (0.37 g).

MS (ESI, $[M+H]^+$) *m/z:* 371.3.

**Step B: Preparation of compound 51B**

Compound 51A (0.37 g), methanesulfonic acid (0.28 g) and dichloromethane (10 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered and concentrated to give compound 51B (0.23 g).

MS (ESI, $[M+H]^+$) *m/z*: 271.3.

**Step C: Preparation of compound 51C**

To a reaction flask were added compound 51B (0.23 g), acetic anhydride (0.13 g), triethylamine (0.87 g) and dichloromethane (50 mL) successively. The mixture was stirred at room temperature. After the reaction was completed, water and dichloromethane were added to extract the reaction solution, and the reaction solution was washed with saturated brine and dried over anhydrous sodium sulfate. The reaction solution was filtered and concentrated to give compound 51C (0.34 g).

MS (ESI, $[M+H]^+$) *m/z*: 313.0.

**Step D: Preparation of compound 51D**

To a reaction flask were added compound 51C (0.34 g), bis(pinacolato)diboron (0.42 g), potassium acetate (0.21 g), [1,1'-bis(diphenylphosphino)ferrocene]palladium dichloride dichloromethane complex (0.18 g) and 1,4-dioxane (50 mL) successively. The reaction solution was stirred at 100 °C under nitrogen atmosphere. After the reaction was completed, the reaction solution was filtered and concentrated. The reaction solution was subjected to column chromatography (dichloromethane/methanol = 20/1) to give compound 51D (0.17 g).

MS (ESI, $[M+H]^+$) *m/z*: 361.5.

**Step E: Preparation of compound 51E**

To a reaction flask were added compound 51D (0.17 g), compound 1C (0.16 g), potassium carbonate (0.89 g), tetrakis(triphenylphosphine)palladium (0.74 g), 1,4-dioxane (25 mL), and water (5 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 51E (0.15 g).

MS (ESI, $[M+H]^+$) *m/z*: 689.6.

**Step F: Preparation of compound 51**

Compound 51E (0.15 g), methanesulfonic acid (0.21 g) and dichloromethane (10 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 51 (0.089 g).

HRMS (ESI, $[M+H]^+$) *m/z*: 589.2825.

$^1H$ NMR (500 MHz, $CDCl_3$) $\delta$ 8.09 - 7.94 (m, 2H), 7.66 (d, *J* = 8.5 Hz, 1H), 7.48 (d, *J* = 7.9 Hz, 2H), 7.32 (d, *J* = 7.8 Hz, 2H), 7.09 - 7.02 (m, 1H), 6.98 (t, *J* = 7.8 Hz, 1H), 6.59 - 6.47 (m, 2H), 6.32 (m, 1H), 4.39 (d, *J* = 6.0 Hz, 2H), 3.88 (dd, *J* = 20.6, 12.8 Hz, 2H), 3.54 (d, *J* = 13.7 Hz, 1H), 3.41 (d, *J* = 11.6 Hz, 1H), 2.80 (q, *J* = 7.0 Hz, 1H), 2.54 (q,

*J* = 9.1 Hz, 3H), 2.21 (m, 2H), 2.10 (dt, *J* = 19.0, 9.3 Hz, 1H), 1.89 (s, 3H), 1.82 - 1.70 (m, 1H).

**Example 52: Preparation of Compound 52**

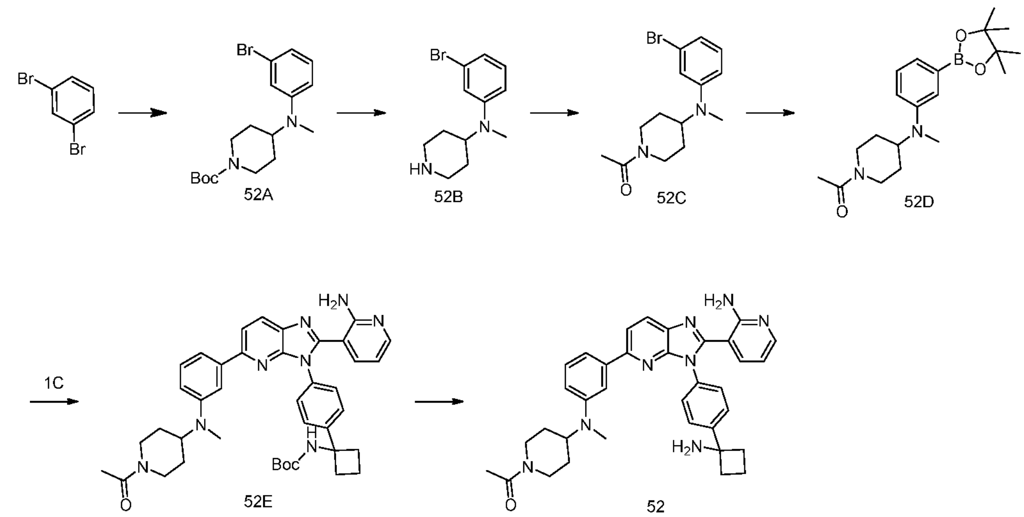


**Step A: Preparation of compound 52A**

To a reaction flask were added 1-*tert*-butoxycarbonyl-4-methylaminopiperidine (2.00 g), 1,3-dibromobenzene (3.30 g), tris(dibenzylideneacetone)dipalladium (0.43 g), 1,1'-binaphthyl-2,2'-diphemyl phosphine (0.44 g), 1,4-dioxane (100 mL) and sodium *tert*-butoxide (1.79 g) successively, and after the addition was completed, the reaction solution was stirred for reaction at 100 °C under nitrogen atmosphere. After the reaction was completed, the reaction solution was concentrated and subjected to column chromatography (petroleum ether/ethyl acetate = 5/1) to give compound 52A (116 g).

MS (ESI, $[M\text{-}t\text{-}Bu+H]^+$) *m*/*z:* 313.4.

**Step B: Preparation of compound 52B**

To a reaction flask containing compound 52A (1.15 g) were added methylene chloride (10 mL) and a solution (15 mL) of 4 M hydrogen chloride in dioxane successively, and after the addition was completed, the reaction solution was stirred for reaction at room temperature. After the reaction was completed, the reaction solution was concentrated to give compound 52B (1.17 g).

MS (ESI, $[M+H]^+$) *m*/*z:* 269.5.

**Step C: Preparation of compound 52C**

To a reaction flask containing compound 52B (1.17 g) were added triethylamine (1.16 g), acetic anhydride (0.78 g) and methylene chloride (40 mL) successively, and after the addition was completed, the reaction solution was stirred for reaction at room temperature. After the reaction was completed, the reaction solution was concentrated and subjected to column chromatography (petroleum ether/ethyl acetate = 1/2) to give compound 52C (0.90 g).

MS (ESI, $[M+H]^+$) *m*/*z*: 311.4.

**Step D: Preparation of compound 52D**

To a reaction flask were added compound 52C (0.88 g), bis(pinacolato)diboron (1.08 g), potassium acetate (0.83 g), [1,1'-bis(diphenylphosphino)ferrocene]palladium dichloride dichloromethane complex (0.23 g) and 1,4-dioxane (50 mL) successively. The reaction solution was stirred for reaction at 100 °C under nitrogen atmosphere. After the reaction was completed, the reaction solution was filtered and concentrated. The reaction solution was subjected to column chromatography (petroleum ether/ethyl acetate = 1/2) to give compound 52D (0.90 g).

MS (ESI, $[M+H]^+$) *m*/*z:* 359.6.

**Step E: Preparation of compound 52E**

To a microwave tube were added compound 52D (200 mg), compound 1C (219 mg), potassium carbonate (169 mg), tetrakis(triphenylphosphine)palladium (47 mg), 1,4-dioxane (10 mL) and water (2 mL) successively. After the addition was completed, the reaction solution was purged with nitrogen gas, and reacted at 140 °C in microwave reactor. After the reaction was completed, the reaction solution was filtered and concentrated. The reaction solution was subjected to column chromatography (dichloromethane/methanol = 20/1) to give compound 52E (216 mg).
MS (ESI, $[M+H]^+$) *m/z:* 687.8.

**Step F: Preparation of compound 52**

To a reaction flask were added compound 52E (206 mg), methanesulfonic acid (0.2 mL) and dichloromethane (10 mL) successively, and the reaction solution was stirred for reaction at room temperature. After the reaction was completed, a saturated aqueous sodium carbonate solution was added to the reaction solution to adjust the pH to 9, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 52 (145 mg).
MS (ESI, $[M+H]^+$) *m/z:* 587.7.
$^1$H NMR (500 MHz, $CDCl_3$) $\delta$ 8.07 (d, *J* = 8.4 Hz, 1H), 7.90 (dd, *J* = 5.2, 1.7 Hz, 1H), 7.72 (d, *J*= 8.4 Hz, 1H), 7.68 - 7.62 (m, 2H), 7.48 - 7.43 (m, 2H), 7.39 (s, 1H), 7.29 (d, *J* = 7.7 Hz, 1H), 7.26 - 7.21 (m, 1H), 7.15 (dd, *J* = 7.8, 1.7 Hz, 1H), 6.82 (dd, *J* = 8.1, 2.5 Hz, 1H), 6.44 (dd, *J* = 7.7, 5.2 Hz, 1H), 4.65 - 4.57 (m, 1H), 3.89 - 3.81 (m, 1H), 3.78 - 3.70 (m, 1H), 3.10 - 3.02 (m, 1H), 2.81 - 2.71 (m,5H), 2.70 - 2.59 (m, 2H), 2.55 - 2.47 (m, 1H), 2.38 - 2.29 (m, 1H), 2.04 (s, 3H), 1.93 -1.84 (m, 1H), 1.81 - 1.71 (m, 2H), 1.66 -1.51 (m, 2H).

**Example 53: Preparation of Compound 53**

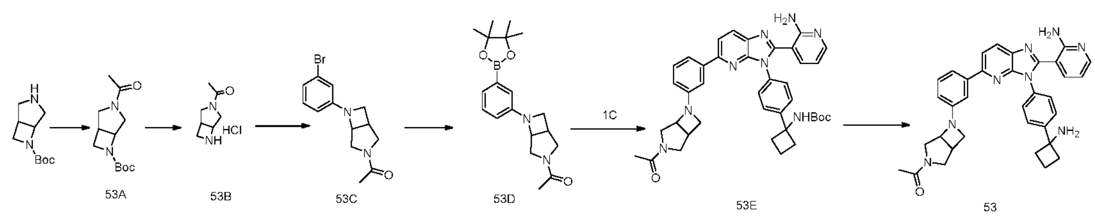


53A 53B 53C 53D 53E 53

**Step A: Preparation of compound 53A**

To a reaction flask were added *tert*-butyl 3,6-diazacyclo[3.2.0]heptane-6-carboxylate (0.35 g), acetyl chloride (0.28 g), triethylamine (0.54 g) and dichloromethane (15 mL) successively. The mixture was stirred at room temperature. After the reaction was completed, water and dichloromethane were added to extract the reaction solution, and the reaction solution was washed with saturated brine and dried over anhydrous sodium sulfate. The reaction solution was filtered and concentrated to give compound 53A (0.45 g).
MS (ESI, $[M+H]^+$) *m/z*: 241.1.

**Step B: Preparation of compound 53B**

To a reaction flask were added compound 53A (0.42 g), hydrochloric acid (3.06 g) and dichloromethane (10 mL) successively. The mixture was stirred at room temperature. After the reaction was completed, the reaction solution was concentrated to give compound 53B (0.4 g).
MS (ESI, $[M+H]^+$) *m/z*: 141.1.

**Step C: Preparation of compound 53C**

To a reaction flask were added 1,1'-binaphthyl-2,2'-diphemyl phosphine (0.20 g), tris(dibenzylideneacetone)dipalladium (0.30 g), 1,3-dibromobenzene (0.43 g), compound 53B (0.29 g), sodium *tert*-butoxide (0.47 g) and dioxane (30 mL) successively under nitrogen atmosphere. After the addition was completed, the reaction solution was stirred at 100 °C. After the reaction was completed, the reaction solution was concentrated and subjected to column chromatography (dichloromethane/methanol = 50/1) to give compound 53C (0.07 g).

MS (ESI, $[M+H]^+$) *m/z:* 295.4.

**Step D: Preparation of compound 53D**

To a reaction flask were added compound 53C (0.12 g), bis(pinacolato)diboron (0.21 g), potassium acetate (0.12 g), [1,1'-bis(diphenylphosphino)ferrocene]palladium dichloride dichloromethane complex (0.066 g) and 1,4-dioxane (20 mL) successively. The reaction solution was stirred at 100 °C under nitrogen atmosphere. After the reaction was completed, the reaction solution was filtered and concentrated. The reaction solution was subjected to column chromatography (dichloromethane/methanol = 40/1) to give compound 53D (0.09 g).

MS (ESI, $[M+H]^+$) *m/z*: 343.6.

**Step E: Preparation of compound 53E**

To a reaction flask were added compound 53D (0.088 g), compound 1C (0.075 g), potassium carbonate (0.042 g), tetrakis(triphenylphosphine)palladium (0.035 g), 1,4-dioxane (10 mL), and water (2 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 40/1) to give compound 53E (0.054 g).

MS (ESI, $[M+H]^+$) *m/z:* 671.8.

**Step F: Preparation of compound 53**

Compound 53E (0.050 g), methanesulfonic acid (0.072 g) and dichloromethane (10 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 40/1) to give compound 53 (0.020 g).

HRMS (ESI, $[M+H]^+$) *m/z*: 571.2924.

$^1$H NMR (500 MHz, $CDCl_3$) $\delta$ 8.08 (t, *J* = 8.4 Hz, 1H), 8.02 (dd, *J* = 8.7, 4.4 Hz, 1H), 7.73 (d, *J* = 8.3 Hz, 1H), 7.68 (d, *J* = 8.2 Hz, 1H), 7.61 (d, *J* = 8.2 Hz, 1H), 7.41 (t, *J* = 8.5 Hz, 2H), 7.34 (d, *J*= 7.7 Hz, 1H), 7.23 (dd, *J* = 14.2, 7.0 Hz, 2H), 7.13 - 7.07 (m, 1H), 6.62 (d, *J* = 9.8 Hz, 2H), 6.37 (dd, *J* = 14.0, 8.0 Hz, 2H), 4.67 - 4.50 (m, 1H), 4.18 (dd, *J* = 19.0, 11.7 Hz, 1H), 3.95 - 3.76 (m, 3H), 3.67 - 3.60 (m, 1H), 3.58 (d,*J* = 6.1 Hz, 1H), 3.34 (dd, *J* = 14.7, 6.2 Hz, 1H), 3.31 - 3.23 (m, 1H), 3.17 (s, 1H), 2.65 (dd, *J* = 39.8, 30.3 Hz, 4H), 2.45 (d, *J* = 39.0 Hz, 2H), 2.12 (s, 1H), 2.00 (s, 1H), 1.85 (s, 1H).

**Example 54: Preparation of Compound 54**

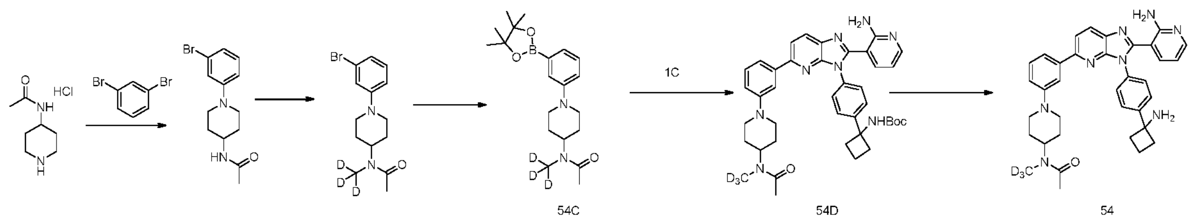


54A 54B 54C 54D 54

**Step A: Preparation of compound 54A**

To a reaction flask were added 1,1'-binaphthyl-2,2'-diphemyl phosphine (3.3 g), tris(dibenzylideneacetone)dipalladium (4.9 g), 1,3-dibromo-2-fluorobenzene (2.6 g), 4-acetylaminopiperidine hydrochloride (3.0 g), sodium *tert*-butoxide (3.6 g) and dioxane (30 mL) successively under nitrogen atmosphere. After the addition was completed, the reaction solution was stirred at 100 °C. After the reaction was completed, the reaction solution was concentrated and subjected to column chromatography (petroleum ether/ethyl acetate = 5/1) to give compound 54A (0.90 g).

MS (ESI, $[M+H]^+$) *m/z:* 297.1.

**Step B: Preparation of compound 54B**

To a reaction flask were added compound 54A (0.90 g), sodium hydride (1.2 g), deuterated iodomethane (3.1 g), and tetrahydrofuran (20 mL) successively. The mixture was stirred at room temperature. After the reaction was completed, a saturated ammonium chloride solution was added to the reaction solution, and water and ethyl acetate were added to extract the reaction solution; the reaction solution was then washed with saturated brine and dried over anhydrous sodium sulfate. The reaction solution was filtered and concentrated to give compound 54B (1.5 g).

MS (ESI, $[M+H]^+$) *m/z*: 314.5.

**Step C: Preparation of compound 54C**

To a reaction flask were added compound 54B (1.5 g), bis(pinacolato)diboron (2.4 g), potassium acetate (1.4 g), [1,1'-bis(diphenylphosphino)ferrocene]palladium dichloride dichloromethane complex (0.57 g) and 1,4-dioxane (50 mL) successively. The reaction solution was stirred at 100 °C under nitrogen atmosphere. After the reaction was completed, the reaction solution was filtered and concentrated. The reaction solution was subjected to column chromatography (dichloromethane/methanol = 20/1) to give compound 54C (0.62 g).

MS (ESI, $[M+H]^+$) *m/z*: 362.1.

**Step D: Preparation of compound 54D**

To a reaction flask were added compound 54C (0.33 g), compound 1C (0.30 g), potassium carbonate (0.17 g), tetrakis(triphenylphosphine)palladium (0.21 g), 1,4-dioxane (30 mL), and water (6 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 54D (0.28 g).

MS (ESI, $[M+H]^+$) *m/z*: 690.8.

**Step E: Preparation of compound 54**

Compound 54D (0.28 g), methanesulfonic acid (0.19 g) and dichloromethane (10 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 54 (0.14 g).

HRMS (ESI, $[M+H]^+$) *m/z*: 590.3460.

$^1H$ NMR (500 MHz, $CDCl_3$) δ 8.08 - 7.96 (m, 2H), 7.70 (dd, *J* = 8.4, 2.1 Hz, 1H), 7.55 (m, 1H), 7.49 (dd, *J* = 8.2, 5.9 Hz, 2H), 7.43 (dd, *J* = 15.3, 7.7 Hz, 1H), 7.40 - 7.32 (m, 2H), 7.25 (q, *J* = 8.1 Hz, 1H), 7.08 (dd, *J* = 7.8, 1.8 Hz, 1H), 6.89 (dd, *J* = 8.2, 2.6 Hz, 1H), 6.56 (s, 2H), 6.32 (dd, *J* = 7.8, 4.8 Hz, 1H), 4.59 (tt, *J* = 12.2, 4.1 Hz, 1H), 3.84 - 3.66 (m, 2H), 2.80 (m, 2H), 2.54 (m, 2H), 2.17 (m, 2H), 2.10 (s, 1H), 2.04 (s, 2H), 1.97 (s, 3H), 1.75 (m, 2H), 1.66 - 1.59 (m, 1H).

**Example 55: Preparation of Compound 55**

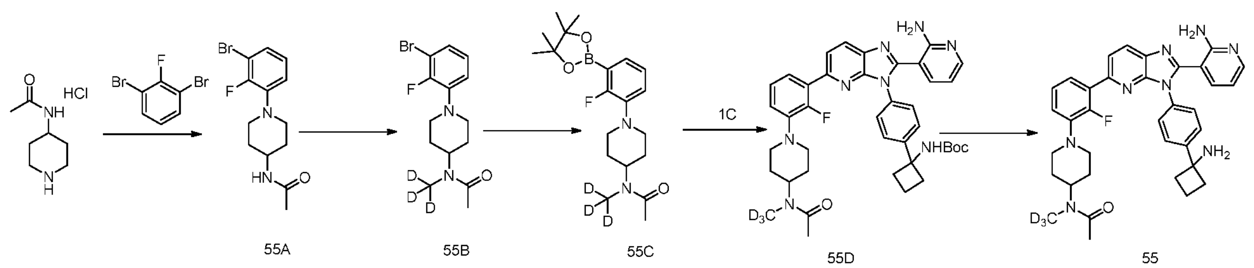


55A 55B 55C 55D 55

**Step A: Preparation of compound 55A**

To a reaction flask were added 1,1'-binaphthyl-2,2'-diphemyl phosphine (3.1 g), tris(dibenzylideneacetone)dipalladium (2.3 g), 1,3-dibromo-2-fluorobenzene (5.1 g), 4-acetylaminopiperidine hydrochloride (3.0 g), sodium *tert*-butoxide (3.6 g) and tetrahydrofuran (30 mL) successively under nitrogen atmosphere. After the addition was completed, the reaction solution was stirred at 100 °C. After the reaction was completed, the reaction solution was concentrated and subjected to column chromatography (petroleum ether/ethyl acetate = 5/1) to give compound 55A (1.0 g).

MS (ESI, $[M+H]^+$) *m/z:* 315.4.

**Step B: Preparation of compound 55B**

To a reaction flask were added compound 55A (1.0 g), sodium hydride (1.1 g), deuterated iodomethane (1.1 g), and tetrahydrofuran (10 mL) successively. The mixture was stirred at room temperature. After the reaction was completed, a saturated ammonium chloride solution was added to the reaction solution, and water and ethyl acetate were added to extract the reaction solution; the reaction solution was then washed with saturated brine and dried over anhydrous sodium sulfate. The reaction solution was filtered and concentrated to give compound 55B (1.4 g).

MS (ESI, $[M+H]^+$) *m/z*: 332.4.

**Step C: Preparation of compound 55C**

To a reaction flask were added compound 55B (1.4 g), bis(pinacolato)diboron (2.0 g), potassium acetate (1.2 g), [1,1'-bis(diphenylphosphino)ferrocene]palladium dichloride dichloromethane complex (0.5 g) and 1,4-dioxane (50 mL) successively. The reaction solution was stirred at 100 °C under nitrogen atmosphere. After the reaction was completed, the reaction solution was filtered and concentrated. The reaction solution was subjected to column chromatography (dichloromethane/methanol = 20/1) to give compound 55C (0.75 g).

MS (ESI, $[M+H]^+$) *m/z*: 380.1.

**Step D: Preparation of compound 55D**

To a reaction flask were added compound 55C (0.35 g), compound 1C (0.30 g), potassium carbonate (0.17 g), tetrakis(triphenylphosphine)palladium (0.21 g), 1,4-dioxane (30 mL), and water (6 mL) successively. After the addition was completed, the reaction solution was purged with nitrogen gas, and reacted at 100 °C. After the reaction was completed, the reaction solution was filtered and concentrated. The reaction solution was subjected to column chromatography (dichloromethane/methanol = 20/1) to give compound 55D (0.28 g).

MS (ESI, $[M+H]^+$) *m/z*: 708.5.

**Step E: Preparation of compound 55**

Compound 55D (0.28 g), methanesulfonic acid (0.19 g) and dichloromethane (10 mL) were added successively to a reaction flask. The mixture was stirred at room temperature. After the reaction was completed, 10% sodium hydroxide solution was added to the reaction solution to adjust the pH to 13, water and dichloromethane were added to extract the reaction solution, and the reaction solution was washed with saturated brine and dried over anhydrous sodium sulfate. The reaction solution was filtered, concentrated, and subjected to column chromatography (dichloromethane/methanol = 10/1) to give compound 55 (0.11 g).

HRMS (ESI, $[M+H]^+$) *m/z*: 608.3345.

$^1$H NMR (500 MHz, $CDCl_3$) δ 8.05 (dd, *J* = 8.3, 5.6 Hz, 1H), 7.99 (dd, *J* = 4.8, 1.9 Hz, 1H), 7.74 (dd, *J* = 8.4, 2.3 Hz, 1H), 7.48 (dd, *J* = 8.5, 2.5 Hz, 2H), 7.46 - 7.40 (m, 1H), 7.33 (dd, *J* = 8.4, 2.2 Hz, 2H), 7.09 - 7.00 (m, 2H), 6.98 - 6.89 (m, 1H), 6.54 (s, 2H), 6.31 (dd, *J* = 7.8, 4.9 Hz, 1H), 4.57 (tt, *J* = 12.3, 4.2 Hz, 1H), 3.53 - 3.36 (m, 2H), 2.86 - 2.66 (m, 2H), 2.53 (dddd, *J* = 12.1, 9.5, 6.2, 2.9 Hz, 2H), 2.20 - 2.14 (m, 3H), 2.09 (s, 2H), 2.04 (s, 3H), 1.86 (m, 2H), 1.74 (m, 1H), 1.66 (m, 2H).

**Experimental Example 1: Inhibitory Effect of Compound on Proliferation of LNcap Cells**

A dish of LNcap cells (human prostate cancer cells with PTEN deficiency) growing at log phase and in good conditions were taken and digested for 3 min by using 1 mL of pancreatin, then the mixture was added with 4 mL of complete culture medium to terminate digestion, and the cells were added to a centrifuge tube. 20 μL of cells were taken and counted, the required number of cells (mL) was pipetted and centrifuged at 1200 rpm for 5 min, the supernatant was discarded, and an appropriate amount of plating medium (RPMI medium + 5% FBS + 1% sodium pyruvate + 1% glutamine) was added to adjust the cell density to $3 \times 10^4$ cells/mL. The cells were seeded on a 96-well plate at 100 μL/well using a multi-channel pipette and cultured in a cell incubator at 37 °C, 5% $CO_2$ and saturated humidity. After incubation overnight, compounds were loaded using a nanoliter pipettor, 2 duplicate wells were set for each concentration,

and cells without compound were used as negative controls. After 72 h, CCK-8 was added at 10 μL/well for incubation for 4 h, then absorbance was measured at 450 nm with an Envision plate reader, and inhibition rate was calculated. Inhibition rate (%) = (mean value of negative control group - mean value of experimental group) / (mean value of negative control group - mean value of blank group) × 100%. A dose-response curve was fitted by four-parameter analysis, with the logarithm of compound concentration as abscissa and inhibition rate as ordinate, so that $IC_{50}$ was calculated. The experimental results are shown in Table 1.

Table 1

| Example | LNcap cells $IC_{50}$ nM | Example | LNcap cells $IC_{50}$ nM |
|---|---|---|---|
| **1** | 16 | **31** | 28 |
| **2** | 34 | **32** | 4.3 |
| **3** | 6.5 | **33** | 11 |
| **6** | 15 | **34** | 2.9 |
| **7** | 48 | **35** | 5.0 |
| **9** | 24 | **36** | 14 |
| **10** | 21 | **37** | 7.6 |
| **11** | 12 | **38** | 11 |
| **13** | 20 | **39** | 11 |
| **16** | 8.7 | **40** | 19 |
| **17** | 12 | **41** | 10 |
| **18** | 18 | **42** | 21 |
| **20** | 15 | **43** | 47 |
| **21** | 3.0 | **44** | 1.7 |
| **23** | 38 | **45** | 1.7 |
| **24** | 41 | **46** | 7.5 |
| **26** | 14 | **47** | 81 |
| **27** | 20 | **48** | 6.9 |
| **28** | 3.0 | **49** | 4.0 |
| **29** | 46 | **54** | 2.2 |
| **30** | 4.8 | **55** | 2.1 |

**Experimental Example 2: Inhibitory Effect of Compound on Phosphorylation of LNcap Cells AKT1 (S473)**

LNcap cells AKT1(S473) growing at log phase were taken, digested with 1 mL of pancreatin for 3 min, and added with 4 mL of complete medium to terminate digestion. The cells were added to a centrifuge tube. 20 μL of cells were taken and counted, the required number of cells (mL) was taken and centrifuged at 1200 rpm for 5 min, and a plating medium (2% FBS + phenol-free red 1640 base medium + 1% sodium pyruvate + 1% glutamine) was added to adjust the cell density to about 1x10E6 cells/mL. The cells were plated (96 wells) at the above cell density (100 μL/well), and cultured in a cell incubator at 37 °C, 5% $CO_2$ overnight; the next day, according to the plate distribution, the corresponding compound was sprayed using a nanoliter pipettor and incubated for 1 h at 37 °C in a cell incubator containing 5% $CO_2$; the supernatant was discarded, and the plate was added with 40 μL of lysis buffer (1×) containing blocking buffer and incubated by shaking at room temperature for 30 min. After the mixture was mixed, 16 μL of lysate was transferred to another 384-well small-volume white plate. The plate was added with 4 μL of pre-mixed antibody (v/v) in assay buffer, covered, centrifuged to mix well, and incubated overnight at room temperature. The 665 nm/620 nm signal value was detected using a PE Envision multi-functional microplate reader, and $IC_{50}$ was calculated by four-parameter fitting.

The results of the inhibitory effect of the compound on the phosphorylation of LNcap cells AKT1 (S473) are shown in table 2.

Table 2

| Example | Phosphorylation of AKT1 (S473) $IC_{50}$ nM | Example | Phosphorylation of AKT1 (S473) $IC_{50}$ nM |
|---|---|---|---|
| **1** | 34 | **32** | 11 |
| **2** | 17 | **33** | 17 |

(continued)

| Example | Phosphorylation of AKT1 (S473) $IC_{50}$ nM | Example | Phosphorylation of AKT1 (S473) $IC_{50}$ nM |
|---|---|---|---|
| **3** | 20 | **34** | 25 |
| **6** | 54 | **35** | 12 |
| **9** | 16 | **37** | 11 |
| **10** | 47 | **38** | 13 |
| **11** | 19 | **39** | 42 |
| **16** | 19 | **41** | 41 |
| **17** | 17 | **44** | 12 |
| **21** | 23 | **45** | 7.7 |
| **28** | 28 | **54** | 10 |
| **30** | 22 | **55** | 7.1 |

**Experimental Example 3: Pharmacokinetic Evaluation of Compound in Mice**

ICR mice, weight 18-22 g, after adaption for 3-5 days, were randomly divided into 4 groups of 9 mice, each group was intragastrically (IG) administered with compound 28 or compound 30 at a dose of 10 mg/kg, or intravenously (IV) administered with compound 28 or compound 30 at a dose of 1 mg/kg.

The animals to be tested (ICR mice) were fasted for 12 h before administration and food was provided 4 h after administration, and water was freely drunk before and after the experiment and during the experiment.

After intragastric administration, about 0.1 mL of blood was taken from eye sockets at 15 min, 30 min, 1 h, 2 h, 3 h, 4 h, 6 h, 8 h, 10 h, and 24 h; after intravenous injection, about 0.1 mL of blood was taken from eye sockets at 5 min, 10 min, 30 min, 1 h, 2 h, 4 h, 6 h, 8 h, 10 h, and 24 h; each mouse was collected at 3-4 time points and 3 mice were collected at each time point, whole blood was collected, placed in centrifuge tubes containing EDTA-$K_2$, and stored at 4 °C within 1 h, and the plasma was separated off by centrifugation at 4 °C and 4000 rpm for 10 min. All the plasma samples were collected and immediately stored at -20 °C for testing. 30 μL of the plasma sample to be tested and a standard curve sample were pipetted and added with 300 μL of acetonitrile solution containing an internal standard (20 ng/mL diazepam), shaken and mixed well for 5 min, and centrifuged at 13,000 rpm for 10 min. 80 μL of supernatant was taken and diluted with 80 μL of ultrapure water. After being mixed well, 2 μL of the resulting sample was taken for liquid chromatography-mass determination, and a chromatogram was recorded.

Oral and intravenous exposure of the compound of the present application was evaluated by *in vivo* pharmacokinetic experiment in mice. The results are shown in the table 3 below.

Table 3 *In vivo* pharmacokinetic parameters of compound in mice

| Compound | | Compound 28 | | Compound 30 | |
|---|---|---|---|---|---|
| Dosage | | IG 10 mg/kg | IV 1 mg/kg | IG 10 mg/kg | IV 1 mg/kg |
| AUC(0-t) | ng*h/mL | 871 | 246 | 2772 | 476 |
| AUC(0-∞) | ng*h/mL | 896 | 253 | 2884 | 482 |
| MRT(0-t) | h | 5.28 | 1.49 | 5.01 | 1.04 |
| t1/2z | h | 5.31 | 1.74 | 6.58 | 1.48 |
| Tmax | h | 2 | / | 2 | / |
| Cmax | ng/mL | 140 | / | 542 | / |
| Vd | L/kg | / | 9.92 | / | 4.43 |
| Cl | L/kg | / | 3.95 | / | 2.08 |
| Absolute bioavailability F% | | 35.42% | | 58.23% | |
| | | | | | |
| Compound | | Compound 44 | | Compound 45 | |
| Dosage | | IG 10 mg/kg | IV 1 mg/kg | IG 10 mg/kg | IV 1 mg/kg |
| AUC(0-t) | ng*h/mL | 24990 | 4837 | 14539 | 4243 |
| AUC(0-∞) | ng*h/mL | 25071 | 4845 | 14543 | 4339 |
| MRT(0-t) | h | 3.58 | 0.817 | 3.95 | 1.176 |

(continued)

| Compound | | Compound 44 | | Compound 45 | |
|---|---|---|---|---|---|
| Dosage | | IG 10 mg/kg | IV 1 mg/kg | IG 10 mg/kg | IV 1 mg/kg |
| t1/2z | h | 3.61 | 1.34 | 2.06 | 1.159 |
| Tmax | h | 2 | / | 0.25 | / |
| Cmax | ng/mL | 4965 | / | 4016 | / |
| Vd | L/kg | / | 0.398 | / | 5.423 |
| Cl | L/kg | / | 0.206 | / | 0.23 |
| Absolute bioavailability F% | | 51.67% | | 34.27% | |

**Experimental Example 4: Pharmacokinetic Evaluation of Compound in Rats**

SD rats, weight 180-220 g, after adaption for 7 days, were randomly divided into 4 groups of 3 mice, each group was intragastrically (IG) administered with compound 28 or compound 30 at a dose of 10 mg/kg, or intravenously (IV) administered with compound 28 or compound 30 at a dose of 1 mg/kg. The animals to be tested (SD rats) were fasted for 12 h before administration and food was provided 4 h after administration, and water was freely drunk before and after the experiment and during the experiment.

After intragastric administration, about 0.1 mL of blood was taken from eye sockets at 15 min, 30 min, 1 h, 2 h, 3 h, 4 h, 6 h, 8 h, 10 h, and 24 h; after intravenous injection, about 0.1 mL of blood was taken from eye sockets at 5 min, 10 min, 30 min, 1 h, 2 h, 4 h, 6 h, 8 h, 10 h, and 24 h; 3 rats were collected at each time point, whole blood was collected, placed in centrifuge tubes containing EDTA-$K_2$, and stored at 4 °C within 1 h, and the plasma was separated off by centrifugation at 4 °C and 4000 rpm for 10 min. All the plasma samples were collected and immediately stored at -20 °C for testing.

30 μL of the plasma sample to be tested and a standard curve sample were pipetted and added with 300 μL of acetonitrile solution containing an internal standard (20 ng/mL diazepam), shaken and mixed well for 5 min, and centrifuged at 13,000 rpm for 10 min. 80 μL of supernatant was taken and diluted with 80 μL of ultrapure water. After being mixed well, 2 μL of the resulting sample was taken for liquid chromatography-mass determination, and a chromatogram was recorded.

Oral and intravenous exposure of the compound of the present application was evaluated by *in vivo* pharmacokinetic experiment in rats. The results are shown in the table 4 below.

Table 4 *In vivo* pharmacokinetic parameters of compound in rats

| Compound | | Compound 28 | | Compound 30 | |
|---|---|---|---|---|---|
| Theoretical dosage | | IG 10 mg/kg | IV 1 mg/kg | IG 10 mg/kg | IV 1 mg/kg |
| AUC(0-t) | ng*h/mL | 362±195 | 145±11.1 | 358±109 | 127±9.69 |
| AUC(0-∞) | ng*h/mL | 372±197 | 174±4.37 | 383±106 | 139±9.21 |
| MRT(0-t) | h | 7.38±0.43 | 2.12±0.099 | 8.15±0.73 | 5.57±0.053 |
| t1/2z | h | 3.93±1.46 | 5.9±2.11 | 5.43±1.82 | 7.73±0.440 |
| Tmax | h | 4.67±1.15 | / | 4.67±1.15 | / |
| Cmax | ng/mL | 32.6±12.4 | / | 31.2±8.80 | / |
| Vd | L/kg | / | 48.8±16.8 | / | 80.8±8.90 |
| Cl | L/kg | / | 5.75±0.146 | / | 7.23±0.496 |
| Absolute bioavailability F% | | 24.97% | | 35.47% | |

**Experimental Example 5: Pharmacodynamics of Compound in Subcutaneous Xenograft Tumor Nude Mouse Model of AN3CA Human Endometrial Cancer Cells**

SPF-grade female BALB/C nude mice (from Changzhou Cavens Laboratory Animal Ltd.) were inoculated subcutaneously at the right side armpit with 5 × $10^6$ AN3CA cells (sourec: Nanjing Cobioer Biosciences Co., Ltd.). When the mean tumor volume reached about 130 $mm^3$, the animals were divided into 3 groups of 6 and dosed as shown in table 5:

Table 5 Administration regimen

| Groups | Dose (mg/kg) | Route of administration | Frequency of administration | Treatment cycle |
|---|---|---|---|---|
| Control group (vehicle control) | ---- | i.g. | qd | 14d |
| Compound 28 group | 20 | i.g. | qd | 14d |
| Compound 30 group | 20 | i.g. | qd | 14d |
| Note: i.g. represents intragastric; qd represents once daily. | | | | |

The day of grouping was determined as d0, and the intragastric administration was started on the grouping day, and the administration volume was 10 mL/kg. The solvent was: D5W (5% glucose solution). The tumor volume was measured 2-3 times a week, meanwhile, the mice were weighed and the data were recorded; the general behavior of the mice was observed and recorded every day. After the experiment was completed, the tumors were removed, weighed and photographed. Calculation formula for tumor volume: Tumor volume ($mm^3$) = 1/2 × (a × $b^2$) (where a represents long diameter and b represents short diameter).

Relative tumor proliferation rate (T/C%) refers to the percentage of the relative tumor volume of the treatment and control groups at a certain time point. The calculation formula is as follows: T/C% = $T_{RTV}/C_{RTV}$ × 100% ($T_{RTV}$: mean relative tumor volume (RTV) for treatment group; $C_{RTV}$: mean RTV for vehicle control group; RTV = $TV_t/TV_0$, where $TV_0$ is the tumor volume at grouping, TVt is the tumor volume after treatment).

Tumor growth inhibition rate (TGI%) was calculated according to the following formula: TGI% = (1 – tumor weight in treatment group/tumor weight in control group) × 100%.

The calculation formula for weight change rate (WCR) (%) is: WCR = ($Wt_t$ - $Wt_0$) / $Wt_0$ × 100%, where $Wt_0$ is the body weight of the animal at grouping (i.e., d0), Wtt is the body weight of the animal at each measurement.

All experimental results are expressed as mean ± SD (mean ± standard deviation). The relative tumor volume of the treatment group was compared with that of the control group for any significant difference by T tests, wherein p < 0.01 indicates a very significant difference.

The results are shown in table 6, and the *in vivo* efficacy results show that both compound 28 and compound 30 have strong *in vivo* tumor inhibition effects.

Table 6 Effect of AKT inhibitors on the subcutaneous xenograft tumor of AN3CA human endometrial cancer cells

| **Groups** | **Dosage** mg/kg | **On day 14 of experiment** | | | | |
|---|---|---|---|---|---|---|
| | | **Tumor volume** ($mm^3$) (mean±SD) | **Relative tumor volume** (mean ± SD) | **T/C (%)** | **TGI (%)** | **Weight change rate** (%) (mean ± SD) |
| Control group Vehicle control | ---- | 2838±498 | 23.7±8.1 | -- | -- | 11.0±3.6 |
| Compound 28 group | 20 | 1267±249 | 10.5±3.4 | 44.3% | 53.2%** | 4.9±2.7 |
| Compound 30 group | 20 | 1242±354 | 9.7±2.2 | 40.9% | 58.4%** | 2.3±3.5 |
| Note: comparison with the control group, where ** denotes p < 0.01. | | | | | | |

## Claims

1. A compound of formula (I) or a pharmaceutically acceptable salt thereof,

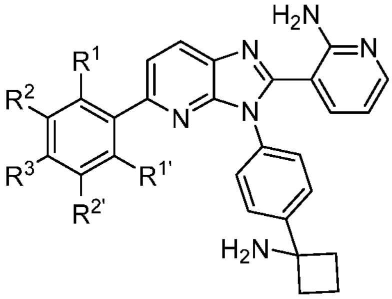

(I)

wherein,

$R^1$ and $R^{1'}$ are each independently selected from the group consisting of hydrogen and halogen;

$R^2$, $R^{2'}$ and $R^3$ are each independently selected from the group consisting of hydrogen, amino, amino-$C_1$-$C_6$ alkyl-, 7-9 membered spiro-heterocyclyl, 10 membered spiro-heterocyclyl whose ring atoms consist of a nitrogen atom and a carbon atom, 7-10 membered fused-heterocyclyl, 7-10 membered bridged heterocyclyl, 5-6 membered heterocyclyl whose ring atoms consist of a nitrogen atom and a carbon atom, 5-6 membered heterocyclyl whose ring atoms contain a silicon atom or a phosphorus atom,

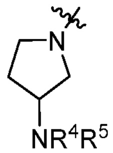 , 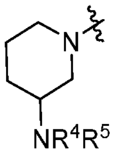 , 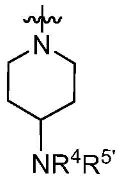 , 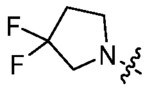 and 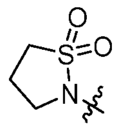 ,

wherein the amino or amino-Ci-$C_6$ alkyl- is substituted with one or more $R^{21}$, and optionally substituted with one or more $R^{21}$, wherein the 5-6 membered heterocyclyl whose ring atoms consist of a nitrogen atom and a carbon atom is substituted with one or more $R^{22}$, and optionally substituted with one or more $R^{22'}$, wherein the 7-9 membered spiro-heterocyclyl, 10 membered spiro-heterocyclyl whose ring atoms consist of a nitrogen atom and a carbon atom, 7-10 membered heterocycloalkenyl or 5-6 membered heterocyclyl whose ring atoms contain a silicon atom or a phosphorus atom is optionally substituted with one or more $R^{23}$, wherein the 7-10 membered bridged heterocyclyl is substituted with one or more $R^{24}$,

or, $R^3$ is connected to $R^2$ or $R^{2'}$, and together with the carbon atoms connected thereto, forms morpholinyl optionally substituted with one or more $R^{25}$,

and $R^2$, $R^{2'}$ and $R^3$ are not hydrogen at the same time;

$R^{21}$ is selected from the group consisting of $C_2$-$C_6$ alkenyl-C(O)-, $C_3$-$C_{12}$ cycloalkyl, 4-12 membered heterocyclyl or $C_1$-$C_6$ alkylacylamino-$C_1$-$C_6$ alkyl-, wherein the $C_3$-$C_{12}$ cycloalkyl, 4-12 membered heterocyclyl or $C_1$-$C_6$ alkylacylamino-$C_1$-$C_6$ alkyl- is optionally substituted with one or more of the following groups selected from the group consisting of: hydroxy, $C_1$-$C_6$ alkyl and $C_1$-$C_6$ alkylacyl; $R^{22}$ is selected from the group consisting of $C_2$-$C_6$ alkynyl-C(O)-, 4-5 membered heterocyclyl or 6 membered heterocyclyl whose ring atoms consist of a nitrogen atom and a sulfur atom, wherein the 4-5 membered heterocyclyl or 6 membered heterocyclyl whose ring atoms consist of a nitrogen atom and a sulfur atom is optionally substituted with one or more of the following groups selected from the group consisting of:

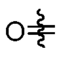 ,

halogen, $C_1$-$C_3$ alkyl and $C_1$-$C_6$ alkylacyl;

$R^{21'}$ and $R^{22'}$ are each independently selected from the group consisting of deuterium and $C_1$-$C_6$ alkyl;

$R^{23}$ and $R^{24}$ are each independently selected from the group consisting of

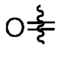 ,

$C_1$-$C_6$ alkyl, $C_1$-$C_6$ alkylsulfonyl, $C_1$-$C_6$ alkylacyl and $C_1$-$C_6$ alkylacyl-N ($C_1$-$C_6$ alkyl)-;
$R^{25}$ is selected from $C_1$-$C_6$ alkylacyl;
$R^4$ is selected from the group consisting of $C_1$-$C_6$ alkylacyl and $C_1$-$C_6$ alkylsulfonyl;
$R^5$ is selected from $C_1$-$C_6$ alkyl;
$R^{5'}$ is selected from the group consisting of $-CD_3$ and $-CH_3$, and when $R^{5'}$ is $-CH_3$, $R^1$ or $R^{1'}$ is halogen.

**2.** The compound of formula (I) or the pharmaceutically acceptable salt thereof according to claim 1, wherein $R^1$ and $R^{1'}$ are each independently selected from the group consisting of hydrogen and fluorine;

or, $R^1$ and $R^{1'}$ are both selected from hydrogen;
or, $R^1$ is selected from hydrogen, and $R^{1'}$ is selected from fluorine;
or, $R^1$ is selected from fluorine, and $R^{1'}$ is selected from hydrogen.

**3.** The compound of formula (I) or the pharmaceutically acceptable salt thereof according to claim 1 or 2, wherein $R^2$, $R^{2'}$ and $R^3$ are each independently selected from the group consisting of hydrogen, amino, amino-$C_1$-$C_4$ alkyl-, 7, 8 or 9 membered spiro-heterocyclyl, 10 membered spiro-heterocyclyl whose ring atoms consist of a nitrogen atom and a carbon atom, 7, 8 or 9 membered fused-heterocyclyl, 7-8 membered bridged heterocyclyl, 5-6 membered monoheterocyclyl whose ring atoms consist of a nitrogen atom and a carbon atom, 5-6 membered monoheterocyclyl whose ring atoms consist of a silicon atom or a phosphorus atom,

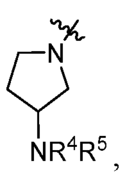 , 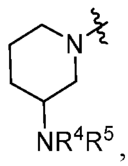 , 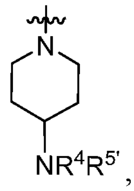 , 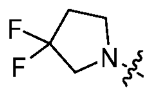

and

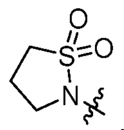 ,

wherein the amino or amino-$C_1$-$C_4$ alkyl- is substituted with one or more $R^{21}$, and optionally substituted with one or more $R^{21}$, wherein the 5-6 membered monoheterocyclyl whose ring atoms consist of a nitrogen atom and a carbon atom is substituted with one or more $R^{22}$, and optionally substituted with one or more $R^{22'}$, wherein the 7, 8 or 9 membered spiro-heterocyclyl, 10 membered spiro-heterocyclyl whose ring atoms consist of a nitrogen atom and a carbon atom, 7, 8 or 9 membered fused-heterocyclyl, or 5-6 membered monoheterocyclyl whose ring atoms contain a silicon atom or a phosphorus atom is optionally substituted with one or more $R^{23}$, wherein the 7-8 membered bridged heterocyclyl is substituted with one or more $R^{24}$,

or, $R^3$ is connected to $R^2$ or $R^{2'}$, and together with the carbon atoms connected thereto, forms morpholinyl optionally substituted with one or more $R^{25}$,
and $R^2$, $R^{2'}$ and $R^3$ are not hydrogen at the same time;
or, $R^2$, $R^{2'}$ and $R^3$ are each independently selected from the group consisting of hydrogen, amino, amino-$C_1$-$C_4$ alkyl-, 7, 8 or 9 membered spiro-heterocycloalkyl, 10 membered spiro-heterocycloalkyl whose ring atoms consist of a nitrogen atom and a carbon atom, 7, 8 or 9 membered fused-heterocycloalkyl, 7-8 membered bridged heterocycloalkyl, 5-6 membered monoheterocycloalkyl whose ring atoms consist of a nitrogen atom and a carbon atom, 5-6 membered monoheterocycloalkyl whose ring atoms contain a silicon atom or a phosphorus atom,

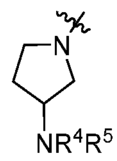, 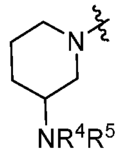, 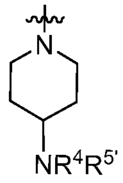,

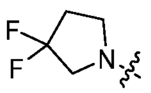 and 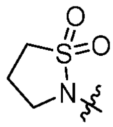,

wherein the amino or amino-$C_1$-$C_4$ alkyl- is substituted with one or more $R^{21}$, and optionally substituted with one or more $R^{21'}$, wherein the 5-6 membered monoheterocycloalkyl whose ring atoms consist of a nitrogen atom and a carbon atom is substituted with one or more $R^{22}$, and optionally substituted with one or more $R^{22'}$, wherein the 7, 8 or 9 membered spiro-heterocycloalkyl, 10 membered spiro-heterocycloalkyl whose ring atoms consist of a nitrogen atom and a carbon atom, 7, 8 or 9 membered fused-heterocycloalkyl, or 5-6 membered monoheterocycloalkyl whose ring atoms contain a silicon atom or a phosphorus atom is optionally substituted by one or more $R^{23}$, wherein the 7-8 membered bridged heterocycloalkyl is substituted by one or more $R^{24}$, or, $R^3$ is connected to $R^2$ or $R^{2'}$, and together with the carbon atoms connected thereto, forms morpholinyl optionally substituted with one or more $R^{25}$,

and $R^2$, $R^{2'}$ and $R^3$ are not hydrogen at the same time.

**4.** The compound of formula (I) or the pharmaceutically acceptable salt thereof according to claim 1 or 2, wherein $R^2$, $R^{2'}$ and $R^3$ are each independently selected from the group consisting of hydrogen, amino, aminomethyl,

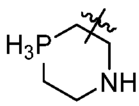, 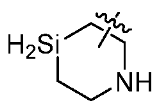, 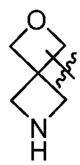, 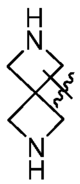, 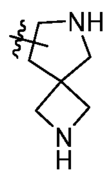, 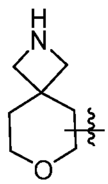, 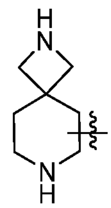,

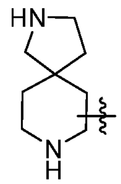, 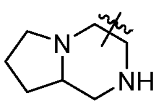, 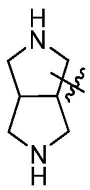, 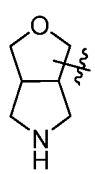, 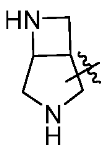, 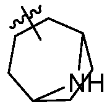, 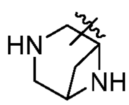, 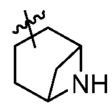, 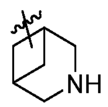,

pyrrolidinyl, piperidinyl, piperazinyl,

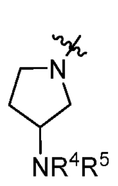, 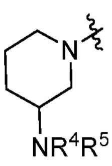, 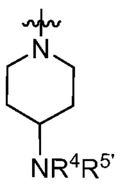, 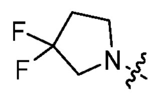 and 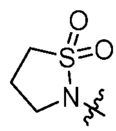,

wherein the amino or aminomethyl is substituted with one or more $R^{21}$, and optionally substituted with one or more $R^{21}$, wherein the pyrrolidinyl, piperidinyl or piperazinyl is substituted with one or more $R^{22}$, and optionally substituted with one or more $R^{22}$, wherein the

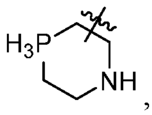, 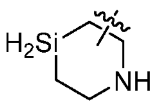,

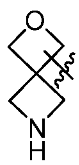, 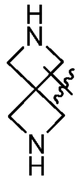, 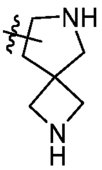, 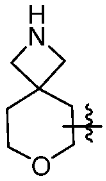, 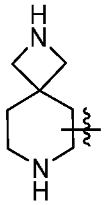, 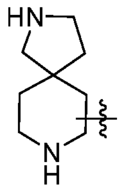, 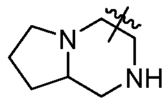, 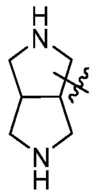, 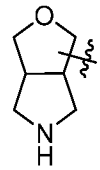

or

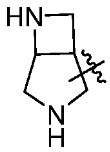

is substituted with one or more $R^{23}$, wherein the

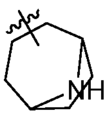, 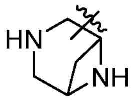, 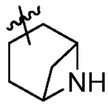

or

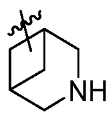

is substituted with one or more $R^{24}$,

or, $R^3$ is connected to $R^2$ or $R^{2'}$, and together with the carbon atoms connected thereto, forms a

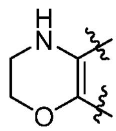

group optionally substituted with one or more $R^{25}$,
and $R^2$, $R^{2'}$ and $R^3$ are not hydrogen at the same time;
or, $R^2$, $R^{2'}$ and $R^3$ are each independently selected from the group consisting of hydrogen, amino, aminomethyl,

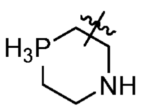, 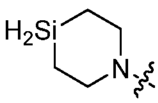, 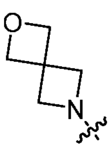, 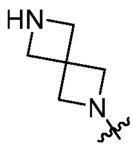, 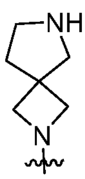, 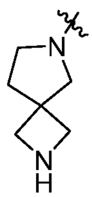, 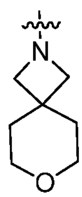, 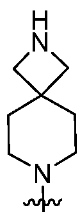,

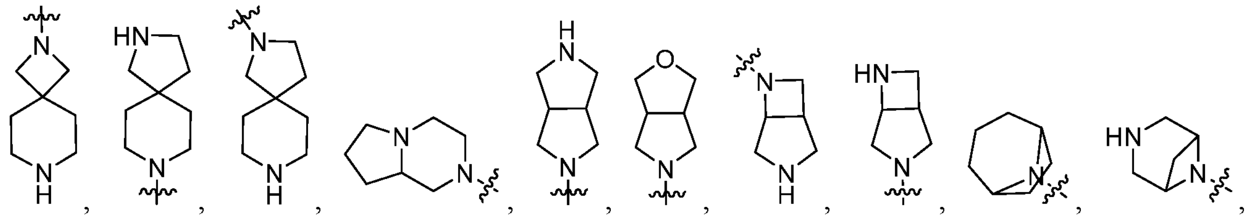,

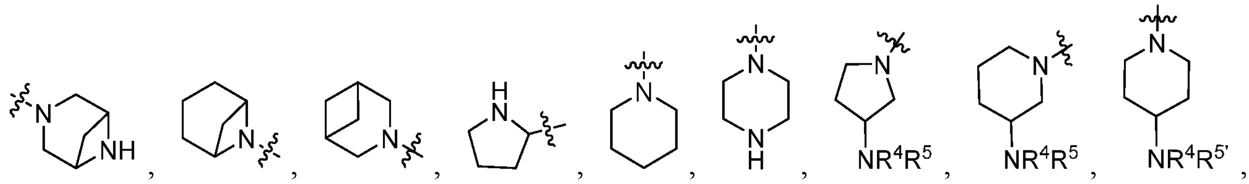,

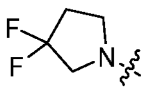 and 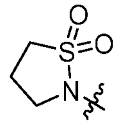,

wherein the amino or aminomethyl is substituted with one or more $R^{21}$, and optionally substituted with one or more $R^{21'}$, wherein the

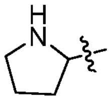, 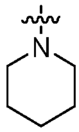 or 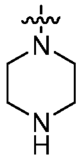

is substituted with one or more $R^{22}$, and optionally substituted with one or more $R^{22}$, wherein the

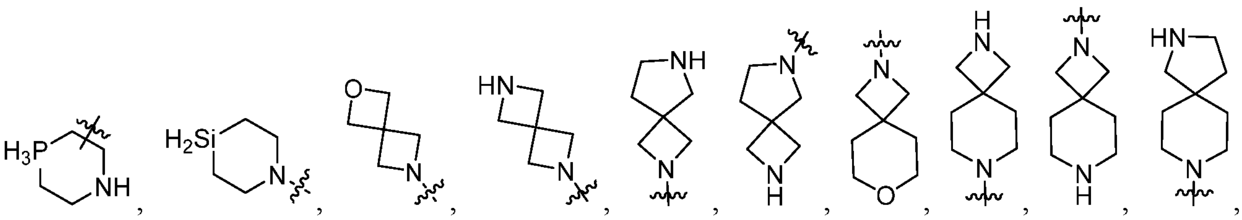,

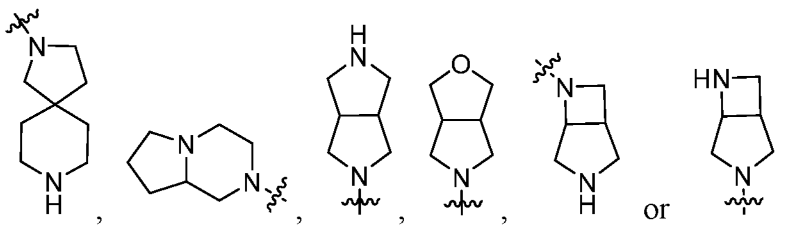 or

is substituted with one or more $R^{23}$, wherein the

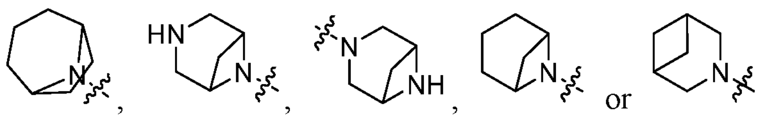 or

is substituted with one or more $R^{24}$,

or, $R^3$ is connected to $R^2$ or $R^{2'}$, and together with the carbon atoms connected thereto, forms a

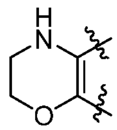

group optionally substituted with one or more $R^{25}$,

and $R^2$, $R^{2'}$ and $R^3$ are not hydrogen at the same time.

**5.** The compound of formula (I) or the pharmaceutically acceptable salt thereof according to any one of claims 1-4, wherein $R^{21}$ is selected from the group consisting of $C_2$-$C_4$ alkenyl-C(O)-, $C_5$-$C_{10}$ cycloalkyl, 4-6 membered heterocyclyl or $C_1$-$C_4$ alkylacylamino-$C_1$-$C_4$ alkyl-, wherein the $C_5$-$C_{10}$ cycloalkyl, 4-6 membered heterocyclyl or $C_1$-$C_4$ alkylacylamino-$C_1$-$C_4$ alkyl- is optionally substituted with one or more of the following groups selected from the group consisting of: hydroxy, $C_1$-$C_6$ alkyl and $C_1$-$C_4$ alkylacyl;

or, $R^{21}$ is selected from the group consisting of $C_2$-$C_3$ alkenyl-C(O)-, $C_{10}$ bridged cycloalkyl, 5 membered or 6 membered monoheterocycloalkyl, or $C_1$-$C_3$ alkylacylamino-$C_1$-$C_3$ alkyl-, wherein the $C_{10}$ bridged cycloalkyl, 5 membered or 6 membered monoheterocycloalkyl, or $C_1$-$C_3$ alkylacylamino-$C_1$-$C_3$ alkyl- is optionally substituted with one or more of the following groups selected from the group consisting of: hydroxy, $C_1$-$C_4$ alkyl and $C_1$-$C_4$ alkylacyl;

or, $R^{21}$ is selected from the group consisting of $H_2C{=}CHC(O)$-,

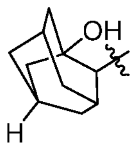

,

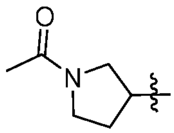

,

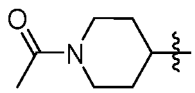

and $CH_3C(O)N(CH_3)$-$CH_3CH_2$-;

or, $R^{21}$ is selected from the group consisting of $H_2C{=}CHC(O)$-,

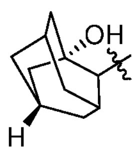

,

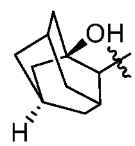

,

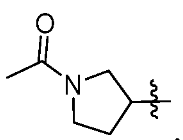

,

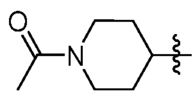

and $CH_3C(O)N(CH_3)$-$CH_3CH_2$-.

**6.** The compound of formula (I) or the pharmaceutically acceptable salt thereof according to any one of claims 1-5, wherein $R^{22}$ is selected from the group consisting of $C_2$-$C_3$ alkynyl-C(O)- and 4 membered or 5 membered heterocyclyl, wherein the 4 membered or 5 membered heterocyclyl is optionally substituted with one or more

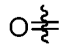,

or, $R^{22}$ is selected from the group consisting of $C_2$-$C_3$ alkynyl-C(O)- and 4 membered or 5 membered heterocycloalkyl, wherein the 4 membered or 5 membered heterocycloalkyl is optionally substituted with one or more

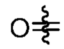,

or, $R^{22}$ is selected from the group consisting of $H_3CC{\equiv}CC(O)$-,

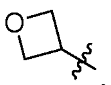, 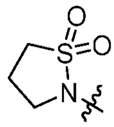 and 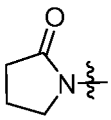.

**7.** The compound of formula (I) or the pharmaceutically acceptable salt thereof according to any one of claims 1-6, wherein $R^{22}$ is selected from the group consisting of $C_2$-$C_3$ alkynyl-C(O)-, 4 membered or 5 membered heterocyclyl and 6 membered heterocyclyl whose ring atoms consist of a nitrogen atom and a sulfur atom, wherein the 4 membered or 5 membered heterocyclyl, or 6 membered heterocyclyl whose ring atoms consist of a nitrogen atom and a sulfur atom is optionally substituted with one or more of the following groups selected from the group consisting of:

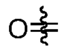,

halogen, $C_1$-$C_3$ alkyl and $C_1$-$C_6$ alkylacyl; $R^{22}$ is selected from the group consisting of $C_2$-$C_3$ alkynyl-C(O)-, 4 membered or 5 membered heterocycloalkyl and

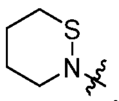,

wherein the 4 membered or 5 membered heterocycloalkyl or

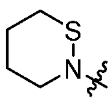

is optionally substituted with one or more of the following groups selected from the group consisting of:

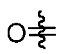,

halogen, $C_1$-$C_3$ alkyl and $C_1$-$C_6$ alkylacyl; or, $R^{22}$ is selected from $H_3CC{\equiv}CC(O)$-,

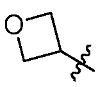, 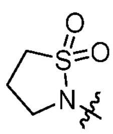, 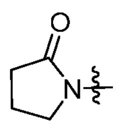, 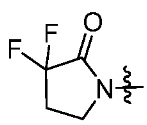, 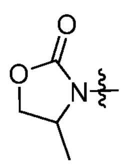, 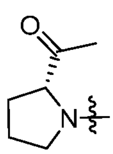,

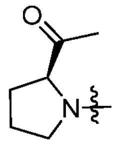 and 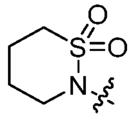 .

8. The compound of formula (I) or the pharmaceutically acceptable salt thereof according to any one of claims 1-7, wherein $R^{21'}$ and $R^{22'}$ are each independently selected from the group consisting of deuterium and $C_1$-$C_4$ alkyl; or, $R^{21'}$ and $R^{22'}$ are each independently selected from the group consisting of deuterium and methyl.

9. The compound of formula (I) or the pharmaceutically acceptable salt thereof according to any one of claims 1-8, wherein $R^{23}$ is selected from the group consisting of

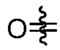,

$C_1$-$C_4$ alkyl, $C_1$-$C_4$ alkylsulfonyl and $C_1$-$C_4$ alkylacyl; or, $R^{23}$ is selected from the group consisting of

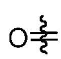,

$CH_3$-, $CH_3S(O)_2$- and $CH_3C(O)$-.

10. The compound of formula (I) or the pharmaceutically acceptable salt thereof according to any one of claims 1-9, wherein $R^{24}$ is selected from $C_1$-$C_4$ alkylacyl-N ($C_1$-$C_4$ alkyl)-;
or, $R^{24}$ is selected from $CH_3C(O)N(CH_3)$-.

11. The compound of formula (I) or the pharmaceutically acceptable salt thereof according to any one of claims 1-10, wherein $R^{25}$ is selected from $C_1$-$C_4$ alkylacyl;
or, $R^{25}$ is selected from $CH_3C(O)$-.

12. The compound of formula (I) or the pharmaceutically acceptable salt thereof according to any one of claims 1-11, wherein $R^4$ is selected from the group consisting of $C_1$-$C_4$ alkylacyl and $C_1$-$C_4$ alkylsulfonyl, and $R^5$ is selected from $C_1$-$C_4$ alkyl;
or, $R^4$ is selected from the group consisting of $CH_3C(O)$- and $CH_3S(O)_2$-, and $R^5$ is selected from methyl.

13. The compound of formula (I) or the pharmaceutically acceptable salt thereof according to any one of claims 1-12, wherein $R^{5'}$ is selected from the group consisting of $-CD_3$ and $-CH_3$, and when $R^{5'}$ is $-CH_3$, $R^1$ or $R^{1'}$ is fluorine.

14. The compound of formula (I) or the pharmaceutically acceptable salt thereof according to any one of claims 1-13, wherein $R^2$, $R^{2'}$ and $R^3$ are each independently selected from the group consisting of hydrogen,

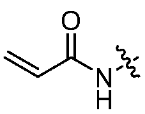, 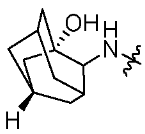, 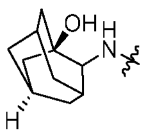, 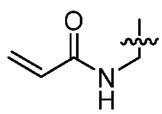, 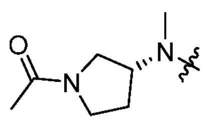,

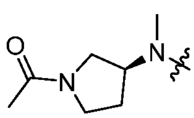, 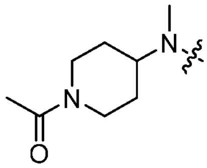, 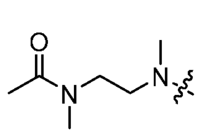, 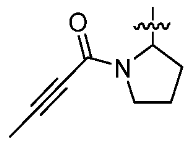, 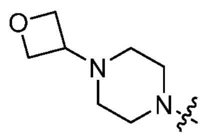,

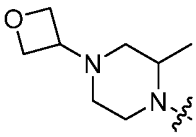, 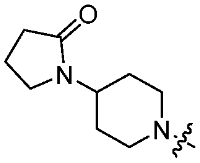, 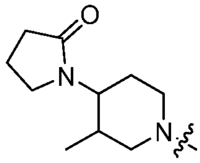, 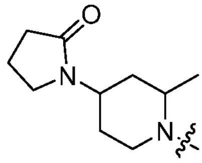, 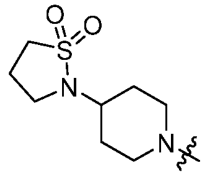,

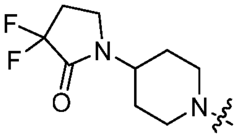, 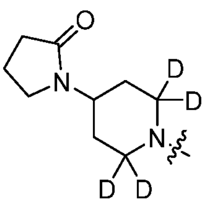, 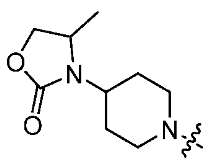, 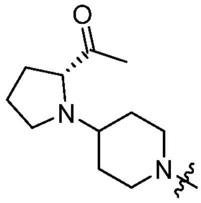, 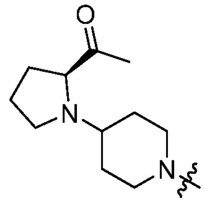,

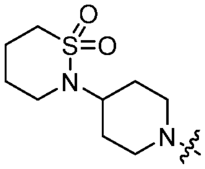, 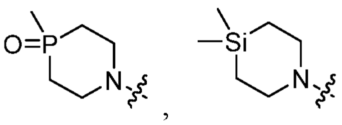, 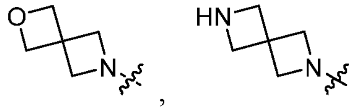, 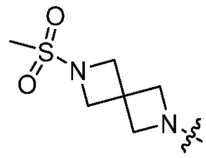,

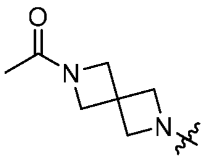, 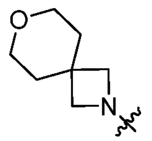, 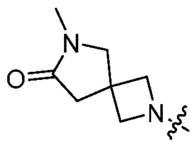, 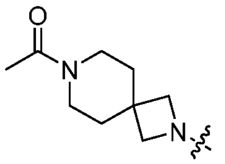, 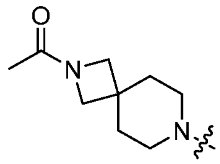,

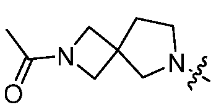, 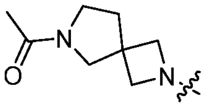, 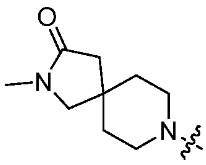, 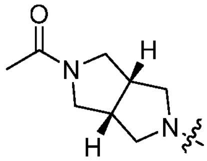, 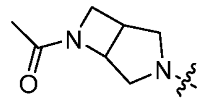,

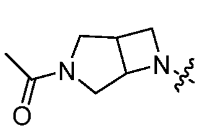, 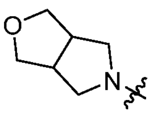, 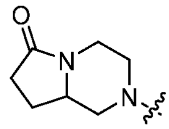, 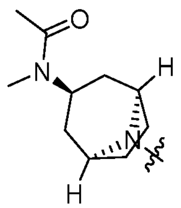, 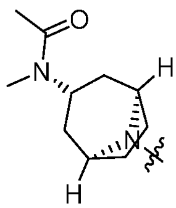, 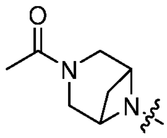,

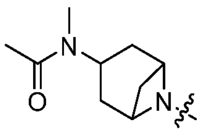, 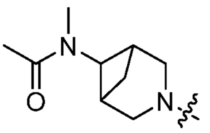, 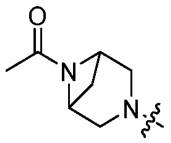, 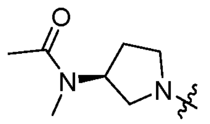, 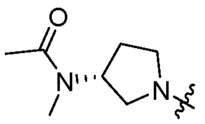,

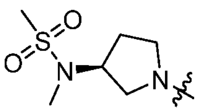, 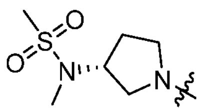, 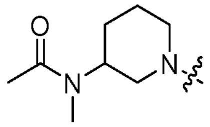, 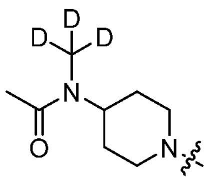, 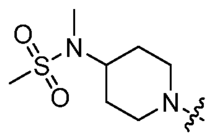,

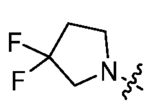 and 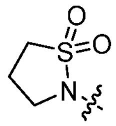,

or $R^3$ is connected to $R^2$ or $R^{2'}$, such that the structural unit

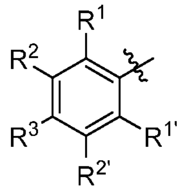

is

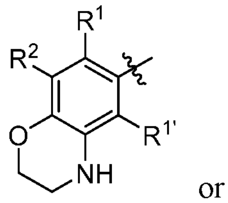 or 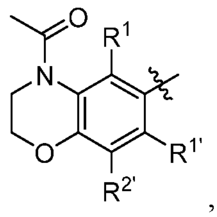,

and $R^2$, $R^{2'}$ and $R^3$ are not hydrogen at the same time.

**15.** A compound of the following formulas or a pharmaceutically acceptable salt thereof,

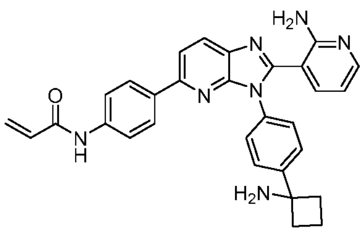

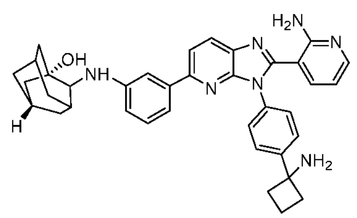

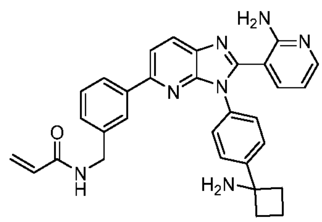

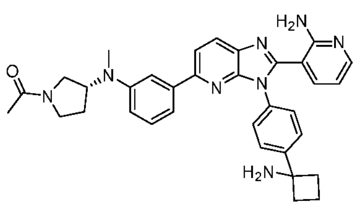

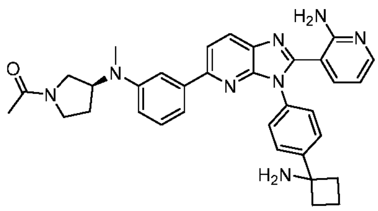

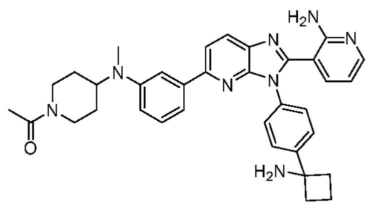

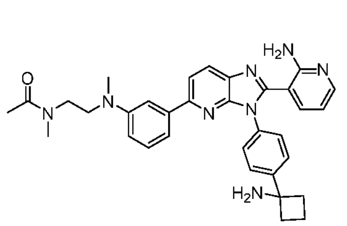

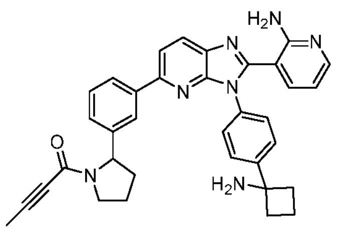

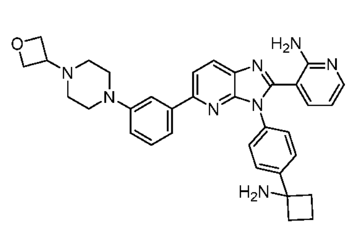

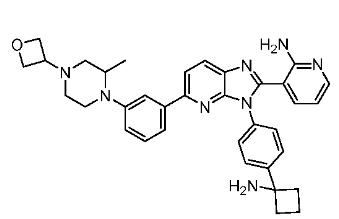
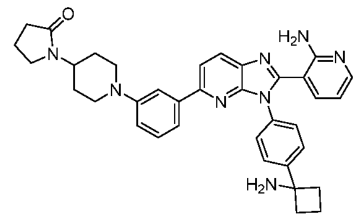
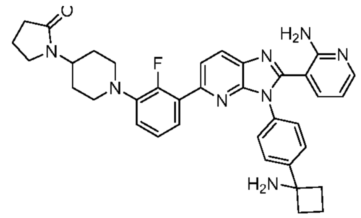

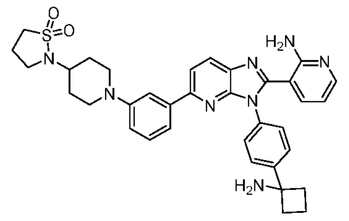
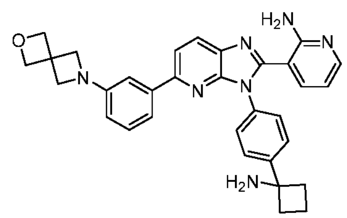
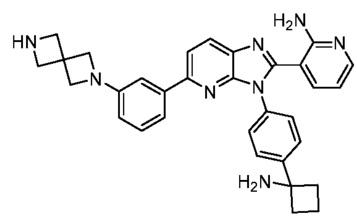

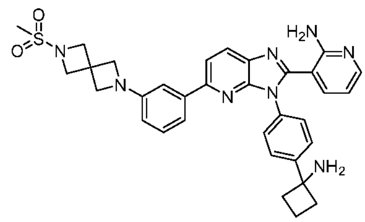
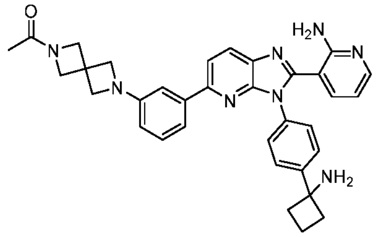
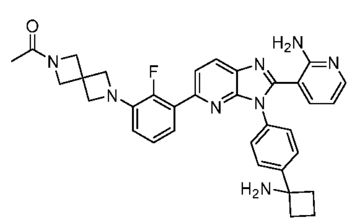

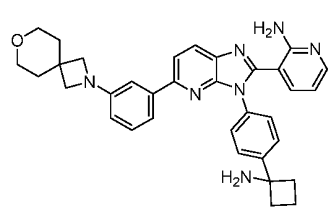
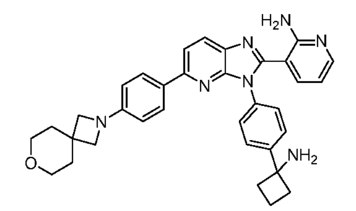
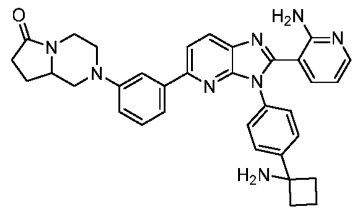

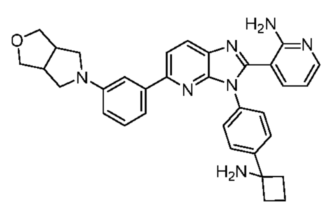
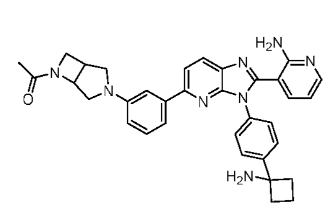
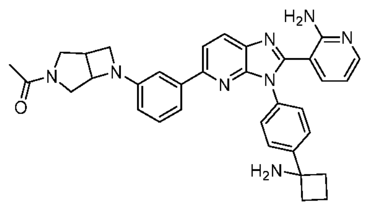

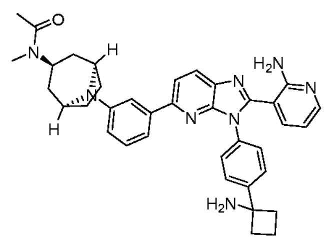
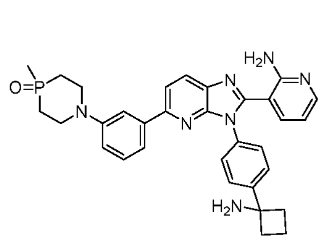
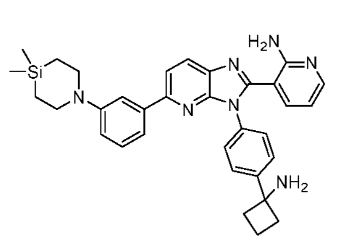

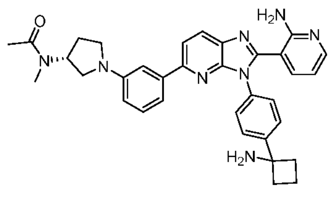
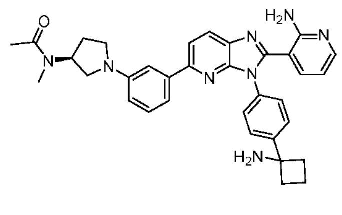
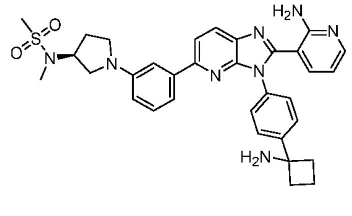

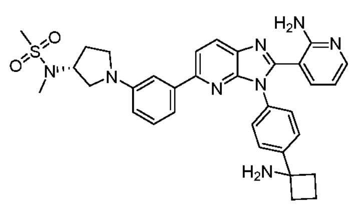
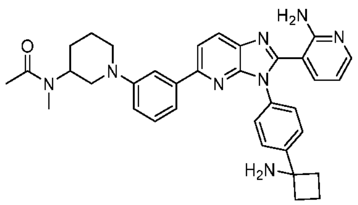
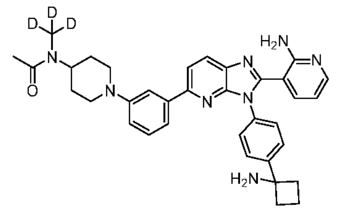
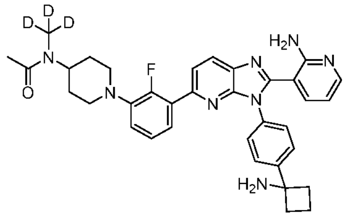
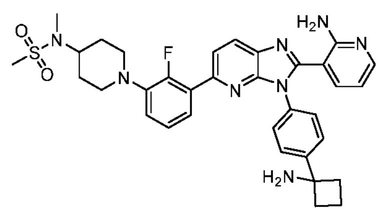
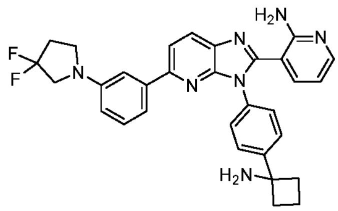
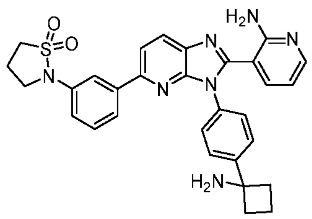
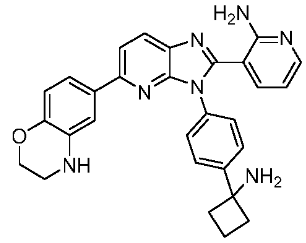
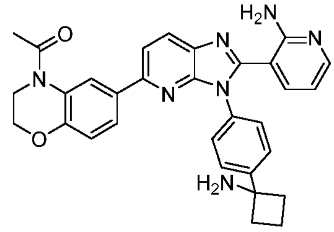
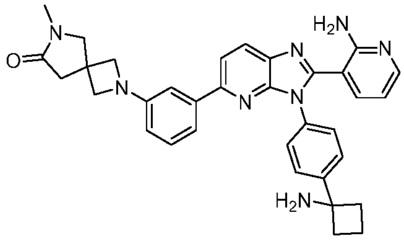
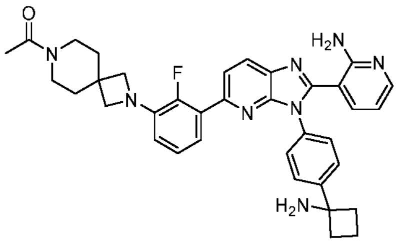
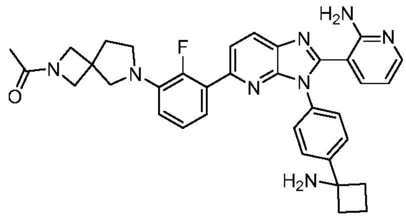
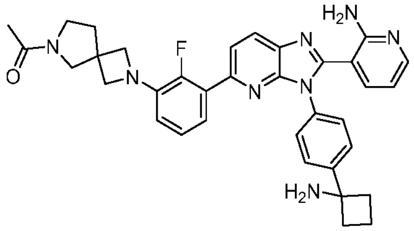
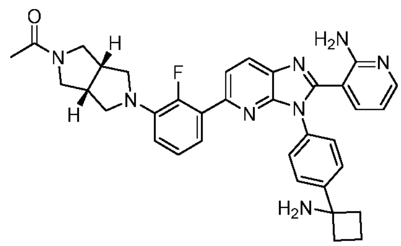
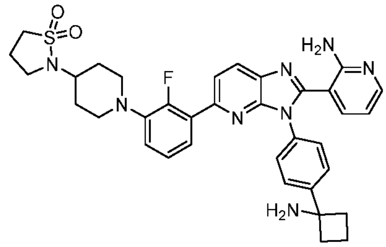
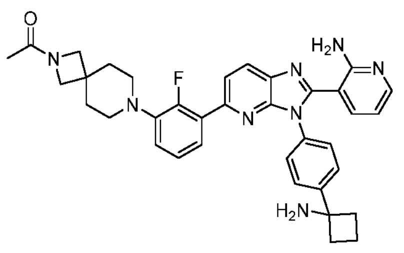
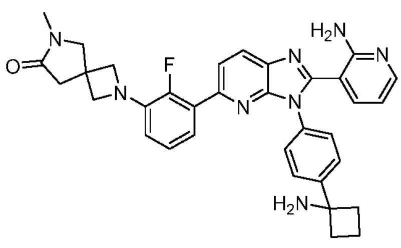
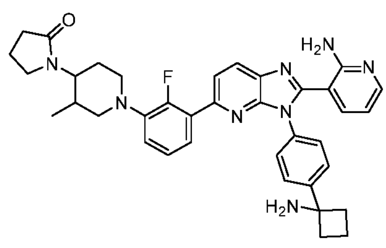

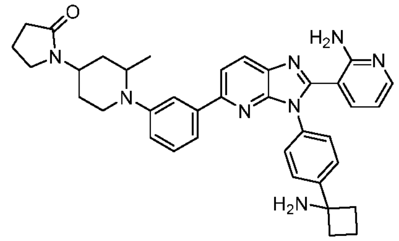
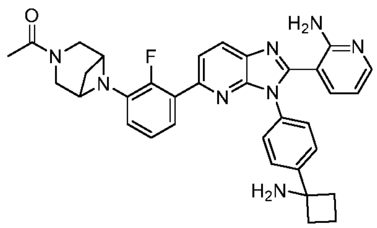
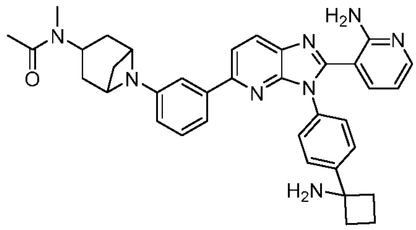
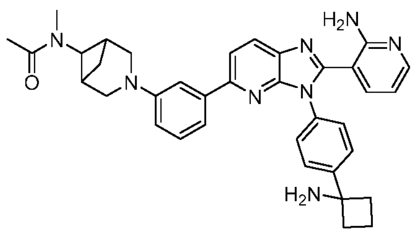
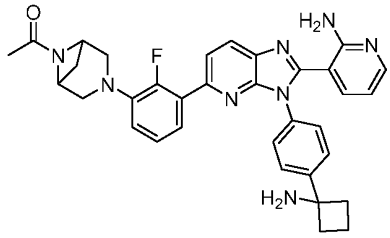
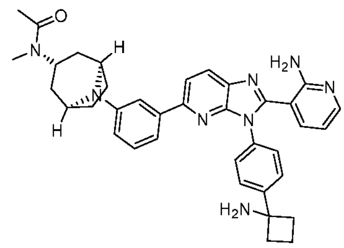
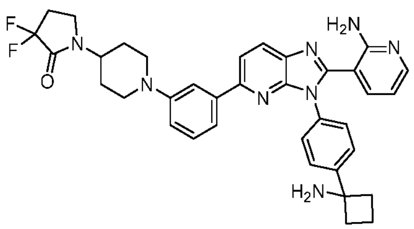
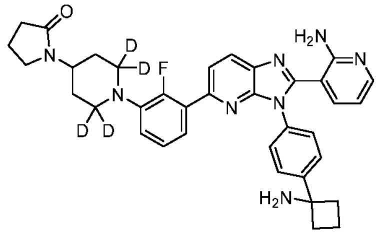
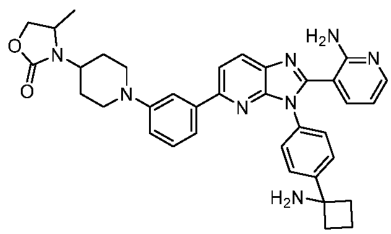
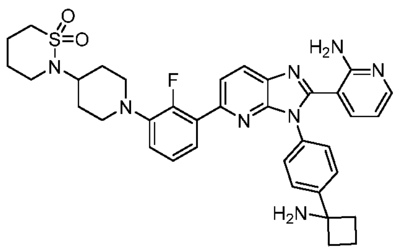
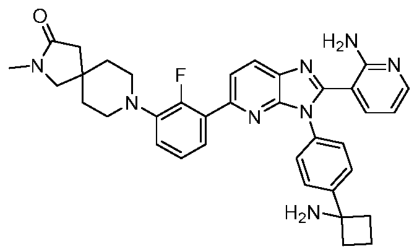
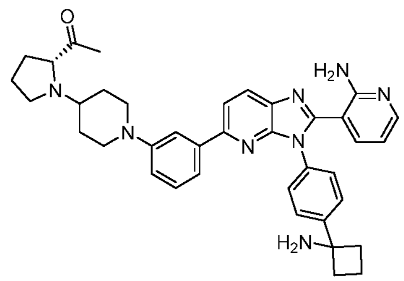
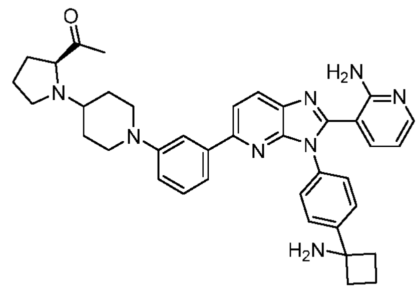
.

**16.** A pharmaceutical composition comprising the compound or the pharmaceutically acceptable salt thereof according to any one of claims 1-15.

**17.** Use of the compound or the pharmaceutically acceptable salt thereof according to any one of claims 1-15 and the pharmaceutical composition according to claim 16 in preparing a medicament for treating a disease mediated by Akt kinase.

**18.** Use of the compound or the pharmaceutically acceptable salt thereof according to any one of claims 1-15 and the pharmaceutical composition according to claim 16 in treating a disease mediated by Akt kinase.

**19.** The use according to claim 17 or the use of the compound or the pharmaceutically acceptable salt thereof according to any one of claims 1-15 and the pharmaceutical composition according to claim 16 in treating a disease mediated by Akt kinase according to claim 18, wherein the disease mediated by Akt kinase is selected from cancer; or, the disease mediated by Akt kinase is selected from the group consisting of prostate cancer and endometrial cancer.

**INTERNATIONAL SEARCH REPORT**

International application No.
**PCT/CN2021/122040**

**A. CLASSIFICATION OF SUBJECT MATTER**

C07D 471/04(2006.01)i; C07D 413/14(2006.01)i; C07D 417/14(2006.01)i; C07D 487/04(2006.01)i; A61K 31/437(2006.01)i; A61K 31/444(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

EPODOC, WPI, CNPAT, CNKI, STN(REGISTRY, CAPLUS, MARPAT): Akt, 蛋白激酶B, PKB, Rac, 咪唑并吡啶, 咪唑[4, 5-b]吡啶, 氨基, 胺, 吡啶, imidazopyridin, imidazopyridinyl, imidazo[4, 5-b]pyridin, imidazo[4, 5-b]pyridinyl, amine, amino, pyridinyl, pyridine, aminopyridine, protein kinases B, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2011082270 A2 (ARQULE, INC. et al.) 07 July 2011 (2011-07-07) claims 1-23, description, paragraphs 00036-00059, description, pages 235-259, compounds 338-340, 351, 364-365, 369-370, 372, 374-376, 380, 382-392, 396-400, 403-477 in table 1, pages 454-463, table 3 | 1-19 |
| X | WO 2012177852 A1 (ARQULE, INC. et al.) 27 December 2012 (2012-12-27) description, paragraphs 0008-0012, 00016-00027, 00046-00074, 000124-000131, pages 24-32, 115, tables 1-2 | 1-19 |
| X | WO 2012177844 A2 (ARQULE, INC.) 27 December 2012 (2012-12-27) claims 1-19, description, paragraphs 00015-00082, pages 14-23, 161, tables 1-2 | 1-19 |
| A | WO 2016037044 A1 (ARQULE, INC.) 10 March 2016 (2016-03-10) description, paragraphs 0008-00010, 000240-000241, embodiments 1-8 | 1-19 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 December 2021** | **29 December 2021** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/122040**

**C. DOCUMENTS CONSIDERED TO BE RELEVANT**

| Category* | Citation of document, with indication, where appropriate, of the relevant passages | Relevant to claim No. |
|---|---|---|
| A | ASHWELL, Mark A. et al. "Discovery and Optimization of a Series of 3-(3-Phenyl-3H-imidazo[4,5-b]pyridin-2-yl)pyridin-2-amines: Orally Bioavailable, Selective, and Potent ATP-Independent Akt Inhibitors" *Journal of Medicinal Chemistry,* Vol. 55, No. 11, 25 April 2012 (2012-04-25), ISSN: 0022-2623, pp. 5291-5310 | 1-19 |
| A | LAPIERRE, Jean-Marc et al. "Discovery of 3(3-(4-(1-Aminocyclobutyl)phenyl)-5-phenyl-3H-imidazo[4,5-b]pyridin-2-yl)pyridin-2-amine (ARQ 092): An Orally Bioavailable, Selective, and Potent Allosteric AKT Inhibitor" *Journal of Medicinal Chemistry,* Vol. 59, No. 13, 15 June 2016 (2016-06-15), ISSN: 0022-2623, pp. 6455-6469 | 1-19 |

Form PCT/ISA/210 (second sheet) (January 2015)

**INTERNATIONAL SEARCH REPORT**
**Information on patent family members**

International application No.
**PCT/CN2021/122040**

| Patent document cited in search report | | | Publication date (day/month/year) | Patent family member(s) | | | Publication date (day/month/year) |
|---|---|---|---|---|---|---|---|
| WO | 2011082270 | A2 | 07 July 2011 | US | 8501770 | B2 | 06 August 2013 |
| | | | | TW | 201132640 | A | 01 October 2011 |
| | | | | RU | 2012132444 | A | 10 February 2014 |
| | | | | MY | 157124 | A | 13 May 2016 |
| | | | | CO | 6511271 | A2 | 31 August 2012 |
| | | | | NZ | 600801 | A | 27 September 2013 |
| | | | | JP | 5774602 | B2 | 09 September 2015 |
| | | | | TW | I585086 | B | 01 June 2017 |
| | | | | EP | 2519522 | A2 | 07 November 2012 |
| | | | | HK | 1177458 | A1 | 23 August 2013 |
| | | | | AU | 2010339533 | B2 | 07 May 2015 |
| | | | | US | 2011172203 | A1 | 14 July 2011 |
| | | | | RU | 2619463 | C2 | 16 May 2017 |
| | | | | WO | 2011082270 | A3 | 17 November 2011 |
| | | | | EP | 2519522 | A4 | 13 March 2013 |
| | | | | KR | 101689421 | B1 | 23 December 2016 |
| | | | | ES | 2531109 | T3 | 10 March 2015 |
| | | | | MX | 2012007367 | A | 03 April 2013 |
| | | | | JP | 2013516419 | A | 13 May 2013 |
| | | | | CN | 102781940 | B | 07 September 2016 |
| | | | | CA | 2785536 | A1 | 07 July 2011 |
| | | | | CN | 102781940 | A | 14 November 2012 |
| | | | | AU | 2010339533 | A1 | 12 July 2012 |
| | | | | IL | 220571 | A | 31 March 2015 |
| | | | | CA | 2785536 | C | 27 February 2018 |
| | | | | SG | 181947 | A1 | 30 July 2012 |
| | | | | DK | 2519522 | T3 | 08 December 2014 |
| | | | | KR | 20120120271 | A | 01 November 2012 |
| | | | | EP | 2519522 | B1 | 24 September 2014 |
| WO | 2012177852 | A1 | 27 December 2012 | US | 8815854 | B2 | 26 August 2014 |
| | | | | TW | 201313710 | A | 01 April 2013 |
| | | | | US | 2012329793 | A1 | 27 December 2012 |
| WO | 2012177844 | A2 | 27 December 2012 | BR | 112013033182 | A2 | 06 September 2016 |
| | | | | EP | 2723741 | A2 | 30 April 2014 |
| | | | | MX | 339862 | B | 15 June 2016 |
| | | | | CN | 103748093 | A | 23 April 2014 |
| | | | | EP | 2723741 | B8 | 21 September 2016 |
| | | | | AU | 2016269408 | B2 | 05 April 2018 |
| | | | | EP | 2723741 | A4 | 03 December 2014 |
| | | | | CN | 103748093 | B | 01 June 2016 |
| | | | | JP | 2014517072 | A | 17 July 2014 |
| | | | | ZA | 201308929 | B | 30 March 2016 |
| | | | | TW | 201311687 | A | 16 March 2013 |
| | | | | CA | 2837727 | A1 | 27 December 2012 |
| | | | | US | 8962619 | B2 | 24 February 2015 |
| | | | | AU | 2012272937 | A1 | 19 December 2013 |
| | | | | US | 8609688 | B2 | 17 December 2013 |
| | | | | EP | 2723741 | B1 | 27 April 2016 |
| | | | | PH | 12013502565 | A1 | 29 May 2017 |
| | | | | DK | 2723741 | T3 | 06 June 2016 |

Form PCT/ISA/210 (patent family annex) (January 2015)

**INTERNATIONAL SEARCH REPORT**
**Information on patent family members**

International application No.
**PCT/CN2021/122040**

| Patent document cited in search report | Publication date (day/month/year) | Patent family member(s) | Publication date (day/month/year) |
|---|---|---|---|
| | | US 2012329791 A1 | 27 December 2012 |
| | | TW I561522 B | 11 December 2016 |
| | | JP 6046710 B2 | 21 December 2016 |
| | | MX 2013015006 A | 08 December 2014 |
| | | IL 265897 D0 | 30 June 2019 |
| | | WO 2012177844 A3 | 04 April 2013 |
| | | ES 2579855 T3 | 17 August 2016 |
| | | KR 20190015600 A | 13 February 2019 |
| | | AU 2012272937 B2 | 29 September 2016 |
| | | CA 2837727 C | 03 December 2019 |
| | | US 2014073635 A1 | 13 March 2014 |
| | | KR 20140040773 A | 03 April 2014 |
| | | AU 2016269408 A1 | 22 December 2016 |
| | | KR 101974673 B1 | 02 May 2019 |
| | | HK 1196371 A1 | 12 December 2014 |
| | | PH 12013502565 B1 | 29 May 2017 |
| | | IL 229719 D0 | 30 January 2014 |
| WO 2016037044 A1 | 10 March 2016 | JP 2020002148 A | 09 January 2020 |
| | | EP 3189036 A4 | 04 April 2018 |
| | | AU 2020203706 A1 | 25 June 2020 |
| | | IL 250715 A | 30 September 2020 |
| | | SG 11201701704 X A | 27 April 2017 |
| | | IL 277028 D0 | 29 October 2020 |
| | | KR 20170045748 A | 27 April 2017 |
| | | CN 111494378 A | 07 August 2020 |
| | | JP 2017526698 A | 14 September 2017 |
| | | EP 3189036 A1 | 12 July 2017 |
| | | CA 2958770 A1 | 10 March 2016 |
| | | AU 2015311751 A1 | 09 March 2017 |
| | | RU 2017111204 A | 05 October 2018 |
| | | MX 2017002892 A | 06 June 2017 |
| | | JP 6574245 B2 | 11 September 2019 |
| | | JP 6837525 B2 | 03 March 2021 |
| | | RU 2711500 C2 | 17 January 2020 |
| | | CN 107074769 B | 07 April 2020 |
| | | US 2016067260 A1 | 10 March 2016 |
| | | US 9949981 B2 | 24 April 2018 |
| | | CN 107074769 A | 18 August 2017 |
| | | IL 250715 D0 | 30 April 2017 |
| | | RU 2019142694 A | 26 February 2020 |
| | | AU 2015311751 B2 | 12 March 2020 |
| | | RU 2017111204 A3 | 25 March 2019 |
| | | BR 112017004459 A2 | 05 December 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011057860
- CN 202110261605


### Non-patent literature cited in the description


- Greene 's Protective Groups in Organic Synthesis. John Wiley & Sons